(19) Europäisches Patentamt
European Patent Office
Office européen des brevets

(11) EP 4 707 387 A2

(12) EUROPEAN PATENT APPLICATION

(43) Date of publication:
**11.03.2026 Bulletin 2026/11**

(21) Application number: **26150104.3**

(22) Date of filing: **23.06.2020**

(51) International Patent Classification (IPC):
***C12N 7/04*** *(2006.01)*

(52) Cooperative Patent Classification (CPC):
**A61K 39/12; A61P 31/14; C12N 7/00; C12N 7/04;**
A61K 2039/5254; C12N 2770/24121;
C12N 2770/24134; C12N 2770/24162; Y02A 50/30

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.06.2019 US 201962866477 P**

(62) Document number(s) of the earlier application(s) in accordance with Art. 76 EPC:
**20833206.4 / 3 990 014**

(71) Applicant: **Codagenix Inc.**
**Farmingdale, NY 11735 (US)**

(72) Inventors:
- **MUELLER, Steffen**
  **Great Neck 11024 (US)**
- **COLEMAN, John Robert**
  **Blauvelt 10913 (US)**
- **STUAFT, Charles**
  **Columbia 21045 (US)**
- **WANG, Ying**
  **South Setauket 11720 (US)**

(74) Representative: **Hutter, Anton**
**Venner Shipley LLP**
**5 Stirling House**
**Stirling Road**
**The Surrey Research Park**
**Guildford GU2 7RF (GB)**

Remarks:
•The complete document including Reference Table(s) and the Sequence Listing(s) can be downloaded from the EPO website
•This application was filed on 02-01-2026 as a divisional application to the application mentioned under INID code 62.

# (54) ATTENUATED DENGUE VIRUSES

(57) The present invention provides for modified Flavivirus such as a modified dengue virus type 1, 2, 3, 4, a combination of these, or a tetravalent combination of these. The modification according to various aspects of the invention results in reduced viral protein expression compared to a parent virus, wherein the reduction in expression is the result of recoding one or more regions of the virus. For example, the prM, or envelope (E) region can be recoded. In various embodiments one or more regions are recoded by reducing the codon pair bias or codon usage bias of the protein-encoding sequence. These modified Flaviviruses are used as vaccine compositions to provide a protective immune response.

FIG. 8B

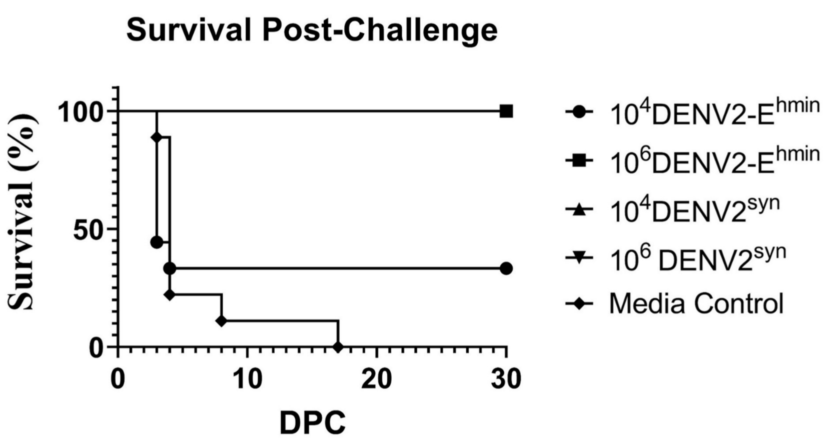




Processed by Luminess, 75001 PARIS (FR)

## Description

CROSS-REFERENCE TO RELATED APPLICATIONS

This application includes a claim of priority under 35 U.S.C. §119(e) to U.S. provisional patent application No. 62/866,477, filed June 25, 2019, the entirety of which is hereby incorporated by reference.

FIELD OF THE INVENTION

This invention provides highly attenuated flaviviruses and particularly, attenuated dengue viruses and vaccines. The attenuated viruses provide protective immunity from challenge by virus of the same lineage, as well as cross protection against heterologous viruses.

BACKGROUND OF THE INVENTION

Dengue virus (DENV) is a single-stranded positive-sense RNA virus belonging to the *Flaviviridae* family, in the same genus (Flavivirus) that includes Zika virus (ZIKV), West Nile virus (WNV), and yellow fever virus (YFV). According to the World Health Organization (WHO) about 2.5 billion people, nearly half the world's population, are now at risk from DENV and estimates that there may be 50 million cases of DENV infection worldwide every year. DENV is closely related phylogenetically to ZIKV and shares a common mosquito vector, *Aedes aegypti,* with both ZIKV and Chikungunya virus (an alphavirus). There is special concern for tourists at risk for DENV infection, with an added concern of importation and spread of the epidemic to places like the southern U.S. The vector for DENV, *Aedes aegypti,* is found throughout the southern U.S. *Aedes albopictus*, the Asian tiger mosquito, is also competent for transmitting DENV. If DENV enters *A. albopictus* populations in the U.S., it will have a vastly expanded range extending into the American Midwest and Northeast.

Flavivirus vaccine development is compounded by the phenomenon of Antibody-Dependent Enhancement (ADE). ADE occurs when prior infection with one flavivirus predisposes an individual to an enhanced severity of disease upon re-infection with a different serotype. During ADE, antibodies against the first virus bind, but do not neutralize the second virus, instead increasing its infectivity. DENV is prevalent in many countries, thus any vaccine strategy should consider the impact on a population with established dengue immunity. It is worth noting that people with underlying DENV immunity could experience increased adverse events from a live DENV vaccine, since their DENV immunity could enhance infectivity of the DENV vaccine strain, leading to increased adverse events. These are plausible scenarios that needs to be considered when developing a DENV vaccine, whether live, inactivated or antigen/VLP/backbone carrier-based. Because of the high likelihood of enhancing flavivirus infection due to ADE, all vaccine development strategies need to consider how any given DENV vaccine might interact with existing antibodies to a flavivirus or a subsequent flavivirus infection.

Dengue virus comprised four serotypes (DENV1-4) of the mosquito-borne Flaviviruses in the family *Flaviviridae*. Dengue viruses are enveloped viruses with an icosahedral virion comprised of C (Core), M(Membrane), and E (Envelope) glycoproteins that is 40-65 nanometers in diameter. The Dengue virus genome is a single positive-strand RNA molecule of 10,000-11,000 bases in length encoding structural (C, prM, E) and nonstructural (NS1, NS2, NS3, NS4, and NS5) proteins. Dengue viruses transcribe and replicate their genome in the cell cytoplasm, with the genome translated into a single polypeptide that is cleaved and processed by both host and viral proteins. Dengue virus is an emerging agent of international concern in the tropics and the individual serotypes are genetically as well as antigenically distinct viruses.

Accordingly, there remains a need in the art to develop a vaccine to prevent or reduce infection.

SUMMARY OF THE INVENTION

It is described herein that recoded dengue viruses made by modification of the E region by large numbers of synonymous nucleotide mutations are highly effective in providing protective immunity against lethal wild type challenge and cross protection against different lineages. Further, the viruses have exceptional safety profiles.

Accordingly, various embodiments of the invention provides an attenuated dengue virus in which expression of viral proteins is reduced through codon-pair deoptimization of the E coding regions. In certain embodiments, E is the only virus protein coding regions targeted. In certain embodiments, when another dengue virus protein encoding region other than E is deoptimized, the reduction is small compared to the reduction of E. According to the invention, reduction in expression of virus proteins of the invention is accomplished by changes in protein encoding sequence, for example by lowering the codon pair bias of the protein-encoding sequence, substituting rare codons, modifying G+C content, modifying CG and/or TA (or UA) dinucleotide content, or combinations. Reduced expression can also be accomplished by modifications to the regulatory sequences of the proteins.

In certain embodiments, reducing the codon-pair bias can comprise identifying a codon pair in the parent protein-encoding sequence having a codon-pair score that can be reduced, and reducing the codon-pair bias by substituting the codon pair with a codon pair that has a lower codon-pair score. In other embodiments, reducing the codon-pair bias comprises rearranging the codons of a parent protein-encoding sequence. In certain embodiments, the E protein-encoding sequence has a codon pair bias less than -0.1, or less than -0.2, or less than -0.3, or less than -0.4. Codon pair bias of a protein-encoding sequence (i.e., an open reading frame) is calculated as described in Coleman et al., 2000 and herein.

In an embodiment of the invention, expression of the E protein-encoding sequence is reduced by replacing one or more codons with synonymous codons that are less frequent in the host.

In an embodiment of the invention, the E protein-encoding sequence DENV serotype are present in a background from a different strain of the same DENV serotype or the homologous strain.

The invention also provides a dengue vaccine composition for inducing a protective immune response in a subject, wherein the vaccine composition comprises virus in which viral translation is reduced while maintaining at least 90% antigenic identity with wt virus. In some embodiments, the viral translation is reduced while maintaining at least 95, 96, 97, 98 or 99% antigenic identity with wt virus. In some embodiments, the viral translation is reduced while maintaining 100% antigenic identity with wt virus.

The invention also provides a method of eliciting a protective immune response in a subject comprising administering to the subject a prophylactically or therapeutically effective dose of a vaccine composition comprising an attenuated dengue virus, wherein expression of viral proteins is reduced by at least 10%. In various embodiments, the expression of viral proteins is reduced by at least 15%, at least 20% or at least 25%. In an embodiment of the invention, an immune response is elicited that is effective against dengue virus of the same lineage as the attenuated virus of the vaccine. In another embodiment, an immune response is elicited that is effective against a heterologous dengue virus.

The invention also provides a method of making an attenuated dengue virus genome comprising a) obtaining the genomic nucleotide b) recoding the envelope-encoding nucleotide sequence to reduce expression and recoding the nonstructural protein 3-encoding nucleotide sequence to reduce expression, and substituting the recoded nucleotide sequences into a dengue virus genome to make an attenuated dengue virus genome. In certain embodiments, only the E region is targeted. In some embodiments, expression of another virus protein encoding region is also reduced.

The invention also provides a method of constructing template dengue virus DNA sequences for transcription of infectious viral RNA genomes by T7 polymerase using overlapping PCR. All dengue genomes with homologous backbone were divided into three fragments starting from 5' end (fragment 1: nt1-3596; fragment 2: nt3030-6959, and fragment 3: nt: nt6851-end) and chemically/biochemically synthesized. Instead of constructing an infectious cDNA clone, a long overlap extension PCR strategy was used to obtain full-length dengue genome (or: the syn-wt and min dengue genomes simultaneously). To construct the heterologous dengue genomes in DENV2 16681 backbone, the first 2.5 Kb fragments, containing the DENV2 16681 5'UTR, C domain and the prM/M, E (wildtype or deoptimized) domain from individual DENV serotype and a small fraction of the DENV2 16681 NS1 domain, were synthesized. The 8 Kb DENV2 16681 backbone containing all NS domains and the 3'UTR were obtained from infectious clones encoding wildtype or deoptimized DENV2 16681. The 2.5 Kb and 8 Kb fragments were fused together using an asymmetric-fusion PCR method.

Various embodiments of the present invention provide for a modified dengue virus, comprising a recoded prM protein, a recoded envelope (E) protein, or both, wherein the recoded prM protein has a reduced codon pair bias compared to its parent prM protein encoding sequence, or has at least 5 codons substituted with synonymous codons less frequently used, or has an increased number of CpG or UpA di-nucleotides compared its parent prM protein encoding sequence, and wherein the recoded E protein has a reduced codon pair bias compared to its parent E protein encoding sequence, or has at least 5 codons substituted with synonymous codons less frequently used, or has an increased number of CpG or UpA di-nucleotides compared its parent E protein encoding sequence.

In various embodiments, the expression of the prM protein or E protein or both can be reduced compared to its parent dengue virus.

In various embodiments, the recoded prM protein has a reduced codon pair bias compared to its parent prM protein encoding sequence. In various embodiments, the recoded prM protein can have at least 5 codons substituted with synonymous codons less frequently used. In various embodiments, the recoded prM protein can have an increased number of CpG or UpA di-nucleotides compared its parent prM protein encoding sequence. In various embodiments, the recoded E protein can have a reduced codon pair bias compared to its parent E protein encoding sequence. In various embodiments, the recoded E protein can have at least 5 codons substituted with synonymous codons less frequently used. In various embodiments, the recoded E protein can have an increased number of CpG or UpA di-nucleotides compared its parent E protein encoding sequence. In various embodiments, each of the recoded prM or E protein-encoding sequence can have a codon pair bias of less than -0.05. In various embodiments, the codon pair bias of each of the recoded prM or E protein-encoding sequence can be reduced by at least 0.05.

In various embodiments, the modified dengue virus can be selected from type 1, type 2, type 3, type 4 or a combination thereof. In various embodiments, the modified dengue virus is a modified tetravalent dengue virus.

Various embodiments of the invention provide for a dengue vaccine composition for inducing a protective immune response in a subject, comprising a modified dengue virus as described above and herein, and a pharmaceutically acceptable excipient or carrier. Various embodiments of the invention provide a method of eliciting an immune response in a subject, comprising: administering to the subject an effective dose of a composition comprising a modified dengue virus as described above and herein, and a pharmaceutically acceptable excipient or carrier.

In various embodiments, the immune response can be a protective immune response, and a prophylactically effective or therapeutically effective dose of a vaccine composition of claims can be administered. In various embodiments, the immune response can be cross-protective against a heterologous dengue virus.

In various embodiments, the method can further comprise administering to the subject at least one adjuvant.

Various embodiments of the present invention provide for a method of eliciting an immune response in a subject in need thereof, comprising: administering a prime dose of (i) an attenuated dengue virus produced by a method other than codon-pair deoptimization or codon deoptimization, or increasing of CpG or UpA di-nucleotides, or (ii) a modified dengue virus comprising a recoded prM protein, a recoded envelope (E) protein, or both, wherein the recoded prM protein has a reduced codon pair bias compared to its parent prM protein encoding sequence, or has at least 5 codons substituted with synonymous codons less frequently used, or has an increased number of CpG or UpA di-nucleotides compared its parent prM protein encoding sequence, and wherein the recoded E protein has a reduced codon pair bias compared to its parent E protein encoding sequence, or has at least 5 codons substituted with synonymous codons less frequently, or has an increased number of CpG or UpA di-nucleotides compared its parent E protein encoding sequence; and administering one or more boost dose of (i) the attenuated dengue virus produced by methods other than codon-pair deoptimization or codon deoptimization, or increasing of CpG or UpA di-nucleotides, or (ii) the modified dengue virus to the subject in need thereof, wherein at least the prime dose or the one or more boost dose is the modified dengue virus.

In various embodiments, a first of the one or more boost dose can be administered about 2 weeks after the prime dose.

In various embodiments, the expression of the prM protein or E protein or both can be reduced compared to its parent dengue virus.

In various embodiments, the recoded prM protein can have a reduced codon pair bias compared to its parent prM protein encoding sequence. In various embodiments, the recoded prM protein can have at least 5 codons substituted with synonymous codons less frequently used. In various embodiments, the recoded prM protein can have an increased number of CpG or UpA di-nucleotides compared its parent prM protein encoding sequence. In various embodiments, the recoded E protein can have a reduced codon pair bias compared to its parent E protein encoding sequence. In various embodiments, the recoded E protein can have at least 5 codons substituted with synonymous codons less frequently used. In various embodiments, the recoded E protein can have an increased number of CpG or UpA di-nucleotides compared its parent E protein encoding sequence. In various embodiments, each of the recoded prM or E protein-encoding sequence has a codon pair bias of less than -0.05. In various embodiments, the codon pair bias of each of the recoded prM or E protein-encoding sequence is reduced by at least 0.05.

In various embodiments, the modified dengue virus is selected from type 1, type 2, type 3, type 4 or a combination thereof. In various embodiments, the modified dengue virus is a modified tetravalent dengue virus.

Various embodiments of the present invention provide for a method of making a modified dengue virus genome comprising: obtaining a nucleotide sequence encoding the envelope protein of a dengue virus; recoding the envelope encoding nucleotide sequence to reduce protein expression, and substituting a nucleic acid having the recoded envelope-encoding nucleotide sequence into a parent dengue virus genome to make a modified dengue virus genome; whereby expression of the recoded envelope-encoding nucleotide sequence is reduced compared to the parent virus.

## BRIEF DESCRIPTION OF THE FIGURES

FIG. 1 depicts rapid construction of SAVE-deoptimized, live-attenuated dengue vaccine candidate with growth in Vero cells under animal component-free conditions. Codon pair bias of the dengue prM/E genes and their SAVE-deoptimized counterparts in relation to the human ORFeome. Codon-Pair Bias (CPB) is expressed as the average codon pair score of a given gene's open reading frame (ORF). Positive and negative CPB value signifies the predominance of statistically over- or under-represented codon-pairs, respectively in an ORF. Red circles indicate the CPB of each of the 14,795 human ORFs, representing the majority of the known, annotated human genes at the time of our analysis (ORFeome). The CPB of wild-type E gene fall within the normal range of human host cell's genes. Following codon pair 'deoptimization' via SAVE, the resulting deoptimized prM/E gene segments were now encoded predominately by under-represented human codon-pairs as evident by their extremely negative CPB, and are drastically different from any human gene. B. cDNA genomes of wild-type and synthetically 'de-optimized' chimeric dengue vaccine variants. The SAVE-deoptimized synthetic E were synthesized *de novo* and using overlapping PCR subcloned individually into the WT DENV genomes - yielding eight independent cDNA genomes each containing a synthetically 'de-optimized' fragment(s). We constructed infectious cDNA genomes for wt DENV1-4 and then recovered fully infectious, replicating virus for the deoptimized DENV vaccine candidates via transfection of RNA into Vero (all DENV WT, E-W/MIN, and E-MIN viruses) or BHK cells.

FIG. 2 depicts diagram of subcloning strategies for decreasing attenuation or increasing immunogenicity by reducing the length of deoptimized sequence in the E encoding region. The second generation of dengue vaccine candidates leverages the flexibility of the SAVE platform to reduce the deoptimized region from full-length (E-min) to approximately half of the length (W-E-Min) while keeping the amino acid sequence 100% identical.

FIG. 3 depicts growth of heterologous backbone dengue vaccine candidates in Vero cells under animal component-free conditions. DENV1-4 E-Min were used to infect Vero cells under animal-component free conditions at a MOI of 0.01 and supernatant titrated daily in a multiple-step growth curve over the course of 10 days post-infection. DENV2, DENV3, and DENV4 candidates reached titers of 1-2 x $10^5$ FFU/ml while DENV1 E-Min titer was reduced by ~1 $\log_{10}$ compared to the others.

FIG. 4A-4C depicts multiple step growth curves that were conducted in Vero cells for synthetic DENV1 WT virus in a full-length DENV1 backbone (FIG. 4A) and attenuated live vaccine candidates DENV1 E-W/MIN (FIG. 4B) and E-MIN (FIG. 4C) derived from DENV1 WT. Synthetic DENV1 WT grows well in Vero cells at 33°C and 37°C. Typical of DENV, a transient reduction in virus yield was observed at 39 °C, however, titers recovered to 3 7°C levels by 7 dpi. DENV1 E-W/MIN, however, reached serviceable titers ~10-fold lower than DENV1 WT at both 33°C and 37°C. DENV1 E-MIN was highly attenuated in Vero cells and only reached detectable titers at 33°C starting on days 10-14 post-infection.

FIG. 5 depicts multiple step growth curves that were conducted in Vero cells for synthetic DENV2 WT virus (in a full-length DENV2 backbone) and attenuated live vaccine candidates DENV2 E-W/MIN and E-MIN derived from DENV2 WT. DENV2 E-W/MIN and E-MIN were both attenuated *in vitro* with a 1-2 $\log_{10}$ FFU/ml reduction for E-W/MIN and a more pronounced 2-3 $\log_{10}$ FFU/ml reduction for DENV2 E-MIN (Figure 3). While growth kinetics were delayed, with regular medium replacement every 1-2 days virus titers reached >$10^6$ FFU/ml for DENV2 E-W/MIN. Importantly, attenuation was correlated with the extent of deoptimization in the E region. While temperature sensitivity (difference between titers at 37°C and 39°C) was similar for DENV2-WT and DENV2 E-W/MIN through day 4 post-infection, there were significant differences on days 5, 6, and 7. Temperature sensitivity was not observed for DENV2 E-MIN because there was no replication at any temperature through 6 DPI. However, starting at day 7-14 DENV2 E-MIN started producing detectable virus (but only at 33°C and 37°C).

FIG. 6 depicts multiple step growth curves that were conducted in Vero cells for synthetic DENV3 WT virus (in a full-length DENV3 backbone) and attenuated live vaccine candidates DENV3 E-W/MIN and E-MIN derived from DENV3 WT. It was apparent that DENV3 E-W/MIN was more temperature sensitive at 39 °C than the WT with a significantly greater difference observable on days 3, 4, 5, and 7 post-infection. Because DENV3 E-MIN had minimal levels of replication in Vero cells at 39°C, the difference in virus yield from 37°C to 39°C were as high as $10^7$ FFU/ml. When grown at permissible temperatures, however, DENV3 E-MIN reached high titers which is promising for cGMP manufacture.

FIG. 7 depicts multiple step growth curves that were conducted in Vero cells for synthetic DENV4 WT virus (in a full-length DENV4 backbone) and attenuated live vaccine candidates DENV4 E-W/MIN and E-MIN derived from DENV4 WT. Both DENV4 WT and E-W/MIN grew to high titers in Vero cells, with the peak DENV4 E-W/MIN titers approximately 10-fold lower but still high (~$10^7$ FFU/ml) at 33°C and 37°C. Both viruses were partially restricted at 39°C, but not to the extent seen with DENV1-3. On day 5 a small but significant (~5-fold) change in titer from 37°C to 39°C compared to WT was observed. DENV4 E-MIN was attenuated in Vero cells with peak titers 100-fold lower than WT. DENV4 E-MIN replication at 33°C and 37°C was similar, however, DENV4 E-MIN was not viable at 39°C with no detectable virus on days 1-14 post-infection.

FIG. 8A-8B depicts evaluation of the attenuation, immunogenicity, and efficacy of DENV2 vaccine candidates in AG129 mice. DENV-2 E-min based on a DENV2 16681 backbone was tested in an immunogenicity/dose escalation study in AG129 mice lacking interferon alpha or gamma receptors. Animals were immunized with DENV-2 E-min or DENV-2 16681 at two different concentrations. Resultant neutralizing antibody titers were determined, and the protective efficacy afforded evaluated against a mouse-adapted lethal strain of DENV2, D2S10.

FIG. 9 depicts constructed D2-E-min and D2-1/2-E-min with E sequence derived from DENV-2/NI/BID-V533/2005 in the heterologous DENV2 16681 backbone to improve the clinical relevance of our DENV2 candidate. We tested D2-1/2-E-min, with the E region consisting of half wt DENV-2/NI/BID-V533/2005 sequence and half CPD, to improve the immunogenicity of our virus. AG129 and BALB/c mice (n=5) were vaccinated with $10^6$ or $10^4$ FFU of D2-E-min, D2-1/2-E-min, or D2-WTE (DENV2 16681 backbone with WT DENV-2/NI/BID-V533/2005 E region). No mortality or morbidity was observed in the DENV2 D2-E-min, D2-1/2-E-min group indicating at least a 100-fold attenuation. The immunogenicity of our candidate was improved by reducing the extent of CPD with PRNT50 values of serum from D2-1/2-E-min being higher compared to D2-E-Min vaccinated AG129 mice. Additionally, high neutralizing antibody titers were maintained with D2-1/2-E-min vaccination as no significant difference was observed between the $10^6$

(GM=3447) and $10^4$ (GM= 2867) FFU vaccinated groups. In immune-competent BALB/c mice, both D2-E-min and D2-1/2-E-min engendered levels of neutralizing antibody comparable to wild-type.

FIG. 10A-10B depicts AG129 mice used to test the immunogenicity and protective efficacy of DENV-3 vaccine candidate DENV D2/D3-E-min, which comprises the DENV-2 strain 16681 backbone with a codon-deoptimized prME cassette from DENV-3/VE/BID-V2268/2008. This study was designed to evaluate the immunogenicity and efficacy of this candidate vaccine against challenge with virulent DENV3 CO360/94. The first immunization was well tolerated by all of the animals with no major associated weight loss and none of the animals developed clinical signs. After lethal challenge with 1.2 x $10^7$ PFU DENV-3 CO360/94, animals in the media control group developed a rapid progressive infection that was lethal in 8/9 animals with the mean day of death (MDD) among the animals that died of 4.0 $\pm$ 0.8 days. Immunization with the higher inoculum of DENV D2/D3-E-min provided significant protection with no lethality or morbidity (as measured by >10% weight loss) among the animals in the group.

FIG. 11A-11B depicts AG129 mice used to test the immunogenicity and protective efficacy of DENV-4 vaccine candidates DENV D2/D4-E-min and D2/D4-1/2-E-min, which comprise the DENV-2 strain 16681 backbone with a codon-deoptimized prME cassette from DENV4. AG 129 Mice were vaccinated with $10^6$ D2/D4-E-min (N=12), $10^6$ D2/D4-1/2-E-min (N=12), $10^6$ D2/D4-WTE, or media as a control (N=9). This study was designed to evaluate the immunogenicity and efficacy of this candidate vaccine against challenge with virulent DENV4 703/4. The first immunization with all strains was well tolerated by all groups of animals with no sustained weight loss over the 5 day observation period, however, in the D2/D4-WTE group one mouse had to be euthanized prior to the second immunization and additional animals died after the second immunization. No animals in any of the other vaccine groups experienced progressive weight loss. Sera were collected on day 35 to test for neutralizing antibodies prior to challenge and on day 30 post-challenge from all surviving animals to test final titers by FRNT50. Using a 96-well plate method with Vero cells, each serum sample was tested in duplicate using serum dilutions from 1:64-1:32784 incubated with 50 FFU of DENV4 WT virus. Each vaccine candidate was immunogenic at 35 DPI with vaccination with D2/D4-E-min (GMT 332.9) and D2/D4-1/2-E-min (GMT 738.4) lower than vaccination with D2/D4-WTE (GMT 1640). At 2 days post-challenge (DPC), viremia was detected in 4/12 mice inoculated with D2/D4-WTE. For all of the mice immunized with the other two viruses, viremia levels were below the limit of detection of the assay (500 genome equivalents/ml). DENV-4 703/4 challenge produced rapid progressive infection with universal lethality in the media control group. In addition, three animals immunized with D2/D4-E-min experienced lethal infection with the other animals in this group remaining healthy for the duration of the study. Importantly, no lethality or evidence of morbidity as determined by weight loss was seen in any of the D2/D4-1/2-E-min immunized animals after DENV-4 703/4 challenge.

FIG. 12 depicts tested DENV1-4 WT as well as vaccine candidates for DENV2-4 for immunogenicity in IFN$\alpha$ receptor knockout mice. All WT viruses were highly immunogenic in these mice, with neutralizing antibody titers >1024. DENV2-E-W/MIN was equally immunogenic to DENV2 WT virus at both a $10^6$ and $10^4$ FFU dose. Immunogenicity of DENV3 viruses waned with decreasing dose and increased deoptimization, however, DENV3 E-W/MIN was still highly immunogenic at a $10^4$ FFU dose (~1024 FRNT50). We noticed a pronounced improvement in the immunogenicity of DENV4 E-W/MIN and WT as compared to the heterologous DENV4 WT in the DENV2 16681 backbone, which was poorly immunogenic in mice.

FIG. 13 depicts immunogenicity and efficacy of tetravalent vaccination in AG129 mice against lethal challenge with DENV3 CO360/94. AG129 mice were vaccinated on days 0 and 21 with $10^6$ IFU monovalent D2/D3-E-min vaccine (N=16; 10 male and 6 female), tetravalent vaccine containing $10^6$ IFU of each of the four monovalent DENV E-min viruses (N=16; 10 male and 6 female), or mock vaccinated with media (N=13; 8 male and 5 female). Serum was collected prior to challenge for measurement of neutralizing antibodies by FRNT50. All of the mice immunized with the monovalent vaccine developed detectable neutralizing antibody titers (range 20 - 160). In mice immunized with the tetravalent vaccine formulation, titers were generally lower (range <20 - 80). On day 35, all remaining mice were challenged with $10^7$ IFU DENV3 CO360/94 delivered by the intraperitoneal route. As anticipated, animals in the media control group developed a rapid progressive infection that was lethal in 8/9 (89%) animals with the mean day of death (MDD) among the animals that died of 4.1 $\pm$ 0.4 days. Immunization with monovalent DENV D2/D3-E-min or the tetravalent vaccine provided significant protection with no lethality or morbidity (as measured by >10% weight loss) among the animals in either group.

FIG. 14 depicts attenuation of homologous backbone viruses - focus size of vaccine strains vs wt - vaccine viruses spread less in vitro as compared to wt. Individual foci areas ($mm^2$) were calculated for Vero cells infected with DENV1-4 WT, E-W/MIN, or E-MIN and incubated for 3 days at temperatures of 33°C, 37°C, or 39°C. We completed analysis of focus-forming unit (FFU) size of each virus using ImageJ software (NIH, v1.52a). Each image of the FFA plates was converted into a binary 8-bit image using ImageJ (an example is presented in Figure 2), and the scale for measuring FFU area was set to the diameter of the wells of 24-well plates (15.5 mm). Using the "analyze particles" function, the area of each FFU was calculated by ImageJ and the results compared by Sidak's multiple comparisons test (GraphPad Prism 8.1.2).

FIG. 15 depicts *in vivo* immunogenicity data for tetravalent homologous backbone vaccine Tetravalent and monovalent homologous backbone dengue vaccine candidates were immunogenic in IFNα receptor knockout mice after vaccination with $10^6$ or $10^4$ FFU delivered by the subcutaneous route on day 0 and 21. Neutralizing antibodies were tested using a focus-reduction neutralization 50% test at days 0, 21, and 35 post-vaccination against each strain of DENV1-4 wt.

DETAILED DESCRIPTION

All references cited herein are incorporated by reference in their entirety as though fully set forth. Unless defined otherwise, technical and scientific terms used herein have the same meaning as commonly understood by one of ordinary skill in the art to which this invention belongs. One skilled in the art will recognize many methods and materials similar or equivalent to those described herein, which could be used in the practice of the present invention. Indeed, the present invention is in no way limited to the methods and materials described. For purposes of the present invention, the following terms are defined below.

As used herein the term "about" when used in connection with a referenced numeric indication means the referenced numeric indication plus or minus up to 5% of that referenced numeric indication, unless otherwise specifically provided for herein. For example, the language "about 50%" covers the range of 45% to 55%. In various embodiments, the term "about" when used in connection with a referenced numeric indication can mean the referenced numeric indication plus or minus up to 4%, 3%, 2%, 1%, 0.5%, or 0.25% of that referenced numeric indication, *if* specifically provided for in the claims.

Codon-Pair Bias (CPB) is expressed as the average codon pair score of a given gene's open reading frame (ORF).

A "subject" means any animal or artificially modified animal. Animals include, but are not limited to, humans, non-human primates, cows, horses, sheep, pigs, dogs, cats, rabbits, ferrets, rodents such as mice, rats and guinea pigs, and birds. Artificially modified animals include, but are not limited to, SCID mice with human immune systems. In a preferred embodiment, the subject is a human. Embodiments of birds are domesticated poultry species, including, but not limited to, chickens, turkeys, ducks, and geese.

A "viral host" means any animal or artificially modified animal, or insect that the virus can infect. Animals include, but are not limited to, humans, non-human primates, cows, horses, sheep, pigs, dogs, cats, rabbits, ferrets, rodents such as mice, rats and guinea pigs, and birds. Artificially modified animals include, but are not limited to, SCID mice with human immune systems. In a specific embodiment, the viral host is a human. Embodiments of birds are domesticated poultry species, including, but not limited to, chickens, turkeys, ducks, and geese. Insects include, but are not limited to mosquitos.

A "prophylactically effective dose" is any amount of a vaccine that, when administered to a subject prone to viral infection or prone to affliction with a virus-associated disorder, induces in the subject an immune response that protects the subject from becoming infected by the virus or afflicted with the disorder. "Protecting" the subject means either reducing the likelihood of the subject's becoming infected with the virus, or lessening the likelihood of the disorder's onset in the subject, by at least two-fold, preferably at least ten-fold, 25-fold, 50-fold, or 100-fold. For example, if a subject has a 1% chance of becoming infected with a virus, a two-fold reduction in the likelihood of the subject becoming infected with the virus would result in the subject having a 0.5% chance of becoming infected with the virus.

As used herein, a "therapeutically effective dose" is any amount of a vaccine that, when administered to a subject afflicted with a disorder against which the vaccine is effective, induces in the subject an immune response that causes the subject to experience a reduction, remission or regression of the disorder and/or its symptoms. In preferred embodiments, recurrence of the disorder and/or its symptoms is prevented. In other preferred embodiments, the subject is cured of the disorder and/or its symptoms.

The present invention relates to attenuated dengue viruses and the production of attenuated dengue viruses that can be used to protect against viral infection and disease. A basic premise in vaccination is adequate delivery of protective antigens to vaccine recipients assuming that a very high dose ("Peptide or Virus-Like Particle") or a dose corresponding to live viral infection ("ChimeriVax") of these traditionally dominant antigenic polypeptides alone are sufficient for adequate vaccine efficacy. Those expectations aside, the present invention benefits from a contrary approach. The invention provides attenuated dengue viruses in which expression of viral proteins is reduced, which have excellent growth properties useful to vaccine production, yet possess an extraordinary safety profile and enhanced protective characteristics. The attenuated viruses proliferate nearly as well as wild type virus, have highly attenuated phenotypes, as revealed by $LD_{50}$ values, are unusually effective in providing protective immunity against challenge by dengue virus of the same strain, and also provide protective immunity against challenge by dengue virus of other strains.

In certain embodiments of the invention, the attenuated dengue viruses of the invention comprise a recoded pre-membrane (prM)/Envelope (E) encoding region. In embodiments wherein the C, NS1, NS2, NS3, NS4, or NS5 protein encoding regions are not recoded does not exclude mutations and other variations in those sequences, but only means that any mutations or variations made in those sequences have little or no effect on attenuation. Little or no effect on attenuation includes one or both of the following: 1) The mutations or variations in the C, NS1, NS2, NS3, NS4, or NS5 encoding regions do not reduce viral replication or viral infectivity more than 20% when the variant C, NS1, NS2, NS3, NS4, or NS5 encoding region is the only variant in a test dengue virus; 2) Mutations or variations in any of the C, NS1, NS2, NS3, NS4, or NS5 encoding regions represent fewer than 10% of the nucleotides in that coding sequence. ***If*** specifically provided for in the claims, little or no effect on attenuation includes one or both of the following: 1) The mutations or variations in the C, NS1, NS2, NS3, NS4, or NS5 encoding regions do not reduce viral replication or viral infectivity more than 10% when the variant C, NS1, NS2, NS3, NS4, or NS5 encoding region is the only variant in a test dengue virus; 2) Mutations or variations in any of the C, NS1, NS2, NS3, NS4, or NS5 encoding regions represent fewer than 5% of the nucleotides in that coding sequence.

In various embodiments, viruses of the invention are attenuated. In embodiments of the invention, compared to wild type, the viruses are at least 10 fold attenuated, at least 50 fold attenuated, or at least 100 fold attenuated, or at least 200 fold attenuated, or at least 500 fold attenuated, or at least 1000 fold attenuated, of at least 2000 fold attenuated in the AG129 mouse model compared to a wild type virus having proteins of the same amino acid sequence but encoded by a different nucleotide sequence.

The attenuated viruses are also highly protective against wild type virus of the same strain. In embodiments of the invention, the protective dose ($PD_{50}$) of the viruses is, when measured by a mouse model, such as exemplified herein.

The attenuated viruses of the invention also exhibit a large margin of safety (i.e., the difference between $LD_{50}$ and $PD_{50}$), thus have high safety factors, defined herein as the ratio of $LD_{50}/PD_{50}$. In certain embodiments of the invention, the safety factor is at least $10^2$, or at least $10^3$, or at least $10^4$, or at least $10^5$, or at least $2\times10^5$, or at least $3\times10^5$, or at least $4\times10^5$or at least $5\times10^5$, or at least $10^6$, or at least $2\times10^6$, or at least $3\times10^6$, or at least $4\times10^6$, or at least $5\times10^6$. In certain embodiments, the safety factor is from $10^2$ to $10^3$, or from $10^3$ to $10^4$, or from $10^4$ to $10^5$, or from $10^5$ to $10^6$.

The attenuated viruses of the invention are also highly protective against heterologous strains of the dengue virus within the same serotype. In certain embodiments of the invention, the protective dose ($PD_{50}$) of an attenuated virus of the invention is less than 1000 PFU, or less than 750 PFU, or less than 500 PFU, or less than 200 PFU, or less than 100 PFU, or less than 50 PFU when measured by a mouse model, such as exemplified herein.

The recoding of E protein encoding sequences of the attenuated viruses of the invention have been made or can be made by one of skill in the art in light of disclosure discussed herein. According to the invention, nucleotide substitutions are engineered in multiple locations in the E coding sequence, wherein the substitutions introduce a plurality of synonymous codons into the genome. In certain embodiments, the synonymous codon substitutions alter codon bias, codon pair bias, the density of infrequent codons or infrequently occurring codon pairs, RNA secondary structure, CG and/or TA (or UA) dinucleotide content, C+G content, translation frame shift sites, translation pause sites, the presence or absence of microRNA recognition sequences or any combination thereof, in the genome. The codon substitutions may be engineered in multiple locations distributed throughout the E coding sequence, or in the multiple locations restricted to a portion of the E coding sequence. Because of the large number of defects (i.e., nucleotide substitutions) involved, the invention provides a means of producing stably attenuated viruses and live vaccines.

As discussed further below, in some embodiments, a virus coding sequence is recoded by substituting one or more codon with synonymous codons used less frequently in the dengue host (e.g., mammals, humans, mosquitoes). In some embodiments, a virus coding sequence is recoded by substituting one or more codons with synonymous codons used less frequently in the dengue virus. In certain embodiments, the number of codons substituted with synonymous codons is at least 5. In some embodiments, at least 10, or at least 20 codons are substituted with synonymous codons. In some embodiments, the number of codons substituted with synonymous codons is at least 30, or at least 40, or at least 50, or at least 75, or at least 100, or at least 125, or at least 150, or at least 175.

In some embodiments, virus codon pairs are recoded to reduce (i.e., lower the value of) codon-pair bias. In certain embodiments, codon-pair bias is reduced by identifying a codon pair in an E coding sequence having a codon-pair score that can be reduced and reducing the codon-pair bias by substituting the codon pair with a codon pair that has a lower codon-pair score. In some embodiments, this substitution of codon pairs takes the form of rearranging existing codons of a sequence. In some such embodiments, a subset of codon pairs is substituted by rearranging a subset of synonymous codons. In other embodiments, codon pairs are substituted by maximizing the number of rearranged synonymous codons. It is noted that while rearrangement of codons leads to codon-pair bias that is reduced (made more negative) for the virus coding sequence overall, and the rearrangement results in a decreased CPS at many locations, there may be accompanying CPS increases at other locations, but on average, the codon pair scores, and thus the CPB of the modified sequence, is reduced. In some embodiments, recoding of codons or codon-pairs can take into account altering the G+C content of the E coding sequence. In some embodiments, recoding of codons or codon-pairs can take into account altering the frequency of CG and/or TA dinucleotides in the E coding sequence.

In certain embodiments, the recoded E protein-encoding sequence has a codon pair bias less than -0.05, or less than -0.06, or less than -0.07, or less than -0.08, or less than -0.09, or less than -0.1, or less than -0.11, or less than -0.12, or less than -0.13, or less than -0.14, or less than -0.15, or less than -0.16, or less than -0.17, or less than -0.18, or less than -0.19, or less than -0.2, or less than -0.25, or less than -0.3, or less than -0.35, or less than -0.4, or less than -0.45, or less than -0.5. In certain embodiments, the codon pair bias of the recoded E protein encoding sequence is reduced by at least 0.05, or at least 0.06, or at least 0.07, or at least 0.08, or at least 0.09, or at least 0.1, or at least 0.11, or at least 0.12, or at least 0.13, or at least 0.14, or at least 0.15, or at least 0.16, or at least 0.17, or at least 0.18, or at least 0.19, or at least 0.2, or at least 0.25, or at least 0.3, or at least 0.35, or at least 0.4, or at least 0.45, or at least 0.5, compared to the parent E protein encoding sequence from which it is derived. In certain embodiments, it is in comparison to an E protein encoding sequence from which the calculation is to be made; for example, the E protein encoding sequence of a wild type virus.

In certain embodiments, rearrangement of synonymous codons of the E protein-encoding sequence provides a codon-pair bias reduction of at least 0.05, or at least 0.06, or at least 0.07, or at least 0.08, or at least 0.09, or at least 0.1, or at least 0.11, or at least 0.12, or at least 0.13, or at least 0.14, or at least 0.15, or at least 0.16, or at least 0.17, or at least 0.18, or at least 0.19, or at least 0.2, or at least 0.25, or at least 0.3, or at least 0.35, or at least 0.4, or at least 0.45, or at least 0.5, compared to the parent E protein encoding sequence from which it is derived. In certain embodiments, it is in comparison to an E protein encoding sequence from which the calculation is to be made; for example, the E protein encoding sequence of a wild type virus.

Usually, these substitutions and alterations are made and reduce expression of the encoded virus proteins without altering the amino acid sequence of the encoded protein. In certain embodiments, the invention also includes alterations in the E coding sequence that result in substitution of non-synonymous codons and amino acid substitutions in the encoded protein, which may or may not be conservative. In various embodiments, there are up to 1, 2, 3, 4, 5, 6, 7, 8, 9, 10, 11, 12, 13, 14, or 15 nucleotide substitutions.

Most amino acids are encoded by more than one codon. See the genetic code in Table 1. For instance, alanine is encoded by GCU, GCC, GCA, and GCG. Three amino acids (Leu, Ser, and Arg) are encoded by six different codons, while only Trp and Met have unique codons. "Synonymous" codons are codons that encode the same amino acid. Thus, for example, CUU, CUC, CUA, CUG, UUA, and UUG are synonymous codons that code for Leu. Synonymous codons are not used with equal frequency. In general, the most frequently used codons in a particular organism are those for which the cognate tRNA is abundant, and the use of these codons enhances the rate and/or accuracy of protein translation. Conversely, tRNAs for the rarely used codons are found at relatively low levels, and the use of rare codons is thought to reduce translation rate and/or accuracy.

*Table 1. Genetic Code*

| U | C | A | G | | |
|---|---|---|---|---|---|
| U | Phe | Ser | Tyr | Cys | U |
| | Phe | Ser | Tyr | Cys | C |
| | Leu | Ser | STOP | STOP | A |
| | Leu | Ser | STOP | Trp | G |
| C | Leu | Pro | His | Arg | U |
| | Leu | Pro | His | Arg | C |
| | Leu | Pro | Gln | Arg | A |
| | Leu | Pro | Gln | Arg | G |
| A | Ile | Thr | Asn | Ser | U |
| | Ile | Thr | Asn | Ser | C |
| | Ile | Thr | Lys | Arg | A |
| | Met | Thr | Lys | Arg | G |
| G | Val | Ala | Asp | Gly | U |
| | Val | Ala | Asp | Gly | C |
| | Val | Ala | Glu | Gly | A |
| | Val | Ala | Glu | Gly | G |

[a] The first nucleotide in each codon encoding a particular amino acid is shown in the left-most column; the second nucleotide is shown in the top row; and the third nucleotide is shown in the right-most column.

*Codon Bias*

As used herein, a "rare" codon is one of at least two synonymous codons encoding a particular amino acid that is present in an mRNA at a significantly lower frequency than the most frequently used codon for that amino acid. Thus, the rare codon may be present at about a 2-fold lower frequency than the most frequently used codon. Preferably, the rare codon is present at least a 3-fold, more preferably at least a 5-fold, lower frequency than the most frequently used codon for the amino acid. Conversely, a "frequent" codon is one of at least two synonymous codons encoding a particular amino acid that is present in an mRNA at a significantly higher frequency than the least frequently used codon for that amino acid. The frequent codon may be present at about a 2-fold, preferably at least a 3-fold, more preferably at least a 5-fold, higher frequency than the least frequently used codon for the amino acid. For example, human genes use the leucine codon CTG 40% of the time, but use the synonymous CTA only 7% of the time (see Table 2). Thus, CTG is a frequent codon, whereas CTA is a rare codon. Roughly consistent with these frequencies of usage, there are 6 copies in the genome for the gene for the tRNA recognizing CTG, whereas there are only 2 copies of the gene for the tRNA recognizing CTA. Similarly, human genes use the frequent codons TCT and TCC for serine 18% and 22% of the time, respectively, but the rare codon TCG only 5% of the time. TCT and TCC are read, via wobble, by the same tRNA, which has 10 copies of its gene in the genome, while TCG is read by a tRNA with only 4 copies. It is well known that those mRNAs that are very actively translated are strongly biased to use only the most frequent codons. This includes genes for ribosomal proteins and glycolytic enzymes. On the other hand, mRNAs for relatively non-abundant proteins may use the rare codons.

*Table 2. Codon usage in Homo sapiens (source: www.kazusa.orjp/codonl)*

| Amino Acid | Codon | Number | /1000 | Fraction |
|---|---|---|---|---|
| Gly | GGG | 636457.00 | 16.45 | 0.25 |
| Gly | GGA | 637120.00 | 16.47 | 0.25 |
| Gly | GGT | 416131.00 | 10.76 | 0.16 |
| Gly | GGC | 862557.00 | 22.29 | 0.34 |
| Glu | GAG | 1532589.00 | 39.61 | 0.58 |
| Glu | GAA | 1116000.00 | 28.84 | 0.42 |
| Asp | GAT | 842504.00 | 21.78 | 0.46 |
| Asp | GAC | 973377.00 | 25.16 | 0.54 |
| Val | GTG | 1091853.00 | 28.22 | 0.46 |
| Val | GTA | 273515.00 | 7.07 | 0.12 |
| Val | GTT | 426252.00 | 11.02 | 0.18 |
| Val | GTC | 562086.00 | 14.53 | 0.24 |
| Ala | GCG | 286975.00 | 7.42 | 0.11 |
| Ala | GCA | 614754.00 | 15.89 | 0.23 |
| Ala | GCT | 715079.00 | 18.48 | 0.27 |
| Ala | GCC | 1079491.00 | 27.90 | 0.40 |
| Arg | AGG | 461676.00 | 11.93 | 0.21 |
| Arg | AGA | 466435.00 | 12.06 | 0.21 |
| Ser | AGT | 469641.00 | 12.14 | 0.15 |
| Ser | AGC | 753597.00 | 19.48 | 0.24 |
| Lys | AAG | 1236148.00 | 31.95 | 0.57 |
| Lys | AAA | 940312.00 | 24.30 | 0.43 |
| Asn | AAT | 653566.00 | 16.89 | 0.47 |
| Asn | AAC | 739007.00 | 19.10 | 0.53 |
| Met | ATG | 853648.00 | 22.06 | 1.00 |
| Ile | ATA | 288118.00 | 7.45 | 0.17 |

(continued)

| Amino Acid | Codon | Number | /1000 | Fraction |
|---|---|---|---|---|
| Ile | ATT | 615699.00 | 15.91 | 0.36 |
| Ile | ATC | 808306.00 | 20.89 | 0.47 |
| Thr | ACG | 234532.00 | 6.06 | 0.11 |
| Thr | ACA | 580580.00 | 15.01 | 0.28 |
| Thr | ACT | 506277.00 | 13.09 | 0.25 |
| Thr | ACC | 732313.00 | 18.93 | 0.36 |
| Trp | TGG | 510256.00 | 13.19 | 1.00 |
| End | TGA | 59528.00 | 1.54 | 0.47 |
| Cys | TGT | 407020.00 | 10.52 | 0.45 |
| Cys | TGC | 487907.00 | 12.61 | 0.55 |
| End | TAG | 30104.00 | 0.78 | 0.24 |
| End | TAA | 38222.00 | 0.99 | 0.30 |
| Tyr | TAT | 470083.00 | 12.15 | 0.44 |
| Tyr | TAC | 592163.00 | 15.30 | 0.56 |
| Leu | TTG | 498920.00 | 12.89 | 0.13 |
| Leu | TTA | 294684.00 | 7.62 | 0.08 |
| Phe | TTT | 676381.00 | 17.48 | 0.46 |
| Phe | TTC | 789374.00 | 20.40 | 0.54 |
| Ser | TCG | 171428.00 | 4.43 | 0.05 |
| Ser | TCA | 471469.00 | 12.19 | 0.15 |
| Ser | TCT | 585967.00 | 15.14 | 0.19 |
| Ser | TCC | 684663.00 | 17.70 | 0.22 |
| Arg | CGG | 443753.00 | 11.47 | 0.20 |
| Arg | CGA | 239573.00 | 6.19 | 0.11 |
| Arg | CGT | 176691.00 | 4.57 | 0.08 |
| Arg | CGC | 405748.00 | 10.49 | 0.18 |
| Gln | CAG | 1323614.00 | 34.21 | 0.74 |
| Gln | CAA | 473648.00 | 12.24 | 0.26 |
| His | CAT | 419726.00 | 10.85 | 0.42 |
| His | CAC | 583620.00 | 15.08 | 0.58 |
| Leu | CTG | 1539118.00 | 39.78 | 0.40 |
| Leu | CTA | 276799.00 | 7.15 | 0.07 |
| Leu | CTT | 508151.00 | 13.13 | 0.13 |
| Leu | CTC | 759527.00 | 19.63 | 0.20 |
| Pro | CCG | 268884.00 | 6.95 | 0.11 |
| Pro | CCA | 653281.00 | 16.88 | 0.28 |
| Pro | CCT | 676401.00 | 17.48 | 0.29 |
| Pro | CCC | 767793.00 | 19.84 | 0.32 |

The propensity for highly expressed genes to use frequent codons is called "codon bias." A gene for a ribosomal protein might use only the 20 to 25 most frequent of the 61 codons, and have a high codon bias (a codon bias close to 1), while a poorly expressed gene might use all 61 codons, and have little or no codon bias (a codon bias close to 0). It is thought that the frequently used codons are codons where larger amounts of the cognate tRNA are expressed, and that use of these codons allows translation to proceed more rapidly, or more accurately, or both. The PV capsid protein, for example, is very actively translated, and has a high codon bias.

*Codon Pair Bias*

In addition, a given organism has a preference for the nearest codon neighbor of a given codon A, referred to a bias in codon pair utilization. A change of codon pair bias, without changing the existing codons, can influence the rate of protein synthesis and production of a protein.

Codon pair bias may be illustrated by considering the amino acid pair Ala-Glu, which can be encoded by 8 different codon pairs. If no factors other than the frequency of each individual codon (as shown in Table 2) are responsible for the frequency of the codon pair, the expected frequency of each of the 8 encodings can be calculated by multiplying the frequencies of the two relevant codons. For example, by this calculation the codon pair GCA-GAA would be expected to occur at a frequency of 0.097 out of all Ala-Glu coding pairs ($0.23 \times 0.42$; based on the frequencies in Table 2). In order to relate the expected (hypothetical) frequency of each codon pair to the actually observed frequency in the human genome the Consensus CDS (CCDS) database of consistently annotated human coding regions, containing a total of 14,795 human genes, was used. This set of genes is the most comprehensive representation of human coding sequences. Using this set of genes, the frequencies of codon usage were re-calculated by dividing the number of occurrences of a codon by the number of all synonymous codons coding for the same amino acid. As expected the frequencies correlated closely with previously published ones such as the ones given in Table 2. Slight frequency variations are possibly due to an oversampling effect in the data provided by the codon usage database at Kazusa DNA Research Institute (www.kazusa.or.jp/codon/codon.html) where 84949 human coding sequences were included in the calculation (far more than the actual number of human genes). The codon frequencies thus calculated were then used to calculate the expected codon-pair frequencies by first multiplying the frequencies of the two relevant codons with each other (see Table 3 expected frequency), and then multiplying this result with the observed frequency (in the entire CCDS data set) with which the amino acid pair encoded by the codon pair in question occurs. In the example of codon pair GCA-GAA, this second calculation gives an expected frequency of 0.098 (compared to 0.097 in the first calculation using the Kazusa dataset). Finally, the actual codon pair frequencies as observed in a set of 14,795 human genes was determined by counting the total number of occurrences of each codon pair in the set and dividing it by the number of all synonymous coding pairs in the set coding for the same amino acid pair (Table 3; observed frequency). Frequency and observed/expected values for the complete set of 3721 ($61^2$) codon pairs, based on the set of 14,795 human genes, are provided herewith as Table 3.

*Table 3. Codon Pair Scores Exemplified by the Amino Pair Ala-Glu*

| amino acid pair | codon pair | expected frequency | observed frequency | obs/exp ratio |
|---|---|---|---|---|
| AE | GCAGAA | 0.098 | 0.163 | 1.65 |
| AE | GCAGAG | 0.132 | 0.198 | 1.51 |
| AE | GCCGAA | 0.171 | 0.031 | 0.18 |
| AE | GCCGAG | 0.229 | 0.142 | 0.62 |
| AE | GCGGAA | 0.046 | 0.027 | 0.57 |
| AE | GCGGAG | 0.062 | 0.089 | 1.44 |
| AE | GCTGAA | 0.112 | 0.145 | 1.29 |
| AE | GCTGAG | 0.150 | 0.206 | 1.37 |
| Total | | 1.000 | 1.000 | |

If the ratio of observed frequency/expected frequency of the codon pair is greater than one the codon pair is said to be overrepresented. If the ratio is smaller than one, it is said to be underrepresented. In the example, the codon pair GCA-GAA is overrepresented 1.65 fold while the coding pair GCC-GAA is more than 5-fold underrepresented.

Many other codon pairs show very strong bias; some pairs are under-represented, while other pairs are over-represented. For instance, the codon pairs GCCGAA (AlaGlu) and GATCTG (AspLeu) are three- to six-fold under-represented (the preferred pairs being GCAGAG and GACCTG, respectively), while the codon pairs GCCAAG (AlaLys) and AATGAA (AsnGlu) are about two-fold over-represented. It is noteworthy that codon pair bias has nothing to do with the frequency of pairs of amino acids, nor with the frequency of individual codons. For instance, the under-represented pair GATCTG (AspLeu) happens to use the most frequent Leu codon, (CTG).

As discussed more fully below, codon pair bias takes into account the score for each codon pair in a coding sequence averaged over the entire length of the coding sequence. According to the invention, codon pair bias is determined by

$$\mathrm{CPB} = \sum_{\mathrm{i}=1}^{\mathrm{k}} \frac{CPSi}{K-1}$$

Accordingly, similar codon pair bias for a coding sequence can be obtained, for example, by minimized codon pair scores over a subsequence or moderately diminished codon pair scores over the full length of the coding sequence.

*Calculation of Codon Pair Bias*

Every individual codon pair of the possible 3721 non-"STOP" containing codon pairs (e.g., GTT-GCT) carries an assigned "codon pair score," or "CPS" that is specific for a given "training set" of genes. The CPS of a given codon pair is defined as the log ratio of the observed number of occurrences over the number that would have been expected in this set of genes (in this example the human genome). Determining the actual number of occurrences of a particular codon pair (or in other words the likelihood of a particular amino acid pair being encoded by a particular codon pair) is simply a matter of counting the actual number of occurrences of a codon pair in a particular set of coding sequences. Determining the expected number, however, requires additional calculations. The expected number is calculated so as to be independent of both amino acid frequency and codon bias similarly to Gutman and Hatfield. That is, the expected frequency is calculated based on the relative proportion of the number of times an amino acid is encoded by a specific codon. A positive CPS value signifies that the given codon pair is statistically over-represented, and a negative CPS indicates the pair is statistically under-represented in the human genome.

To perform these calculations within the human context, the most recent Consensus CDS (CCDS) database of consistently annotated human coding regions, containing a total of 14,795 genes, was used. This data set provided codon and codon pair, and thus amino acid and amino-acid pair frequencies on a genomic scale.

The paradigm of Federov et al. (2002), was used to further enhanced the approach of Gutman and Hatfield (1989). This allowed calculation of the expected frequency of a given codon pair independent of codon frequency and non-random associations of neighboring codons encoding a particular amino acid pair. The detailed equations used to calculate CPB are disclosed in WO 2008/121992 and WO 2011/044561, which are incorporated by reference.

$$S(P_{ij}) = \ln\left(\frac{N_O(P_{ij})}{N_E(P_{ij})}\right) = \ln\left(\frac{N_O(P_{ij})}{F(C_i)F(C_j)N_O(X_{ij})}\right)$$

In the calculation, $P_{ij}$ is a codon pair occurring with a frequency of $N_O(P_{ij})$ in its synonymous group. $C_i$ and $C_j$ are the two codons comprising $P_{ij}$, occurring with frequencies $F(C_i)$ and $F(C_j)$ in their synonymous groups respectively. More explicitly, $F(C_i)$ is the frequency that corresponding amino acid $X_i$ is coded by codon $C_i$ throughout all coding regions and $F(C_i)=N_O(C_j)/N_O(X_i)$, where $N_O(C_i)$ and $N_O(X_i)$ are the observed number of occurrences of codon $C_i$ and amino acid $X_i$ respectively. $F(C_j)$ is calculated accordingly. Further, $N_O(X_{ij})$ is the number of occurrences of amino acid pair $X_{ij}$ throughout all coding regions. The codon pair bias score $S(P_{ij})$ of $P_{ij}$ was calculated as the log-odds ratio of the observed frequency $N_o(P_{ij})$ over the expected number of occurrences of $N_e(P_{ij})$.

Using the formula above, it was then determined whether individual codon pairs in individual coding sequences are over- or under-represented when compared to the corresponding genomic $N_e(P_{ij})$ values that were calculated by using the entire human CCDS data set. This calculation resulted in positive $S(P_{ij})$ score values for over-represented and negative values for under-represented codon pairs in the human coding regions.

The "combined" codon pair bias of an individual coding sequence was calculated by averaging all codon pair scores according to the following formula:

$$S(P_{ij}) = \sum_{l=1}^{k} \frac{S(P_{ij})l}{k-1}$$

The codon pair bias of an entire coding region is thus calculated by adding all of the individual codon pair scores comprising the region and dividing this sum by the length of the coding sequence.

*Calculation of Codon Pair Bias, Implementation of Algorithm to Alter Codon-Pair Bias.*

An algorithm was developed to quantify codon pair bias. Every possible individual codon pair was given a "codon pair score", or "CPS". CPS is defined as the natural log of the ratio of the observed over the expected number of occurrences of each codon pair over all human coding regions, where humans represent the host species of the instant vaccine virus to be recoded.

$$CPS = \ln\left(\frac{F(AB)_O}{\frac{F(A) \times F(B)}{F(X) \times F(Y)} \times F(XY)}\right)$$

Although the calculation of the observed occurrences of a particular codon pair is straightforward (the actual count within the gene set), the expected number of occurrences of a codon pair requires additional calculation. We calculate this expected number to be independent both of amino acid frequency and of codon bias, similar to Gutman and Hatfield. That is, the expected frequency is calculated based on the relative proportion of the number of times an amino acid is encoded by a specific codon. A positive CPS value signifies that the given codon pair is statistically over-represented, and a negative CPS indicates the pair is statistically under-represented in the human genome.

Using these calculated CPSs, any coding region can then be rated as using over- or under-represented codon pairs by taking the average of the codon pair scores, thus giving a Codon Pair Bias (CPB) for the entire gene.

$$CPB = \sum_{i=1}^{k} \frac{CPSi}{k-1}$$

The CPB has been calculated for all annotated human genes using the equations shown and plotted (FIG. 1). Each point in the graph corresponds to the CPB of a single human gene. The peak of the distribution has a positive codon pair bias of 0.07, which is the mean score for all annotated human genes. Also, there are very few genes with a negative codon pair bias. Equations established to define and calculate CPB were then used to manipulate this bias.

Algorithm for Reducing Codon-Pair Bias to Attenuate.

Recoding of protein-encoding sequences may be performed with or without the aid of a computer, using, for example, a gradient descent, or simulated annealing, or other minimization routine. An example of the procedure that rearranges codons present in a starting sequence can be represented by the following steps:

(1) Obtain wildtype viral genome sequence.
(2) Select protein coding sequences to target for attenuated design.
(3) Lock down known or conjectured DNA segments with non-coding functions.
(4) Select desired codon distribution for remaining amino acids in redesigned proteins.
(5) Perform random shuffle of at least two synonymous unlocked codon positions and calculate codon-pair score.
(6) Further reduce (or increase) codon-pair score optionally employing a simulated annealing procedure.
(7) Inspect resulting design for excessive secondary structure and unwanted restriction site:

- if yes-> go to step (5) or correct the design by replacing problematic regions with wildtype sequences and go to step (8).

(8) Synthesize DNA sequence corresponding to virus design.
(9) Create viral construct and assess viral phenotype:

- if too attenuated, prepare subclone construct and go to 9;
- if insufficiently attenuated, go to 2.

Attenuation of viruses by reducing codon pair bias is disclosed in WO 2008/121992 and WO 2011/044561, which are incorporated by reference.

Methods of obtaining full-length Flavivirus or dengue genome sequence or codon pair deoptimized sequences embedded in a wild-type Flavivirus or dengue genome sequence can include for example, constructing an infectious cDNA clone, using an overlap extension PCR strategy, or long PCR-based fusion strategy.

***Modified Flavivirus***

Various embodiments of the invention provide for a modified Flavivirus virus in which expression of viral proteins is reduced compared to a parent virus. The reduction in expression is the result of recoding the prM, or envelope (E) region or both. In some embodiments the parent virus is a wild type Flavivirus, and thus, comparisons are made to the wild-type virus or sequences in the wild-type virus.

In various embodiments, the E protein-encoding sequence is recoded by reducing the codon pair bias or codon usage bias of the protein-encoding sequence. In various embodiments, reducing the codon-pair bias comprises identifying a codon pair in the parent protein-encoding sequence having a codon-pair score that can be reduced, and reducing the codon-pair bias by substituting the codon pair with a codon pair that has a lower codon-pair score. In other embodiments, reducing the codon-pair bias comprises rearranging the codons of a parent protein-encoding sequence.

In various embodiments, each of the recoded prM/E protein-encoding sequence have a codon pair bias less than, -0.05, -0.1, or less than -0.2, or less than -0.3, or less than -0.4.

In certain embodiments, the recoded prM protein-encoding sequence has a codon pair bias less than -0.05, or less than -0.06, or less than -0.07, or less than -0.08, or less than -0.09, or less than -0.1, or less than -0.11, or less than -0.12, or less than -0.13, or less than -0.14, or less than -0.15, or less than -0.16, or less than -0.17, or less than -0.18, or less than -0.19, or less than -0.2, or less than -0.25, or less than -0.3, or less than -0.35, or less than -0.4, or less than -0.45, or less than -0.5.

In certain embodiments, the recoded E protein-encoding sequence has a codon pair bias less than -0.05, or less than -0.06, or less than -0.07, or less than -0.08, or less than -0.09, or less than -0.1, or less than -0.11, or less than -0.12, or less than -0.13, or less than -0.14, or less than -0.15, or less than -0.16, or less than -0.17, or less than -0.18, or less than -0.19, or less than -0.2, or less than -0.25, or less than -0.3, or less than -0.35, or less than -0.4, or less than -0.45, or less than -0.5.

In certain embodiments, the codon pair bias of the recoded prM protein encoding sequence is reduced by at least 0.05, or at least 0.06, or at least 0.07, or at least 0.08, or at least 0.09, or at least 0.1, or at least 0.11, or at least 0.12, or at least 0.13, or at least 0.14, or at least 0.15, or at least 0.16, or at least 0.17, or at least 0.18, or at least 0.19, or at least 0.2, or at least 0.25, or at least 0.3, or at least 0.35, or at least 0.4, or at least 0.45, or at least 0.5, compared to the parent prM protein encoding sequence from which it is derived.

In certain embodiments, the codon pair bias of the recoded E protein encoding sequence is reduced by at least 0.05, or at least 0.06, or at least 0.07, or at least 0.08, or at least 0.09, or at least 0.1, or at least 0.11, or at least 0.12, or at least 0.13, or at least 0.14, or at least 0.15, or at least 0.16, or at least 0.17, or at least 0.18, or at least 0.19, or at least 0.2, or at least 0.25, or at least 0.3, or at least 0.35, or at least 0.4, or at least 0.45, or at least 0.5, compared to the parent E protein encoding sequence from which it is derived. In certain embodiments, it is in comparison to an E protein encoding sequence from which the calculation is to be made; for example, the E protein encoding sequence of a wild type virus.

In various embodiments, the E protein-encoding sequence is recoded by increasing the number of CpG or UpA di nucleotides compared to its parent virus. In various embodiments, the E protein-encoding sequence is recoded by modifying G+C content compared to its parent virus.

In various embodiments, the E protein-encoding sequence is recoded by replacing one or more codons with synonymous codons that are less frequent in the viral host; for example, human.

In various embodiments, the E protein-encoding sequence is recoded by replacing one or more codons with synonymous codons that are less frequent in the virus itself.

In some embodiments, the number of codons substituted in the prM protein encoding sequence with synonymous codons is at least 5, or at least 10, or at least 30, or at least 30, or at least 40, or at least 50, or at least 75, or at least 100, or at least 150.

In some embodiments, the number of codons substituted in the E protein encoding sequence with synonymous codons is at least 5, or at least 10, or at least 30, or at least 30, or at least 40, or at least 50, or at least 75, or at least 100, or at least 125 or at least 150, or at least 175.

In various embodiments, the parent virus is a Flavivirus selected from the group consisting of dengue fever virus, West Nile virus, yellow fever virus, Japanese encephalitis virus, Spondweni virus, Zika virus, Saint Louis encephalitis virus, and Powassan virus. In various embodiments, the parent virus is a natural isolate. In various embodiments, the parent virus is a mutant of a natural isolate.

*Modified Dengue Viruses*

Various embodiments of the present invention provide for modified dengue viruses as the modified Flavivirus.

In various embodiments, a modified dengue virus is provided in which expression of viral proteins is reduced compared to a parent virus, wherein the reduction in expression is the result of recoding the prM, or envelope (E) region, or both. In some embodiments, a parent dengue virus is a wild-type dengue virus. As such, comparisons can be made in reference to a wild-type dengue virus.

In various embodiments, one or both of the E protein-encoding sequence is recoded by reducing the codon pair bias or codon usage bias of the protein-encoding sequence. In various embodiments, reducing the codon-pair bias comprises identifying a codon pair in the parent protein-encoding sequence having a codon-pair score that can be reduced, and reducing the codon-pair bias by substituting the codon pair with a codon pair that has a lower codon-pair score. In various embodiments, reducing the codon-pair bias comprises rearranging the codons of a parent protein-encoding sequence.

Various embodiments of the present invention provide for a modified dengue virus, comprising a recoded prM protein, a recoded envelope (E) protein, or both, wherein the recoded prM protein has a reduced codon pair bias compared to its parent prM protein encoding sequence, or has at least 5 codons substituted with synonymous codons less frequently used, or has an increased number of CpG or UpA di-nucleotides compared its parent prM protein encoding sequence, and wherein the recoded E protein has a reduced codon pair bias compared to its parent E protein encoding sequence, or has at least 5 codons substituted with synonymous codons less frequently used, or has an increased number of CpG or UpA di-nucleotides compared its parent E protein encoding sequence. In various embodiments, "its parent protein encoding sequence" is "a wild-type dengue protein encoding sequence", for example, "a wild-type dengue E protein encoding sequence", "a wild-type dengue prM protein encoding sequence".

In various embodiments, the expression of the prM protein or E protein or both are reduced compared to its parent dengue virus.

In various embodiments, the recoded prM protein has a reduced codon pair bias compared to its parent prM protein encoding sequence. In various embodiments, the codon pair bias of the recoded prM encoding sequence is reduced by at least 0.05. In certain embodiments, the codon pair bias of the recoded prM protein encoding sequence of the dengue virus is reduced by at least 0.06, or at least 0.07, or at least 0.08, or at least 0.09, or at least 0.1, or at least 0.11, or at least 0.12, or at least 0.13, or at least 0.14, or at least 0.15, or at least 0.16, or at least 0.17, or at least 0.18, or at least 0.19, or at least 0.2, or at least 0.25, or at least 0.3, or at least 0.35, or at least 0.4, or at least 0.45, or at least 0.5, compared to the parent dengue virus' prM protein encoding sequence from which it is derived. In certain embodiments, it is in comparison to a prM protein encoding sequence from which the calculation is to be made; for example, a prM protein encoding sequence of a wild-type dengue virus.

In various embodiments, the recoded prM protein has at least 5 codons substituted with synonymous codons less frequently used. In various embodiments, the recoded prM protein has at least 10, 20, 25, 30, 35, 40, 45, 50 or 55 codons substituted with synonymous codons less frequently used. In some embodiments, the substitution with synonymous codons less frequently used are one that are less frequently used in the viral host; for example, human, mosquitos. In some embodiments, the substitution with synonymous codons less frequently used are one that are less frequently used in the virus itself.

In various embodiments, the recoded prM protein has an increased number of CpG or UpA di-nucleotides compared its parent prM protein encoding sequence. In various embodiments, the recoded prM protein has an increase of 15-55 CpG or UpA di-nucleotides compared its parent prM protein encoding sequence. In various embodiments, the recoded prM protein has an increase of about 15, 20, 25, 30, 35, 40, 45 or 55 CpG or UpA di-nucleotides compared its parent prM protein encoding sequence.

In various embodiments, the recoded E protein has a reduced codon pair bias compared to its parent E protein encoding sequence. In various embodiments, the codon pair bias of E protein-encoding sequence is reduced by at least 0.05 compared to its parent E protein encoding sequence. In certain embodiments, the codon pair bias of the recoded E protein encoding sequence of the dengue virus is reduced by at least 0.06, or at least 0.07, or at least 0.08, or at least 0.09, or at least 0.1, or at least 0.11, or at least 0.12, or at least 0.13, or at least 0.14, or at least 0.15, or at least 0.16, or at least 0.17, or at least 0.18, or at least 0.19, or at least 0.2, or at least 0.25, or at least 0.3, or at least 0.35, or at least 0.4, or at least 0.45, or at least 0.5, compared to its parent dengue virus' E protein encoding sequence from which it is derived. In certain embodiments, it is in comparison to an E protein encoding sequence from which the calculation is to be made; for example, the E protein encoding sequence of a wild-type dengue virus.

In various embodiments, the recoded E protein has at least 5 codons substituted with synonymous codons less frequently used. In various embodiments, the recoded E protein has at least 10, 20, 25, 30, 35, 40, 45, 50, 55, 60, 65, 70, 75, 80, 85, 90, 95, 100, 125, 150, or 175 codons substituted with synonymous codons less frequently used. In some embodiments, the substitution with synonymous codons less frequently used are one that are less frequently used in the viral host; for example, human, mosquitos. In some embodiments, the substitution with synonymous codons less frequently used are one that are less frequently used in the virus itself.

In various embodiments, the recoded E protein has an increased number of CpG or UpA di-nucleotides compared its parent E protein encoding sequence. In various embodiments, the recoded prM protein has an increase of 5-12 CpG or UpA di-nucleotides compared its parent E protein encoding sequence. In various embodiments, the recoded prM protein has an increase of about 5, 6, 7, 8, 9, 10 11 or 12 CpG or UpA di-nucleotides compared its parent E protein encoding sequence.

In various embodiments, each of the recoded prM or E protein-encoding sequence has a codon pair bias of less than -0.05. In certain embodiments, the recoded prM protein encoding sequence has a codon pair bias of less than -0.05, or less than -0.06, or less than -0.07, or less than -0.08, or less than -0.09, or less than -0.1, or less than -0.11, or less than -0.12, or less than -0.13, or less than -0.14, or less than -0.15, or less than -0.16, or less than -0.17, or less than -0.18, or less than -0.19, or less than -0.2, or less than -0.25, or less than -0.3, or less than -0.35, or less than -0.4, or less than -0.45, or less than -0.5. In certain embodiments, the recoded E protein encoding sequence has a codon pair bias of less than -0.05, or less than -0.06, or less than -0.07, or less than -0.08, or less than -0.09, or less than -0.1, or less than -0.11, or less than -0.12, or less than -0.13, or less than -0.14, or less than -0.15, or less than -0.16, or less than -0.17, or less than -0.18, or less than -0.19, or less than -0.2, or less than -0.25, or less than -0.3, or less than -0.35, or less than -0.4, or less than -0.45, or less than -0.5.

In various embodiments, the modified dengue virus is selected from type 1, type 2, type 3, type 4 or a combination thereof. For example, type 1 and type 2, type 1 and type 3, type 1 and type 4, type 2 and type 3, type 2 and type 4, or type 3 and type 4. Additional examples are type 1, type 2 and type 3; type 1, type 3 and type 4; or type 2, type 3 and type 4. In various embodiments, the modified dengue virus is a modified tetravalent dengue virus (i.e., type 1, type 2, type 3 and type 4).

*Methods*

Various embodiments provide for a method of eliciting an immune response in a subject, comprising: administering to the subject an effective dose of a composition comprising a modified dengue virus of the present invention as described above and herein.

In various embodiments, the immune response is a protective immune response, and a prophylactically effective or therapeutically effective dose from $10^3$ to $10^7$ of a vaccine composition of claims is administered. In various embodiments, the immune response is a protective immune response, and a prophylactically effective or therapeutically effective dose of $10^3$, $10^4$, $10^5$, $10^6$, or $10^7$ of a vaccine composition of claims is administered. In various embodiments, the method further comprises administering to the subject at least one adjuvant. In various embodiments, the immune response is cross-protective against a heterologous dengue virus.

Various embodiments provide for a method of eliciting an immune response in a subject in need thereof, comprising: administering a prime dose of (i) an attenuated dengue virus produced by a method other than codon-pair deoptimization or codon deoptimization, or increasing of CpG or UpA di-nucleotides, or (ii) a modified dengue virus comprising a recoded prM protein, a recoded envelope (E) protein, or both, wherein the recoded prM protein has a reduced codon pair bias compared to its parent prM protein encoding sequence, or has at least 5 codons substituted with synonymous codons less frequently used, or has an increased number of CpG or UpA di-nucleotides compared its parent prM protein encoding sequence, and wherein the recoded E protein has a reduced codon pair bias compared to its parent E protein encoding sequence, or has at least 5 codons substituted with synonymous codons less frequently, or has an increased number of CpG or UpA di-nucleotides compared its parent E protein encoding sequence; and administering one or more boost dose of (i) the attenuated dengue virus produced by methods other than codon-pair deoptimization, or codon deoptimization, or increasing of CpG or UpA di-nucleotides or (ii) the modified dengue virus to the subject in need thereof, wherein at least the prime dose or the one or more boost dose is the modified dengue virus.

In various embodiments, a first of the one or more boost dose is administered about 2 weeks after the prime dose.

In various embodiments, the expression of the prM protein or E protein or both are reduced compared to its parent dengue virus. In various embodiments the parent dengue virus is a wild-type dengue virus.

In various embodiments, the recoded prM protein has a reduced codon pair bias compared to its parent prM protein encoding sequence. In various embodiments, the codon pair bias of the recoded prM encoding sequence is reduced by at least 0.05. In certain embodiments, the codon pair bias of the recoded prM protein encoding sequence of the dengue virus is reduced by at least 0.06, or at least 0.07, or at least 0.08, or at least 0.09, or at least 0.1, or at least 0.11, or at least 0.12, or at least 0.13, or at least 0.14, or at least 0.15, or at least 0.16, or at least 0.17, or at least 0.18, or at least 0.19, or at least 0.2, or at least 0.25, or at least 0.3, or at least 0.35, or at least 0.4, or at least 0.45, or at least 0.5, compared to the parent dengue virus' prM protein encoding sequence from which it is derived. In certain embodiments, it is in comparison to a prM protein encoding sequence from which the calculation is to be made; for example, a prM protein encoding sequence of a wild-type dengue virus.

In various embodiments, the recoded prM protein has at least 5 codons substituted with synonymous codons less frequently used. In various embodiments, the recoded prM protein has at least 10, 20, 25, 30, 35, 40, 45, 50 or 55 codons substituted with synonymous codons less frequently used. In some embodiments, the substitution with synonymous codons less frequently used are one that are less frequently used in the viral host; for example, human, mosquitos. In some embodiments, the substitution with synonymous codons less frequently used are one that are less frequently used in the virus itself.

In various embodiments, the recoded prM protein has an increased number of CpG or UpA di-nucleotides compared its parent prM protein encoding sequence. In various embodiments, the recoded prM protein has an increase of 15-55 CpG or UpA di-nucleotides compared its parent prM protein encoding sequence. In various embodiments, the recoded prM protein has an increase of about 15, 20, 25, 30, 35, 40, 45 or 55 CpG or UpA di-nucleotides compared its parent prM protein encoding sequence. In certain embodiments, it is in comparison to a prM protein encoding sequence from which the calculation is to be made; for example, a prM protein encoding sequence of a wild-type dengue virus.

In various embodiments, the recoded E protein has a reduced codon pair bias compared to its parent E protein encoding sequence. In various embodiments, the codon pair bias of E protein-encoding sequence is reduced by at least 0.05 compared to its parent E protein encoding sequence. In certain embodiments, the codon pair bias of the recoded E protein encoding sequence of the dengue virus is reduced by at least 0.06, or at least 0.07, or at least 0.08, or at least 0.09, or at least 0.1, or at least 0.11, or at least 0.12, or at least 0.13, or at least 0.14, or at least 0.15, or at least 0.16, or at least 0.17, or at least 0.18, or at least 0.19, or at least 0.2, or at least 0.25, or at least 0.3, or at least 0.35, or at least 0.4, or at least 0.45, or at least 0.5, compared to its parent dengue virus' E protein encoding sequence from which it is derived. In certain embodiments, it is in comparison to an E protein encoding sequence from which the calculation is to be made; for example, the E protein encoding sequence of a wild-type dengue virus.

In various embodiments, the recoded E protein has at least 5 codons substituted with synonymous codons less frequently used. In various embodiments, the recoded E protein has at least 10, 20, 25, 30, 35, 40, 45, 50, 55, 60, 65, 70, 75, 80, 85, 90, 95, 100, 125, 150, or 175 codons substituted with synonymous codons less frequently used. In some embodiments, the substitution with synonymous codons less frequently used are one that are less frequently used in the viral host; for example, human, mosquitos. In some embodiments, the substitution with synonymous codons less frequently used are one that are less frequently used in the virus itself.

In various embodiments, the recoded E protein has an increased number of CpG or UpA di-nucleotides compared its parent E protein encoding sequence. In various embodiments, the recoded prM protein has an increase of 5-12 CpG or UpA di-nucleotides compared its parent E protein encoding sequence. In various embodiments, the recoded prM protein has an increase of about 5, 6, 7, 8, 9, 10 11 or 12 CpG or UpA di-nucleotides compared its parent E protein encoding sequence. In certain embodiments, it is in comparison to an E protein encoding sequence from which the calculation is to be made; for example, the E protein encoding sequence of a wild-type dengue virus.

In various embodiments, each of the recoded prM or E protein-encoding sequence has a codon pair bias of less than -0.05. In certain embodiments, the recoded prM protein encoding sequence has a codon pair bias of less than -0.05, or less than -0.06, or less than -0.07, or less than -0.08, or less than -0.09, or less than -0.1, or less than -0.11, or less than -0.12, or less than -0.13, or less than -0.14, or less than -0.15, or less than -0.16, or less than -0.17, or less than -0.18, or less than -0.19, or less than -0.2, or less than -0.25, or less than -0.3, or less than -0.35, or less than -0.4, or less than -0.45, or less than -0.5. In certain embodiments, the recoded E protein encoding sequence has a codon pair bias of less than -0.05, or less than -0.06, or less than -0.07, or less than -0.08, or less than -0.09, or less than -0.1, or less than -0.11, or less than -0.12, or less than -0.13, or less than -0.14, or less than -0.15, or less than -0.16, or less than -0.17, or less than -0.18, or less than -0.19, or less than -0.2, or less than -0.25, or less than -0.3, or less than -0.35, or less than -0.4, or less than -0.45, or less than -0.5.

In various embodiments, the modified dengue virus is selected from type 1, type 2, type 3, type 4 or a combination thereof. For example, type 1 and type 2, type 1 and type 3, type 1 and type 4, type 2 and type 3, type 2 and type 4, or type 3 and type 4. Additional examples are type 1, type 2 and type 3; type 1, type 3 and type 4; or type 2, type 3 and type 4. In various embodiments, the modified dengue virus is a modified tetravalent dengue virus (i.e., type 1, type 2, type 3 and type 4).

*Methods of Making Modified Flavivirus virus genome*

Various embodiments of the present invention provide for a method of making a modified Flavivirus virus genome. The method comprises obtaining the nucleotide sequence encoding the envelope protein of a Flavivirus virus and the nucleotide sequence encoding the nonstructural 3 proteins of a Flavivirus virus; recoding the envelope encoding nucleotide sequence to reduce protein expression and recoding the nonstructural protein 3-encoding nucleotide sequence to reduce protein expression, and substituting a nucleic acid having the recoded envelope-encoding nucleotide sequence and a nucleic acid having the recoded nonstructural protein 3-encoding nucleotide sequence into a parent Flavivirus virus genome to make a modified Flavivirus virus genome; whereby expression of the recoded envelope-encoding nucleotide sequence and expression of the recoded nonstructural protein 3-encoding nucleotide sequence is reduced compared to the parent virus.

Various embodiments of the present invention provide for a method of making a modified dengue virus genome comprising: obtaining the nucleotide sequence encoding the envelope protein of a dengue virus and the nucleotide sequence encoding the nonstructural 3 proteins of a dengue virus; recoding the envelope encoding nucleotide sequence to reduce protein expression and recoding the nonstructural protein 3-encoding nucleotide sequence to reduce protein expression, and substituting a nucleic acid having the recoded envelope-encoding nucleotide sequence and a nucleic acid having the recoded nonstructural protein 3-encoding nucleotide sequence into a parent dengue virus genome to make a modified dengue virus genome; whereby expression of the recoded envelope-encoding nucleotide sequence and expression of the recoded nonstructural protein 3-encoding nucleotide sequence is reduced compared to the parent virus.

Various embodiments of the present invention provide for a method of making a modified dengue virus genome comprising: obtaining a nucleotide sequence encoding the envelope protein of a dengue virus; recoding the envelope encoding nucleotide sequence to reduce protein expression, and substituting a nucleic acid having the recoded envelope-encoding nucleotide sequence into a parent dengue virus genome to make a modified dengue virus genome; whereby expression of the recoded envelope-encoding nucleotide sequence is reduced compared to the parent virus.

According to various embodiments the invention, viral attenuation is accomplished by reducing expression viral proteins through codon pair deoptimization of E coding sequence. One way to reduce expression of the coding sequences is by a reduction in codon pair bias, but other methods can also be used, alone or in combination. While codon bias may be changed, adjusting codon pair bias is particularly advantageous. For example, attenuating a virus through codon bias generally requires elimination of common codons, and so the complexity of the nucleotide sequence is reduced. In contrast, codon pair bias reduction or minimization can be accomplished while maintaining far greater sequence diversity, and consequently greater control over nucleic acid secondary structure, annealing temperature, and other physical and biochemical properties.

Codon pair bias of a protein-encoding sequence (i.e., an open reading frame) is calculated as set forth above and described in Coleman et al., 2008.

Viral attenuation and induction or protective immune responses can be confirmed in ways that are well known to one of ordinary skill in the art, including but not limited to, the methods and assays disclosed herein. Non-limiting examples include plaque assays, growth measurements, reduced lethality in test animals, and protection against subsequent infection with a wild type virus.

In various embodiments, the invention provides viruses that are highly attenuated, and induce immunity against a plurality of dengue types and/or subtypes. Such dengue virus varieties include viruses in serogroups 1, 2, 3, and 4. Examples of attenuated dengue protein coding sequences are provided below.

*Table 4. Reduced-Expression Dengue Virus Genes*

| | WT Coding Sequence | Recoded Coding Sequence |
|---|---|---|
| Gene | SEQ ID NO: | SEQ ID NO: |
| DENV1-WT | 1 | |
| DENV1-E-Min | | 2 |
| DENV2-WT | 3 | |
| DENV2-E-Min | | 4 |
| DENV3-WT | 5 | |
| DENV3-E-Min | | 6 |
| DENV4-WT | 7 | |
| DENV4-E-Min | | 8 |
| DENV1-E-W/Min | | 9 |
| DENV2-E-W/Min | | 10 |
| DENV3-E-W/Min | | 11 |
| DENV4-E-W/Min | | 12 |

### *Compositions*

Various embodiments provide for a Flavivirus composition for inducing an immune response in a subject, which comprises the modified Flavivirus of the present invention as described herein.

Various embodiments provide for a Flavivirus vaccine composition for inducing a protective immune response in a subject, which comprises the modified Flavivirus of the present invention as described herein.

Various embodiments provide for a modified dengue virus composition for inducing an immune response in a subject, comprising a modified dengue virus of the present invention as described above and herein, and a pharmaceutically acceptable excipient or carrier.

Various embodiments provide for a dengue vaccine composition for inducing a protective immune response in a subject, comprising a modified dengue virus of the present invention as described above and herein, and a pharmaceutically acceptable excipient or carrier.

In various embodiments, the modified dengue virus is selected from type 1, type 2, type 3, type 4 or a combination thereof.

In various embodiments, the modified dengue virus is a type 1 and type 2 modified dengue virus; a type 1 and type 3 modified dengue virus; a type 1 and type 4 modified dengue virus; a type 2 and type 3 modified dengue virus; a type 2 and type 4 modified dengue virus; a type 3 and type 4 modified dengue virus.

In various embodiments, the modified dengue virus is a type 1, type 2 and type 3 modified dengue virus; a type 1, type 2 and type 4 modified dengue virus; a type 1, type 3 and type 4 modified dengue virus; or a type 2, type 3 and type 4 modified dengue virus.

In various embodiments, the modified dengue virus modified tetravalent dengue virus; (type 1, type 2, type 3 and type 4).

Non-limiting examples of wild-type and modified dengue viruses are herein:

SEQ ID NO:1 - DENV-1-VN-BID-V1774-2007(WT)

AGTTGTTAGTCTACGTGGACCGACAAGAACAGTTTCGAATCGGAAGCTTGCTTAACGTAGTTCT
AACAGTTTTTTATTAGAGAGCAGATCTCTGATGAACAACCAACGGAAAAAGACGGCTCGACCG
TCTTTCAATATGCTGAAACGCGCGAGAAACCGCGTGTCAACTGTTTCACAGTTGGCGAAGAGA
TTCTCAAAAGGATTGCTCTCAGGCCAAGGACCCATGAAATTGGTGATGGCTTTCATAGCATTCC
TAAGATTTCTAGCCATACCCCCAACAGCAGGAATTTTGGCTAGATGGGGTTCATTCAAGAAGA
GTGGAGCGATCAAAGTGCTACGGGGTTTCAAGAAAGAAATCTCAAACATGTTGAATATAATGA
ATAGAAGGAAAAGATCTGTGACCATGCTCCTTATGCTGATGCCTACAGCCTTGGCGTTCCATTT
GACTACACGAGGGGGAGAGCCGCACATGATAGTCAGCAAGCAGGAAAGAGGAAAGTCACTCT
TGTTTAAGACCTCAGCAGGTGTCAACATGTGCACCCTTATAGCGATGGATTTGGGAGAGTTATG
TGAGGACACAATGACTTACAAATGCCCTCGAATCACTGAAACTGAACCAGATGACGTTGATTG
TTGGTGTAATGCCACAGACACATGGGTGACCTATGGAACATGTTCCCAAACTGGCGAGCACCG
ACGAGACAAACGTTCCGTCGCACTGGCCCCACACGTGGGACTTGGTTTGGAAACAAGAACCGA

AACGTGGATGTCCTCTGAAGGCGCTTGGAAACAGATACAAAGAGTGGAGACTTGGGCCCTGA GACACCCAGGATTCACGGTGATAGCCCTTTTTCTAGCACATGCCATAGGAACATCCATCACCC AGAAAGGGATTATTTTCATTTTGTTAATGCTGGTAACACCATCCATGGCCATGCGATGCGTGGG AATAGGCAGCAGGGACTTCGTGGAAGGACTGTCAGGAGCAACTTGGGTAGATGTGGTACTGG AACATGGAAGTTGCGTCACCACCATGGCAAAAGACAAACCAACATTGGACATTGAACTCTTGA AGACGGAAGTCACAAACCCTGCCGTCCTGCGCAAACTGTGCATTGAAGCTAAAATATCAAACA CCACCACCGACTCAAGATGTCCAACACAAGGAGAAGCCACACTAGTGGAAGAACAAGACGCG AACTTTGTGTGTCGACGAACGTTTGTGGACAGAGGCTGGGGCAATGGCTGTGGCCTCTTCGGA AAAGGAAGCCTAATAACGTGTGCAAAGTTCAAGTGTGTGACAAAACTGGAAGGAAAGATAGT TCAATATGAGAACTTGAAATATTCAGTAATAGTCACCGTTCACACCGGAGACCAGCATCAAGT GGGAAATGAAAGCACAGAACATGGGACAACTGCAACTATAACACCTCAAGCTCCTACGACGG AAATACAGCTGACCGACTACGGAGCTCTTACATTGGATTGTTCACCTAGAACAGGACTAGACT TTAATGAAATGGTGTTGTTGACAATGAAAGAAAAATCATGGCTAGTCCACAAACAATGGTTTT TAGACCTACCACTGCCTTGGACCTCGGGAGCTTCAACATCACAAGAGACTTGGAACAGACAAG ATTTGCTGGTGACATTTAAGACAGCTCATGCAAAGAAGCAGGAAGTAGTCGTACTAGGATCAC AAGAAGGAGCAATGCACACTGCGTTGACCGGAGCGACAGAAATCCAAACGTCTGGAACGACA ACAATTTTTGCAGGACACTTGAAATGCAGACTAAAGATGGACAAACTGACTCTAAAAGGGATG TCATATGTGATGTGCACAGGCTCATTCAAGCTAGAGAAAGAAGTGGCTGAGACCCAGCATGGA ACCGTTCTAGTGCAGATTAAATACGAAGGAACAGATGCACCATGCAAGATCCCTTTTTCGACC CAAGATGAAAAAGGAGTAACCCAGAATGGGAGATTGATAACAGCTAACCCCATAGTTACTGA CAAAGAAAAACCAGTCAACATTGAGGCAGAACCGCCCTTTGGTGAGAGTTACATCGTAATAGG AGCAGGTGAAAAAGCTTTGAAACTAAGCTGGTTCAAGAAAGGAAGCAGCATAGGGAAAATGT TTGAGGCAACTGCCAGAGGAGCACGAAGGATGGCCATACTGGGAGACACCGCATGGGACTTT GGTTCTATAGGAGGAGTGTTCACGTCTGTTGGAAAATTAGTACACCAGATTTTTGGAACTGCAT ATGGAGTTTTGTTCAGCGGTGTTTCCTGGACCATGAAAATAGGAATAGGGGTTCTGCTGACATG GCTGGGATTAAACTCAAGGAGCACGTCCCTTTCGATGACGTGCATTGCAGTTGGCCTAGTAAC ACTATACCTAGGAGTCATGGTTCAGGCGGATTCAGGATGTGTAATTAATTGGAAAGGTAGAGA ACTCAAGTGTGGAAGTGGCATTTTTGTCACCAATGAAGTTCACACTTGGACAGAGCAATACAA ATTTCAAGCTGACTCCCCTAAGAGACTATCAGCAGCCATCGGGAAGGCATGGGAGGAGGGTGT GTGTGGAATTCGATCAGCAACTCGTCTCGAGAACATCATGTGGAAGCAAATATCAAATGAACT GAATCACATCTTACTTGAAAATGATATGAAATTCACAGTGGTTGTAGGAGATGTTGCTGGGAT CTTGGCTCAAGGAAAGAAAATGATTAGGCCACAACCCATGGAATACAAATACTCGTGGAAAA GCTGGGGAAAGGCTAAAATCATAGGGGCAGATGTACAGAACACCACCTTCATCATCGATGGCC CAAACACCCCAGAATGCCCTGATGACCAAAGAGCATGGAACATTTGGGAAGTTGAGGACTAT GGATTTGGAATTTTCACGACAAACATATGGCTGAAATTGCGTGATTCCTACACCCAAGTGTGTG ACCACCGGCTAATGTCAGCTGCCATCAAGGACAGCAAGGCAGTTCACGCTGACATGGGGTACT GGATAGAAAGTGAAAAGAACGAGACCTGGAAGCTGGCAAGAGCCTCATTCATAGAAGTTAAA

ACATGTATCTGGCCAAAATCCCACACTCTATGGAGCAATGGAGTTCTGGAAAGTGAAATGATA
ATTCCAAAGATCTATGGAGGACCAATATCTCAGCACAACTACAGACCAGGATATTTTACACAA
GCAGCAGGGCCGTGGCACCTAGGCAAGTTGGAACTGGATTTTGATTTGTGTGAGGGTACCACA
GTTGTTGTGGATGAACATTGTGGAAATCGAGGACCATCTCTTAGGACCACAACAGTCACAGGA
AAGATAATTCATGAATGGTGTTGCAGATCTTGCACGCTACCACCCTTACGTTTCAGAGGAGAA
GATGGGTGCTGGTACGGTATGGAAATCAGACCAGTCAAGGAAAAGGAAGAAAATCTAGTCAA
ATCAATGGTCTCTGCAGGGTCAGGGGAAGTGGACAGCTTTTCACTAGGACTGCTATGCATATC
AATAATGATCGAGGAGGTGATGAGATCCAGATGGAGTAGAAGAATGCTGATGACTGGAACAC
TGGCTGTGTTCTTCCTTCTCATAATGGGACAATTGACATGGAACGATCTGATCAGATTATGCAT
CATGGTTGGAGCCAACGCTTCCGACAGGATGGGGATGGGAACGACGTACCTAGCCCTGATGGC
CACTTTTAAAATGAGACCGATGTTCGCTGTAGGGCTATTATTTCGCAGACTAACATCCAGAGAA
GTTCTTCTTCTAACAATTGGATTGAGTCTAGTGGCATCTGTGGAGTTACCAAATTCCTTGGAGG
AGCTGGGGGATGGACTTGCAATGGGCATTATGATTTTAAAATTATTGACTGACTTTCAATCACA
TCAGCTGTGGGCTACCTTGCTGTCCTTGACATTTATCAAAACAACGTTTTCCTTGCACTACGCAT
GGAAGACAATGGCTATGGTACTGTCAATTGTATCTCTCTTCCCTTTATGCCTGTCCACGACCTC
CCAAAAAACAACATGGCTTCCGGTGCTATTGGGATCCCTTGGATGCAAACCACTAACCATGTTT
CTTATAGCAGAAAACAAAATCTGGGGAAGGAGAAGTTGGCCCCTCAATGAAGGAATCATGGC
TGTTGGAATAGTCAGCATCCTACTAAGTTCACTCCTCAAAAATGATGTACCGCTAGCTGGGCCA
CTAATAGCTGGAGGCATGCTAATAGCATGTTATGTTATATCTGGAAGCTCAGCCGACCTATCAT
TAGAGAAAGCGGCTGAGGTCTCCTGGGAAGAAGAAGCAGAACACTCTGGTGCCTCACACAAC
ATATTAGTGGAAGTCCAAGATGATGGAACCATGAAGATAAAGGATGAAGAGAGAGATGACAC
GCTAACCATTCTCCTTAAAGCAACTCTGTTAGCAGTTTCAGGGGTGTACCCATTATCAATACCA
GCGACCCTTTTCGTGTGGTACTTTTGGCAGAAAAAGAAACAGAGATCTGGAGTGTTATGGGAC
ACACCCAGCCCTCCAGAAGTGGAAAGAGCAGTTCTTGATGATGGTATCTATAGAATTATGCAG
AGAGGACTGTTGGGCAGGTCCCAAGTAGGGGTAGGAGTTTTCCAAGAAAACGTGTTCCACACA
ATGTGGCATGTCACCAGGGGAGCTGTACTCATGTATCAAGGGAAGAGATTGGAACCGAGCTGG
GCCAGTGTCAAAAAAGACCTGATCTCATATGGAGGAGGTTGGAGGCTTCAAGGATCCTGGAAC
ACAGGAGAAGAAGTGCAGGTGATTGCTGTTGAACCAGGGAAAAACCCCAAAAATGTACAAAC
AGCGCCGGGCACCTTTAAGACCCCTGAAGGTGAAGTTGGAGCCATTGCCCTAGACTTTAAACC
TGGCACATCTGGATCTCCCATCGTGAACAGAGAAGGAAAAATAGTAGGTCTTTATGGAAATGG
AGTAGTGACAACAAGTGGAACCTACGTCAGTGCCATAGCTCAAGCCAAAGCATCACAAGAAG
GGCCCCTACCAGAGATTGAAGACGAGGTGTTTAGGAAAAGAAACTTAACAATAATGGACCTA
CATCCAGGATCGGGGAAAACAAGAAGATATCTTCCAGCCATAGTCCGTGAGGCCATAAAAAG
GAAGCTGCGCACACTAATCCTGGCTCCCACAAGGGTTGTCGCTTCCGAAATGGCAGAGGCGCT
CAAGGGAATGCCAATAAGGTACCAAACAACAGCAGTGAAGAGTGAACATACAGGAAAAGAG
ATAGTTGACCTCATGTGTCACGCCACTTTCACCATGCGCCTCCTGTCTCCCGTAAGAGTTCCCA
ATTACAACATGATCATCATGGATGAAGCACATTTCACCGATCCATCCAGTATAGCGGCCAGAG

GGTACATCTCAACCCGAGTGGGCATGGGTGAAGCAGCTGCAATCTTCATGACAGCCACTCCCCCAGGATCAGTGGAGGCCTTTCCACAGAGCAACGCAGTTATCCAAGATGAGGAAAGAGACATTCCTGAGAGATCATGGAACTCAGGCTATGAGTGGATCACTGACTTCCCAGGTAAAACAGTTTGGTTTGTTCCAAGCATTAAATCAGGAAATGACATTGCCAACTGCTTAAGAAAGAATGGAAACGGGTGATTCAATTGAGCAGGAAAACCTTTGATACAGAGTACCAAAAAACAAAAAACAACGACTGGGACTACGTCGTCACAACAGACATCTCCGAAATGGGAGCAAATTTCCGAGCCGACAGGGTGATAGACCCAAGACGGTGTCTGAAACCGGTAATACTAAAAGATGGTCCAGAGCGTGTCATTTTAGCAGGACCAATGCCAGTGACTGTGGCCAGTGCCGCTCAGAGGAGAGGAAGAATTGGAAGGAACCACAATAAGGAAGGTGATCAGTACATCTACATGGGACAGCCTTTAAACAACGATGAAGATCACGCTCACTGGACAGAAGCAAAAATGCTCCTTGATAATATAAACACACCAGAAGGGATTATCCCAGCCCTCTTTGAGCCAGAGAGAGAAAAGAGTGCAGCAATAGACGGGGAATACAGACTGCGGGGTGAAGCAAGGAAAACGTTTGTGGAGCTCATGAGAAGAGGAGATCTACCTGTCTGGCTATCCTACAAAGTTGCCTCAGAAGGCTTCCAGTACTCTGACAGAAGATGGTGCTTTGACGGGGAAAGGAACAACCAGGTGTTGGAGGAGAACATGGACGTGGAGATCTGGACAAAAGAAGGAGAAAGAAAGAAACTACGACCCCGCTGGCTGGATGCCAGAACATACTCAGACCCACTAGCCCTGCGCGAGTTTAAAGAGTTTGCAGCAGGGAGAAGAAGCGTCTCAGGTGATTTAATATTAGAAATAGGGAAGCTTCCACAACACTTGACGCAAAGGGCCCAGAATGCCCTGGACAACCTGGTTATGTTGCACAACTCCGAACAAGGAGGCAGAGCCTATAGACATGCAATGGAAGAACTGCCAGACACCATAGAAACGTTGATGCTCCTAGCTTTGATAGCTGTGTTAACTGGTGGAGTGACACTGTTCTTCCTATCAGGAAGGGGCCTAGGGAAAACATCTATCGGCCTACTCTGCGTAATGGCTTCAAGCGTACTGCTATGGATGGCCAGTGTGGAGCCCCATTGGATAGCGGCCTCCATCATACTGGAGTTCTTCCTGATGGTGCTGCTTATTCCAGAGCCAGACAGACAACGCACTCCGCAGGACAACCAGCTGGCATATGTGGTGATAGGTTTGTTATTCATGATACTGACAGTAGCAGCCAATGAGATGGGACTGCTGGAAACCACAAAGAAAGACTTAGGGATTGGCCATGTGGCTGTTGAAAATCACCACCATGCCGCAATGCTGGACGTAGACTTACATCCAGCTTCAGCCTGGACCCTCTATGCAGTGGCCACAACAATTATCACTCCCATGATGAGGCACACAATCGAAAACACAACGGCAAACATTTCCCTGACAGCCATTGCAAACCAGGCAGCTATATTGATGGGACTTGACAAAGGATGGCCAATATCGAAGATGGACATAGGAGTTCCACTTCTCGCCTTGGGGTGCTATTCCCAGGTGAATCCACTGACGCTGACAGCGGCGGTATTGATGCTAGTGGCTCATTACGCCATAATTGGACCTGGACTGCAAGCAAAAGCTACTAGAGAAGCTCAAAAAAGGACAGCGGCCGGAATAATGAAAAATCCAACCGTTGATGGAATTGTTGCAATAGATTTGGACCCTGTGGTTTATGATGCAAAATTTGAAAAACAACTAGGCCAAATAATGTTGTTGATACTATGCACATCACAGATCCTCTTGATGCGGACTACATGGGCCTTGTGCGAGTCCATCACACTGGCCACTGGACCTCTGACCACGCTTTGGGAGGGATCTCCAGGAAAATTTTGGAACACCACGATAGCGGTTTCCATGGCAAACATTTTCAGAGGAAGTTATCTAGCAGGAGCAGGTCTGGCCTTCTCATTAATGAAATCTCTAGGAGGAGGTAGGAGAGGCACGGGAGCCCAAGGGGAAACACTGGGAGAGAAATGGAAAAGACAGCTGAACCAACTGAGCAAGTCAGAATTTAACACCTATAAAAGGAGTGGGATTATGGAAGTGGACAGATCCGAAGCCAAAGAGGGATTGAAAAGAGGAGAAACAACCAAACATGCA

GTGTCGAGAGGAACCGCTAAACTGAGGTGGTTCGTGGAGAGGAACCTTGTGAAACCAGAAGG
GAAAGTCATAGACCTCGGTTGTGGAAGAGGTGGCTGGTCATACTATTGCGCTGGGCTGAAGAA
AGTCACAGAAGTGAAGGGGTATACAAAAGGAGGACCTGGACATGAGGAACCAATCCCAATGG
CGACCTATGGATGGAACCTAGTAAAGCTGCACTCTGGGAAAGACGTATTTTTTATACCACCTG
AGAAATGTGACACCCTTTTGTGTGATATTGGTGAGTCCTCTCCAAACCCAACTATAGAGGAAG
GAAGAACGCTACGCGTCCTAAAGATGGTGGAACCATGGCTCAGAGGAAACCAATTTTGCATAA
AAATTCTGAATCCCTACATGCCAAGTGTGGTGGAAACTCTGGAGCAAATGCAAAGAAAACATG
GAGGAATGCTAGTGCGAAATCCACTTTCAAGAAATTCTACTCATGAAATGTATTGGGTTTCATG
TGGAACAGGAAACATTGTGTCAGCAGTAAACATGACATCTAGAATGTTGCTAAATCGATTCAC
AATGGCTCACAGGAAACCAACATATGAAAGAGACGTGGACCTAGGCGCCGGAACAAGACATG
TGGCAGTGGAACCAGAGGTAGCCAACCTAGATATCATTGGCCAGAGGATAGAGAACATAAAA
CATGAACATAAGTCAACATGGCATTATGATGAGGACAATCCATATAAAACATGGGCCTATCAT
GGATCATATGAGGTCAAGCCATCAGGATCAGCCTCATCCATGGTCAATGGCGTGGTGAAACTG
CTCACCAAACCATGGGATGTCATCCCTATGGTCACACAAATAGCCATGACTGACACTACACCC
TTTGGACAACAGAGGGTGTTTAAAGAGAAAGTTGACACACGCACACCAAAAGCAAAACGGGG
CACAGCACAAATCATGGAGGTGACAGCCAAGTGGTTATGGGGTTTTCTTTCTAGAAACAAGAA
ACCAAGAATTTGCACAAGAGAGGAGTTCACAAGAAAAGTTAGGTCAAACGCAGCCATTGGAG
CAGTGTTCGTTGATGAAAATCAATGGAACTCAGCAAAAGAAGCAGTGGAAGATGAGCGGTTCT
GGGACCTTGTGCATAGAGAGAGGGAGCTTCACAAACAGGGAAAATGTGCTACGTGTGTTTACA
ACATGATGGGGAAGAGAGAGAAAAAGCTAGGAGAGTTCGGAAAGGCAAAAGGAAGTCGTGC
AATATGGTACATGTGGTTGGGAGCACGCTTTCTAGAGTTCGAAGCTCTTGGTTTCATGAACGAA
GATCACTGGTTCAGCAGAGAGAATTCACTCAGCGGAGTGGAAGGAGAAGGACTCCACAAACT
TGGATATATACTCAGAGACATATCAAAGATTCCAGGGGGAAACATGTATGCAGATGACACAGC
CGGATGGGATACAAGGATAACAGAGGATGACCTTCAGAATGAGGCCAGAATTACTGACATCA
TGGAACCCGAACATGCCCTCCTGGCTAAGTCAATCTTCAAGTTAACCTACCAAAATAAGGTGG
TAAGGGTACAGAGACCAGCAAAAAATGGAACCGTGATGGATGTCATATCCAGACGTGACCAG
AGAGGAAGTGGTCAGGTCGGAACTTATGGCTTAAACACTTTCACCAACATGGAAGCCCAGCTG
ATAAGACAAATGGAGTCTGAGGGAATCTTTTCACCCAGCGAATTAGAGACCCCAAATTTAGCC
GAGAGAGTTCTCGACTGGCTGGAAAAATATGGCGTCGAAAGGCTGAAAAGAATGGCAATCAG
CGGAGATGACTGCGTAGTGAAACCAATTGATGATAGGTTTGCAACAGCCTTGACAGCTCTGAA
TGATATGGGAAAAGTAAGAAAAGATATACCACAATGGGAACCTTCAAAAGGATGGAATGATT
GGCAACAAGTGCCTTTTTGTTCACACCACTTCCACCAGCTGATTATGAAGGATGGGAGGGAAA
TAGTGGTGCCATGCCGCAACCAAGATGAACTTGTGGGTAGGGCTAGAGTATCACAAGGTGCTG
GATGGAGCCTGAGAGAAACTGCATGCCTAGGCAAGTCATATGCACAAATGTGGCAGCTGATGT
ACTTCCACAGGAGAGACCTGAGACTAGCCGCTAATGCTATCTGTTCAGCCGTTCCAGTTGATTG
GATCCCAACCAGCCGTACCACCTGGTCGATCCATGCCCATCACCAATGGATGACAACAGAAGA
CATGCTGTCAGTGTGGAATAGGGTTTGGATAGAGGAAAACCCATGGATGGAGGACAAAACCC

ACATATCCAGTTGGGGAGATGTTCCATATTTAGGAAAAAGGGAAGACCAATGGTGTGGATCCC
TGATAGGCTTAACAGCAAGGGCCACCTGGGCCACCAACATACAAGTGGCCATAAACCAAGTG
AGAAGACTAATTGGGAATGAGAATTATCTAGATTACATGACATCAATGAAGAGATTCAAGAAC
GAGAGTGATCCCGAAGGGGCACTCTGGTGAGTCAACACATTTACAAAATAAAGGAAAATAAG
AAATCAAACAAGGCAAGAAGTCAGGCCGGATTAAGCCATAGTACGGTAAGAGCTATGCTGCC
TGTGAGCCCCGTCTAAGGACGTAAAATGAAGTCAGGCCGAAAGCCACGGCTTGAGCAAACCG
TGCTGCCTGTAGCTCCATCGTGGGGATGTAAAAACCTGGGAGGCTGCAACCCATGGAAGCTGT
ACGCATGGGGTAGCAGACTAGTGGTTAGAGGAGACCCCTCCCGAAACATAACGCAGCAGCGG
GGCCCAACACCAGGGGAAGCTGTACCCTGGTGGTAAGGACTAGAGGTTAGAGGAGACCCCCC
GCATAACAATAAACAGCATATTGACGCTGGGAGAGACCAGAGATCCTGCTGTCTCTACAGCAT
CATTCCAGGCACAGAACGCCAGAAAATGGAATGGTGCTGTTGAATCAACAGGTTCT

SEQ ID NO:2 - DENV-1-VN-BID-V1774-2007-EMin

AGTTGTTAGTCTACGTGGACCGACAAGAACAGTTTCGAATCGGAAGCTTGCTTAACGTAGTTCT
AACAGTTTTTATTAGAGAGCAGATCTCTGATGAACAACCAACGGAAAAAGACGGCTCGACCG
TCTTTCAATATGCTGAAACGCGCGAGAAACCGCGTGTCAACTGTTTCACAGTTGGCGAAGAGA
TTCTCAAAAGGATTGCTCTCAGGCCAAGGACCCATGAAATTGGTGATGGCTTTCATAGCATTCC
TAAGATTTCTAGCCATACCCCCAACAGCAGGAATTTTGGCTAGATGGGGTTCATTCAAGAAGA
GTGGAGCGATCAAAGTGCTACGGGGTTTCAAGAAAGAAATCTCAAACATGTTGAATATAATGA
ATAGAAGGAAAAGATCTGTGACCATGCTCCTTATGCTGATGCCTACAGCCTTGGCGTTCCATTT
GACTACACGAGGGGGAGAGCCGCACATGATAGTCAGCAAGCAGGAAAGAGGAAAGTCACTCT
TGTTTAAGACCTCAGCAGGTGTCAACATGTGCACCCTTATAGCGATGGATTTGGGAGAGTTATG
TGAGGACACAATGACTTACAAATGCCCTCGAATCACTGAAACTGAACCAGATGACGTTGATTG
TTGGTGTAATGCCACAGACACATGGGTGACCTATGGAACATGTTCCCAAACTGGCGAGCACCG
ACGAGACAAACGTTCCGTCGCACTGGCCCCACACGTGGGACTTGGTTTGGAAACAAGAACCGA
AACGTGGATGTCCTCTGAAGGCGCTTGGAAACAGATACAAAGAGTGGAGACTTGGGCCCTGA
GACACCCAGGATTCACGGTGATAGCCCTTTTTCTAGCACATGCCATAGGAACATCCATCACCC
AGAAAGGGATTATTTTCATTTTGTTAATGCTGGTAACACCATCCATGGCCATGCGATGCGTAGG
GATAGGGTCACGCGATTTCGTCGAAGGACTATCCGGAGCGACATGGGTCGACGTCGTACTCGA
ACACGGATCATGCGTTACGACTATGGCTAAAGACAAACCTACACTAGACATAGAGTTACTGAA
AACCGAAGTTACGAATCCCGCCGTACTGCGAAAATTGTGTATCGAAGCGAAAATTAGCAATAC
GACTACAGACTCTAGGTGTCCTACACAAGGCGAAGCGACATTGGTCGAAGAACAGGACGCTA
ACTTCGTATGTAGACGAACATTCGTCGATAGGGGGTGGGGAAACGGATGCGGATTGTTCGGTA
AAGGATCACTGATTACATGCGCAAAGTTCAAATGCGTTACGAAACTGGAAGGTAAAATAGTGC
AATACGAAAACCTAAAGTATAGCGTAATCGTTACAGTGCATACCGGAGACCAACATCAGGTCG
GAAACGAATCAACCGAACACGGAACAACCGCAACAATTACACCACAGGCACCTACAACCGAA
ATTCAACTAACCGATTACGGAGCTCTTACACTAGATTGCTCACCTAGAACCGGATTGGACTTTA

ACGAAATGGTACTGTTGACTATGAAGGAGAAATCATGGTTAGTGCATAAACAATGGTTTCTAG
ACCTACCACTACCATGGACTAGCGGAGCGAGTACGTCACAGGAAACATGGAATAGACAGGAC
CTATTGGTGACATTTAAGACCGCACACGCTAAGAAGCAGGAAGTCGTAGTGTTAGGGTCACAA
GAGGGAGCAATGCATACCGCACTAACCGGAGCAACCGAAATACAGACTAGCGGAACTACAAC
GATTTTTGCCGGTCACCTGAAATGTAGACTGAAGATGGACAAACTGACACTTAAAGGAATGTC
ATACGTTATGTGTACGGGATCCTTTAAGCTCGAAAAAGAGGTTGCCGAAACGCAACACGGAAC
AGTGCTAGTGCAAATTAAATATGAGGGAACCGACGCACCATGTAAGATACCGTTTAGTACGCA
AGACGAAAAGGGCGTTACGCAAAACGGAAGACTGATTACCGCTAACCCTATCGTTACAGACA
AAGAGAAACCCGTTAACATAGAGGCCGAACCACCTTTTGGCGAATCATATATAGTGATAGGCG
CAGGCGAAAAAGCACTAAAGCTGTCATGGTTCAAAAAAGGATCTAGCATAGGGAAGATGTTC
GAAGCAACCGCTAGGGGAGCTAGACGAATGGCAATACTGGGAGATACCGCATGGGACTTCGG
ATCGATAGGGGGAGTGTTTACTAGCGTAGGTAAGTTGGTGCATCAGATATTCGGAACCGCATA
CGGAGTGTTGTTTAGCGGAGTGTCATGGACTATGAAGATAGGGATAGGAGTGCTATTGACATG
GCTCGGACTGAATTCGAGATCGACTAGCCTATCTATGACATGCATAGCCGTCGGACTGGTTAC
ACTGTATCTAGGCGTAATGGTGCAAGCAGATTCAGGATGTGTAATTAATTGGAAAGGTAGAGA
ACTCAAGTGTGGAAGTGGCATTTTTGTCACCAATGAAGTTCACACTTGGACAGAGCAATACAA
ATTTCAAGCTGACTCCCCTAAGAGACTATCAGCAGCCATCGGGAAGGCATGGGAGGAGGGTGT
GTGTGGAATTCGATCAGCAACTCGTCTCGAGAACATCATGTGGAAGCAAATATCAAATGAACT
GAATCACATCTTACTTGAAAATGATATGAAATTCACAGTGGTTGTAGGAGATGTTGCTGGGAT
CTTGGCTCAAGGAAAGAAAATGATTAGGCCACAACCCATGGAATACAAATACTCGTGGAAAA
GCTGGGGAAAGGCTAAAATCATAGGGGCAGATGTACAGAACACCACCTTCATCATCGATGGCC
CAAACACCCCAGAATGCCCTGATGACCAAAGAGCATGGAACATTTGGGAAGTTGAGGACTAT
GGATTTGGAATTTTCACGACAAACATATGGCTGAAATTGCGTGATTCCTACACCCAAGTGTGTG
ACCACCGGCTAATGTCAGCTGCCATCAAGGACAGCAAGGCAGTTCACGCTGACATGGGGTACT
GGATAGAAAGTGAAAAGAACGAGACCTGGAAGCTGGCAAGAGCCTCATTCATAGAAGTTAAA
ACATGTATCTGGCCAAAATCCCACACTCTATGGAGCAATGGAGTTCTGGAAAGTGAAATGATA
ATTCCAAAGATCTATGGAGGACCAATATCTCAGCACAACTACAGACCAGGATATTTTACACAA
GCAGCAGGGCCGTGGCACCTAGGCAAGTTGGAACTGGATTTTGATTTGTGTGAGGGTACCACA
GTTGTTGTGGATGAACATTGTGGAAATCGAGGACCATCTCTTAGGACCACAACAGTCACAGGA
AAGATAATTCATGAATGGTGTTGCAGATCTTGCACGCTACCACCCTTACGTTTCAGAGGAGAA
GATGGGTGCTGGTACGGTATGGAAATCAGACCAGTCAAGGAAAAGGAAGAAAATCTAGTCAA
ATCAATGGTCTCTGCAGGGTCAGGGGAAGTGGACAGCTTTTCACTAGGACTGCTATGCATATC
AATAATGATCGAGGAGGTGATGAGATCCAGATGGAGTAGAAGAATGCTGATGACTGGAACAC
TGGCTGTGTTCTTCCTTCTCATAATGGGACAATTGACATGGAACGATCTGATCAGATTATGCAT
CATGGTTGGAGCCAACGCTTCCGACAGGATGGGGATGGGAACGACGTACCTAGCCCTGATGGC
CACTTTTAAAATGAGACCGATGTTCGCTGTAGGGCTATTATTTCGCAGACTAACATCCAGAGAA
GTTCTTCTTCTAACAATTGGATTGAGTCTAGTGGCATCTGTGGAGTTACCAAATTCCTTGGAGG

AGCTGGGGGATGGACTTGCAATGGGCATTATGATTTTAAAATTATTGACTGACTTTCAATCACA
TCAGCTGTGGGCTACCTTGCTGTCCTTGACATTTATCAAAACAACGTTTTCCTTGCACTACGCAT
GGAAGACAATGGCTATGGTACTGTCAATTGTATCTCTCTTCCCTTTATGCCTGTCCACGACCTC
CCAAAAAACAACATGGCTTCCGGTGCTATTGGGATCCCTTGGATGCAAACCACTAACCATGTTT
CTTATAGCAGAAAACAAAATCTGGGGAAGGAGAAGTTGGCCCCTCAATGAAGGAATCATGGC
TGTTGGAATAGTCAGCATCCTACTAAGTTCACTCCTCAAAAATGATGTACCGCTAGCTGGGCCA
CTAATAGCTGGAGGCATGCTAATAGCATGTTATGTTATATCTGGAAGCTCAGCCGACCTATCAT
TAGAGAAAGCGGCTGAGGTCTCCTGGGAAGAAGAAGCAGAACACTCTGGTGCCTCACACAAC
ATATTAGTGGAAGTCCAAGATGATGGAACCATGAAGATAAAGGATGAAGAGAGAGATGACAC
GCTAACCATTCTCCTTAAAGCAACTCTGTTAGCAGTTTCAGGGGTGTACCCATTATCAATACCA
GCGACCCTTTTCGTGTGGTACTTTTGGCAGAAAAAGAAACAGAGATCTGGAGTGTTATGGGAC
ACACCCAGCCCTCCAGAAGTGGAAAGAGCAGTTCTTGATGATGGTATCTATAGAATTATGCAG
AGAGGACTGTTGGGCAGGTCCCAAGTAGGGGTAGGAGTTTTCCAAGAAAACGTGTTCCACACA
ATGTGGCATGTCACCAGGGGAGCTGTACTCATGTATCAAGGGAAGAGATTGGAACCGAGCTGG
GCCAGTGTCAAAAAAGACCTGATCTCATATGGAGGAGGTTGGAGGCTTCAAGGATCCTGGAAC
ACAGGAGAAGAAGTGCAGGTGATTGCTGTTGAACCAGGGAAAAACCCCAAAAATGTACAAAC
AGCGCCGGGCACCTTTAAGACCCCTGAAGGTGAAGTTGGAGCCATTGCCCTAGACTTTAAACC
TGGCACATCTGGATCTCCCATCGTGAACAGAGAAGGAAAAATAGTAGGTCTTTATGGAAATGG
AGTAGTGACAACAAGTGGAACCTACGTCAGTGCCATAGCTCAAGCCAAAGCATCACAAGAAG
GGCCCCTACCAGAGATTGAAGACGAGGTGTTTAGGAAAAGAAACTTAACAATAATGGACCTA
CATCCAGGATCGGGGAAAACAAGAAGATATCTTCCAGCCATAGTCCGTGAGGCCATAAAAAG
GAAGCTGCGCACACTAATCCTGGCTCCCACAAGGGTTGTCGCTTCCGAAATGGCAGAGGCGCT
CAAGGGAATGCCAATAAGGTACCAAACAACAGCAGTGAAGAGTGAACATACAGGAAAAGAG
ATAGTTGACCTCATGTGTCACGCCACTTTCACCATGCGCCTCCTGTCTCCCGTAAGAGTTCCCA
ATTACAACATGATCATCATGGATGAAGCACATTTCACCGATCCATCCAGTATAGCGGCCAGAG
GGTACATCTCAACCCGAGTGGGCATGGGTGAAGCAGCTGCAATCTTCATGACAGCCACTCCCC
CAGGATCAGTGGAGGCCTTTCCACAGAGCAACGCAGTTATCCAAGATGAGGAAAGAGACATT
CCTGAGAGATCATGGAACTCAGGCTATGAGTGGATCACTGACTTCCCAGGTAAAACAGTTTGG
TTTGTTCCAAGCATTAAATCAGGAAATGACATTGCCAACTGCTTAAGAAAGAATGGGAAACGG
GTGATTCAATTGAGCAGGAAAACCTTTGATACAGAGTACCAAAAAACAAAAAACAACGACTG
GGACTACGTCGTCACAACAGACATCTCCGAAATGGGAGCAAATTTCCGAGCCGACAGGGTGAT
AGACCCAAGACGGTGTCTGAAACCGGTAATACTAAAAGATGGTCCAGAGCGTGTCATTTTAGC
AGGACCAATGCCAGTGACTGTGGCCAGTGCCGCTCAGAGGAGAGGAAGAATTGGAAGGAACC
ACAATAAGGAAGGTGATCAGTACATCTACATGGGACAGCCTTTAAACAACGATGAAGATCAC
GCTCACTGGACAGAAGCAAAAATGCTCCTTGATAATATAAACACACCAGAAGGGATTATCCCA
GCCCTCTTTGAGCCAGAGAGAGAAAAGAGTGCAGCAATAGACGGGGAATACAGACTGCGGGG
TGAAGCAAGGAAAACGTTTGTGGAGCTCATGAGAAGAGGAGATCTACCTGTCTGGCTATCCTA

CAAAGTTGCCTCAGAAGGCTTCCAGTACTCTGACAGAAGATGGTGCTTTGACGGGGAAAGGAA
CAACCAGGTGTTGGAGGAGAACATGGACGTGGAGATCTGGACAAAAGAAGGAGAAAGAAAG
AAACTACGACCCCGCTGGCTGGATGCCAGAACATACTCAGACCCACTAGCCCTGCGCGAGTTT
AAAGAGTTTGCAGCAGGGAGAAGAAGCGTCTCAGGTGATTTAATATTAGAAATAGGGAAGCT
TCCACAACACTTGACGCAAAGGGCCCAGAATGCCCTGGACAACCTGGTTATGTTGCACAACTC
CGAACAAGGAGGCAGAGCCTATAGACATGCAATGGAAGAACTGCCAGACACCATAGAAACGT
TGATGCTCCTAGCTTTGATAGCTGTGTTAACTGGTGGAGTGACACTGTTCTTCCTATCAGGAAG
GGGCCTAGGGAAAACATCTATCGGCCTACTCTGCGTAATGGCTTCAAGCGTACTGCTATGGAT
GGCCAGTGTGGAGCCCCATTGGATAGCGGCCTCCATCATACTGGAGTTCTTCCTGATGGTGCTG
CTTATTCCAGAGCCAGACAGACAACGCACTCCGCAGGACAACCAGCTGGCATATGTGGTGATA
GGTTTGTTATTCATGATACTGACAGTAGCAGCCAATGAGATGGGACTGCTGGAAACCACAAAG
AAAGACTTAGGGATTGGCCATGTGGCTGTTGAAAATCACCACCATGCCGCAATGCTGGACGTA
GACTTACATCCAGCTTCAGCCTGGACCCTCTATGCAGTGGCCACAACAATTATCACTCCCATGA
TGAGGCACACAATCGAAAACACAACGGCAAACATTTCCCTGACAGCCATTGCAAACCAGGCA
GCTATATTGATGGGACTTGACAAAGGATGGCCAATATCGAAGATGGACATAGGAGTTCCACTT
CTCGCCTTGGGGTGCTATTCCCAGGTGAATCCACTGACGCTGACAGCGGCGGTATTGATGCTAG
TGGCTCATTACGCCATAATTGGACCTGGACTGCAAGCAAAAGCTACTAGAGAAGCTCAAAAA
GGACAGCGGCCGGAATAATGAAAAATCCAACCGTTGATGGAATTGTTGCAATAGATTTGGACC
CTGTGGTTTATGATGCAAAATTTGAAAAACAACTAGGCCAAATAATGTTGTTGATACTATGCAC
ATCACAGATCCTCTTGATGCGGACTACATGGGCCTTGTGCGAGTCCATCACACTGGCCACTGG
ACCTCTGACCACGCTTTGGGAGGGATCTCCAGGAAAATTTTGGAACACCACGATAGCGGTTTC
CATGGCAAACATTTTCAGAGGAAGTTATCTAGCAGGAGCAGGTCTGGCCTTCTCATTAATGAA
ATCTCTAGGAGGAGGTAGGAGAGGCACGGGAGCCCAAGGGGAAACACTGGGAGAGAAATGG
AAAAGACAGCTGAACCAACTGAGCAAGTCAGAATTTAACACCTATAAAAGGAGTGGGATTAT
GGAAGTGGACAGATCCGAAGCCAAAGAGGGATTGAAAAGAGGAGAAACAACCAAACATGCA
GTGTCGAGAGGAACCGCTAAACTGAGGTGGTTCGTGGAGAGGAACCTTGTGAAACCAGAAGG
GAAAGTCATAGACCTCGGTTGTGGAAGAGGTGGCTGGTCATACTATTGCGCTGGGCTGAAGAA
AGTCACAGAAGTGAAGGGGTATACAAAAGGAGGACCTGGACATGAGGAACCAATCCCAATGG
CGACCTATGGATGGAACCTAGTAAAGCTGCACTCTGGGAAAGACGTATTTTTATACCACCTG
AGAAATGTGACACCCTTTTGTGTGATATTGGTGAGTCCTCTCCAAACCCAACTATAGAGGAAG
GAAGAACGCTACGCGTCCTAAAGATGGTGGAACCATGGCTCAGAGGAAACCAATTTTGCATAA
AAATTCTGAATCCCTACATGCCAAGTGTGGTGGAAACTCTGGAGCAAATGCAAAGAAAACATG
GAGGAATGCTAGTGCGAAATCCACTTTCAAGAAATTCTACTCATGAAATGTATTGGGTTTCATG
TGGAACAGGAAACATTGTGTCAGCAGTAAACATGACATCTAGAATGTTGCTAAATCGATTCAC
AATGGCTCACAGGAAACCAACATATGAAAGAGACGTGGACCTAGGCGCCGGAACAAGACATG
TGGCAGTGGAACCAGAGGTAGCCAACCTAGATATCATTGGCCAGAGGATAGAGAACATAAAA
CATGAACATAAGTCAACATGGCATTATGATGAGGACAATCCATATAAAACATGGGCCTATCAT

GGATCATATGAGGTCAAGCCATCAGGATCAGCCTCATCCATGGTCAATGGCGTGGTGAAACTG
CTCACCAAACCATGGGATGTCATCCCTATGGTCACACAAATAGCCATGACTGACACTACACCC
TTTGGACAACAGAGGGTGTTTAAAGAGAAAGTTGACACACGCACACCAAAAGCAAAACGGGG
CACAGCACAAATCATGGAGGTGACAGCCAAGTGGTTATGGGGTTTTCTTTCTAGAAACAAGAA
ACCAAGAATTTGCACAAGAGAGGAGTTCACAAGAAAAGTTAGGTCAAACGCAGCCATTGGAG
CAGTGTTCGTTGATGAAAATCAATGGAACTCAGCAAAAGAAGCAGTGGAAGATGAGCGGTTCT
GGGACCTTGTGCATAGAGAGAGGGAGCTTCACAAACAGGGAAAATGTGCTACGTGTGTTTACA
ACATGATGGGGAAGAGAGAGAAAAAGCTAGGAGAGTTCGGAAAGGCAAAAGGAAGTCGTGC
AATATGGTACATGTGGTTGGGAGCACGCTTTCTAGAGTTCGAAGCTCTTGGTTTCATGAACGAA
GATCACTGGTTCAGCAGAGAGAATTCACTCAGCGGAGTGGAAGGAGAAGGACTCCACAAACT
TGGATATATACTCAGAGACATATCAAAGATTCCAGGGGGAAACATGTATGCAGATGACACAGC
CGGATGGGATACAAGGATAACAGAGGATGACCTTCAGAATGAGGCCAGAATTACTGACATCA
TGGAACCCGAACATGCCCTCCTGGCTAAGTCAATCTTCAAGTTAACCTACCAAAATAAGGTGG
TAAGGGTACAGAGACCAGCAAAAAATGGAACCGTGATGGATGTCATATCCAGACGTGACCAG
AGAGGAAGTGGTCAGGTCGGAACTTATGGCTTAAACACTTTCACCAACATGGAAGCCCAGCTG
ATAAGACAAATGGAGTCTGAGGGAATCTTTTCACCCAGCGAATTAGAGACCCCAAATTTAGCC
GAGAGAGTTCTCGACTGGCTGGAAAAATATGGCGTCGAAAGGCTGAAAAGAATGGCAATCAG
CGGAGATGACTGCGTAGTGAAACCAATTGATGATAGGTTTGCAACAGCCTTGACAGCTCTGAA
TGATATGGGAAAAGTAAGAAAAGATATACCACAATGGGAACCTTCAAAAGGATGGAATGATT
GGCAACAAGTGCCTTTTTGTTCACACCACTTCCACCAGCTGATTATGAAGGATGGGAGGGAAA
TAGTGGTGCCATGCCGCAACCAAGATGAACTTGTGGGTAGGGCTAGAGTATCACAAGGTGCTG
GATGGAGCCTGAGAGAAACTGCATGCCTAGGCAAGTCATATGCACAAATGTGGCAGCTGATGT
ACTTCCACAGGAGAGACCTGAGACTAGCCGCTAATGCTATCTGTTCAGCCGTTCCAGTTGATTG
GATCCCAACCAGCCGTACCACCTGGTCGATCCATGCCCATCACCAATGGATGACAACAGAAGA
CATGCTGTCAGTGTGGAATAGGGTTTGGATAGAGGAAAACCCATGGATGGAGGACAAAACCC
ACATATCCAGTTGGGGAGATGTTCCATATTTAGGAAAAAGGGAAGACCAATGGTGTGGATCCC
TGATAGGCTTAACAGCAAGGGCCACCTGGGCCACCAACATACAAGTGGCCATAAACCAAGTG
AGAAGACTAATTGGGAATGAGAATTATCTAGATTACATGACATCAATGAAGAGATTCAAGAAC
GAGAGTGATCCCGAAGGGGCACTCTGGTGAGTCAACACATTTACAAAATAAAGGAAAATAAG
AAATCAAACAAGGCAAGAAGTCAGGCCGGATTAAGCCATAGTACGGTAAGAGCTATGCTGCC
TGTGAGCCCCGTCTAAGGACGTAAAATGAAGTCAGGCCGAAAGCCACGGCTTGAGCAAACCG
TGCTGCCTGTAGCTCCATCGTGGGGATGTAAAAACCTGGGAGGCTGCAACCCATGGAAGCTGT
ACGCATGGGGTAGCAGACTAGTGGTTAGAGGAGACCCCTCCCGAAACATAACGCAGCAGCGG
GGCCCAACACCAGGGGAAGCTGTACCCTGGTGGTAAGGACTAGAGGTTAGAGGAGACCCCCC
GCATAACAATAAACAGCATATTGACGCTGGGAGAGACCAGAGATCCTGCTGTCTCTACAGCAT
CATTCCAGGCACAGAACGCCAGAAAATGGAATGGTGCTGTTGAATCAACAGGTTCT

SEQ ID NO:3 - DENV-2-NI-BID-V533-2005

AGTTGTTAGTCTACGTGGACCGACAAAGACAGATTCTTTGAGGGAGCTAAGCTCAACGTAGTT
CTAACAGTTTTTTAATTAGAGAGCAGATCTCTGATGAATAACCAACGAAAAAAGGCGAGAAGT
ACGCCTTTCAATATGCTGAAACGCGAGAGAAACCGCGTGTCAACTGTGCAACAGCTGACAAAG
AGATTCTCACTTGGAATGCTGCAAGGACGCGGACCATTAAAACTGTTCATGGCCCTTGTGGCGT
TCCTTCGTTTCCTAACAATCCCACCAACAGCAGGGATACTAAAAAGATGGGGAACGATCAAAA
AATCAAAAGCTATCAATGTTTTGAGAGGGTTCAGGAAAGAGATTGGAAGGATGCTGAACATCT
TGAACAAGAGACGCAGGACAGCAGGCGTGATTGTTATGTTGATTCCAACAGCGATGGCGTTCC
ATTTAACCACACGCAATGGAGAACCACACATGATCGTTGGTAGGCAGGAGAAAGGAAAAGT
CTTCTGTTCAAAACAGAGGATGGTGTTAACATGTGTACTCTCATGGCCATAGACCTTGGTGAAT
TGTGTGAAGATACAATCACGTACAAGTGTCCTCTCCTCAGACAAAATGAACCAGAAGACATAG
ATTGTTGGTGCAACTCTACGTCCACATGGGTAACTTATGGGACATGTACCACCACAGGAGAAC
ACAGAAGAGAAAAAAGATCAGTGGCGCTCGTTCCACATGTAGGTATGGGACTGGAGACACGA
ACTGAAACATGGATGTCATCAGAAGGGGCCTGGAAACATGTTCAGAGAATTGAAACCTGGATC
TTGAGACATCCAGGTTTTACCATAATGGCAGCAATCCTGGCATACACCATAGGAACGACACAT
TTCCAAAGGGCCTTGATTTTCATTTTACTGACAGCTGTCGCTCCTTCAATGACAATGCGCTGCA
TAGGAATATCAAATAGAGACTTCGTAGAAGGGGTTTCAGGAGGAAGCTGGGTTGACATAGTCT
TAGAACATGGAAGTTGTGTGACGACGATGGCAAAAAACAAACCAACATTGGATTTTGAACTGA
TAAAAACAGAAGCCAAACAACCTGCCACTCTAAGGAAGTACTGTATAGAAGCAAAGCTGACC
AACACAACAACAGAATCGCGTTGCCCAACACAAGGGGAACCCAGTCTAAATGAGGAGCAGGA
CAAAAGGTTCATCTGCAAACACTCCATGGTAGACAGAGGATGGGGAAATGGATGTGGATTATT
TGGAAAGGGAGGCATTGTGACCTGTGCTATGTTTACATGCAAAAAGAACATGGAAGGAAAAG
TCGTGCAGCCAGAAAATTTGGAATACACCATCGTGATAACACCTCACTCAGGAGAGGAGCACG
CTGTAGGTAATGACACAGGAAAGCATGGCAAGGAAATCAAAATAACACCACAGAGCTCCATC
ACAGAAGCAGAACTGACAGGCTATGGCACTGTCACGATGGAGTGCTCTCCGAGAACGGGCCTC
GACTTCAATGAGATGGTACTGCTGCAGATGGAAGACAAAGCTTGGCTGGTGCACAGGCAATGG
TTCCTAGACCTGCCGTTACCATGGCTACCCGGAGCGGACACACAAGGATCAAATTGGATACAG
AAAGAGACATTGGTCACTTTCAAAAATCCCCACGCGAAGAAACAGGATGTCGTTGTCTTAGGG
TCTCAAGAAGGGGCCATGCACACGGCACTCACAGGGGCCACAGAAATCCAGATGTCATCAGG
AAACTTACTGTTCACAGGACATCTCAAGTGCAGGCTGAGAATGGACAAACTACAGCTCAAAGG
AATGTCATACTCTATGTGTACAGGAAAGTTTAAAATTGTGAAGGAAATAGCAGAAACACAACA
TGGAACAATAGTTATCAGAGTACAATATGAAGGGGACGGTTCTCCATGCAAGATCCCTTTTGA
GATAACAGATTTGGAAAAAAGACACGTCTTAGGTCGCTTGATTACAGTTAACCCAATCGTAAC
AGAAAAAGATAGCCCAGTCAACATAGAAGCAGAACCTCCATTCGGAGACAGCTACATCATTAT
AGGAGTAGAGCCGGGACAATTGAAACTCAATTGGTTTAAGAAGGGAAGTTCCATCGGCCAAA
TGTTTGAGACAACAATGAGAGGAGCAAAGAGAATGGCCATTTTAGGTGACACAGCCTGGGAC
TTTGGATCCCTGGGAGGAGTGTTTACATCTATAGGAAAGGCTCTCCACCAAGTTTTCGGAGCAA

TCTATGGGGCTGCTTTTAGTGGGGTCTCATGGACTATGAAAATCCTCATAGGAGTCATCATCAC
ATGGATAGGAATGAATTCACGTAGCACCTCACTGTCTGTGTCGCTAGTATTGGTGGGCGTTGTG
ACACTGTACCTGGGAGCTATGGTGCAAGCTGATAGTGGTTGCGTTGTGAGCTGGAAAAATAAA
GAACTGAAATGTGGCAGCGGGATCTTCATCACAGATAACGTACACACATGGACAGAACAATAT
AAGTTCCAACCAGAATCCCCTTCAAAACTAGCTTCAGCTATCCAAAAAGCTCATGAAGAGGGC
ATTTGTGGAATCCGCTCAGTAACAAGATTGGAGAATCTGATGTGGAAACAAATAACACCAGAA
TTGAATCATATTCTATCAGAAAATGAGGTAAAGTTGACCATTATGACAGGAGACATTAGAGGA
ATCATGCAGGCAGGAAAACGATCCTTGCGGCCCCAGCCCACTGAGCTGAAGTACTCATGGAAA
ACATGGGGAAAGGCGAAAATGCTCTCCACAGAGTCTCACAATCAGACCTTTCTTATTGATGGC
CCTGAAACAGCAGAATGCCCCAACACAAACAGAGCCTGGAACTCGCTGGAAGTTGAAGACTA
TGGTTTTGGAGTTTTCACCACCAATATATGGCTGAAATTGAGAGAAAAACAGGATGTATTTTGT
GACTCAAAACTCATGTCAGCGGCCATTAAAGACAACAGAGCCGTTCATGCTGATATGGGTTAT
TGGATAGAAAGTGCACTCAATGACACATGGAAGATGGAGAAAGCCTCCTTCATTGAAGTTAAA
AGCTGCCACTGGCCAAAGTCACACACCCTTTGGAGCAATGGAGTATTAGAAAGTGAGATGATA
ATCCCAAAAAATTTTGCCGGGCCAGTGTCACAACACAACTACAGACCAGGCTACCATACACAA
ACAGCAGGACCTTGGCATCTAGGTAAGCTTGAGATGGACTTTGATCTCTGCGAAGGAACTACA
GTGGTGGTGACTGAGGACTGTGGAAATAGAGGACCCTCTTTAAGAACGACCACTGCCTCTGGA
AAACTCATAACAGAATGGTGCTGCCGATCCTGCACACTACCACCTCTAAGATACAGAGGTGAG
GATGGATGCTGGTACGGGATGGAAATCAGACCATTGAAAGAGAAAGAAGAGAATTTGGTCAA
CTCCTTGGTCACAGCCGGACATGGGCAGATTGACAACTTTTCACTAGGAGTCTTGGGAATGGC
ACTGTTCCTGGAAGAAATGCTCAGGACCCGAATAGGAACGAAACATGCAATACTGCTAGTTGC
AGTATCTTTTGTGACATTGATTACTGGGAACATGTCTTTTAGAGACCTGGGAAGAGTGATGGTT
ATGGTGGGCGCTACCATGACGGATGACATAGGTATGGGAGTGACTTATCTTGCCCTACTAGCA
GCTTTTAAGGTTAGACCAACTTTTGCAGCTGGACTACTCTTAAGAAAACTGACCTCCAAGGAAT
TGATGATGGCCACCATAGGAATCGCACTCCTTTCCCAAAGCACCATACCAGAGACCATTCTTG
AACTGACTGATGCATTAGCCCTGGGCATGATGGTCCTCAAAATAGTGAGAAATATGGAAAAAT
ACCAATTGGCAGTGACTATCATGGCTATTTCATGTGTCCCAAATGCAGTGATACTGCAAAACGC
ATGGAAGGTGAGTTGCACAATATTGGCAGCGGTGTCCGTTTCACCACTGCTCTTAACATCCTCA
CAGCAGAAAGCGGATTGGATACCACTGGCATTGACGATAAAAGGTCTCAATCCAACAGCCATT
TTTTTAACAACTCTCTCGAGGACCAGCAAGAAAAGGAGCTGGCCGCTAAATGAAGCTATCATG
GCAGTTGGGATGGTGAGCATTTTAGCCAGTTCTCTCCTAAAGAATGATATTCCTATGACAGGTC
CATTAGTGGCTGGAGGACTCCTCACCGTATGTTACGTGCTCACTGGACGATCGGCCGATTTGGA
ACTGGAGAGAGCTGCCGATGTAAAATGGGAAGATCAGGCAGAAATATCAGGAAGCAGCCCAA
TCCTGTCAATAACAATATCAGAAGATGGCAGCATGTCGATAAAAAATGAAGAGGAAGAACAA
ACACTGACCATACTCATCAGAACGGGATTGTTGGTGATCTCAGGAGTCTTTCCAGTATCGATAC
CAATTACGGCAGCAGCATGGTACCTGTGGGAAGTAAAGAAACAACGGGCTGGAGTACTGTGG
GACGTCCCTTCACCCCCACCAGTGGAAAAAGCCGAACTGGAGGATGGAGCCTACAGAATCAA

GCAAAGAGGGATCCTTGGATATTCTCAGATTGGAGCCGGAGTTTACAAAGAAGGAACATTCCA
TACAATGTGGCACGTCACACGTGGTGCTGTTCTGATGCATAGAGGGAAGAGGATTGAACCATC
ATGGGCAGATGTCAAGAAAGACCTAATATCATATGGAGGAGGCTGGAAGCTAGAAGGAGAAT
GGAAGGAAGGAGAGGAAGTCCAAGTCCTGGCATTGGAACCTGGAAAAAATCCAAGAGCCGTC
CAAACGAAACCTGGAATATTCAAAACCAACACCGGAACCATAGGCGCCGTATCTCTGGACTTT
TCCCCTGGAACGTCAGGATCTCCAATCGTCGACAGAAAAGGAAAAGTTGTGGGTCTTTACGGT
AATGGTGTTGTCACAAGGAGTGGAGCATATGTAAGTGCTATAGCCCAGACCGAAAAAAGCATT
GAAGACAATCCAGAGATCGAAGATGACATTTTCCGAAAGAAAAGATTGACCATCATGGACCTC
CATCCAGGAGCAGGAAAGACAAAAAGATACCTTCCAGCCATAGTTAGAGAAGCCATAAAACG
TGGCTTGAGAACATTGATCCTGGCTCCCACTAGAGTAGTGGCAGCTGAAATGGAGGAAGCTCT
TAGAGGACTTCCAATAAGATACCAAACTACAGCCATCAAAACCGAGCATACCGGGCGGGAGA
TCGTGGACCTAATGTGTCATGCCACATTTACTATGAGGCTGTTATCACCAGTCAGAGTGCCAAA
TTACAACCTGATCATCATGGACGAAGCCCACTTCACAGACCCAGCAAGTATAGCAGCTAGAGG
ATACATTTCAACTCGAGTAGAGATGGGTGAAGCAGCCGGGATTTTCATGACAGCCACTCCTCC
GGGAAGTAGAGACCCATTTCCTCAGAGCAATGCACCAATTATGGATGAGGAAAGAGAAATCC
CTGAGCGTTCATGGAATTCAGGACACGAATGGGTCACGGATTTTAAGGGGAAGACTGTTTGGT
TTGTTCCAAGTATAAAAGCAGGAAATGATATAGCAGCTTGTCTTAGGAAAAATGGAAAGAAA
GTGATACAACTCAGTAGGAAGACTTTTGACTCTGAGTATGTTAAGACTAGAGCCAATGATTGG
GACTTTGTGGTCACAACTGACATTTCAGAAATGGGTGCCAACTTCAAGGCTGAGAGGGTTATA
GACCCCAGACGTTGCATGAAACCAGTTATACTAACAGATGGCGAGGAGCGGGTGATCTTGGCT
GGACCTATGCCAGTGACCCACTCTAGTGCAGCGCAAAGAAGAGGGAGAATAGGAAGAAATCC
AAAAAATGAAAATGACCAGTACATATACATGGGGGAACCTCTTGAAAATGATGAAGACTGTG
CACATTGGAAAGAAGCTAAAATGCTCCTAGATAACATCAACACACCTGAAGGAATCATTCCTA
GTATGTTCGAACCAGAGCGTGAAAAAGTGGATGCCATTGATGGTGAATACCGTTTGAGAGGAG
AAGCAAGGAAAACCTTTGTGGACCTAATGAGAAGAGGGGACTTACCAGTCTGGTTGGCCTACA
AAGTGGCAGCTGAAGGCATCAACTACGCAGACAGAAAGTGGTGTTTTGATGGAATTAAGAAC
AACCAAATACTGGAAGAAAATATGGAAGTGGAAATCTGGACAAAAGAAGGGGAAAGGAAAA
AATTAAAACCCAGATGGTTGGATGCTAGGATCTATTCTGACCCACTAGCACTAAAAGAATTCA
AGGAATTTGCAGCTGGAAGAAAATCTTTGACCCTGAACCTAATCACAGAAATGGGTAGGCTTC
CAACTTTCATGACTCAGAAAGCAAGAAACGCACTGGACAACCTGGCTGTGCTGCATACGGCTG
AGGCAGGTGGAAGGGCGTACAATCATGCTCTCAGTGAACTGCCGGAGACCCTGGAGACACTG
CTCCTACTGACACTTCTGGCAACAGTCACAGGAGGAATCTTCTTATTCTTAATGAGCGGAAAAG
GTATAGGGAAGATGACCCTGGGAATGTGTTGCATAATCACGGCTAGTATCCTCCTATGGTATG
CACAGATACAACCACACTGGATAGCAGCTTCAATAATACTGGAGTTTTTCTCATAGTTTTGCT
CATTCCAGAACCAGAAAAACAGAGAACACCCCAAGACAACCAATTGACCTACGTTGTCATAGC
CATCCTCACAGTGGTGGCCGCAACCATGGCAAACGAGATGGGTTTCCTGGAAAAAACCAAGA
AAGACCTCGGATTGGGAAGCATTACAACCCAGGAATCTGAGAGCAATATCCTGGACATAGATC

TACGCCCTGCATCAGCATGGACGCTGTATGCCGTAGCTACAACATTTGTCACACCAATGTTGAG ACATAGCATTGAAAATTCCTCAGTGAATGTCTCCCTAACAGCCATTGCTAACCAAGCTACAGTG CTAATGGGTCTTGGGAAAGGATGGCCATTGTCAAAGATGGACATTGGAGTTCCCCTCCTTGCC ATTGGATGCTATTCACAAGTCAACCCTATAACTCTCACAGCAGCTCTCCTTTTATTGGTAGCAC ATTATGCCATTATAGGGCCAGGACTTCAAGCAAAAGCAACCAGAGAAGCCCAGAAAAGAGCA GCAGCAGGCATCATGAAAAACCCAACAGTCGATGGAATAACAGTGATTGACCTAGAACCAAT ACCCTATGATCCAAAATTTGAAAAGCAGTTAGGACAAGTAATGCTCCTAATCCTCTGCGTGACT CAAGTATTAATGATGAGGACTACATGGGCTTTATGTGAGGCTCTAACCCTAGCGACCGGGCCC ATCTCCACACTGTGGGAAGGAAATCCAGGGAGGTTTTGGAACACTACCATTGCAGTGTCAATG GCTAACATCTTTAGGGGGAGCTACTTGGCCGGAGCTGGACTTCTCTTTTCCATCATGAAAAACA CAACAAACACAAGAAGAGGAACTGGCAACATAGGAGAGACACTTGGAGAAAAATGGAAAAG TCGATTAAACGCACTGGGGAAAAGTGAATTTCAAATCTACAAGAAAAGTGGAATCCAGGAAG TGGATAGAACCCTAGCAAAAGAAGGTATCAAAAGAGGAGAAACGGACCACCATGCTGTGTCG CGAGGTTCAGCAAAACTGAGATGGTTCGTCGAGAGAAATATGGTCACACCGGAAGGGAAGGT GGTGGATCTCGGTTGCGGCAGAGGGGGCTGGTCATACTATTGCGGGGGACTAAAGAATGTAAG AGAAGTCAAAGGCCTAACAAAAGGAGGACCAGGACATGAAGAACCCATCCCCATGTCAACAT ATGGGTGGAATCTAGTGCGTCTGCAAAGTGGAGTTGACGTTTTCTTCACCCCGCCAGAAAAGT GTGATACATTGTTGTGTGACATAGGGGAGTCGTCACCAAATCCCACGATAGAAGCAGGACGAA CACTCAGAGTCCTCAACTTAGTGGAAAATTGGTTGAACAATAACACCCAATTTTGCATAAAGG TCCTCAACCCATATATGCCTTCAGTCATAGAAAAAATGGAAGCATTACAAAGGAAATATGGAG GAGCCTTAGTGAGGAATCCACTCTCACGAAACTCCACGCATGAAATGTACTGGGTATCTAATG CCACCGGGAACATAGTGTCATCAGTGAACATGATTTCAAGGATGTTGATTAACAGATTCACAA TGAAACACAAGAAAGCCACTTACGAGCCAGATGTTGACCTAGGAAGTGGAACCCGCAACATT GGAATTGAAAGTGAGATACCAAATCTAGACATAATAGGAAAGAGAATAGAGAAAATAAAACA AGAGCATGAAACATCATGGCACTATGATCAAGACCACCCATACAAAACGTGGGCTTACCATGG CAGCTATGAAACAAAACAAACTGGATCAGCATCATCTATGGTGAACGGAGTGGTCAGATTGCT GACAAAACCTTGGGACGTCGTCCCTATGGTGACACAGATGGCAATGACAGACACGACTCCTTT TGGACAACAGCGCGTTTTCAAAGAGAAAGTAGACACGAGAACCCAAGAACCGAAGGAAGGCA CAAAGAAACTGATGAAAATTACGGCAGAGTGGCTTTGGAAAGAACTAGGAAAGAAAAAGACA CCTAGGATGTGTACCAGAGAAGAATTCACAAGAAAAGTGAGAAGCAATGCAGCCTTGGGGGC CATATTCACTGATGAGAACAAATGGAAATCGGCACGTGAGGCTGTTGAAGATGGTAGGTTCTG GGAGCTGGTTGACAGGGAAAGAAATCTCCATCTTGAAGGAAAGTGTGAAACATGTGTGTACA ACATGATGGGAAAAAGAGAGAAGAAACTAGGGGAGTTCGGCAAGGCAAAAGGTAGCAGAGC CATATGGTACATGTGGCTTGGAGCACGCTTCTTAGAGTTTGAAGCCCTAGGATTCCTGAATGAA GATCACTGGTTCTCCAGAGGGAACTCCCTGAGTGGAGTGGAAGGAGAAGGGCTGCACAGGCT AGGCTACATTTTAAGAGACGTGGGCAAGAAGGAAGGGGGGGCAATGTACGCCGATGATACAG CAGGATGGGACACAAGAATCACACTAGAAGACTTAAAAAATGAAGAAATGGTAACGAACCAC

ATGAAAGGAGAACACAAGAAACTAGCCGAGGCCATATTCAAACTAACGTACCAAAACAAGGT
GGTGCGTGTGCAAAGACCAACACCAAGAGGTACAGTAATGGATATCATATCGAGAAGAGACC
AAAGAGGCAGTGGGCAAGTCGGCACCTATGGCCTTAATACCTTCACCAATATGGAAGCCCAAT
TAATTAGACAGATGGAGGGAGAAGGAATCTTCAAAAGCATTCAGCACCTGACAGCCACAGAA
GAAATCGCTGTACAGAACTGGCTAGCAAGAGTGGGGCGTGAAAGGCTATCAAGAATGGCAAT
CAGTGGAGATGATTGTGTTGTAAAACCTATAGATGACAGATTTGCAAGTGCTTTAACAGCTCTA
AATGACATGGGAAAAGTTAGAAAAGATATACAACAATGGGAACCTTCAAGAGGATGGAACGA
TTGGACACAAGTGCCTTTCTGTTCACACCACTTTCATGAGTTAGTCATGAAAGATGGTCGCGTG
CTCGTAGTCCCATGCAGAAACCAAGATGAACTGATTGGTAGAGCCCGAATTTCCCAGGGAGCT
GGGTGGTCTTTGAAAGAGACGGCCTGTTTGGGAAAGTCTTACGCCCAAATGTGGACTCTGATG
TACTTCCACAGACGTGACCTCAGACTGGCGGCAAATGCCATCTGCTCGGCAGTCCCGTCACATT
GGGTTCCAACAAGTCGAACAACCTGGTCCATACACGCTAAGCATGAATGGATGACGACGGAA
GACATGCTGGCAGTCTGGAACAGGGTGTGGATCCAAGAAAACCCGTGGATGGAAGATAAAAC
TCCAGTGGAATCATGGGAAGAAGTCCCATACTTGGGAAAAAGAGAAGACCAATGGTGCGGCT
CATTGATTGGGCTAACAAGCAGGGCTACCTGGGCAAAGAACATCCAAACAGCAATAAATCAA
GTCAGATCCCTTATAGGCAATGAGGAATACACAGACTACATGCCATCCATGAAGAGATTTAGA
AGGGAAGAGGAAGAGGCAGGTGTCCTGTGGTAGAAGGCAAAACTAACATGAAACAAGGCTAA
AAGTCAGGTCGGATTAAGCCATAGTACGGAAAAAACTATGCTACCTGTGAGCCCCGTCCAAGG
ACGTTAAAAGAAGTCAGGCCATCACAAAATGCCACAGCTTGAGTAAACTGTGCAGCCTGTAGC
TCCACCTGAGGAGGTGTAAAAAACCTGGGAGGCCACAAACCATGGAAGCTGTACGCATGGCG
TAGTGGACTAGCGGTTAGAGGAGACCCCTCCCTTACAAATCGCAGCAACAACGGGGGCCCA
AGGTGAGATGAAGCTGTAATCTCACTGGAAGGACTAGAGGTTAGAGGAGACCCCCCCAAAAC
AAAAAACAGCATATTGACGCTGGGAAAGACCAGAGATCCTGCTGTCTCCTCAGCATCATTCCA
GGCACAGAACGCCAGAAAATGGAATGGTGCTGTTGAATCAACAGGTTCT

SEQ ID NO: 4 - DENV-2-NI-BID-V533-2005-E-Min

AGTTGTTAGTCTACGTGGACCGACAAAGACAGATTCTTTGAGGGAGCTAAGCTCAACGTAGTT
CTAACAGTTTTTTAATTAGAGAGCAGATCTCTGATGAATAACCAACGAAAAAAGGCGAGAAGT
ACGCCTTTCAATATGCTGAAACGCGAGAGAAACCGCGTGTCAACTGTGCAACAGCTGACAAAG
AGATTCTCACTTGGAATGCTGCAAGGACGCGGACCATTAAAACTGTTCATGGCCCTTGTGGCGT
TCCTTCGTTTCCTAACAATCCCACCAACAGCAGGGATACTAAAAAGATGGGGAACGATCAAAA
AATCAAAAGCTATCAATGTTTTGAGAGGGTTCAGGAAAGAGATTGGAAGGATGCTGAACATCT
TGAACAAGAGACGCAGGACAGCAGGCGTGATTGTTATGTTGATTCCAACAGCGATGGCGTTCC
ATTTAACCACACGCAATGGAGAACCACACATGATCGTTGGTAGGCAGGAGAAAGGGAAAAGT
CTTCTGTTCAAAACAGAGGATGGTGTTAACATGTGTACTCTCATGGCCATAGACCTTGGTGAAT
TGTGTGAAGATACAATCACGTACAAGTGTCCTCTCCTCAGACAAAATGAACCAGAAGACATAG
ATTGTTGGTGCAACTCTACGTCCACATGGGTAACTTATGGGACATGTACCACCACAGGAGAAC

ACAGAAGAGAAAAAAGATCAGTGGCGCTCGTTCCACATGTAGGTATGGGACTGGAGACACGA
ACTGAAACATGGATGTCATCAGAAGGGGCCTGGAAACATGTTCAGAGAATTGAAACCTGGATC
TTGAGACATCCAGGTTTTACCATAATGGCAGCAATCCTGGCATACACCATAGGAACGACACAT
TTCCAAAGGGCCTTGATTTTCATTTTACTGACAGCTGTCGCTCCTTCAATGACAATGAGATGCA
TAGGGATATCGAATCGCGATTTCGTCGAAGGCGTATCCGGAGGGTCATGGGTCGACATCGTAC
TCGAACACGGATCATGCGTTACGACTATGGCTAAGAATAAGCCTACACTAGACTTCGAACTGA
TTAAGACAGAGGCTAAGCAACCGGCAACATTGCGTAAGTACTGTATCGAAGCGAAACTGACTA
ACACTACAACCGAATCTAGATGCCCTACACAGGGCGAACCTAGTCTGAACGAAGAGCAAGAC
AAAAGGTTTATATGCAAACACTCTATGGTCGATCGCGGATGGGGAAACGGATGCGGATTGTTC
GGTAAGGGGGGAATCGTTACATGCGCTATGTTTACATGTAAAAAAAATATGGAGGGAAAGGT
CGTGCAACCAGAGAATCTCGAATATACAATCGTAATCACACCGCATAGCGGAGAGGAACACG
CAGTCGGAAACGATACCGGAAAACACGGAAAAGAGATTAAGATTACACCACAGAGCTCCATA
ACCGAAGCCGAACTGACAGGGTACGGAACAGTGACAATGGAATGCTCACCTAGAACAGGCCT
AGACTTTAACGAAATGGTGTTACTGCAAATGGAAGACAAAGCATGGTTAGTGCATAGGCAATG
GTTTTTAGACCTACCACTACCATGGTTGCCCGGAGCCGATACACAGGGATCGAACTGGATACA
GAAAGAGACACTCGTTACGTTTAAAAACCCACACGCTAAAAAACAGGACGTAGTCGTACTCGG
ATCACAGGAAGGCGCAATGCATACCGCATTGACAGGCGCTACAGAGATACAGATGTCTAGCG
GAAATCTGTTGTTTACAGGGCATCTGAAATGTAGACTGAGAATGGACAAACTGCAATTGAAGG
GAATGTCATACTCTATGTGTACGGGTAAGTTTAAGATAGTCAAAGAGATAGCCGAAACACAAC
ACGGAACAATCGTAATTAGGGTGCAATACGAAGGCGACGGGTCACCATGTAAGATACCATTCG
AAATTACAGACCTCGAAAAAAGACACGTACTCGGAAGACTGATAACAGTGAATCCGATCGTTA
CGGAAAAAGACTCACCCGTTAATATCGAAGCCGAACCACCATTCGGAGACTCATACATAATAA
TCGGAGTCGAACCCGGACAATTGAAACTGAATTGGTTTAAAAAAGGGTCATCAATCGGACAAA
TGTTCGAAACAACTATGAGAGGCGCTAAGCGTATGGCTATACTCGGAGACACAGCATGGGACT
TCGGATCCTTAGGGGGAGTGTTTACGTCAATCGGTAAGGCACTACACCAGGTATTCGGAGCGA
TATACGGAGCCGCATTTAGCGGAGTGTCGTGGACAATGAAGATACTGATCGGAGTGATAATCA
CATGGATCGGAATGAATAGTAGGTCTACAAGTCTATCCGTTAGCTTAGTGTTAGTCGGAGTCGT
TACACTGTATCTAGGCGCTATGGTGCAAGCCGATAGTGGTTGCGTTGTGAGCTGGAAAAATAA
AGAACTGAAATGTGGCAGCGGGATCTTCATCACAGATAACGTACACACATGGACAGAACAAT
ATAAGTTCCAACCAGAATCCCCTTCAAAACTAGCTTCAGCTATCCAAAAAGCTCATGAAGAGG
GCATTTGTGGAATCCGCTCAGTAACAAGATTGGAGAATCTGATGTGGAAACAAATAACACCAG
AATTGAATCATATTCTATCAGAAAATGAGGTAAAGTTGACCATTATGACAGGAGACATTAGAG
GAATCATGCAGGCAGGAAAACGATCCTTGCGGCCCCAGCCCACTGAGCTGAAGTACTCATGGA
AAACATGGGGAAAGGCGAAAATGCTCTCCACAGAGTCTCACAATCAGACCTTTCTTATTGATG
GCCCTGAAACAGCAGAATGCCCCAACACAAACAGAGCCTGGAACTCGCTGGAAGTTGAAGAC
TATGGTTTTGGAGTTTTCACCACCAATATATGGCTGAAATTGAGAGAAAAACAGGATGTATTTT
GTGACTCAAAACTCATGTCAGCGGCCATTAAAGACAACAGAGCCGTTCATGCTGATATGGGTT

ATTGGATAGAAAGTGCACTCAATGACACATGGAAGATGGAGAAAGCCTCCTTCATTGAAGTTA
AAAGCTGCCACTGGCCAAAGTCACACACCCTTTGGAGCAATGGAGTATTAGAAAGTGAGATGA
TAATCCCAAAAAATTTTGCCGGGCCAGTGTCACAACACAACTACAGACCAGGCTACCATACAC
AAACAGCAGGACCTTGGCATCTAGGTAAGCTTGAGATGGACTTTGATCTCTGCGAAGGAACTA
CAGTGGTGGTGACTGAGGACTGTGGAAATAGAGGACCCTCTTTAAGAACGACCACTGCCTCTG
GAAAACTCATAACAGAATGGTGCTGCCGATCCTGCACACTACCACCTCTAAGATACAGAGGTG
AGGATGGATGCTGGTACGGGATGGAAATCAGACCATTGAAAGAGAAAGAAGAGAATTTGGTC
AACTCCTTGGTCACAGCCGGACATGGGCAGATTGACAACTTTTCACTAGGAGTCTTGGGAATG
GCACTGTTCCTGGAAGAAATGCTCAGGACCCGAATAGGAACGAAACATGCAATACTGCTAGTT
GCAGTATCTTTTGTGACATTGATTACTGGGAACATGTCTTTTAGAGACCTGGGAAGAGTGATGG
TTATGGTGGGCGCTACCATGACGGATGACATAGGTATGGGAGTGACTTATCTTGCCCTACTAGC
AGCTTTTAAGGTTAGACCAACTTTTGCAGCTGGACTACTCTTAAGAAAACTGACCTCCAAGGA
ATTGATGATGGCCACCATAGGAATCGCACTCCTTTCCCAAAGCACCATACCAGAGACCATTCTT
GAACTGACTGATGCATTAGCCCTGGGCATGATGGTCCTCAAAATAGTGAGAAATATGGAAAAA
TACCAATTGGCAGTGACTATCATGGCTATTTCATGTGTCCCAAATGCAGTGATACTGCAAAACG
CATGGAAGGTGAGTTGCACAATATTGGCAGCGGTGTCCGTTTCACCACTGCTCTTAACATCCTC
ACAGCAGAAAGCGGATTGGATACCACTGGCATTGACGATAAAAGGTCTCAATCCAACAGCCAT
TTTTTTAACAACTCTCTCGAGGACCAGCAAGAAAAGGAGCTGGCCGCTAAATGAAGCTATCAT
GGCAGTTGGGATGGTGAGCATTTTAGCCAGTTCTCTCCTAAAGAATGATATTCCTATGACAGGT
CCATTAGTGGCTGGAGGACTCCTCACCGTATGTTACGTGCTCACTGGACGATCGGCCGATTTGG
AACTGGAGAGAGCTGCCGATGTAAAATGGGAAGATCAGGCAGAAATATCAGGAAGCAGCCCA
ATCCTGTCAATAACAATATCAGAAGATGGCAGCATGTCGATAAAAAATGAAGAGGAAGAACA
AACACTGACCATACTCATCAGAACGGGATTGTTGGTGATCTCAGGAGTCTTTCCAGTATCGATA
CCAATTACGGCAGCAGCATGGTACCTGTGGGAAGTAAAGAAACAACGGGCTGGAGTACTGTG
GGACGTCCCTTCACCCCCACCAGTGGAAAAAGCCGAACTGGAGGATGGAGCCTACAGAATCA
AGCAAAGAGGGATCCTTGGATATTCTCAGATTGGAGCCGGAGTTTACAAAGAAGGAACATTCC
ATACAATGTGGCACGTCACACGTGGTGCTGTTCTGATGCATAGAGGGAAGAGGATTGAACCAT
CATGGGCAGATGTCAAGAAAGACCTAATATCATATGGAGGAGGCTGGAAGCTAGAAGGAGAA
TGGAAGGAAGGAGAGGAAGTCCAAGTCCTGGCATTGGAACCTGGAAAAAATCCAAGAGCCGT
CCAAACGAAACCTGGAATATTCAAAACCAACACCGGAACCATAGGCGCCGTATCTCTGGACTT
TTCCCCTGGAACGTCAGGATCTCCAATCGTCGACAGAAAAGGAAAAGTTGTGGGTCTTTACGG
TAATGGTGTTGTCACAAGGAGTGGAGCATATGTAAGTGCTATAGCCCAGACCGAAAAAAGCAT
TGAAGACAATCCAGAGATCGAAGATGACATTTTCCGAAAGAAAAGATTGACCATCATGGACCT
CCATCCAGGAGCAGGAAAGACAAAAAGATACCTTCCAGCCATAGTTAGAGAAGCCATAAAAC
GTGGCTTGAGAACATTGATCCTGGCTCCCACTAGAGTAGTGGCAGCTGAAATGGAGGAAGCTC
TTAGAGGACTTCCAATAAGATACCAAACTACAGCCATCAAAACCGAGCATACCGGGCGGGAG
ATCGTGGACCTAATGTGTCATGCCACATTTACTATGAGGCTGTTATCACCAGTCAGAGTGCCAA

ATTACAACCTGATCATCATGGACGAAGCCCACTTCACAGACCCAGCAAGTATAGCAGCTAGAG
GATACATTTCAACTCGAGTAGAGATGGGTGAAGCAGCCGGGATTTTCATGACAGCCACTCCTC
CGGGAAGTAGAGACCCATTTCCTCAGAGCAATGCACCAATTATGGATGAGGAAAGAGAAATC
CCTGAGCGTTCATGGAATTCAGGACACGAATGGGTCACGGATTTTAAGGGGAAGACTGTTTGG
TTTGTTCCAAGTATAAAAGCAGGAAATGATATAGCAGCTTGTCTTAGGAAAAATGGAAAGAAA
GTGATACAACTCAGTAGGAAGACTTTTGACTCTGAGTATGTTAAGACTAGAGCCAATGATTGG
GACTTTGTGGTCACAACTGACATTTCAGAAATGGGTGCCAACTTCAAGGCTGAGAGGGTTATA
GACCCCAGACGTTGCATGAAACCAGTTATACTAACAGATGGCGAGGAGCGGGTGATCTTGGCT
GGACCTATGCCAGTGACCCACTCTAGTGCAGCGCAAAGAAGAGGGAGAATAGGAAGAAATCC
AAAAAATGAAAATGACCAGTACATATACATGGGGGAACCTCTTGAAAATGATGAAGACTGTG
CACATTGGAAAGAAGCTAAAATGCTCCTAGATAACATCAACACACCTGAAGGAATCATTCCTA
GTATGTTCGAACCAGAGCGTGAAAAAGTGGATGCCATTGATGGTGAATACCGTTTGAGAGGAG
AAGCAAGGAAAACCTTTGTGGACCTAATGAGAAGAGGGGACTTACCAGTCTGGTTGGCCTACA
AAGTGGCAGCTGAAGGCATCAACTACGCAGACAGAAAGTGGTGTTTTGATGGAATTAAGAAC
AACCAAATACTGGAAGAAAATATGGAAGTGGAAATCTGGACAAAAGAAGGGGAAAGGAAAA
AATTAAAACCCAGATGGTTGGATGCTAGGATCTATTCTGACCCACTAGCACTAAAAGAATTCA
AGGAATTTGCAGCTGGAAGAAAATCTTTGACCCTGAACCTAATCACAGAAATGGGTAGGCTTC
CAACTTTCATGACTCAGAAAGCAAGAAACGCACTGGACAACCTGGCTGTGCTGCATACGGCTG
AGGCAGGTGGAAGGGCGTACAATCATGCTCTCAGTGAACTGCCGGAGACCCTGGAGACACTG
CTCCTACTGACACTTCTGGCAACAGTCACAGGAGGAATCTTCTTATTCTTAATGAGCGGAAAAG
GTATAGGGAAGATGACCCTGGGAATGTGTTGCATAATCACGGCTAGTATCCTCCTATGGTATG
CACAGATACAACCACACTGGATAGCAGCTTCAATAATACTGGAGTTTTTCTCATAGTTTTGCT
CATTCCAGAACCAGAAAAACAGAGAACACCCCAAGACAACCAATTGACCTACGTTGTCATAGC
CATCCTCACAGTGGTGGCCGCAACCATGGCAAACGAGATGGGTTTCCTGGAAAAAACCAAGA
AAGACCTCGGATTGGGAAGCATTACAACCCAGGAATCTGAGAGCAATATCCTGGACATAGATC
TACGCCCTGCATCAGCATGGACGCTGTATGCCGTAGCTACAACATTTGTCACACCAATGTTGAG
ACATAGCATTGAAAATTCCTCAGTGAATGTCTCCCTAACAGCCATTGCTAACCAAGCTACAGTG
CTAATGGGTCTTGGGAAAGGATGGCCATTGTCAAAGATGGACATTGGAGTTCCCCTCCTTGCC
ATTGGATGCTATTCACAAGTCAACCCTATAACTCTCACAGCAGCTCTCCTTTTATTGGTAGCAC
ATTATGCCATTATAGGGCCAGGACTTCAAGCAAAAGCAACCAGAGAAGCCCAGAAAAGAGCA
GCAGCAGGCATCATGAAAAACCCAACAGTCGATGGAATAACAGTGATTGACCTAGAACCAAT
ACCCTATGATCCAAAATTTGAAAAGCAGTTAGGACAAGTAATGCTCCTAATCCTCTGCGTGACT
CAAGTATTAATGATGAGGACTACATGGGCTTTATGTGAGGCTCTAACCCTAGCGACCGGGCCC
ATCTCCACACTGTGGGAAGGAAATCCAGGGAGGTTTTGGAACACTACCATTGCAGTGTCAATG
GCTAACATCTTTAGGGGGAGCTACTTGGCCGGAGCTGGACTTCTCTTTTCCATCATGAAAAACA
CAACAAACACAAGAAGAGGAACTGGCAACATAGGAGAGACACTTGGAGAAAAATGGAAAAG
TCGATTAAACGCACTGGGGAAAAGTGAATTTCAAATCTACAAGAAAAGTGGAATCCAGGAAG

TGGATAGAACCCTAGCAAAAGAAGGTATCAAAAGAGGAGAAACGGACCACCATGCTGTGTCG CGAGGTTCAGCAAAACTGAGATGGTTCGTCGAGAGAAATATGGTCACACCGGAAGGGAAGGT GGTGGATCTCGGTTGCGGCAGAGGGGGCTGGTCATACTATTGCGGGGGACTAAAGAATGTAAG AGAAGTCAAAGGCCTAACAAAAGGAGGACCAGGACATGAAGAACCCATCCCCATGTCAACAT ATGGGTGGAATCTAGTGCGTCTGCAAAGTGGAGTTGACGTTTCTTCACCCCGCCAGAAAAGT GTGATACATTGTTGTGTGACATAGGGGAGTCGTCACCAAATCCCACGATAGAAGCAGGACGAA CACTCAGAGTCCTCAACTTAGTGGAAAATTGGTTGAACAATAACACCCAATTTTGCATAAAGG TCCTCAACCCATATATGCCTTCAGTCATAGAAAAAATGGAAGCATTACAAAGGAAATATGGAG GAGCCTTAGTGAGGAATCCACTCTCACGAAACTCCACGCATGAAATGTACTGGGTATCTAATG CCACCGGGAACATAGTGTCATCAGTGAACATGATTTCAAGGATGTTGATTAACAGATTCACAA TGAAACACAAGAAAGCCACTTACGAGCCAGATGTTGACCTAGGAAGTGGAACCCGCAACATT GGAATTGAAAGTGAGATACCAAATCTAGACATAATAGGAAAGAGAATAGAGAAAATAAAACA AGAGCATGAAACATCATGGCACTATGATCAAGACCACCCATACAAAACGTGGGCTTACCATGG CAGCTATGAAACAAAACAAACTGGATCAGCATCATCTATGGTGAACGGAGTGGTCAGATTGCT GACAAAACCTTGGGACGTCGTCCCTATGGTGACACAGATGGCAATGACAGACACGACTCCTTT TGGACAACAGCGCGTTTTCAAAGAGAAAGTAGACACGAGAACCCAAGAACCGAAGGAAGGCA CAAAGAAACTGATGAAAATTACGGCAGAGTGGCTTTGGAAAGAACTAGGAAAGAAAAAGACA CCTAGGATGTGTACCAGAGAAGAATTCACAAGAAAAGTGAGAAGCAATGCAGCCTTGGGGGC CATATTCACTGATGAGAACAAATGGAAATCGGCACGTGAGGCTGTTGAAGATGGTAGGTTCTG GGAGCTGGTTGACAGGGAAAGAAATCTCCATCTTGAAGGAAAGTGTGAAACATGTGTGTACA ACATGATGGGAAAAAGAGAGAAGAAACTAGGGGAGTTCGGCAAGGCAAAAGGTAGCAGAGC CATATGGTACATGTGGCTTGGAGCACGCTTCTTAGAGTTTGAAGCCCTAGGATTCCTGAATGAA GATCACTGGTTCTCCAGAGGGAACTCCCTGAGTGGAGTGGAAGGAGAAGGGCTGCACAGGCT AGGCTACATTTTAAGAGACGTGGGCAAGAAGGAAGGGGGGGCAATGTACGCCGATGATACAG CAGGATGGGACACAAGAATCACACTAGAAGACTTAAAAAATGAAGAAATGGTAACGAACCAC ATGAAAGGAGAACACAAGAAACTAGCCGAGGCCATATTCAAACTAACGTACCAAAACAAGGT GGTGCGTGTGCAAAGACCAACACCAAGAGGTACAGTAATGGATATCATATCGAGAAGAGACC AAAGAGGCAGTGGGCAAGTCGGCACCTATGGCCTTAATACCTTCACCAATATGGAAGCCCAAT TAATTAGACAGATGGAGGGAGAAGGAATCTTCAAAAGCATTCAGCACCTGACAGCCACAGAA GAAATCGCTGTACAGAACTGGCTAGCAAGAGTGGGGCGTGAAAGGCTATCAAGAATGGCAAT CAGTGGAGATGATTGTGTTGTAAAACCTATAGATGACAGATTTGCAAGTGCTTTAACAGCTCTA AATGACATGGGAAAAGTTAGAAAAGATATACAACAATGGGAACCTTCAAGAGGATGGAACGA TTGGACACAAGTGCCTTTCTGTTCACACCACTTTCATGAGTTAGTCATGAAAGATGGTCGCGTG CTCGTAGTCCCATGCAGAAACCAAGATGAACTGATTGGTAGAGCCCGAATTTCCCAGGGAGCT GGGTGGTCTTTGAAAGAGACGGCCTGTTTGGGAAAGTCTTACGCCCAAATGTGGACTCTGATG TACTTCCACAGACGTGACCTCAGACTGGCGGCAAATGCCATCTGCTCGGCAGTCCCGTCACATT GGGTTCCAACAAGTCGAACAACCTGGTCCATACACGCTAAGCATGAATGGATGACGACGGAA

GACATGCTGGCAGTCTGGAACAGGGTGTGGATCCAAGAAAACCCGTGGATGGAAGATAAAAC
TCCAGTGGAATCATGGGAAGAAGTCCCATACTTGGGAAAAAGAGAAGACCAATGGTGCGGCT
CATTGATTGGGCTAACAAGCAGGGCTACCTGGGCAAAGAACATCCAAACAGCAATAAATCAA
GTCAGATCCCTTATAGGCAATGAGGAATACACAGACTACATGCCATCCATGAAGAGATTTAGA
AGGGAAGAGGAAGAGGCAGGTGTCCTGTGGTAGAAGGCAAAACTAACATGAAACAAGGCTAA
AAGTCAGGTCGGATTAAGCCATAGTACGGAAAAAACTATGCTACCTGTGAGCCCCGTCCAAGG
ACGTTAAAAGAAGTCAGGCCATCACAAAATGCCACAGCTTGAGTAAACTGTGCAGCCTGTAGC
TCCACCTGAGGAGGTGTAAAAAACCTGGGAGGCCACAAACCATGGAAGCTGTACGCATGGCG
TAGTGGACTAGCGGTTAGAGGAGACCCCTCCCTTACAAATCGCAGCAAACAACGGGGGCCCA
AGGTGAGATGAAGCTGTAATCTCACTGGAAGGACTAGAGGTTAGAGGAGACCCCCCCAAAAC
AAAAAACAGCATATTGACGCTGGGAAAGACCAGAGATCCTGCTGTCTCCTCAGCATCATTCCA
GGCACAGAACGCCAGAAAATGGAATGGTGCTGTTGAATCAACAGGTTCT

SEQ ID NO: 5 - DENV-3-VE-BID-V2268-2008

AGTTGTTAGTCTACGTGGACCGACAAGAACAGTTTCGACTCGGAAGCTTGCTTAACGTAGTGCT
AACAGTTTTTTATTAGAGAGCAGATCTCTGATGAACAACCAACGGAAGAAGACGGGAAAACC
GTCTATCAATATGCTGAAACGCGTGAGAAACCGTGTGTCAACTGGATCACAGTTGGCGAAGAG
ATTCTCAAAAGGACTGCTGAACGGCCAGGGACCAATGAAATTGGTTATGGCGTTCATAGCTTT
CCTCAGATTTCTAGCCATTCCACCAACAGCAGGAGTCTTGGCTAGATGGGGAACCTTCAAGAA
GTCGGGGGCCATTAAGGTCCTGAAAGGCTTTAAGAAGGAGATCTCAAACATGCTGAGCATAAT
CAACAAACGGAAAAAGACATCGCTCTGTCTCATGATGATATTGCCAGCAGCACTTGCTTTCCA
CTTGACTTCACGAGATGGAGAGCCGCGCATGATTGTGGGGAAGAATGAAAGAGGAAAATCCC
TACTTTTTAAGACAGCCTCTGGAATTAACATGTGCACACTCATAGCCATGGACTTGGGAGAGAT
GTGTGATGACACGGTCACTTACAAATGCCCCCATATTACCGAAGTGGAACCTGAAGACATTGA
CTGCTGGTGCAACCTCACATCAACATGGGTGACTTATGGAACGTGCAATCAAGCCGGAGAGCA
TAGACGCGATAAGAGATCAGTGGCGTTAGCTCCCCATGTCGGCATGGGACTGGATACACGCAC
CCAAACTTGGATGTCGGCTGAAGGAGCTTGGAGGCAAGTCGAGAAGGTAGAGACATGGGCCC
TTAGGCACCCAGGGTTCACCATACTAGCCCTATTTCTTGCCCATTACATAGGCACTTCCTTGAC
CCAGAAGGTGGTTATTTTTATACTGCTAATGCTGGTCACCCCATCCATGACAATGAGATGTGTG
GGAGTAGGAAACAGAGATTTTGTGGAAGGACTATCAGGAGCTACGTGGGTTGACGTGGTGCTC
GAGCACGGGGGGTGTGTGACCACCATGGCTAAGAACAAGCCCACGTTGGATATAGAGCTTCA
GAAGACCGAGGCCACCCAACTGGCGACCCTAAGGAAGCTATGCATTGAGGGGAAAATCACCA
ACATAACAACTGACTCAAGATGTCCTACCCAAGGGGAAGCGGTTTTGCCTGAGGAGCAGGACC
AAAACTACGTGTGTAAGCATACATACGTAGACAGAGGCTGGGGGAACGGATGTGGTTTGTTTG
GTAAGGGAAGCTTGGTAACATGTGCGAAATTTCAATGCCTGGAACCAATAGAGGGAAAAGTG
GTGCAATATGAGAACCTCAAATACACCGTTATCATCACAGTGCACACAGGAGACCAACACCAG
GTGGGAAATGAAACGCAGGGAGTCACGGCTGAGATAACACCTCAGGCATCAACCACTGAAGC

CATCTTGCCTGAATATGGAACCCTTGGGCTAGAATGCTCACCACGGACAGGTTTGGACTTCAAT
GAAATGATCTTGCTAACAATGAAGAACAAAGCATGGATGGTACATAGACAATGGTTTTTGAC
CTACCTCTACCATGGACATCAGGAGCTACAACGGAAACACCAACCTGGAACAGGAAGGAGCT
TCTTGTGACATTTAAAAACGCACATGCGAAGAAACAAGAAGTAGTTGTTCTTGGGTCGCAAGA
GGGAGCAATGCATACCGCATTGACAGGAGCCACAGAAATCCAAAACTCAGGAGGCACAAGCA
TTTTTGCGGGGCACTTAAAATGTAGACTTAAGATGGACAAATTAGAACTCAAGGGGATGAGCT
ATGCAATGTGCACGAATACCTTTGTGTTGAAGAAAGAAGTCTCAGAAACGCAGCATGGGACAA
TACTTATCAAGGTCGAGTACAAAGGGGAAGATGTACCTTGCAAGATTCCTTTCTCCACAGAGG
ATGGACAAGGGAAAGCTCACAATGGCAGACTGATTACAGCCAACCCAGTGGTGACTAAGAAG
GAGGAGCCTGTCAATATTGAGGCTGAACCTCCTTTTGGGGAAAGCAATATAGTAATTGGAATT
GGAGACAACGCCTTGAAAATCAACTGGTACAAGAAGGGAAGCTCTATTGGGAAGATGTTCGA
GGCCACTGCTAGAGGTGCAAGGCGCATGGCCATCTTGGGAGACACAGCTTGGGACTTTGGATC
AGTGGGTGGTGTTCTGAACTCATTAGGCAAAATGGTGCACCAAATATTCGGAAGTGCTTACAC
AGCCCTATTCAGTGGAGTCTCTTGGGTAATGAAAATTGGAATAGGAGTTCTCTTGACTTGGATA
GGGTTGAATTCAAAAAACACATCCATGTCATTTTCATGCATTGCGATAGGAATCATCACACTCT
ATCTGGGAGCTGTGGTGCAAGCTGACATGGGGTGTGTTATAAACTGGAAAGGCAAAGAACTCA
AGTGTGGAAGTGGAATCTTCGTCACCAACGAGGTCCATACCTGGACAGAGCAATACAAATTCC
AAGCAGACTCCCCAAAAAGATTGGCGACAGCCATTGCAGGCGCTTGGGAGAATGGAGTGTGC
GGAATTAGGTCAACAACCAGAATGGAGAATCTCCTGTGGAAGCAAATAGCCAATGAACTGAA
CTACATATTGTGGGAAAACAATATCAAATTAACGGTTGTTGTGGGCGATATAATTGGGGTCTTA
GAGCAAGGGAAAAGAACACTAACACCACAACCCATGGAGCTAAAATACTCATGGAAAACGTG
GGGAAAGGCAAAAATAGTGACAGCTGAAACACAAAATTCTTCTTTCATAATAGATGGACCAA
ACACACCGGAGTGTCCAAGTGCCTCAAGAGCATGGAATGTGTGGGAGGTGGAAGATTACGGG
TTCGGAGTCTTCACAACCAACATATGGCTGAAACTCCGAGAGGTGTATACCCAACTATGTGAC
CATAGGTTAATGTCGGCAGCCGTCAAGGATGAAAGGGCCGTACATGCCGACATGGGCTATTGG
ATAGAAAGTCAAAAGAATGGAAGTTGGAAGCTAGAAAAAGCATCCCTCATAGAGGTGAAAAC
CTGCACATGGCCAAAATCACATACCCTTTGGAGTAATGGTGTGTTAGAGAGTGACATGATCAT
TCCAAAAAGTCTAGCTGGTCCTATCTCGCAACACAACTACAGGCCCGGGTACCACACCCAGAC
GGCGGGACCTTGGCATTTAGGAAAATTAGAGCTGGACTTCAACTATTGTGAAGGAACAACAGT
TGTCATCACAGAAAACTGTGGGACAAGAGGCCCATCATTGAGAACAACAACAGTGTCAGGGA
AGTTAATACACGAATGGTGCTGCCGCTCGTGCACACTTCCTCCCCTGCGATACATGGGAGAAG
ACGGTTGCTGGTATGGCATGGAAATCAGACCCATCAGTGAGAAAGAAGAAAACATGGTAAAG
TCTTTAGTCTCAGCGGGAAGTGGAGAGGTGGACAACTTCACAATGGGTGTCTTGTGTTTGGCA
ATCCTCTTTGAAGAGGTGATGAGAGGAAAATTTGGGAAGAAACACATGATTGCGGGGGTTTTC
TTCACGTTTGTACTCCTTCTCTCAGGGCAAATAACATGGAGAGATATGGCGCACACACTAATAA
TGATTGGGTCCAATGCATCTGACAGGATGGGAATGGGCGTCACCTACCTAGCTTTAATTGCAA
CATTTAAAATCCAGCCATTTTTGGCTTTGGGATTTTTCCTAAGAAAACTGACATCCAGAGAAAA

TTTATTGTTAGGAGTTGGGCTGGCTATGGCAACAACGTTACAACTGCCAGAGGACATTGAACA
AATGGCAAATGGAATCGCTCTGGGGCTCATGGCTCTCAAACTGATAACACAATTTGAAACATA
CCAACTATGGACAGCATTAATCTCCTTAACGTGTTCAAATACAATTTTTACGTTGACTGTTGCC
TGGAGAACAGCCACCCTGATTTTGGCTGGAGTTTCACTTTTACCAGTGTGCCAGTCTTCGAGTA
TGAGGAAAACAGACTGGCTTCCAATGACAGTGGCAGCTATGGGAGTTCCACCTCTACCACTTT
TTATTTTTAGCTTAAAAGACACACTCAAAAGGAGAAGCTGGCCACTGAATGAAGGGGTGATGG
CTGTTGGGCTTGTGAGCATTCTGGCCAGTTCTCTCCTTAGAAATGATGTACCCATGGCTGGACC
ATTAGTGGCCGGGGGCTTGCTGATAGCGTGCTACGTCATAACTGGCACGTCAGCAGACCTCAC
CGTAGAAAAAGCAGCAGATGTAACATGGGAGGAAGAGGCTGAGCAAACAGGAGTGTCCCACA
ACTTAATGATCACAGTTGATGATGATGGAACAATGAGAATAAAAGATGATGAGACTGAGAAT
ATCTTAACAGTGCTTTTGAAAACAGCATTACTAATAGTGTCAGGAATCTTTCCATACTCCATAC
CCGCAACATTGTTGGTCTGGCATACTTGGCAAAAGCAAACCCAAAGATCCGGCGTTCTATGGG
ATGTACCCAGCCCTCCAGAGACACAGAAAGCAGAACTGGAAGAAGGGGTCTATAGGATCAAA
CAGCAAGGAATTTTTGGGAAAACCCAAGTAGGGGTTGGAGTACAGAAAGAAGGAGTCTTTCA
CACCATGTGGCACGTTACAAGAGGGGCAGTGTTGACATATAATGGGAAAAGACTGGAACCAA
ATTGGGCTAGCGTGAAAAAAGATCTGATTTCATACGGAGGAGGATGGAGATTGAGCGCACAA
TGGCAAAAGGGGGAGGAGGTGCAGGTTATTGCCGTAGAGCCTGGGAAGAACCCAAAGAACTT
TCAAACCATGCCAGGCACTTTTCAGACTACAACAGGGGAAATAGGAGCAATTGCACTGGATTT
CAAGCCCGGAACTTCAGGATCTCCTATCATAAACAGAGAGGGAAAGGTAGTGGGACTGTATG
GCAATGGAGTGGTTACAAAGAATGGTGGCTACGTCAGCGGAATAGCGCAAACGAATGCAGAA
CCAGATGGACCGACACCAGAATTGGAAGAAGAGATGTTCAAAAAGCGAAATCTAACCATAAT
GGATCTTCATCCTGGGTCAGGAAAGACACGGAAATACCTTCCAGCTATTGTTAGAGAGGCAAT
CAAGAGACGTTTGAGAACTCTAATTCTGGCACCAACAAGGGTGGTTGCAGCTGAGATGGAAGA
AGCATTGAAAGGGCTCCCAATAAGGTACCAAACAACAGCAACAAAATCTGAACACACAGGAA
GAGAGATTGTTGATCTGATGTGCCACGCAACGTTCACAATGCGTCTGCTGTCACCAGTTAGGGT
TCCAAACTATAACTTGATAATAATGGATGAAGCCCATTTCACAGACCCAGCCAGTATAGCTGC
TAGAGGGTACATATCAACTCGTGTTGGAATGGGAGAAGCAGCCGCAATATTCATGACAGCAAC
GCCCCCTGGAACAGCTGATGCCTTTCCCCAGAGCAACGCTCCAATTCAAGATGAAGAAAGGGA
CATACCAGAACGCTCATGGAATTCAGGCAATGAATGGATAACCGACTTCGCTGGGAAAACGGT
GTGGTTTGTCCCCAGCATTAAAGCCGGAAATGACATAGCAAATTGCCTGCGGAAAAACGGGAA
AAAGGTCATTCAACTTAGTAGGAAGACTTTTGACACAGAATATCAGAAAACCAAACTGAATGA
TTGGGACTTTGTGGTGACGACTGACATTTCAGAAATGGGGGCCAATTTCAAAGCAGATAGAGT
GATCGACCCAAGAAGATGTCTCAAACCAGTGATCCTGACAGATGGACCAGAGCGGGTGATCCT
GGCTGGACCAATGCCAGTCACCGCAGCGAGTGCTGCGCAAAGGAGAGGGAGAGTTGGCAGGA
ACCCACAAAAAGAAAATGACCAGTACATATTCACGGGCCAGCCTCTCAACAATGATGAAGAC
CATGCTCACTGGACAGAAGCAAAAATGCTGCTGGACAACATTAACACACCAGAAGGGATCAT
ACCAGCTCTCTTTGAGCCAGAAAGGGAGAAGTCAGCCGCCATAGACGGTGAGTATCGCCTGAA

AGGTGAGTCCAGGAAGACTTTCGTGGAACTCATGAGGAGGGGTGACCTTCCAGTCTGGTTAGCCCATAAAGTAGCATCAGAAGGGATCAAATATACAGATAGAAAATGGTGCTTTGATGGACAACGTAATAATCAAATTTTAGAAGAGAACATGGATGTGGAAATCTGGACAAAGGAAGGAGAAAAGAAAAAATTGAGACCTAGGTGGCTTGATGCTCGCACCTATTCAGATCCCTTAGCACTCAAGGAATTCAAGGACTTTGCGGCTGGCAGGAAGTCAATAGCCCTTGATCTTGTGACAGAAATAGGAAGAGTGCCTTCACACCTAGCTCATAGAACGAGAAACGCTCTGGACAATCTGGTGATGCTGCATACGTCAGAACATGGCGGTAAGGCCTACAGGCATGCGGTGGAGGAACTACCAGAGACAATGGAAACACTCCTACTCTTGGGACTCATGATCTTGTTGACAGGTGGAGCAATGCTTTTCTTAATATCAGGTAAAGGGATTGGAAAGACTTCAATAGGACTCATTTGTGTAATTGCTTCCAGCGGCATGTTGTGGATGGCCGAAATCCCACTCCAATGGATCGCGTCGGCCATAGTCCTGGAGTTTTTATGATGGTGTTGCTTATACCAGAACCAGAGAAGCAGAGAACCCCCCAAGACAACCAACTCGCATATGTCGTGATAGGCATACTTACACTGGCAGCAATAATAGCAGCCAATGAAATGGGATTGTTGGAAACTACAAAGAGAGATTTAGGAATGTCTAAGGAGCCAGGTGTTGTTTCTCCAACCAGCTATTTAGATGTGGACTTGCACCCAGCATCAGCCTGGACATTGTACGCTGTGGCCACTACAGTAATAACACCAATGTTAAGACATACCATAGAGAATTCCACAGCAAATGTGTCCTTGGCAGCTATAGCCAACCAGGCAGTGGTCCTGATGGGTTTGGACAAAGGATGGCCAATATCAAAAATGGACTTAGGAGTACCCCTGCTGGCATTGGGTTGCTATTCACAAGTGAACCCACTGACTCTAACAGCGGCAGTACTCTTGCTGATCACACATTATGCCATTATAGGTCCAGGATTGCAGGCAAAAGCCACCCGTGAAGCTCAGAAAAGGACAGCTGCTGGAATAATGAAGAATCCAACAGTGGATGGGATAATGACAATAGACCTAGATCCTGTAATATATGATTCAAAATTTGAAAAGCAACTGGGACAGGTTATGCTCCTGGTTTTGTGTGCAGTTCAACTTTTGTTAATGAGAACATCATGGGCCTTGTGTGAAGCTTTAACCCTAGCTACAGGACCAATAACAACACTCTGGGAAGGATCACCTGGGAAGTTTTGGAACACCACGATAGCTGTTTCCATGGCGAACATTTTTAGAGGGAGCTATTTAGCAGGAGCTGGGCTTGCTTTCTCTATTATGAAATCAGTTGGAACAGGGAAAAGAGGAACAGGCTCACAGGGTGAAACTTTGGGAGAAAAATGGAAAAAGAAGTTAAATCAATTATCCCGGAAAGAGTTTGACCTTTACAAGAAATCTGGAATCACTGAGGTGGATAGAACAGAAGCCAAAGAAGGGTTGAAAAGAGGAGAAATAACACATCATGCCGTGTCCAGAGGTAGTGCAAAACTTCAATGGTTTGTGGAGAGAAACATGGTCATTCCCGAAGGAAGAGTTATAGACTTGGGCTGTGGAAGAGGAGGCTGGTCATATTACTGTGCAGGACTGAAAAAAGTCACAGAAGTGCGAGGATACACAAAAGGCGGTCCAGGACACGAAGAACCAGTACCTATGTCCACATATGGATGGAACATAGTTAAGTTAATGAGTGGAAAGGATGTGTTTTATCTTCCACCTGAAAAGTGTGACACCCTGTTGTGCGACATTGGAGAATCTTCACCAAGCCCAACAGTGGAAGAAAGCAGAACTATAAGAGTTTTGAAGATGGTTGAACCATGGCTAAAGAACAACCAATTTTGTATTAAAGTATTGAACCCTTACATGCCAACTGTGATTGAGCACCTAGAAAGACTACAAAGGAAACATGGAGGAATGCTTGTGAGAAATCCACTTTCACGAAACTCCACGCACGAAATGTACTGGATATCCAATGGCACAGGTAACATTGTCGCTTCAGTCAACATGGTATCTAGACTGCTACTGAACAGGTTCACGATGACACACAGAAGACCCACCATTGAGAAAGATGTGGATTTAGGAGCAGGAACTCGACATGTTAATGCGGAACCAGAAACACCCAACATGGATGTCATTGGGGAAAGAATAAAAAGGATCAAGGAGG

AGCATAATTCAACATGGCACTACGATGACGAAAACCCCTACAAAACGTGGGCTTACCATGGAT
CTTATGAAGTCAAAGCCACAGGCTCAGCCTCCTCCATGATAAATGGAGTCGTGAAACTCCTCA
CTAAACCATGGGATGTGGTGCCCATGGTGACACAGATGGCAATGACAGATACAACTCCATTTG
GCCAGCAGAGAGTCTTTAAAGAGAAAGTGGACACCAGGACACCCAGGTCCATGCCAGGAACA
AGAAGGGTTATGGGGATCACAGCGGAGTGGCTCTGGAGAACCCTGGGAAGGAATAAAAAACC
CAGGTTATGCACAAGGGAAGAGTTTACAAAAAAGGTCAGAACTAACGCAGCCATGGGAGCTG
TTTTCACAGAGGAGAACCAATGGGACAGCGCGAAAGCTGCTGTTGAGGATGAGGATTTTTGGA
AACTTGTGGACAGAGAACGTGAACTCCACAAACTGGGCAAGTGTGGAAGCTGTGTTTACAACA
TGATGGGTAAGAGAGAGAAGAAACTTGGAGAGTTTGGCAAAGCAAAAGGCAGTAGAGCTATA
TGGTACATGTGGTTGGGAGCCAGGTACCTTGAGTTCGAAGCCCTTGGATTCTTAAATGAAGAC
CACTGGTTCTCGCGTGAGAACTCTTACAGTGGAGTGGAAGGAGAAGGACTGCACAAGCTAGGC
TATATATTAAGGGACATTTCCAAGATACCCGGAGGAGCTATGTATGCTGATGACACAGCTGGT
TGGGACACAAGAATAACAGAAGATGACCTGCACAATGAGGAAAAGATCACACAGCAAATGGA
CCCTGAACACAGGCAGTTAGCGAACGCTATATTTAAGCTCACATACCAAAACAAAGTGGTCAA
AGTTCAACGACCGACTCCAACAGGCACGGTAATGGATATCATATCTAGGAAAGACCAAAGAG
GCAGTGGACAGGTAGGAACTTATGGTCTGAATACATTCACCAACATGGAAGCCCAGTTAATCA
GACAAATGGAAGGAGAAGGTGTGCTGTCAAAGGCAGACCTCGAGAACCCTCATCTGCCAGAG
AAGAAAATTACACAATGGTTGGAAACCAAAGGAGTGGAGAGGTTAAAAAGAATGGCCATAAG
TGGGGATGACTGCGTGGTGAAACCAATCGATGACAGGTTCGCTAATGCCCTGCTCGCTCTGAA
CGACATGGGAAAGGTTCGGAAAGACATACCTCAATGGCAGCCATCAAAGGGATGGCATGATT
GGCAACAGGTTCCTTTCTGCTCCCACCACTTTCATGAATTGATCATGAAAGATGGAAGAAAGTT
GGTGGTTCCCTGCAGACCCCAGGACGAACTAATAGGAAGGGCAAGAATCTCTCAAGGAGCGG
GATGGAGCCTTAGAGAAACCGCATGTCTGGGGAAAGCCTACGCTCAAATGTGGAGTCTCATGT
ATTTTCACAGAAGAGACCTCAGACTAGCATCCAACGCCATATGTTCAGCAGTACCAGTCCACT
GGGTCCCCACAAGTAGAACGACATGGTCTATTCACGCTCACCATCAGTGGATGACCACAGAAG
ACATGCTTACTGTCTGGAACAGAGTGTGGATCGAGGACAATCCATGGATGGAAGACAAAACTC
CAGTCACAACCTGGGAAAATGTTCCATATCTAGGGAAGAGAGAAGACCAATGGTGCGGATCA
CTTATTGGTCTCACTTCCAGAGCAACCTGGGCCCAGAACATACCCACAGCAATTCAACAGGTT
AGAAGCCTTATAGGCAATGAAGAGTTTCTGGACTACATGCCTTCAATGAAGAGATTTAGGAAG
GAGGAGGAGTCGGAGGGAGCCATTTGGTAAAACGTAGGAAGTGAAAAAGAGGTTAACTGTCA
GGCCATATTAAGCCACAGTACGGAAGAAGCTGTGCTGCCTGTGAGCCCCGTCCAAGGACGTTA
AAAGAAGAAGTCAGGCCCCAAAGCCACGGTTTGAGCAAACCGTGCTGCCTGTAGCTCCGTCGT
GGGGACGTAAAACCTGGGAGGCTGCAAACTGTGGAAGCTGTACGCACGGTGTAGCAGACTAG
CGGTTAGAGGAGACCCCTCCCATGACACAACGCAGCAGCGGGGCCCGAGCACTGAGGGAAGC
TGTACCTCCTTGCAAAGGACTAGAGGTTAGAGGAGACCCCCCGCAAATAAAAACAGCATATTG
ACGCTGGGAGAGACCAGAGATCCTGCTGTCTCCTCAGCATCATTCCAGGCACAGAACGCCAGA
AAATGGAATGGTGCTGTTGAATCAACAGGTTCT

SEQ ID NO: 6 - DENV-3-VE-BID-V2268-2008-E-Min

AGTTGTTAGTCTACGTGGACCGACAAGAACAGTTTCGACTCGGAAGCTTGCTTAACGTAGTGCT
AACAGTTTTTTATTAGAGAGCAGATCTCTGATGAACAACCAACGGAAGAAGACGGGAAAACC
GTCTATCAATATGCTGAAACGCGTGAGAAACCGTGTGTCAACTGGATCACAGTTGGCGAAGAG
ATTCTCAAAAGGACTGCTGAACGGCCAGGGACCAATGAAATTGGTTATGGCGTTCATAGCTTT
CCTCAGATTTCTAGCCATTCCACCAACAGCAGGAGTCTTGGCTAGATGGGGAACCTTCAAGAA
GTCGGGGGCCATTAAGGTCCTGAAAGGCTTTAAGAAGGAGATCTCAAACATGCTGAGCATAAT
CAACAAACGGAAAAAGACATCGCTCTGTCTCATGATGATATTGCCAGCAGCACTTGCTTTCCA
CTTGACTTCACGAGATGGAGAGCCGCGCATGATTGTGGGGAAGAATGAAAGAGGAAAATCCC
TACTTTTTAAGACAGCCTCTGGAATTAACATGTGCACACTCATAGCCATGGACTTGGGAGAGAT
GTGTGATGACACGGTCACTTACAAATGCCCCCATATTACCGAAGTGGAACCTGAAGACATTGA
CTGCTGGTGCAACCTCACATCAACATGGGTGACTTATGGAACGTGCAATCAAGCCGGAGAGCA
TAGACGCGATAAGAGATCAGTGGCGTTAGCTCCCCATGTCGGCATGGGACTGGATACACGCAC
CCAAACTTGGATGTCGGCTGAAGGAGCTTGGAGGCAAGTCGAGAAGGTAGAGACATGGGCCC
TTAGGCACCCAGGGTTCACCATACTAGCCCTATTTCTTGCCCATTACATAGGCACTTCCTTGAC
CCAGAAGGTGGTTATTTTTATACTGCTAATGCTGGTCACCCCATCCATGACAATGAGATGCGTG
GGCGTAGGGAATAGAGACTTTGTAGAGGGATTGAGCGGAGCGACATGGGTCGACGTCGTACT
CGAACACGGGGGGTGCGTGACGACTATGGCTAAGAATAAGCCTACACTCGATATCGAACTGCA
AAAAACCGAAGCGACACAATTGGCGACATTGCGCAAACTATGTATCGAAGGTAAGATTACGA
ACATTACAACCGATAGTAGATGCCCTACACAAGGCGAAGCCGTTCTACCTGAGGAGCAAGACC
AAAACTACGTATGTAAGCATACATACGTTGATAGGGGGTGGGGGAACGGATGCGGATTGTTCG
GTAAGGGGTCATTGGTCACATGCGCTAAGTTCCAATGCTTAGAGCCTATCGAGGGTAAGGTGG
TACAATACGAAAACCTTAAGTATACCGTGATAATTACAGTTCATACCGGAGACCAACACCAAG
TGGGAAACGAGACACAGGGAGTGACAGCCGAAATTACGCCTCAGGCTAGTACAACCGAAGCG
ATACTACCCGAATACGGAACATTGGGGTTGGAGTGTTCACCTAGAACCGGATTGGACTTTAAC
GAAATGATACTGTTGACTATGAAAAACAAGGCATGGATGGTGCATAGGCAATGGTTTTTTGAC
CTACCATTGCCTTGGACTAGCGGAGCGACAACCGAAACACCTACATGGAATAGAAAAGAGCTC
CTAGTGACATTTAAAAACGCTCACGCTAAGAAGCAGGAAGTGGTCGTTTTAGGGTCACAGGAG
GGAGCTATGCATACCGCACTAACCGGAGCGACTGAGATACAGAATAGCGGAGGGACATCAAT
CTTCGCCGGACACCTTAAGTGTAGATTGAAAATGGACAAACTTGAGCTTAAGGGGATGTCATA
CGCTATGTGTACGAACACATTCGTGCTGAAAAAAGAGGTAAGCGAAACGCAACACGGAACGA
TACTGATTAAGGTTGAGTATAAGGGCGAAGACGTCCCATGTAAGATACCTTTTTCGACAGAGG
ACGGACAGGGTAAGGCCCATAACGGAAGACTGATTACCGCTAACCCAGTGGTGACCAAAAAA
GAGGAACCAGTCAATATCGAAGCCGAACCTCCATTCGGAGAGTCAAACATAGTGATAGGGAT
AGGCGATAACGCACTTAAGATTAACTGGTACAAAAAAGGGTCATCAATCGGTAAGATGTTTGA
GGCAACCGCTAGGGGGGCTAGACGGATGGCCATACTCGGAGACACCGCATGGGACTTCGGAT

CCGTGGGGGGGGTACTTAACTCACTCGGTAAGATGGTGCACCAAATTTTCGGATCCGCATATA CCGCTCTATTTAGCGGAGTGTCATGGGTGATGAAAATCGGAATCGGAGTTCTATTGACATGGA TCGGATTGAACTCTAAGAATACATCTATGTCATTTTCATGTATCGCAATCGGAATTATTACGCT ATATCTCGGAGCCGTAGTGCAAGCCGACATGGGGTGTGTTATAAACTGGAAAGGCAAAGAACT CAAGTGTGGAAGTGGAATCTTCGTCACCAACGAGGTCCATACCTGGACAGAGCAATACAAATT CCAAGCAGACTCCCCAAAAAGATTGGCGACAGCCATTGCAGGCGCTTGGGAGAATGGAGTGT GCGGAATTAGGTCAACAACCAGAATGGAGAATCTCCTGTGGAAGCAAATAGCCAATGAACTG AACTACATATTGTGGGAAAACAATATCAAATTAACGGTTGTTGTGGGCGATATAATTGGGGTC TTAGAGCAAGGGAAAAGAACACTAACACCACAACCCATGGAGCTAAAATACTCATGGAAAAC GTGGGGAAAGGCAAAAATAGTGACAGCTGAAACACAAAATTCTTCTTTCATAATAGATGGACC AAACACACCGGAGTGTCCAAGTGCCTCAAGAGCATGGAATGTGTGGGAGGTGGAAGATTACG GGTTCGGAGTCTTCACAACCAACATATGGCTGAAACTCCGAGAGGTGTATACCCAACTATGTG ACCATAGGTTAATGTCGGCAGCCGTCAAGGATGAAAGGGCCGTACATGCCGACATGGGCTATT GGATAGAAAGTCAAAAGAATGGAAGTTGGAAGCTAGAAAAAGCATCCCTCATAGAGGTGAAA ACCTGCACATGGCCAAAATCACATACCCTTTGGAGTAATGGTGTGTTAGAGAGTGACATGATC ATTCCAAAAAGTCTAGCTGGTCCTATCTCGCAACACAACTACAGGCCCGGGTACCACACCCAG ACGGCGGGACCTTGGCATTTAGGAAAATTAGAGCTGGACTTCAACTATTGTGAAGGAACAACA GTTGTCATCACAGAAAACTGTGGGACAAGAGGCCCATCATTGAGAACAACAACAGTGTCAGG GAAGTTAATACACGAATGGTGCTGCCGCTCGTGCACACTTCCTCCCCTGCGATACATGGGAGA AGACGGTTGCTGGTATGGCATGGAAATCAGACCCATCAGTGAGAAAGAAGAAAACATGGTAA AGTCTTTAGTCTCAGCGGGAAGTGGAGAGGTGGACAACTTCACAATGGGTGTCTTGTGTTTGG CAATCCTCTTTGAAGAGGTGATGAGAGGAAAATTTGGGAAGAAACACATGATTGCGGGGGTTT TCTTCACGTTTGTACTCCTTCTCTCAGGGCAAATAACATGGAGAGATATGGCGCACACACTAAT AATGATTGGGTCCAATGCATCTGACAGGATGGGAATGGGCGTCACCTACCTAGCTTTAATTGC AACATTTAAAATCCAGCCATTTTTGGCTTTGGGATTTTTCCTAAGAAAACTGACATCCAGAGAA AATTTATTGTTAGGAGTTGGGCTGGCTATGGCAACAACGTTACAACTGCCAGAGGACATTGAA CAAATGGCAAATGGAATCGCTCTGGGGCTCATGGCTCTCAAACTGATAACACAATTTGAAACA TACCAACTATGGACAGCATTAATCTCCTTAACGTGTTCAAATACAATTTTTACGTTGACTGTTG CCTGGAGAACAGCCACCCTGATTTTGGCTGGAGTTTCACTTTTACCAGTGTGCCAGTCTTCGAG TATGAGGAAAACAGACTGGCTTCCAATGACAGTGGCAGCTATGGGAGTTCCACCTCTACCACT TTTTATTTTTAGCTTAAAAGACACACTCAAAAGGAGAAGCTGGCCACTGAATGAAGGGGTGAT GGCTGTTGGGCTTGTGAGCATTCTGGCCAGTTCTCTCCTTAGAAATGATGTACCCATGGCTGGA CCATTAGTGGCCGGGGGCTTGCTGATAGCGTGCTACGTCATAACTGGCACGTCAGCAGACCTC ACCGTAGAAAAAGCAGCAGATGTAACATGGGAGGAAGAGGCTGAGCAAACAGGAGTGTCCCA CAACTTAATGATCACAGTTGATGATGATGGAACAATGAGAATAAAAGATGATGAGACTGAGA ATATCTTAACAGTGCTTTTGAAAACAGCATTACTAATAGTGTCAGGAATCTTTCCATACTCCAT ACCCGCAACATTGTTGGTCTGGCATACTTGGCAAAAGCAAACCCAAAGATCCGGCGTTCTATG

GGATGTACCCAGCCCTCCAGAGACACAGAAAGCAGAACTGGAAGAAGGGGTCTATAGGATCA AACAGCAAGGAATTTTTGGGAAAACCCAAGTAGGGGTTGGAGTACAGAAAGAAGGAGTCTTT CACACCATGTGGCACGTTACAAGAGGGGCAGTGTTGACATATAATGGGAAAAGACTGGAACC AAATTGGGCTAGCGTGAAAAAAGATCTGATTTCATACGGAGGAGGATGGAGATTGAGCGCAC AATGGCAAAAGGGGGAGGAGGTGCAGGTTATTGCCGTAGAGCCTGGGAAGAACCCAAAGAAC TTTCAAACCATGCCAGGCACTTTTCAGACTACAACAGGGGAAATAGGAGCAATTGCACTGGAT TTCAAGCCCGGAACTTCAGGATCTCCTATCATAAACAGAGAGGGAAAGGTAGTGGGACTGTAT GGCAATGGAGTGGTTACAAAGAATGGTGGCTACGTCAGCGGAATAGCGCAAACGAATGCAGA ACCAGATGGACCGACACCAGAATTGGAAGAAGAGATGTTCAAAAAGCGAAATCTAACCATAA TGGATCTTCATCCTGGGTCAGGAAAGACACGGAAATACCTTCCAGCTATTGTTAGAGAGGCAA TCAAGAGACGTTTGAGAACTCTAATTCTGGCACCAACAAGGGTGGTTGCAGCTGAGATGGAAG AAGCATTGAAAGGGCTCCCAATAAGGTACCAAACAACAGCAACAAAATCTGAACACACAGGA AGAGAGATTGTTGATCTGATGTGCCACGCAACGTTCACAATGCGTCTGCTGTCACCAGTTAGG GTTCCAAACTATAACTTGATAATAATGGATGAAGCCCATTTCACAGACCCAGCCAGTATAGCT GCTAGAGGGTACATATCAACTCGTGTTGGAATGGGAGAAGCAGCCGCAATATTCATGACAGCA ACGCCCCCTGGAACAGCTGATGCCTTTCCCCAGAGCAACGCTCCAATTCAAGATGAAGAAAGG GACATACCAGAACGCTCATGGAATTCAGGCAATGAATGGATAACCGACTTCGCTGGGAAAAC GGTGTGGTTTGTCCCCAGCATTAAAGCCGGAAATGACATAGCAAATTGCCTGCGGAAAAACGG GAAAAAGGTCATTCAACTTAGTAGGAAGACTTTTGACACAGAATATCAGAAAACCAAACTGA ATGATTGGGACTTTGTGGTGACGACTGACATTTCAGAAATGGGGGCCAATTTCAAAGCAGATA GAGTGATCGACCCAAGAAGATGTCTCAAACCAGTGATCCTGACAGATGGACCAGAGCGGGTG ATCCTGGCTGGACCAATGCCAGTCACCGCAGCGAGTGCTGCGCAAAGGAGAGGGAGAGTTGG CAGGAACCCACAAAAAGAAAATGACCAGTACATATTCACGGGCCAGCCTCTCAACAATGATG AAGACCATGCTCACTGGACAGAAGCAAAAATGCTGCTGGACAACATTAACACACCAGAAGGG ATCATACCAGCTCTCTTTGAGCCAGAAAGGGAGAAGTCAGCCGCCATAGACGGTGAGTATCGC CTGAAAGGTGAGTCCAGGAAGACTTTCGTGGAACTCATGAGGAGGGGTGACCTTCCAGTCTGG TTAGCCCATAAAGTAGCATCAGAAGGGATCAAATATACAGATAGAAAATGGTGCTTTGATGGA CAACGTAATAATCAAATTTTAGAAGAGAACATGGATGTGGAAATCTGGACAAAGGAAGGAGA AAAGAAAAAATTGAGACCTAGGTGGCTTGATGCTCGCACCTATTCAGATCCCTTAGCACTCAA GGAATTCAAGGACTTTGCGGCTGGCAGGAAGTCAATAGCCCTTGATCTTGTGACAGAAATAGG AAGAGTGCCTTCACACCTAGCTCATAGAACGAGAAACGCTCTGGACAATCTGGTGATGCTGCA TACGTCAGAACATGGCGGTAAGGCCTACAGGCATGCGGTGGAGGAACTACCAGAGACAATGG AAACACTCCTACTCTTGGGACTCATGATCTTGTTGACAGGTGGAGCAATGCTTTTCTTAATATC AGGTAAAGGGATTGGAAAGACTTCAATAGGACTCATTTGTGTAATTGCTTCCAGCGGCATGTT GTGGATGGCCGAAATCCCACTCCAATGGATCGCGTCGGCCATAGTCCTGGAGTTTTTTATGATG GTGTTGCTTATACCAGAACCAGAGAAGCAGAGAACCCCCCAAGACAACCAACTCGCATATGTC GTGATAGGCATACTTACACTGGCAGCAATAATAGCAGCCAATGAAATGGGATTGTTGGAAACT

ACAAAGAGAGATTTAGGAATGTCTAAGGAGCCAGGTGTTGTTTCTCCAACCAGCTATTTAGAT
GTGGACTTGCACCCAGCATCAGCCTGGACATTGTACGCTGTGGCCACTACAGTAATAACACCA
ATGTTAAGACATACCATAGAGAATTCCACAGCAAATGTGTCCTTGGCAGCTATAGCCAACCAG
GCAGTGGTCCTGATGGGTTTGGACAAAGGATGGCCAATATCAAAAATGGACTTAGGAGTACCC
CTGCTGGCATTGGGTTGCTATTCACAAGTGAACCCACTGACTCTAACAGCGGCAGTACTCTTGC
TGATCACACATTATGCCATTATAGGTCCAGGATTGCAGGCAAAAGCCACCCGTGAAGCTCAGA
AAAGGACAGCTGCTGGAATAATGAAGAATCCAACAGTGGATGGGATAATGACAATAGACCTA
GATCCTGTAATATATGATTCAAAATTTGAAAAGCAACTGGGACAGGTTATGCTCCTGGTTTTGT
GTGCAGTTCAACTTTTGTTAATGAGAACATCATGGGCCTTGTGTGAAGCTTTAACCCTAGCTAC
AGGACCAATAACAACACTCTGGGAAGGATCACCTGGGAAGTTTTGGAACACCACGATAGCTGT
TTCCATGGCGAACATTTTTAGAGGGAGCTATTTAGCAGGAGCTGGGCTTGCTTTCTCTATTATG
AAATCAGTTGGAACAGGGAAAAGAGGAACAGGCTCACAGGGTGAAACTTTGGGAGAAAAATG
GAAAAAGAAGTTAAATCAATTATCCCGGAAAGAGTTTGACCTTTACAAGAAATCTGGAATCAC
TGAGGTGGATAGAACAGAAGCCAAAGAAGGGTTGAAAAGAGGAGAAATAACACATCATGCCG
TGTCCAGAGGTAGTGCAAAACTTCAATGGTTTGTGGAGAGAAACATGGTCATTCCCGAAGGAA
GAGTTATAGACTTGGGCTGTGGAAGAGGAGGCTGGTCATATTACTGTGCAGGACTGAAAAAAG
TCACAGAAGTGCGAGGATACACAAAAGGCGGTCCAGGACACGAAGAACCAGTACCTATGTCC
ACATATGGATGGAACATAGTTAAGTTAATGAGTGGAAAGGATGTGTTTTATCTTCCACCTGAA
AAGTGTGACACCCTGTTGTGCGACATTGGAGAATCTTCACCAAGCCCAACAGTGGAAGAAAGC
AGAACTATAAGAGTTTTGAAGATGGTTGAACCATGGCTAAAGAACAACCAATTTTGTATTAAA
GTATTGAACCCTTACATGCCAACTGTGATTGAGCACCTAGAAAGACTACAAAGGAAACATGGA
GGAATGCTTGTGAGAAATCCACTTTCACGAAACTCCACGCACGAAATGTACTGGATATCCAAT
GGCACAGGTAACATTGTCGCTTCAGTCAACATGGTATCTAGACTGCTACTGAACAGGTTCACG
ATGACACACAGAAGACCCACCATTGAGAAAGATGTGGATTTAGGAGCAGGAACTCGACATGT
TAATGCGGAACCAGAAACACCCAACATGGATGTCATTGGGGAAAGAATAAAAAGGATCAAGG
AGGAGCATAATTCAACATGGCACTACGATGACGAAAACCCCTACAAAACGTGGGCTTACCATG
GATCTTATGAAGTCAAAGCCACAGGCTCAGCCTCCTCCATGATAAATGGAGTCGTGAAACTCC
TCACTAAACCATGGGATGTGGTGCCCATGGTGACACAGATGGCAATGACAGATACAACTCCAT
TTGGCCAGCAGAGAGTCTTTAAAGAGAAAGTGGACACCAGGACACCCAGGTCCATGCCAGGA
ACAAGAAGGGTTATGGGGATCACAGCGGAGTGGCTCTGGAGAACCCTGGGAAGGAATAAAAA
ACCCAGGTTATGCACAAGGGAAGAGTTTACAAAAAAGGTCAGAACTAACGCAGCCATGGGAG
CTGTTTTCACAGAGGAGAACCAATGGGACAGCGCGAAAGCTGCTGTTGAGGATGAGGATTTTT
GGAAACTTGTGGACAGAGAACGTGAACTCCACAAACTGGGCAAGTGTGGAAGCTGTGTTTACA
ACATGATGGGTAAGAGAGAGAAGAAACTTGGAGAGTTTGGCAAAGCAAAAGGCAGTAGAGCT
ATATGGTACATGTGGTTGGGAGCCAGGTACCTTGAGTTCGAAGCCCTTGGATTCTTAAATGAA
GACCACTGGTTCTCGCGTGAGAACTCTTACAGTGGAGTGGAAGGAGAAGGACTGCACAAGCTA
GGCTATATATTAAGGGACATTTCCAAGATACCCGGAGGAGCTATGTATGCTGATGACACAGCT

GGTTGGGACACAAGAATAACAGAAGATGACCTGCACAATGAGGAAAAGATCACACAGCAAATGGACCCTGAACACAGGCAGTTAGCGAACGCTATATTTAAGCTCACATACCAAAACAAAGTGGTCAAAGTTCAACGACCGACTCCAACAGGCACGGTAATGGATATCATATCTAGGAAAGACCAAAGAGGCAGTGGACAGGTAGGAACTTATGGTCTGAATACATTCACCAACATGGAAGCCCAGTTAATCAGACAAATGGAAGGAGAAGGTGTGCTGTCAAAGGCAGACCTCGAGAACCCTCATCTGCCAGAGAAGAAAATTACACAATGGTTGGAAACCAAAGGAGTGGAGAGGTTAAAAAGAATGGCCATAAGTGGGGATGACTGCGTGGTGAAACCAATCGATGACAGGTTCGCTAATGCCCTGCTCGCTCTGAACGACATGGGAAAGGTTCGGAAAGACATACCTCAATGGCAGCCATCAAAGGGATGGCATGATTGGCAACAGGTTCCTTTCTGCTCCCACCACTTTCATGAATTGATCATGAAAGATGGAAGAAAGTTGGTGGTTCCCTGCAGACCCCAGGACGAACTAATAGGAAGGGCAAGAATCTCTCAAGGAGCGGGATGGAGCCTTAGAGAAACCGCATGTCTGGGGAAAGCCTACGCTCAAATGTGGAGTCTCATGTATTTTCACAGAAGAGACCTCAGACTAGCATCCAACGCCATATGTTCAGCAGTACCAGTCCACTGGGTCCCCACAAGTAGAACGACATGGTCTATTCACGCTCACCATCAGTGGATGACCACAGAAGACATGCTTACTGTCTGGAACAGAGTGTGGATCGAGGACAATCCATGGATGGAAGACAAAACTCCAGTCACAACCTGGGAAAATGTTCCATATCTAGGGAAGAGAGAAGACCAATGGTGCGGATCACTTATTGGTCTCACTTCCAGAGCAACCTGGGCCCAGAACATACCCACAGCAATTCAACAGGTTAGAAGCCTTATAGGCAATGAAGAGTTTCTGGACTACATGCCTTCAATGAAGAGATTTAGGAAGGAGGAGGAGTCGGAGGGAGCCATTTGGTAAAACGTAGGAAGTGAAAAAGAGGTTAACTGTCAGGCCATATTAAGCCACAGTACGGAAGAAGCTGTGCTGCCTGTGAGCCCCGTCCAAGGACGTTAAAAGAAGAAGTCAGGCCCCAAAGCCACGGTTTGAGCAAACCGTGCTGCCTGTAGCTCCGTCGTGGGGACGTAAAACCTGGGAGGCTGCAAACTGTGGAAGCTGTACGCACGGTGTAGCAGACTAGCGGTTAGAGGAGACCCCTCCCATGACACAACGCAGCAGCGGGGCCCGAGCACTGAGGGAAGCTGTACCTCCTTGCAAAGGACTAGAGGTTAGAGGAGACCCCCCGCAAATAAAAACAGCATATTGACGCTGGGAGAGACCAGAGATCCTGCTGTCTCCTCAGCATCATTCCAGGCACAGAACGCCAGAAAATGGAATGGTGCTGTTGAATCAACAGGTTCT

SEQ ID NO:7 - DENV4_Syn_WT

AGTTGTTAGTCTGTGTGGACCGACAAGGACAGTTCCAAATCGGAAGCTTGCTTAACACAGTTCTAACAGTTTGTTTAAATAGAGAGCAGATCTCTGGAAAAATGAACCAACGAAAAAAGGTGGTTAGACCACCTTTCAATATGCTGAAACGCGAGAGAAACCGCGTATCAACCCCTCAAGGGTTGGTGAAGAGATTCTCAACCGGACTTTTTTCTGGGAAAGGACCCTTACGGATGGTGCTAGCATTCATCACGTTTTTGCGAGTCCTTTCCATCCCACCAACAGCAGGGATTCTGAAGAGATGGGGACAGTTGAAGAAAAATAAGGCCATCAAGATACTGATTGGATTCAGGAAGGAGATAGGCCGCATGCTGAACATCTTGAACGGGAGAAAAAGGTCAACGATAACATTGTTGTGCTTGATTCCCACCGTAATGGCGTTTCACTTGTCAACAAGAGATGGCGAACCCCTCATGATAGTGGCAAAACATGAAAGGGGGAGACCTCTCTTGTTTAAGACAACAGAGGGGATCAACAAATGCACTCTCATTGCCATGGACTTGGGTGAAATGTGTGAGGACACTGTCACGTATAAATGCCCCCTACTGGTCAATACCGAACCTGAAGACA

TTGATTGCTGGTGCAACCTCACGTCTACCTGGGTCATGTATGGGACATGCACCCAGAGCGGAG
AACGGAGACGAGAGAAGCGCTCAGTAGCTTTGACACCACATTCAGGAATGGGATTGGAAACA
AGAGCTGAGACATGGATGTCATCGGAAGGGGCTTGGAAGCATGCTCAGAGAGTAGAGAGCTG
GATACTCAGAAACCCAGGATTTGCGCTCTTGGCAGGATTTATGGCTTATATGATTGGGCAAAC
AGGAATCCAGCGAACTGTCTTCTTTGTCCTAATGATGCTGGTCGCCCCATCCTACGGAATGCGG
TGCGTAGGAGTAGGAAACAGAGACTTTGTGGAAGGAGTCTCAGGTGGAGCATGGGTCGACCT
GGTGCTAGAACATGGAGGATGCGTCACAACCATGGCCCAGGGAAAACCAACCTTGGATTTTGA
ACTGACTAAGACAACAGCTAAGGAAGTGGCTCTGTTAAGAACCTATTGCATTGAAGCCTCAAT
ATCAAACATAACTACGGCAACAAGATGTCCAACGCAAGGAGAGCCTTATCTGAAAGAGGAAC
AGGACCAACAGTACATCTGCCGGAGAGATGTGGTAGACAGAGGATGGGGCAATGGCTGTGGC
TTGTTTGGAAAAGGAGGAGTTGTGACATGTGCGAAGTTTTCATGTTCGGGGAAGATAACAGGC
AATCTGGTCCAAATTGAGAACCTTGAATACACAGTGGTTGTAACAGTCCACAATGGAGACACC
CATGCAGTAGGAAATGACACATCCAATCATGGAGTTACAGCCACGATAACTCCCAGGTCACCA
TCGGTAGAAGTCAAATTGCCGGACTATGGAGAACTAACACTCGATTGTGAACCCAGGTCTGGA
ATTGACTTTAATGAGATGATCCTAATGAAAATGAAAAAGAAAACATGGCTCGTGCATAAGCAA
TGGTTTTTGGATCTGCCTCTTCCATGGACAGCAGGAGCAGACACATCAGAGGTTCACTGGAATT
ACAAAGAGAGAATGGTGACGTTCAAGGTTCCTCATGCCAAGAGACAGGATGTGACAGTGCTG
GGATCTCAGGAAGGAGCCATGCATTCTGCCCTCGCTGGAGCCACAGAAGTGGACTCCGGTGAT
GGAAATCACATGTTTGCAGGACATCTCAAGTGCAAAGTCCGTATGGAGAAATTGAGAATCAAG
GGAATGTCATACACGATGTGTTCAGGAAAGTTTTCAATTGACAAAGAGATGGCAGAAACACAG
CATGGGACAACAGTGGTGAAAGTCAAGTATGAAGGTGCTGGAGCTCCGTGTAAAGTCCCCATA
GAGATAAGAGATGTAAACAAGGAAAAAGTGGTTGGGCGCGTTATCTCATCCACCCCTTTGGCT
GAGAATACCAACAGTGTAACCAACATAGAATTAGAACCCCCCTTTGGGGACAGCTACATTGTG
ATAGGTGTTGGAAACAGCGCATTAACACTCCATTGGTTCAGGAAAGGGAGTTCCATTGGCAAG
ATGTTTGAGTCCACATACAGAGGTGCAAAACGAATGGCCATTCTAGGTGAAACAGCTTGGGAT
TTTGGTTCCGTTGGTGGACTGTTCACATCATTGGGAAAGGCTGTACACCAGGTTTTTGGAAGCG
TGTATACAACCATGTTTGGAGGAGTCTCATGGATGATTAGAATCCTAATTGGGTTCTTAGTGTT
GTGGATTGGCACGAATTCAAGGAACACTTCAATGGCCATGACGTGCATAGCTGTTGGAGGAAT
TACTCTGTTTTTGGGCTTCACAGTTCAAGCGGACATGGGTTGTGTGGTGTCATGGAGTGGGAGA
GAATTGAAGTGTGGAAGCGGAATTTTTGTGGTTGACAACGTGCACACTTGGACAGAACAGTAC
AAATTCCAACCAGAGTCCCCAGCGAGACTAGCGTCTGCAATATTAAATGCCCACAAAGATGGG
GTCTGTGGAATTAGATCAACCACGAGGCTGGAAAATGTTATGTGGAAGCAAATAACCAATGAG
CTAAACTATGTTCTCTGGGAAGGAGGACATGATCTCACTGTAGTGGCTGGGGATGTGAAAGGG
GTGTTGACCAAGGGCAAGAGAGCACTCACACCCCCAGCGAGTGATCTGAAATATTCATGGAAG
ACATGGGGGAAAGCAAAAATCTTCACCCCTGAAGCAAGAAACAGCACATTTTTAATAGACGG
ACCAGACACCTCTGAATGCCCCAATGAACGAAGGGCATGGAATTCTTTTGAGGTGGAAGACTA
TGGATTTGGCATGTTCACGACCAACATATGGATGAAATTCCGAGAAGGAAGTTCAGAAGTGTG

TGACCACAGGTTAATGTCAGCTGCAATTAAAGACCAGAAAGCTGTGCATGCTGACATGGGTTATTGGATAGAGAGCTCAAAAAACCAGACCTGGCAGATAGAGAGAGCATCTCTTATTGAAGTGAAAACATGTCTGTGGCCCAAGACCCATACACTGTGGAGCAATGGAGTGCTGGAAAGCCAGATGCTTATTCCAAAATCATATGCAGGCCCTTTTTCACAGCACAATTACCGCCAGGGCTATGCTACGCAAACCGTGGGTCCATGGCACTTAGGCAAACTAGAGATAGACTTTGGAGAATGCCCCGGAACAACAGTCACAATTCAGGAGAATTGTGACCATAGAGGCCCATCTTTGAGGACCACCACTGCATCTGGAAAACTAGTCACGCAATGGTGTTGCCGCTCCTGCACGATGCCCCCCTTAAGGTTCTTAGGAGAAGATGGGTGCTGGTATGGGATGGAGATTAGGCCCTTGAGTGAAAAAGAAGAGAACATGGTCAAATCACAGGTGACGGCCGGACAGGGCACATCGGAAACTTTTTCAATGGGTCTGTTGTGCCTGACCTTGTTTGTGGAAGAATGCTTGAGGAGAAGAGTCACCAGGAAACACATGATATTAGCTGTGGTAATCACTCTTTGTGCTATCATCCTGGGGGGCCTCACATGGATGGACTTGCTACGAGCCCTCATCATGTTGGGGGACACTATGTCTGGTAGAATAGGAGGACAGACCCACCTAGCCATCATGGCAGTGTTCAAGATGTCACCGGGATACGTGCTGGGTGTGTTTTTAAGGAAACTCACTTCAAGAGAGACAGCACTAATGGTAATAGGAATGGCCATGACAACAACACTTTCAATTCCACATGACCTCATGGAACTCATTGATGGAATATCACTAGGACTAATTTTGCTAAAAATAGTAACACAGTTTGACAACACCCAAGTGGGAACCTTAGCTCTTTCCTTGACTTTCATAAGATCAACAATGTCATTGGTCATGGCTTGGAGGACCATTATGGCTGTGTTGTTTGTGGTCACACTCATTCCTTTGTGCAGGACAAGCTGTCTTCAAAAACAGTCTCATTGGGTAGAAATAACAGCACTCATCCTAGGAGCCCAAGCTCTGCCAGTGTACCTAATGACTCTTATGAAAGGAGCCTCAAGAAGATCTTGGCCTCTTAACGAAGGCATAATGGCTGTGGGTTTGGTTAGTCTCTTAGGAAGCGCTCTTTTAAAGAATGATGTCCCTTTAGCTGGCCCAATGGTGGCAGGAGGCTTACTTCTGGCGGCTTACGTAATGAGTGGCAGCTCAGCAGATCTGTCACTAGAGAAGGCCGCTAATGTGCAGTGGGATGAAATGGCAGACATAACAGGCTCAAGTCCAATCATAGAAGTGAAGCAAGATGAGGATGGCTCTTTCTCCATACGGGACGTCGAGGAAACCAATATGATAACCCTTTTGGTGAAACTGGCACTGATAACGGTGTCAGGTCTCTACCCCTTGGCAATTCCAATCACAATGACCTTATGGTACATGTGGCAAGTGAAAACACAAAGATCAGGAGCCCTGTGGGACGTCCCTTCACCCGCCGCCACTCAAAAAGCCGCACTGTCTGAAGGAGTGTACAGGATCATGCAAAGAGGGTTATTCGGGAAAACTCAGGTTGGAGTAGGGATACACATGGAAGGTGTATTTCACACAATGTGGCATGTTACAAGAGGATCGGTGATCTGCCACGAGACTGGGAGATTGGAGCCATCTTGGGCTGATGTCAGGAATGACATGATATCATACGGTGGGGGATGGAGGCTTGGAGATAAATGGGACAAAGAAGAAGACGTTCAGGTCCTCGCTATAGAACCAGGGAAAAATCCCAAACATGTCCAAACGAAACCTGGCCTTTTCAAGACCCTAACTGGAGAAATTGGAGCAGTAACATTAGATTTCAAACCCGGAACGTCTGGTTCTCCCATTATCAACAGGAAAGGAAAAGTCATCGGACTCTATGGAAATGGAGTGGTCACCAAATCAGGTGATTACGTCAGTGCCATAACACAAGCCGAAAGAATTGGAGAGCCAGATTATGAAGTGGATGAGGACATTTTTCGAAAGAAAAGACTAACTATAATGGACTTACACCCCGGAGCCGGAAAGACAAAAAGAATTCTTCCATCAATAGTGAGAGAAGCCTTAAAAAGGAGGCTGCGAACTTTGATTTTGGCTCCCACGAGAGTGGTGGCGGCCGAGATGGAAGAGGCCCTACGTGGACTGCCAATCCGTTACCAAACCCCAGCTGTAAAATCAGAACACACAGGAAGAGAGA

TTGTAGACCTCATGTGCCATGCAACCTTCACAACAAGACTTTTGTCATCAACCAGAGTTCCAAA CTACAACCTTATAGTAATGGATGAAGCACATTTCACCGATCCTTCCAGTGTCGCGGCTAGAGG ATACATTTCGACCAGGGTGGAAATGGGAGAGGCAGCAGCCATCTTCATGACCGCAACCCCTCC CGGAGCGACAGATCCCTTTCCCCAGAGCAACAGCCCAATAGAAGACATCGAGAGAGAGATTC CGGAAAGGTCATGGAACACAGGGTTCGACTGGATAACAGACTACCAAGGGAAAACTGTGTGG TTTGTTCCCAGCATAAAAGCTGGAAATGACATTGCAAATTGTTTGAGAAAGTCGGGAAAGAAA GTTATCCAGTTGAGTAGGAAAACCTTTGATACAGAATATCCAAAAACGAAACTCACGGACTGG GACTTTGTGGTCACTACAGACATATCTGAAATGGGGGCTAACTTTAGAGCTGGGAGAGTGATA GACCCTAGAAGATGCCTCAAGCCAGTTATCCTAACAGATGGGCCAGAGAGAGTCATCTTAGCA GGTCCTATTCCAGTGACTCCAGCAAGCGCTGCTCAGAGAAGAGGGCGAATAGGAAGGAACCC AGCACAAGAAGACGACCAATACGTTTTCTCCGGAGACCCACTAAAAAATGATGAAGACCATG CCCACTGGACAGAAGCAAAGATGCTGCTTGACAATATCTACACCCCAGAAGGGATCATTCCAA CATTGTTTGGTCCGGAAAGGGAAAAAACCCAAGCTATTGATGGAGAGTTTCGCCTCAGAGGGG AACAAAGGAAGACTTTTGTGGAATTAATGAGGAGAGGAGACCTTCCGGTGTGGCTGAGTTATA AGGTAGCTTCTGCTGGCATTTCTTACAAAGATCGGGAATGGTGCTTTACTGGGGAAAGAAATA ACCAAATTTTAGAAGAAAACATGGAGGTTGAAATTTGGACTAGAGAGGGAGAAAAGAAAAAA CTGAGGCCAAAATGGTTAGATGCACGTGTATACGCTGACCCCATGGCTTTGAAGGATTTCAAG GAGTTTGCCAGTGGAAGGAAGAGTATAACTCTCGACATCCTAACAGAGATCGCCAGTTTGCCA ACTTACCTTTCCTCTAGGGCCAAGCTCGCCCTTGACAACATAGTTATGCTCCACACAACAGAAA GAGGAGGGAGGGCCTATCAACATGCCCTGAACGAACTTCCGGAGTCACTGGAAACACTCATGC TTGTAGCCTTACTAGGAGCTATGACAGCAGGTATCTTCCTGTTTTTCATGCAAGGGAAAGGAAT AGGGAAATTGTCAATGGGTTTGATAACCATTGCGGTGGCTAGTGGCTTGCTCTGGGTAGCAGA AATTCAACCCCAGTGGATAGCGGCCTCAATCATACTGGAGTTTTTTCTCATGGTACTGTTGATA CCAGAACCAGAAAAACAAAGGACCCCACAAGACAATCAATTGATCTACGTCATATTGACCATT CTCACCATTATTGGTCTAATAGCAGCCAACGAGATGGGGCTGATAGAAAAAACAAAAACGGA TTTTGGGTTTTACCAGGTAAAAACAGAAACCACCATCCTCGATGTGGACCTGAGACCAGCTTC AGCATGGACGCTCTATGCGGTAGCCACCACAATTCTGACTCCCATGCTGAGACACACCATAGA AAATACGTCGGCCAACCTATCTTTAGCAGCCATCGCCAACCAGGCAGCCGTCCTAATGGGGCT TGGAAAAGGATGGCCACTCCACAGAATGGACCTCGGTGTGCCGCTGTTAGCAATGGGATGCTA TTCTCAAGTGAACCCAACAACCTTGACAGCATCCTTAGTCATGCTTTTAGTCCATTATGCAATA ATAGGCCCAGGATTGCAGGCAAAAGCCACAAGAGAGGCCCAGAAAAGGACAGCTGCTGGAAT CATGAAAAATCCCACAGTGGACGGGATAACAGTGATAGATCTAGAACCAATATCCTATGACCC AAAATTTGAAAAGCAATTAGGGCAGGTCATGTTACTCGTCTTGTGTGCTGGACAACTACTCTTG ATGAGAACAACATGGGCTTTCTGTGAAGTTTTGACTTTGGCCACAGGACCAATCTTGACCTTGT GGGAGGGCAACCCGGGAAGGTTTTGGAACACGACCATAGCCGTATCTACCGCCAACATTTTCA GGGGAAGTTATTTGGCAGGAGCTGGACTGGCTTTTTCACTCATAAAGAATGCACAAACCCCCA GGAGGGGAACTGGGACCACAGGAGAGACACTGGGAGAGAAGTGGAAGAGACAGCTAAACTC

ATTAGACAGGAAAGAGTTTGAAGAGTATAAAAGAAGTGGAATACTAGAAGTGGACAGGACTG
AAGCCAAGTCCGCCCTGAAAGATGGGTCTAAAATCAAGCATGCAGTATCTAGAGGGTCCAGTA
AGATCAGATGGATTGTTGAGAGAGGGATGGTAAAGCCAAAGGGGAAAGTTGTAGATCTTGGC
TGTGGGAGAGGAGGATGGTCTTATTACATGGCGACACTCAAGAACGTGACTGAAGTGAAAGG
GTATACAAAAGGAGGTCCAGGACATGAAGAACCGATTCCCATGGCTACTTATGGCTGGAATTT
GGTCAAACTCCATTCAGGGGTTGACGTGTTCTACAAACCCACAGAGCAAGTGGACACCCTGCT
CTGTGATATTGGGGAGTCATCTTCTAATCCAACAATAGAGGAAGGAAGAACATTAAGAGTTTT
GAAGATGGTGGAGCCATGGCTCTCTTCAAAACCTGAATTCTGCATCAAAGTCCTCAACCCCTA
CATGCCAACAGTCATAGAGGAGCTGGAGAAACTGCAGAGAAAACACGGTGGGAACCTTGTCA
GATGCCCGCTGTCCAGGAACTCCACCCATGAGATGTATTGGGTGTCAGGAGCGTCGGGAAATA
TTGTGAGCTCTGTGAACACAACATCAAAGATGTTGTTGAACAGGTTCACAACAAGGCATAGGA
AACCCACTTATGAGAAGGACGTAGATCTTGGGGCAGGAACGAGAAGTGTCTCTACTGAAACA
GAAAAACCAGACATGACAATCATTGGGAGAAGGCTTCAGCGATTGCAAGAGGAGCACAAAGA
AACATGGCATTATGATCAGGAAAACCCATACAGAACCTGGGCGTATCATGGAAGCTATGAAGC
TCCTTCGACAGGCTCTGCATCCTCCATGGTGAACGGGGTGGTGAAACTGCTAACAAAACCCTG
GGATGTGATTCCAATGGTGACTCAGTTAGCCATGACAGATACAACCCCTTTTGGGCAACAAAG
AGTGTTCAAAGAGAAGGTGGATACCAGAACGCCGCAACCAAAACCAGGCACACGAATGGTTA
TGACCACGACAGCCAATTGGCTATGGGCCCTCCTTGGAAAGAAGAAAAATCCCAGACTGTGTA
CAAGGGAAGAGTTCATCTCAAAAGTTAGATCAAACGCAGCCATAGGCGCAGTCTTTCAGGAAG
AACAAGGATGGACATCAGCCAGTGAAGCTGTGAATGACAGCCGGTTTTGGGAACTGGTTGACA
AAGAAAGGGCCCTTCACCAGGAAGGGAAATGTGAATCGTGTGTCTATAACATGATGGGAAAA
CGTGAGAAAAAGTTAGGAGAGTTTGGCAGAGCCAAGGGAAGCCGAGCAATCTGGTACATGTG
GCTGGGAGCGCGGTTTCTGGAATTTGAAGCCCTGGGTTTTTTGAATGAAGATCACTGGTTTGGC
AGAGAAAATTCATGGAGTGGAGTGGAAGGGGAAGGTCTGCACAGATTGGGATACATCCTGGA
GGAGATAGACAAGAAGGATGGAGACCTAATGTATGCTGATGACACAGCAGGTTGGGACACAA
GAATCACTGAGGATGACCTTCAAAATGAGGAACTGATCACGGAACAGATGGCTCCCCACCACA
AGATCCTAGCCAAAGCCATTTTCAAACTAACCTATCAAAACAAAGTGGTGAAAGTCCTCAGAC
CCACACCGAGAGGAGCGGTGATGGATATCATATCCAGGAAAGACCAAAGAGGTAGTGGACAA
GTTGGAACATATGGTTTGAACACATTCACCAACATGGAAGTTCAACTCATCCGCCAAATGGAA
GCTGAAGGAGTCATCACACAAGATGACATGCAGAACCCAAAAGGGTTGAAAGAAAGAGTTGA
GAAATGGCTGAGAGAGTGTGGTGTCGACAGGTTAAAGAGGATGGCAATCAGTGGAGACGATT
GCGTGGTGAAGCCCCTAGATGAGAGGTTTGGCACTTCCCTCCTCTTCTTGAACGACATGGGAA
AGGTGAGGAAAGACATTCCGCAGTGGGAACCATCTAAGGGATGGAAAAACTGGCAAGAGGTT
CCTTTTTGCTCCCATCACTTTCACAAGATCTTTATGAAGGATGGCCGCTCACTAGTTGTTCCATG
TAGAAACCAGGATGAACTGATAGGGAGAGCCAGAATCTCGCAGGGGGCTGGATGGAGCTTAA
GAGAAACAGCCTGCCTGGGCAAAGCTTACGCCCAGATGTGGTCGCTTATGTACTTCCACAGAA
GGGATCTGCGTTTAGCCTCCATGGCCATATGCTCAGCAGTTCCAACGGAATGGTTTCCAACAAG

CAGAACAACATGGTCAATCCATGCTCATCACCAATGGATGACCACTGAAGACATGCTCAAAGT
GTGGAACAGAGTGTGGATAGAAGACAACCCCAATATGATTGACAAGACTCCAGTCCATTCGTG
GGAAGATATACCTTACCTAGGGAAAAGAGAGGATTTGTGGTGTGGATCCCTGATTGGACTTTC
TTCTAGAGCCACCTGGGCGAAGAACATTCACACGGCCATAACTCAGGTCAGGAACTTGATCGG
AAAAGAGGAATACGTGGATTACATGCCAGTAATGAAAAGATACAGTGCTCCTTCAGAGAGTG
AAGGAGTTCTGTAATTACCAACAACAAACACCAAAGGCTATTGAAGTCAGGCCACTTGTGCCA
CGGTTTGAGCAAACCGTGCTGCCTGTAGCTCCGCCAATAATGGGAGGCGTAATAATCCCTAGG
GAGGCCATGCGCCACGGAAGCTGTACGCGTGGCATATTGGACTAGCGGTTAGAGGAGACCCCT
CCCATCACTGACAAAACGCAGCAAAAGGGGGCCCGAAGCCAGGAGGAAGCTGTACTCCTGGT
GGAAGGACTAGAGGTTAGAGGAGACCCCCCCAACACAAAAACAGCATATTGACGCTGGGAAA
GACCAGAGATCCTGCTGTCTCTACAACATCAATCCAGGCACAGAGCGCCGCAAGATGGATTGG
TGTTGTTGATCCAACAGGTTCT

SEQ ID NO:8 - DENV-4-EMin

AGTTGTTAGTCTGTGTGGACCGACAAGGACAGTTCCAAATCGGAAGCTTGCTTAACACAGTTCT
AACAGTTTGTTTAAATAGAGAGCAGATCTCTGGAAAAATGAACCAACGAAAAAAGGTGGTTA
GACCACCTTTCAATATGCTGAAACGCGAGAGAAACCGCGTATCAACCCCTCAAGGGTTGGTGA
AGAGATTCTCAACCGGACTTTTTTCTGGGAAAGGACCCTTACGGATGGTGCTAGCATTCATCAC
GTTTTTGCGAGTCCTTTCCATCCCACCAACAGCAGGGATTCTGAAGAGATGGGGACAGTTGAA
GAAAAATAAGGCCATCAAGATACTGATTGGATTCAGGAAGGAGATAGGCCGCATGCTGAACA
TCTTGAACGGGAGAAAAAGGTCAACGATAACATTGTTGTGCTTGATTCCCACCGTAATGGCGT
TTCACTTGTCAACAAGAGATGGCGAACCCCTCATGATAGTGGCAAAACATGAAAGGGGGAGA
CCTCTCTTGTTTAAGACAACAGAGGGGATCAACAAATGCACTCTCATTGCCATGGACTTGGGTG
AAATGTGTGAGGACACTGTCACGTATAAATGCCCCCTACTGGTCAATACCGAACCTGAAGACA
TTGATTGCTGGTGCAACCTCACGTCTACCTGGGTCATGTATGGGACATGCACCCAGAGCGGAG
AACGGAGACGAGAGAAGCGCTCAGTAGCTTTGACACCACATTCAGGAATGGGATTGGAAACA
AGAGCTGAGACATGGATGTCATCGGAAGGGGCTTGGAAGCATGCTCAGAGAGTAGAGAGCTG
GATACTCAGAAACCCAGGATTTGCGCTCTTGGCAGGATTTATGGCTTATATGATTGGGCAAAC
AGGAATCCAGCGAACTGTCTTCTTTGTCCTAATGATGCTGGTCGCCCCATCCTACGGAATGAGA
TGCGTAGGCGTAGGTAATCGCGATTTCGTTGAGGGAGTGTCAGGCGGAGCATGGGTCGATCTG
GTACTCGAACACGGAGGATGCGTTACGACTATGGCACAGGGAAAACCGACATTGGACTTTGAG
TTGACTAAGACAACCGCTAAAGAGGTCGCACTATTGCGAACATACTGTATCGAAGCGTCAATT
TCGAATATTACAACCGCTACTAGGTGTCCTACACAGGGCGAACCATACCTTAAAGAGGAACAG
GACCAACAGTATATTTGTAGAAGAGACGTAGTCGATAGGGGGTGGGGAAACGGATGCGGATT
GTTCGGTAAGGGGGGAGTCGTTACATGCGCTAAGTTCTCATGTTCCGGTAAGATTACCGGTAA
CTTAGTGCAAATTGAGAATCTCGAATATACCGTAGTCGTTACAGTGCATAACGGAGACACACA
CGCAGTCGGAAACGATACATCTAACCATGGCGTAACCGCTACAATTACACCTAGGTCACCATC

CGTTGAGGTTAAGTTGCCCGATTACGGCGAACTGACACTCGATTGCGAACCTAGATCCGGAAT
AGACTTTAACGAAATGATACTGATGAAAATGAAAAAAAAGACATGGTTAGTGCATAAGCAAT
GGTTTCTCGACTTACCCCTACCTTGGACCGCCGGAGCCGATACTAGCGAAGTGCACTGGAATT
ACAAAGAGAGAATGGTTACATTTAAAGTGCCACACGCTAAGAGACAGGACGTTACAGTGTTA
GGGTCACAGGAGGGAGCTATGCATAGCGCACTAGCCGGAGCAACCGAAGTCGATAGCGGAGA
CGGAAATCATATGTTCGCCGGACATCTGAAATGCAAAGTGAGAATGGAGAAATTGCGGATTAA
GGGAATGTCATACACTATGTGTTCCGGTAAGTTTTCGATTGACAAAGAGATGGCCGAAACGCA
ACACGGAACAACAGTCGTTAAGGTTAAGTACGAAGGAGCCGGAGCTCCGTGTAAAGTGCCAA
TCGAAATTAGAGACGTTAACAAAGAGAAAGTGGTCGGAAGAGTGATATCTAGTACACCCCTA
GCCGAAAATACGAATTCCGTTACGAATATCGAACTCGAACCCCCCTTTGGAGACTCATACATA
GTGATAGGAGTGGGTAATTCCGCACTCACGTTGCACTGGTTCAGAAAGGGATCATCAATCGGG
AAGATGTTCGAATCAACATATAGGGGAGCGAAAAGAATGGCTATATTGGGCGAAACCGCATG
GGACTTCGGATCAGTCGGAGGACTGTTTACGAGTCTGGGTAAGGCCGTACATCAGGTATTCGG
ATCAGTGTATACAACAATGTTTGGCGGAGTGTCATGGATGATACGGATACTGATAGGGTTTCT
AGTGTTGTGGATCGGAACGAACTCACGTAATACGTCTATGGCTATGACATGTATTGCCGTAGG
GGGGATTACACTGTTTCTGGGATTTACAGTGCAAGCAGACATGGGTTGTGTGGTGTCATGGAG
TGGGAGAGAATTGAAGTGTGGAAGCGGAATTTTTGTGGTTGACAACGTGCACACTTGGACAGA
ACAGTACAAATTCCAACCAGAGTCCCCAGCGAGACTAGCGTCTGCAATATTAAATGCCCACAA
AGATGGGGTCTGTGGAATTAGATCAACCACGAGGCTGGAAAATGTTATGTGGAAGCAAATAA
CCAATGAGCTAAACTATGTTCTCTGGGAAGGAGGACATGATCTCACTGTAGTGGCTGGGGATG
TGAAAGGGGTGTTGACCAAGGGCAAGAGAGCACTCACACCCCCAGCGAGTGATCTGAAATAT
TCATGGAAGACATGGGGGAAAGCAAAAATCTTCACCCCTGAAGCAAGAAACAGCACATTTTTA
ATAGACGGACCAGACACCTCTGAATGCCCCAATGAACGAAGGGCATGGAATTCTTTTGAGGTG
GAAGACTATGGATTTGGCATGTTCACGACCAACATATGGATGAAATTCCGAGAAGGAAGTTCA
GAAGTGTGTGACCACAGGTTAATGTCAGCTGCAATTAAAGACCAGAAAGCTGTGCATGCTGAC
ATGGGTTATTGGATAGAGAGCTCAAAAAACCAGACCTGGCAGATAGAGAGAGCATCTCTTATT
GAAGTGAAAACATGTCTGTGGCCCAAGACCCATACACTGTGGAGCAATGGAGTGCTGGAAAG
CCAGATGCTTATTCCAAAATCATATGCAGGCCCTTTTTCACAGCACAATTACCGCCAGGGCTAT
GCTACGCAAACCGTGGGTCCATGGCACTTAGGCAAACTAGAGATAGACTTTGGAGAATGCCCC
GGAACAACAGTCACAATTCAGGAGAATTGTGACCATAGAGGCCCATCTTTGAGGACCACCACT
GCATCTGGAAAACTAGTCACGCAATGGTGTTGCCGCTCCTGCACGATGCCCCCCTTAAGGTTCT
TAGGAGAAGATGGGTGCTGGTATGGGATGGAGATTAGGCCCTTGAGTGAAAAAGAAGAGAAC
ATGGTCAAATCACAGGTGACGGCCGGACAGGGCACATCGGAAACTTTTTCAATGGGTCTGTTG
TGCCTGACCTTGTTTGTGGAAGAATGCTTGAGGAGAAGAGTCACCAGGAAACACATGATATTA
GCTGTGGTAATCACTCTTTGTGCTATCATCCTGGGGGGCCTCACATGGATGGACTTGCTACGAG
CCCTCATCATGTTGGGGGACACTATGTCTGGTAGAATAGGAGGACAGACCCACCTAGCCATCA
TGGCAGTGTTCAAGATGTCACCGGGATACGTGCTGGGTGTGTTTTTAAGGAAACTCACTTCAAG

AGAGACAGCACTAATGGTAATAGGAATGGCCATGACAACAACACTTTCAATTCCACATGACCT CATGGAACTCATTGATGGAATATCACTAGGACTAATTTTGCTAAAAATAGTAACACAGTTTGA CAACACCCAAGTGGGAACCTTAGCTCTTTCCTTGACTTTCATAAGATCAACAATGTCATTGGTC ATGGCTTGGAGGACCATTATGGCTGTGTTGTTTGTGGTCACACTCATTCCTTTGTGCAGGACAA GCTGTCTTCAAAAACAGTCTCATTGGGTAGAAATAACAGCACTCATCCTAGGAGCCCAAGCTC TGCCAGTGTACCTAATGACTCTTATGAAAGGAGCCTCAAGAAGATCTTGGCCTCTTAACGAAG GCATAATGGCTGTGGGTTTGGTTAGTCTCTTAGGAAGCGCTCTTTTAAAGAATGATGTCCCTTT AGCTGGCCCAATGGTGGCAGGAGGCTTACTTCTGGCGGCTTACGTAATGAGTGGCAGCTCAGC AGATCTGTCACTAGAGAAGGCCGCTAATGTGCAGTGGGATGAAATGGCAGACATAACAGGCT CAAGTCCAATCATAGAAGTGAAGCAAGATGAGGATGGCTCTTTCTCCATACGGGACGTCGAGG AAACCAATATGATAACCCTTTTGGTGAAACTGGCACTGATAACGGTGTCAGGTCTCTACCCCTT GGCAATTCCAATCACAATGACCTTATGGTACATGTGGCAAGTGAAAACACAAAGATCAGGAGC CCTGTGGGACGTCCCTTCACCCGCCGCCACTCAAAAAGCCGCACTGTCTGAAGGAGTGTACAG GATCATGCAAAGAGGGTTATTCGGGAAAACTCAGGTTGGAGTAGGGATACACATGGAAGGTG TATTTCACACAATGTGGCATGTTACAAGAGGATCGGTGATCTGCCACGAGACTGGGAGATTGG AGCCATCTTGGGCTGATGTCAGGAATGACATGATATCATACGGTGGGGGATGGAGGCTTGGAG ATAAATGGGACAAAGAAGAAGACGTTCAGGTCCTCGCTATAGAACCAGGGAAAAATCCCAAA CATGTCCAAACGAAACCTGGCCTTTTCAAGACCCTAACTGGAGAAATTGGAGCAGTAACATTA GATTTCAAACCCGGAACGTCTGGTTCTCCCATTATCAACAGGAAAGGAAAAGTCATCGGACTC TATGGAAATGGAGTGGTCACCAAATCAGGTGATTACGTCAGTGCCATAACACAAGCCGAAAG AATTGGAGAGCCAGATTATGAAGTGGATGAGGACATTTTTCGAAAGAAAAGACTAACTATAAT GGACTTACACCCCGGAGCCGGAAAGACAAAAAGAATTCTTCCATCAATAGTGAGAGAAGCCTT AAAAAGGAGGCTGCGAACTTTGATTTTGGCTCCCACGAGAGTGGTGGCGGCCGAGATGGAAG AGGCCCTACGTGGACTGCCAATCCGTTACCAAACCCCAGCTGTAAAATCAGAACACACAGGAA GAGAGATTGTAGACCTCATGTGCCATGCAACCTTCACAACAAGACTTTTGTCATCAACCAGAG TTCCAAACTACAACCTTATAGTAATGGATGAAGCACATTTCACCGATCCTTCCAGTGTCGCGGC TAGAGGATACATTTCGACCAGGGTGGAAATGGGAGAGGCAGCAGCCATCTTCATGACCGCAA CCCCTCCCGGAGCGACAGATCCCTTTCCCCAGAGCAACAGCCCAATAGAAGACATCGAGAGAG AGATTCCGGAAAGGTCATGGAACACAGGGTTCGACTGGATAACAGACTACCAAGGGAAAACT GTGTGGTTTGTTCCCAGCATAAAAGCTGGAAATGACATTGCAAATTGTTTGAGAAAGTCGGGA AAGAAAGTTATCCAGTTGAGTAGGAAAACCTTTGATACAGAATATCCAAAAACGAAACTCACG GACTGGGACTTTGTGGTCACTACAGACATATCTGAAATGGGGGCTAACTTTAGAGCTGGGAGA GTGATAGACCCTAGAAGATGCCTCAAGCCAGTTATCCTAACAGATGGGCCAGAGAGAGTCATC TTAGCAGGTCCTATTCCAGTGACTCCAGCAAGCGCTGCTCAGAGAAGAGGGCGAATAGGAAG GAACCCAGCACAAGAAGACGACCAATACGTTTTCTCCGGAGACCCACTAAAAAATGATGAAG ACCATGCCCACTGGACAGAAGCAAAGATGCTGCTTGACAATATCTACACCCCAGAAGGGATCA TTCCAACATTGTTTGGTCCGGAAAGGGAAAAAACCCAAGCTATTGATGGAGAGTTTCGCCTCA

GAGGGGAACAAAGGAAGACTTTTGTGGAATTAATGAGGAGAGGAGACCTTCCGGTGTGGCTG
AGTTATAAGGTAGCTTCTGCTGGCATTTCTTACAAAGATCGGGAATGGTGCTTTACTGGGGAAA
GAAATAACCAAATTTTAGAAGAAAACATGGAGGTTGAAATTTGGACTAGAGAGGGAGAAAAG
AAAAAACTGAGGCCAAAATGGTTAGATGCACGTGTATACGCTGACCCCATGGCTTTGAAGGAT
TTCAAGGAGTTTGCCAGTGGAAGGAAGAGTATAACTCTCGACATCCTAACAGAGATCGCCAGT
TTGCCAACTTACCTTTCCTCTAGGGCCAAGCTCGCCCTTGACAACATAGTTATGCTCCACACAA
CAGAAAGAGGAGGGAGGGCCTATCAACATGCCCTGAACGAACTTCCGGAGTCACTGGAAACA
CTCATGCTTGTAGCCTTACTAGGAGCTATGACAGCAGGTATCTTCCTGTTTTTCATGCAAGGGA
AAGGAATAGGGAAATTGTCAATGGGTTTGATAACCATTGCGGTGGCTAGTGGCTTGCTCTGGG
TAGCAGAAATTCAACCCCAGTGGATAGCGGCCTCAATCATACTGGAGTTTTTCTCATGGTACT
GTTGATACCAGAACCAGAAAAACAAAGGACCCCACAAGACAATCAATTGATCTACGTCATATT
GACCATTCTCACCATTATTGGTCTAATAGCAGCCAACGAGATGGGGCTGATAGAAAAAACAAA
AACGGATTTTGGGTTTTACCAGGTAAAAACAGAAACCACCATCCTCGATGTGGACCTGAGACC
AGCTTCAGCATGGACGCTCTATGCGGTAGCCACCACAATTCTGACTCCCATGCTGAGACACAC
CATAGAAAATACGTCGGCCAACCTATCTTTAGCAGCCATCGCCAACCAGGCAGCCGTCCTAAT
GGGGCTTGGAAAAGGATGGCCACTCCACAGAATGGACCTCGGTGTGCCGCTGTTAGCAATGGG
ATGCTATTCTCAAGTGAACCCAACAACCTTGACAGCATCCTTAGTCATGCTTTTAGTCCATTAT
GCAATAATAGGCCCAGGATTGCAGGCAAAAGCCACAAGAGAGGCCCAGAAAAGGACAGCTGC
TGGAATCATGAAAAATCCCACAGTGGACGGGATAACAGTGATAGATCTAGAACCAATATCCTA
TGACCCAAAATTTGAAAAGCAATTAGGGCAGGTCATGTTACTCGTCTTGTGTGCTGGACAACT
ACTCTTGATGAGAACAACATGGGCTTTCTGTGAAGTTTTGACTTTGGCCACAGGACCAATCTTG
ACCTTGTGGGAGGGCAACCCGGGAAGGTTTTGGAACACGACCATAGCCGTATCTACCGCCAAC
ATTTTCAGGGGAAGTTATTTGGCAGGAGCTGGACTGGCTTTTTCACTCATAAAGAATGCACAA
ACCCCCAGGAGGGGAACTGGGACCACAGGAGAGACACTGGGAGAGAAGTGGAAGAGACAGC
TAAACTCATTAGACAGGAAAGAGTTTGAAGAGTATAAAAGAAGTGGAATACTAGAAGTGGAC
AGGACTGAAGCCAAGTCCGCCCTGAAAGATGGGTCTAAAATCAAGCATGCAGTATCTAGAGG
GTCCAGTAAGATCAGATGGATTGTTGAGAGAGGGATGGTAAAGCCAAAGGGGAAAGTTGTAG
ATCTTGGCTGTGGGAGAGGAGGATGGTCTTATTACATGGCGACACTCAAGAACGTGACTGAAG
TGAAAGGGTATACAAAAGGAGGTCCAGGACATGAAGAACCGATTCCCATGGCTACTTATGGCT
GGAATTTGGTCAAACTCCATTCAGGGGTTGACGTGTTCTACAAACCCACAGAGCAAGTGGACA
CCCTGCTCTGTGATATTGGGGAGTCATCTTCTAATCCAACAATAGAGGAAGGAAGAACATTAA
GAGTTTTGAAGATGGTGGAGCCATGGCTCTCTTCAAAACCTGAATTCTGCATCAAAGTCCTCAA
CCCCTACATGCCAACAGTCATAGAGGAGCTGGAGAAACTGCAGAGAAAACACGGTGGGAACC
TTGTCAGATGCCCGCTGTCCAGGAACTCCACCCATGAGATGTATTGGGTGTCAGGAGCGTCGG
GAAATATTGTGAGCTCTGTGAACACAACATCAAAGATGTTGTTGAACAGGTTCACAACAAGGC
ATAGGAAACCCACTTATGAGAAGGACGTAGATCTTGGGGCAGGAACGAGAAGTGTCTCTACTG
AAACAGAAAAACCAGACATGACAATCATTGGGAGAAGGCTTCAGCGATTGCAAGAGGAGCAC

AAAGAAACATGGCATTATGATCAGGAAAACCCATACAGAACCTGGGCGTATCATGGAAGCTATGAAGCTCCTTCGACAGGCTCTGCATCCTCCATGGTGAACGGGGTGGTGAAACTGCTAACAAAACCCTGGGATGTGATTCCAATGGTGACTCAGTTAGCCATGACAGATACAACCCCTTTTGGGCAACAAAGAGTGTTCAAAGAGAAGGTGGATACCAGAACGCCGCAACCAAAACCAGGCACACGAATGGTTATGACCACGACAGCCAATTGGCTATGGGCCCTCCTTGGAAAGAAGAAAAATCCCAGACTGTGTACAAGGGAAGAGTTCATCTCAAAAGTTAGATCAAACGCAGCCATAGGCGCAGTCTTTCAGGAAGAACAAGGATGGACATCAGCCAGTGAAGCTGTGAATGACAGCCGGTTTTGGGAACTGGTTGACAAAGAAAGGCCCTTCACCAGGAAGGGAAATGTGAATCGTGTGTCTATAACATGATGGGAAAACGTGAGAAAAAGTTAGGAGAGTTTGGCAGAGCCAAGGGAAGCCGAGCAATCTGGTACATGTGGCTGGGAGCGCGGTTTCTGGAATTTGAAGCCCTGGGTTTTTTGAATGAAGATCACTGGTTTGGCAGAGAAAATTCATGGAGTGGAGTGGAAGGGGAAGGTCTGCACAGATTGGGATACATCCTGGAGGAGATAGACAAGAAGGATGGAGACCTAATGTATGCTGATGACACAGCAGGTTGGGACACAAGAATCACTGAGGATGACCTTCAAAATGAGGAACTGATCACGGAACAGATGGCTCCCCACCACAAGATCCTAGCCAAAGCCATTTTCAAACTAACCTATCAAAACAAAGTGGTGAAAGTCCTCAGACCCACACCGAGAGGAGCGGTGATGGATATCATATCCAGGAAAGACCAAAGAGGTAGTGGACAAGTTGGAACATATGGTTTGAACACATTCACCAACATGGAAGTTCAACTCATCCGCCAAATGGAAGCTGAAGGAGTCATCACACAAGATGACATGCAGAACCCAAAAGGGTTGAAAGAAAGAGTTGAGAAATGGCTGAGAGAGTGTGGTGTCGACAGGTTAAAGAGGATGGCAATCAGTGGAGACGATTGCGTGGTGAAGCCCCTAGATGAGAGGTTTGGCACTTCCCTCCTCTTCTTGAACGACATGGGAAAGGTGAGGAAAGACATTCCGCAGTGGGAACCATCTAAGGGATGGAAAAACTGGCAAGAGGTTCCTTTTTGCTCCCATCACTTTCACAAGATCTTTATGAAGGATGGCCGCTCACTAGTTGTTCCATGTAGAAACCAGGATGAACTGATAGGGAGAGCCAGAATCTCGCAGGGGGCTGGATGGAGCTTAAGAGAAACAGCCTGCCTGGGCAAAGCTTACGCCCAGATGTGGTCGCTTATGTACTTCCACAGAAGGGATCTGCGTTTAGCCTCCATGGCCATATGCTCAGCAGTTCCAACGGAATGGTTTCCAACAAGCAGAACAACATGGTCAATCCATGCTCATCACCAATGGATGACCACTGAAGACATGCTCAAAGTGTGGAACAGAGTGTGGATAGAAGACAACCCCAATATGATTGACAAGACTCCAGTCCATTCGTGGGAAGATATACCTTACCTAGGGAAAAGAGAGGATTTGTGGTGTGGATCCCTGATTGGACTTTCTTCTAGAGCCACCTGGGCGAAGAACATTCACACGGCCATAACTCAGGTCAGGAACTTGATCGGAAAAGAGGAATACGTGGATTACATGCCAGTAATGAAAAGATACAGTGCTCCTTCAGAGAGTGAAGGAGTTCTGTAATTACCAACAACAAACACCAAAGGCTATTGAAGTCAGGCCACTTGTGCCACGGTTTGAGCAAACCGTGCTGCCTGTAGCTCCGCCAATAATGGGAGGCGTAATAATCCCTAGGGAGGCCATGCGCCACGGAAGCTGTACGCGTGGCATATTGGACTAGCGGTTAGAGGAGACCCCTCCCATCACTGACAAAACGCAGCAAAAGGGGGCCCGAAGCCAGGAGGAAGCTGTACTCCTGGTGGAAGGACTAGAGGTTAGAGGAGACCCCCCCAACACAAAAACAGCATATTGACGCTGGGAAAGACCAGAGATCCTGCTGTCTCTACAACATCAATCCAGGCACAGAGCGCCGCAAGATGGATTGGTGTTGTTGATCCAACAGGTTCT

SEQ ID NO:9 - DENV-1-VN-BID-V1774-2007-E-W/Min

AGTTGTTAGTCTACGTGGACCGACAAGAACAGTTTCGAATCGGAAGCTTGCTTAACGTAGTTCT
AACAGTTTTTATTAGAGAGCAGATCTCTGATGAACAACCAACGGAAAAAGACGGCTCGACCG
TCTTTCAATATGCTGAAACGCGCGAGAAACCGCGTGTCAACTGTTTCACAGTTGGCGAAGAGA
TTCTCAAAAGGATTGCTCTCAGGCCAAGGACCCATGAAATTGGTGATGGCTTTCATAGCATTCC
TAAGATTTCTAGCCATACCCCCAACAGCAGGAATTTTGGCTAGATGGGGTTCATTCAAGAAGA
GTGGAGCGATCAAAGTGCTACGGGGTTTCAAGAAAGAAATCTCAAACATGTTGAATATAATGA
ATAGAAGGAAAAGATCTGTGACCATGCTCCTTATGCTGATGCCTACAGCCTTGGCGTTCCATTT
GACTACACGAGGGGGAGAGCCGCACATGATAGTCAGCAAGCAGGAAAGAGGAAAGTCACTCT
TGTTTAAGACCTCAGCAGGTGTCAACATGTGCACCCTTATAGCGATGGATTTGGGAGAGTTATG
TGAGGACACAATGACTTACAAATGCCCTCGAATCACTGAAACTGAACCAGATGACGTTGATTG
TTGGTGTAATGCCACAGACACATGGGTGACCTATGGAACATGTTCCCAAACTGGCGAGCACCG
ACGAGACAAACGTTCCGTCGCACTGGCCCCACACGTGGGACTTGGTTTGGAAACAAGAACCGA
AACGTGGATGTCCTCTGAAGGCGCTTGGAAACAGATACAAAGAGTGGAGACTTGGGCCCTGA
GACACCCAGGATTCACGGTGATAGCCCTTTTTCTAGCACATGCCATAGGAACATCCATCACCC
AGAAAGGGATTATTTTCATTTTGTTAATGCTGGTAACACCATCCATGGCCATGCGATGCGTGGG
AATAGGCAGCAGGGACTTCGTGGAAGGACTGTCAGGAGCAACTTGGGTAGATGTGGTACTGG
AACATGGAAGTTGCGTCACCACCATGGCAAAAGACAAACCAACATTGGACATTGAACTCTTGA
AGACGGAAGTCACAAACCCTGCCGTCCTGCGCAAACTGTGCATTGAAGCTAAAATATCAAACA
CCACCACCGACTCAAGATGTCCAACACAAGGAGAAGCCACACTAGTGGAAGAACAAGACGCG
AACTTTGTGTGTCGACGAACGTTTGTGGACAGAGGCTGGGGCAATGGCTGTGGCCTCTTCGGA
AAAGGAAGCCTAATAACGTGTGCAAAGTTCAAGTGTGTGACAAAACTGGAAGGAAAGATAGT
TCAATATGAGAACTTGAAATATTCAGTAATAGTCACCGTTCACACCGGAGACCAGCATCAAGT
GGGAAATGAAAGCACAGAACATGGGACAACTGCAACTATAACACCTCAAGCTCCTACGACGG
AAATACAGCTGACCGACTACGGAGCTCTTACATTGGATTGTTCACCTAGAACAGGACTAGACT
TTAATGAAATGGTGTTGTTGACAATGAAAGAAAAATCATGGCTAGTCCACAAACAATGGTTTT
TAGACCTACCACTGCCTTGGACCTCGGGAGCTTCAACATCACAAGAGACTTGGAACAGACAAG
ATTTGCTGGTGACATTTAAGACAGCTCATGCAAAGAAGCAGGAAGTAGTAGTGTTAGGGTCAC
AAGAGGGAGCAATGCATACCGCACTAACCGGAGCAACCGAAATACAGACTAGCGGAACTACA
ACGATTTTTGCCGGTCACCTGAAATGTAGACTGAAGATGGACAAACTGACACTTAAAGGAATG
TCATACGTTATGTGTACGGGATCCTTTAAGCTCGAAAAAGAGGTTGCCGAAACGCAACACGGA
ACAGTGCTAGTGCAAATTAAATATGAGGGAACCGACGCACCATGTAAGATACCGTTTAGTACG
CAAGACGAAAAGGGCGTTACGCAAAACGGAAGACTGATTACCGCTAACCCTATCGTTACAGA
CAAAGAGAAACCCGTTAACATAGAGGCCGAACCACCTTTTGGCGAATCATATATAGTGATAGG
CGCAGGCGAAAAAGCACTAAAGCTGTCATGGTTCAAAAAAGGATCTAGCATAGGGAAGATGT
TCGAAGCAACCGCTAGGGGAGCTAGACGAATGGCAATACTGGGAGATACCGCATGGGACTTC
GGATCGATAGGGGGAGTGTTTACTAGCGTAGGTAAGTTGGTGCATCAGATATTCGGAACCGCA

TACGGAGTGTTGTTTAGCGGAGTGTCATGGACTATGAAGATAGGGATAGGAGTGCTATTGACA
TGGCTCGGACTGAATTCGAGATCGACTAGCCTATCTATGACATGCATAGCCGTCGGACTGGTTA
CACTGTATCTAGGCGTAATGGTGCAAGCAGATTCAGGATGTGTAATTAATTGGAAAGGTAGAG
AACTCAAGTGTGGAAGTGGCATTTTTGTCACCAATGAAGTTCACACTTGGACAGAGCAATACA
AATTTCAAGCTGACTCCCCTAAGAGACTATCAGCAGCCATCGGGAAGGCATGGGAGGAGGGT
GTGTGTGGAATTCGATCAGCAACTCGTCTCGAGAACATCATGTGGAAGCAAATATCAAATGAA
CTGAATCACATCTTACTTGAAAATGATATGAAATTCACAGTGGTTGTAGGAGATGTTGCTGGG
ATCTTGGCTCAAGGAAAGAAAATGATTAGGCCACAACCCATGGAATACAAATACTCGTGGAA
AAGCTGGGGAAAGGCTAAAATCATAGGGGCAGATGTACAGAACACCACCTTCATCATCGATG
GCCCAAACACCCCAGAATGCCCTGATGACCAAAGAGCATGGAACATTTGGGAAGTTGAGGAC
TATGGATTTGGAATTTTCACGACAAACATATGGCTGAAATTGCGTGATTCCTACACCCAAGTGT
GTGACCACCGGCTAATGTCAGCTGCCATCAAGGACAGCAAGGCAGTTCACGCTGACATGGGGT
ACTGGATAGAAAGTGAAAAGAACGAGACCTGGAAGCTGGCAAGAGCCTCATTCATAGAAGTT
AAAACATGTATCTGGCCAAAATCCCACACTCTATGGAGCAATGGAGTTCTGGAAAGTGAAATG
ATAATTCCAAAGATCTATGGAGGACCAATATCTCAGCACAACTACAGACCAGGATATTTTACA
CAAGCAGCAGGGCCGTGGCACCTAGGCAAGTTGGAACTGGATTTTGATTTGTGTGAGGGTACC
ACAGTTGTTGTGGATGAACATTGTGGAAATCGAGGACCATCTCTTAGGACCACAACAGTCACA
GGAAAGATAATTCATGAATGGTGTTGCAGATCTTGCACGCTACCACCCTTACGTTTCAGAGGA
GAAGATGGGTGCTGGTACGGTATGGAAATCAGACCAGTCAAGGAAAAGGAAGAAAATCTAGT
CAAATCAATGGTCTCTGCAGGGTCAGGGGAAGTGGACAGCTTTTCACTAGGACTGCTATGCAT
ATCAATAATGATCGAGGAGGTGATGAGATCCAGATGGAGTAGAAGAATGCTGATGACTGGAA
CACTGGCTGTGTTCTTCCTTCTCATAATGGGACAATTGACATGGAACGATCTGATCAGATTATG
CATCATGGTTGGAGCCAACGCTTCCGACAGGATGGGGATGGGAACGACGTACCTAGCCCTGAT
GGCCACTTTTAAAATGAGACCGATGTTCGCTGTAGGGCTATTATTTCGCAGACTAACATCCAGA
GAAGTTCTTCTTCTAACAATTGGATTGAGTCTAGTGGCATCTGTGGAGTTACCAAATTCCTTGG
AGGAGCTGGGGGATGGACTTGCAATGGGCATTATGATTTTAAAATTATTGACTGACTTTCAATC
ACATCAGCTGTGGGCTACCTTGCTGTCCTTGACATTTATCAAAACAACGTTTTCCTTGCACTAC
GCATGGAAGACAATGGCTATGGTACTGTCAATTGTATCTCTCTTCCCTTTATGCCTGTCCACGA
CCTCCCAAAAAACAACATGGCTTCCGGTGCTATTGGGATCCCTTGGATGCAAACCACTAACCA
TGTTTCTTATAGCAGAAAACAAAATCTGGGGAAGGAGAAGTTGGCCCCTCAATGAAGGAATCA
TGGCTGTTGGAATAGTCAGCATCCTACTAAGTTCACTCCTCAAAAATGATGTACCGCTAGCTGG
GCCACTAATAGCTGGAGGCATGCTAATAGCATGTTATGTTATATCTGGAAGCTCAGCCGACCT
ATCATTAGAGAAAGCGGCTGAGGTCTCCTGGGAAGAAGAAGCAGAACACTCTGGTGCCTCAC
ACAACATATTAGTGGAAGTCCAAGATGATGGAACCATGAAGATAAAGGATGAAGAGAGAGAT
GACACGCTAACCATTCTCCTTAAAGCAACTCTGTTAGCAGTTTCAGGGGTGTACCCATTATCAA
TACCAGCGACCCTTTTCGTGTGGTACTTTTGGCAGAAAAAGAAACAGAGATCTGGAGTGTTAT
GGGACACACCCAGCCCTCCAGAAGTGGAAAGAGCAGTTCTTGATGATGGTATCTATAGAATTA

TGCAGAGAGGACTGTTGGGCAGGTCCCAAGTAGGGGTAGGAGTTTTCCAAGAAAACGTGTTCC ACACAATGTGGCATGTCACCAGGGGAGCTGTACTCATGTATCAAGGGAAGAGATTGGAACCG AGCTGGGCCAGTGTCAAAAAAGACCTGATCTCATATGGAGGAGGTTGGAGGCTTCAAGGATCC TGGAACACAGGAGAAGAAGTGCAGGTGATTGCTGTTGAACCAGGGAAAAACCCCAAAAATGT ACAAACAGCGCCGGGCACCTTTAAGACCCCTGAAGGTGAAGTTGGAGCCATTGCCCTAGACTT TAAACCTGGCACATCTGGATCTCCCATCGTGAACAGAGAAGGAAAAATAGTAGGTCTTTATGG AAATGGAGTAGTGACAACAAGTGGAACCTACGTCAGTGCCATAGCTCAAGCCAAAGCATCAC AAGAAGGGCCCCTACCAGAGATTGAAGACGAGGTGTTTAGGAAAAGAAACTTAACAATAATG GACCTACATCCAGGATCGGGGAAAACAAGAAGATATCTTCCAGCCATAGTCCGTGAGGCCATA AAAAGGAAGCTGCGCACACTAATCCTGGCTCCCACAAGGGTTGTCGCTTCCGAAATGGCAGAG GCGCTCAAGGGAATGCCAATAAGGTACCAAACAACAGCAGTGAAGAGTGAACATACAGGAAA AGAGATAGTTGACCTCATGTGTCACGCCACTTTCACCATGCGCCTCCTGTCTCCCGTAAGAGTT CCCAATTACAACATGATCATCATGGATGAAGCACATTTCACCGATCCATCCAGTATAGCGGCC AGAGGGTACATCTCAACCCGAGTGGGCATGGGTGAAGCAGCTGCAATCTTCATGACAGCCACT CCCCCAGGATCAGTGGAGGCCTTTCCACAGAGCAACGCAGTTATCCAAGATGAGGAAAGAGA CATTCCTGAGAGATCATGGAACTCAGGCTATGAGTGGATCACTGACTTCCCAGGTAAAACAGT TTGGTTTGTTCCAAGCATTAAATCAGGAAATGACATTGCCAACTGCTTAAGAAAGAATGGGAA ACGGGTGATTCAATTGAGCAGGAAAACCTTTGATACAGAGTACCAAAAAACAAAAAACAACG ACTGGGACTACGTCGTCACAACAGACATCTCCGAAATGGGAGCAAATTTCCGAGCCGACAGGG TGATAGACCCAAGACGGTGTCTGAAACCGGTAATACTAAAAGATGGTCCAGAGCGTGTCATTT TAGCAGGACCAATGCCAGTGACTGTGGCCAGTGCCGCTCAGAGGAGAGGAAGAATTGGAAGG AACCACAATAAGGAAGGTGATCAGTACATCTACATGGGACAGCCTTTAAACAACGATGAAGA TCACGCTCACTGGACAGAAGCAAAAATGCTCCTTGATAATATAAACACACCAGAAGGGATTAT CCCAGCCCTCTTTGAGCCAGAGAGAGAAAAGAGTGCAGCAATAGACGGGGAATACAGACTGC GGGGTGAAGCAAGGAAAACGTTTGTGGAGCTCATGAGAAGAGGAGATCTACCTGTCTGGCTAT CCTACAAAGTTGCCTCAGAAGGCTTCCAGTACTCTGACAGAAGATGGTGCTTTGACGGGGAAA GGAACAACCAGGTGTTGGAGGAGAACATGGACGTGGAGATCTGGACAAAAGAAGGAGAAAG AAAGAAACTACGACCCCGCTGGCTGGATGCCAGAACATACTCAGACCCACTAGCCCTGCGCGA GTTTAAAGAGTTTGCAGCAGGGAGAAGAAGCGTCTCAGGTGATTTAATATTAGAAATAGGGAA GCTTCCACAACACTTGACGCAAAGGGCCCAGAATGCCCTGGACAACCTGGTTATGTTGCACAA CTCCGAACAAGGAGGCAGAGCCTATAGACATGCAATGGAAGAACTGCCAGACACCATAGAAA CGTTGATGCTCCTAGCTTTGATAGCTGTGTTAACTGGTGGAGTGACACTGTTCTTCCTATCAGG AAGGGGCCTAGGGAAAACATCTATCGGCCTACTCTGCGTAATGGCTTCAAGCGTACTGCTATG GATGGCCAGTGTGGAGCCCCATTGGATAGCGGCCTCCATCATACTGGAGTTCTTCCTGATGGTG CTGCTTATTCCAGAGCCAGACAGACAACGCACTCCGCAGGACAACCAGCTGGCATATGTGGTG ATAGGTTTGTTATTCATGATACTGACAGTAGCAGCCAATGAGATGGGACTGCTGGAAACCACA AAGAAAGACTTAGGGATTGGCCATGTGGCTGTTGAAAATCACCACCATGCCGCAATGCTGGAC

GTAGACTTACATCCAGCTTCAGCCTGGACCCTCTATGCAGTGGCCACAACAATTATCACTCCCA
TGATGAGGCACACAATCGAAAACACAACGGCAAACATTTCCCTGACAGCCATTGCAAACCAG
GCAGCTATATTGATGGGACTTGACAAAGGATGGCCAATATCGAAGATGGACATAGGAGTTCCA
CTTCTCGCCTTGGGGTGCTATTCCCAGGTGAATCCACTGACGCTGACAGCGGCGGTATTGATGC
TAGTGGCTCATTACGCCATAATTGGACCTGGACTGCAAGCAAAAGCTACTAGAGAAGCTCAAA
AAAGGACAGCGGCCGGAATAATGAAAAATCCAACCGTTGATGGAATTGTTGCAATAGATTTGG
ACCCTGTGGTTTATGATGCAAAATTTGAAAAACAACTAGGCCAAATAATGTTGTTGATACTATG
CACATCACAGATCCTCTTGATGCGGACTACATGGGCCTTGTGCGAGTCCATCACACTGGCCACT
GGACCTCTGACCACGCTTTGGGAGGGATCTCCAGGAAAATTTTGGAACACCACGATAGCGGTT
TCCATGGCAAACATTTTCAGAGGAAGTTATCTAGCAGGAGCAGGTCTGGCCTTCTCATTAATGA
AATCTCTAGGAGGAGGTAGGAGAGGCACGGGAGCCCAAGGGGAAACACTGGGAGAGAAATG
GAAAAGACAGCTGAACCAACTGAGCAAGTCAGAATTTAACACCTATAAAAGGAGTGGGATTA
TGGAAGTGGACAGATCCGAAGCCAAAGAGGGATTGAAAAGAGGAGAAACAACCAAACATGC
AGTGTCGAGAGGAACCGCTAAACTGAGGTGGTTCGTGGAGAGGAACCTTGTGAAACCAGAAG
GGAAAGTCATAGACCTCGGTTGTGGAAGAGGTGGCTGGTCATACTATTGCGCTGGGCTGAAGA
AAGTCACAGAAGTGAAGGGGTATACAAAAGGAGGACCTGGACATGAGGAACCAATCCCAATG
GCGACCTATGGATGGAACCTAGTAAAGCTGCACTCTGGGAAAGACGTATTTTTATACCACCT
GAGAAATGTGACACCCTTTTGTGTGATATTGGTGAGTCCTCTCCAAACCCAACTATAGAGGAA
GGAAGAACGCTACGCGTCCTAAAGATGGTGGAACCATGGCTCAGAGGAAACCAATTTTGCATA
AAAATTCTGAATCCCTACATGCCAAGTGTGGTGGAAACTCTGGAGCAAATGCAAAGAAAACAT
GGAGGAATGCTAGTGCGAAATCCACTTTCAAGAAATTCTACTCATGAAATGTATTGGGTTTCAT
GTGGAACAGGAAACATTGTGTCAGCAGTAAACATGACATCTAGAATGTTGCTAAATCGATTCA
CAATGGCTCACAGGAAACCAACATATGAAAGAGACGTGGACCTAGGCGCCGGAACAAGACAT
GTGGCAGTGGAACCAGAGGTAGCCAACCTAGATATCATTGGCCAGAGGATAGAGAACATAAA
ACATGAACATAAGTCAACATGGCATTATGATGAGGACAATCCATATAAAACATGGGCCTATCA
TGGATCATATGAGGTCAAGCCATCAGGATCAGCCTCATCCATGGTCAATGGCGTGGTGAAACT
GCTCACCAAACCATGGGATGTCATCCCTATGGTCACACAAATAGCCATGACTGACACTACACC
CTTTGGACAACAGAGGGTGTTTAAAGAGAAAGTTGACACACGCACACCAAAAGCAAAACGGG
GCACAGCACAAATCATGGAGGTGACAGCCAAGTGGTTATGGGGTTTTCTTTCTAGAAACAAGA
AACCAAGAATTTGCACAAGAGAGGAGTTCACAAGAAAAGTTAGGTCAAACGCAGCCATTGGA
GCAGTGTTCGTTGATGAAAATCAATGGAACTCAGCAAAAGAAGCAGTGGAAGATGAGCGGTT
CTGGGACCTTGTGCATAGAGAGAGGGAGCTTCACAAACAGGGAAAATGTGCTACGTGTGTTTA
CAACATGATGGGGAAGAGAGAGAAAAAGCTAGGAGAGTTCGGAAAGGCAAAAGGAAGTCGT
GCAATATGGTACATGTGGTTGGGAGCACGCTTTCTAGAGTTCGAAGCTCTTGGTTTCATGAACG
AAGATCACTGGTTCAGCAGAGAGAATTCACTCAGCGGAGTGGAAGGAGAAGGACTCCACAAA
CTTGGATATATACTCAGAGACATATCAAAGATTCCAGGGGGAAACATGTATGCAGATGACACA
GCCGGATGGGATACAAGGATAACAGAGGATGACCTTCAGAATGAGGCCAGAATTACTGACAT

CATGGAACCCGAACATGCCCTCCTGGCTAAGTCAATCTTCAAGTTAACCTACCAAAATAAGGTGGTAAGGGTACAGAGACCAGCAAAAAATGGAACCGTGATGGATGTCATATCCAGACGTGACCAGAGAGGAAGTGGTCAGGTCGGAACTTATGGCTTAAACACTTTCACCAACATGGAAGCCCAGCTGATAAGACAAATGGAGTCTGAGGGAATCTTTTCACCCAGCGAATTAGAGACCCCAAATTTAGCCGAGAGAGTTCTCGACTGGCTGGAAAAATATGGCGTCGAAAGGCTGAAAAGAATGGCAATCAGCGGAGATGACTGCGTAGTGAAACCAATTGATGATAGGTTTGCAACAGCCTTGACAGCTCTGAATGATATGGGAAAAGTAAGAAAAGATATACCACAATGGGAACCTTCAAAAGGATGGAATGATTGGCAACAAGTGCCTTTTTGTTCACACCACTTCCACCAGCTGATTATGAAGGATGGGAGGGAAATAGTGGTGCCATGCCGCAACCAAGATGAACTTGTGGGTAGGGCTAGAGTATCACAAGGTGCTGGATGGAGCCTGAGAGAAACTGCATGCCTAGGCAAGTCATATGCACAAATGTGGCAGCTGATGTACTTCCACAGGAGAGACCTGAGACTAGCCGCTAATGCTATCTGTTCAGCCGTTCCAGTTGATTGGATCCCAACCAGCCGTACCACCTGGTCGATCCATGCCCATCACCAATGGATGACAACAGAAGACATGCTGTCAGTGTGGAATAGGGTTTGGATAGAGGAAAACCCATGGATGGAGGACAAAACCCACATATCCAGTTGGGGAGATGTTCCATATTTAGGAAAAAGGGAAGACCAATGGTGTGGATCCCTGATAGGCTTAACAGCAAGGGCCACCTGGGCCACCAACATACAAGTGGCCATAAACCAAGTGAGAAGACTAATTGGGAATGAGAATTATCTAGATTACATGACATCAATGAAGAGATTCAAGAACGAGAGTGATCCCGAAGGGGCACTCTGGTGAGTCAACACATTTACAAAATAAAGGAAAATAAGAAATCAAACAAGGCAAGAAGTCAGGCCGGATTAAGCCATAGTACGGTAAGAGCTATGCTGCCTGTGAGCCCCGTCTAAGGACGTAAAATGAAGTCAGGCCGAAAGCCACGGCTTGAGCAAACCGTGCTGCCTGTAGCTCCATCGTGGGGATGTAAAAACCTGGGAGGCTGCAACCCATGGAAGCTGTACGCATGGGGTAGCAGACTAGTGGTTAGAGGAGACCCCTCCCGAAACATAACGCAGCAGCGGGGCCCAACACCAGGGGAAGCTGTACCCTGGTGGTAAGGACTAGAGGTTAGAGGAGACCCCCCGCATAACAATAAACAGCATATTGACGCTGGGAGAGACCAGAGATCCTGCTGTCTCTACAGCATCATTCCAGGCACAGAACGCCAGAAAATGGAATGGTGCTGTTGAATCAACAGGTTCT

SEQ ID NO:10 - DENV-2-NI-BID-V533-2005-E-W/Min

AGTTGTTAGTCTACGTGGACCGACAAAGACAGATTCTTTGAGGGAGCTAAGCTCAACGTAGTTCTAACAGTTTTTTAATTAGAGAGCAGATCTCTGATGAATAACCAACGAAAAAAGGCGAGAAGTACGCCTTTCAATATGCTGAAACGCGAGAGAAACCGCGTGTCAACTGTGCAACAGCTGACAAAGAGATTCTCACTTGGAATGCTGCAAGGACGCGGACCATTAAAACTGTTCATGGCCCTTGTGGCGTTCCTTCGTTTCCTAACAATCCCACCAACAGCAGGGATACTAAAAAGATGGGGAACGATCAAAAAATCAAAAGCTATCAATGTTTTGAGAGGGTTCAGGAAAGAGATTGGAAGGATGCTGAACATCTTGAACAAGAGACGCAGGACAGCAGGCGTGATTGTTATGTTGATTCCAACAGCGATGGCGTTCCATTTAACCACACGCAATGGAGAACCACACATGATCGTTGGTAGGCAGGAGAAAGGGAAAAGTCTTCTGTTCAAAACAGAGGATGGTGTTAACATGTGTACTCTCATGGCCATAGACCTTGGTGAATTGTGTGAAGATACAATCACGTACAAGTGTCCTCTCCTCAGACAAAATGAACCAGAAGACATAGATTGTTGGTGCAACTCTACGTCCACATGGGTAACTTATGGGACATGTACCACCACAGGAGAAC

ACAGAAGAGAAAAAAGATCAGTGGCGCTCGTTCCACATGTAGGTATGGGACTGGAGACACGA
ACTGAAACATGGATGTCATCAGAAGGGGCCTGGAAACATGTTCAGAGAATTGAAACCTGGATC
TTGAGACATCCAGGTTTTACCATAATGGCAGCAATCCTGGCATACACCATAGGAACGACACAT
TTCCAAAGGGCCTTGATTTTCATTTTACTGACAGCTGTCGCTCCTTCAATGACAATGCGCTGCA
TAGGAATATCAAATAGAGACTTCGTAGAAGGGGTTTCAGGAGGAAGCTGGGTTGACATAGTCT
TAGAACATGGAAGTTGTGTGACGACGATGGCAAAAAACAAACCAACATTGGATTTTGAACTGA
TAAAAACAGAAGCCAAACAACCTGCCACTCTAAGGAAGTACTGTATAGAAGCAAAGCTGACC
AACACAACAACAGAATCGCGTTGCCCAACACAAGGGGAACCCAGTCTAATGAGGAGCAGGAC
AAAAGGTTCATCTGCAAACACTCCATGGTAGACAGAGGATGGGGAAATGGATGTGGATTATTT
GGAAAGGGAGGCATTGTGACCTGTGCTATGTTTACATGCAAAAAGAACATGGAAGGAAAAGT
CGTGCAGCCAGAAAATTTGGAATACACCATCGTGATAACACCTCACTCAGGAGAGGAGCACGC
TGTAGGTAATGACACAGGAAAGCATGGCAAGGAAATCAAAATAACACCACAGAGCTCCATCA
CAGAAGCAGAACTGACAGGCTATGGCACTGTCACGATGGAGTGCTCTCCGAGAACGGGCCTCG
ACTTCAATGAGATGGTACTGCTGCAGATGGAAGACAAAGCTTGGCTGGTGCACAGGCAATGGT
TCCTAGACCTGCCGTTACCATGGCTACCCGGAGCGGACACACAAGGATCAAATTGGATACAGA
AAGAGACATTGGTCACTTTCAAAAATCCCCACGCGAAGAAACAGGACGTAGTCGTACTCGGAT
CACAGGAAGGCGCAATGCATACCGCATTGACAGGCGCTACAGAGATACAGATGTCTAGCGGA
AATCTGTTGTTTACAGGGCATCTGAAATGTAGACTGAGAATGGACAAACTGCAATTGAAGGGA
ATGTCATACTCTATGTGTACGGGTAAGTTTAAGATAGTCAAAGAGATAGCCGAAACACAACAC
GGAACAATCGTAATTAGGGTGCAATACGAAGGCGACGGGTCACCATGTAAGATACCATTCGA
AATTACAGACCTCGAAAAAAGACACGTACTCGGAAGACTGATAACAGTGAATCCGATCGTTAC
GGAAAAAGACTCACCCGTTAATATCGAAGCCGAACCACCATTCGGAGACTCATACATAATAAT
CGGAGTCGAACCCGGACAATTGAAACTGAATTGGTTTAAAAAAGGGTCATCAATCGGACAAAT
GTTCGAAACAACTATGAGAGGCGCTAAGCGTATGGCTATACTCGGAGACACAGCATGGGACTT
CGGATCCTTAGGGGGAGTGTTTACGTCAATCGGTAAGGCACTACACCAGGTATTCGGAGCGAT
ATACGGAGCCGCATTTAGCGGAGTGTCGTGGACAATGAAGATACTGATCGGAGTGATAATCAC
ATGGATCGGAATGAATAGTAGGTCTACAAGTCTATCCGTTAGCTTAGTGTTAGTCGGAGTCGTT
ACACTGTATCTAGGCGCTATGGTGCAAGCCGATAGTGGTTGCGTTGTGAGCTGGAAAAATAAA
GAACTGAAATGTGGCAGCGGGATCTTCATCACAGATAACGTACACACATGGACAGAACAATAT
AAGTTCCAACCAGAATCCCCTTCAAAACTAGCTTCAGCTATCCAAAAAGCTCATGAAGAGGGC
ATTTGTGGAATCCGCTCAGTAACAAGATTGGAGAATCTGATGTGGAAACAAATAACACCAGAA
TTGAATCATATTCTATCAGAAAATGAGGTAAAGTTGACCATTATGACAGGAGACATTAGAGGA
ATCATGCAGGCAGGAAAACGATCCTTGCGGCCCCAGCCCACTGAGCTGAAGTACTCATGGAAA
ACATGGGGAAAGGCGAAAATGCTCTCCACAGAGTCTCACAATCAGACCTTTCTTATTGATGGC
CCTGAAACAGCAGAATGCCCCAACACAAACAGAGCCTGGAACTCGCTGGAAGTTGAAGACTA
TGGTTTTGGAGTTTTCACCACCAATATATGGCTGAAATTGAGAGAAAAACAGGATGTATTTTGT
GACTCAAAACTCATGTCAGCGGCCATTAAAGACAACAGAGCCGTTCATGCTGATATGGGTTAT

TGGATAGAAAGTGCACTCAATGACACATGGAAGATGGAGAAAGCCTCCTTCATTGAAGTTAAA AGCTGCCACTGGCCAAAGTCACACACCCTTTGGAGCAATGGAGTATTAGAAAGTGAGATGATA ATCCCAAAAAATTTTGCCGGGCCAGTGTCACAACACAACTACAGACCAGGCTACCATACACAA ACAGCAGGACCTTGGCATCTAGGTAAGCTTGAGATGGACTTTGATCTCTGCGAAGGAACTACA GTGGTGGTGACTGAGGACTGTGGAAATAGAGGACCCTCTTTAAGAACGACCACTGCCTCTGGA AAACTCATAACAGAATGGTGCTGCCGATCCTGCACACTACCACCTCTAAGATACAGAGGTGAG GATGGATGCTGGTACGGGATGGAAATCAGACCATTGAAAGAGAAAGAAGAGAATTTGGTCAA CTCCTTGGTCACAGCCGGACATGGGCAGATTGACAACTTTTCACTAGGAGTCTTGGGAATGGC ACTGTTCCTGGAAGAAATGCTCAGGACCCGAATAGGAACGAAACATGCAATACTGCTAGTTGC AGTATCTTTTGTGACATTGATTACTGGGAACATGTCTTTTAGAGACCTGGGAAGAGTGATGGTT ATGGTGGGCGCTACCATGACGGATGACATAGGTATGGGAGTGACTTATCTTGCCCTACTAGCA GCTTTTAAGGTTAGACCAACTTTTGCAGCTGGACTACTCTTAAGAAAACTGACCTCCAAGGAAT TGATGATGGCCACCATAGGAATCGCACTCCTTTCCCAAAGCACCATACCAGAGACCATTCTTG AACTGACTGATGCATTAGCCCTGGGCATGATGGTCCTCAAAATAGTGAGAAATATGGAAAAAT ACCAATTGGCAGTGACTATCATGGCTATTTCATGTGTCCCAAATGCAGTGATACTGCAAAACGC ATGGAAGGTGAGTTGCACAATATTGGCAGCGGTGTCCGTTTCACCACTGCTCTTAACATCCTCA CAGCAGAAAGCGGATTGGATACCACTGGCATTGACGATAAAAGGTCTCAATCCAACAGCCATT TTTTTAACAACTCTCTCGAGGACCAGCAAGAAAAGGAGCTGGCCGCTAAATGAAGCTATCATG GCAGTTGGGATGGTGAGCATTTTAGCCAGTTCTCTCCTAAAGAATGATATTCCTATGACAGGTC CATTAGTGGCTGGAGGACTCCTCACCGTATGTTACGTGCTCACTGGACGATCGGCCGATTTGGA ACTGGAGAGAGCTGCCGATGTAAAATGGGAAGATCAGGCAGAAATATCAGGAAGCAGCCCAA TCCTGTCAATAACAATATCAGAAGATGGCAGCATGTCGATAAAAAATGAAGAGGAAGAACAA ACACTGACCATACTCATCAGAACGGGATTGTTGGTGATCTCAGGAGTCTTTCCAGTATCGATAC CAATTACGGCAGCAGCATGGTACCTGTGGGAAGTAAAGAAACAACGGGCTGGAGTACTGTGG GACGTCCCTTCACCCCCACCAGTGGAAAAAGCCGAACTGGAGGATGGAGCCTACAGAATCAA GCAAAGAGGGATCCTTGGATATTCTCAGATTGGAGCCGGAGTTTACAAAGAAGGAACATTCCA TACAATGTGGCACGTCACACGTGGTGCTGTTCTGATGCATAGAGGGAAGAGGATTGAACCATC ATGGGCAGATGTCAAGAAAGACCTAATATCATATGGAGGAGGCTGGAAGCTAGAAGGAGAAT GGAAGGAAGGAGAGGAAGTCCAAGTCCTGGCATTGGAACCTGGAAAAAATCCAAGAGCCGTC CAAACGAAACCTGGAATATTCAAAACCAACACCGGAACCATAGGCGCCGTATCTCTGGACTTT TCCCCTGGAACGTCAGGATCTCCAATCGTCGACAGAAAAGGAAAAGTTGTGGGTCTTTACGGT AATGGTGTTGTCACAAGGAGTGGAGCATATGTAAGTGCTATAGCCCAGACCGAAAAAAGCATT GAAGACAATCCAGAGATCGAAGATGACATTTTCCGAAAGAAAAGATTGACCATCATGGACCTC CATCCAGGAGCAGGAAAGACAAAAAGATACCTTCCAGCCATAGTTAGAGAAGCCATAAAACG TGGCTTGAGAACATTGATCCTGGCTCCCACTAGAGTAGTGGCAGCTGAAATGGAGGAAGCTCT TAGAGGACTTCCAATAAGATACCAAACTACAGCCATCAAAACCGAGCATACCGGGCGGGAGA TCGTGGACCTAATGTGTCATGCCACATTTACTATGAGGCTGTTATCACCAGTCAGAGTGCCAAA

TTACAACCTGATCATCATGGACGAAGCCCACTTCACAGACCCAGCAAGTATAGCAGCTAGAGG ATACATTTCAACTCGAGTAGAGATGGGTGAAGCAGCCGGGATTTTCATGACAGCCACTCCTCC GGGAAGTAGAGACCCATTTCCTCAGAGCAATGCACCAATTATGGATGAGGAAAGAGAAATCC CTGAGCGTTCATGGAATTCAGGACACGAATGGGTCACGGATTTTAAGGGGAAGACTGTTTGGT TTGTTCCAAGTATAAAAGCAGGAAATGATATAGCAGCTTGTCTTAGGAAAAATGGAAAGAAA GTGATACAACTCAGTAGGAAGACTTTTGACTCTGAGTATGTTAAGACTAGAGCCAATGATTGG GACTTTGTGGTCACAACTGACATTTCAGAAATGGGTGCCAACTTCAAGGCTGAGAGGGTTATA GACCCCAGACGTTGCATGAAACCAGTTATACTAACAGATGGCGAGGAGCGGGTGATCTTGGCT GGACCTATGCCAGTGACCCACTCTAGTGCAGCGCAAAGAAGAGGGAGAATAGGAAGAAATCC AAAAAATGAAAATGACCAGTACATATACATGGGGGAACCTCTTGAAAATGATGAAGACTGTG CACATTGGAAAGAAGCTAAAATGCTCCTAGATAACATCAACACACCTGAAGGAATCATTCCTA GTATGTTCGAACCAGAGCGTGAAAAAGTGGATGCCATTGATGGTGAATACCGTTTGAGAGGAG AAGCAAGGAAAACCTTTGTGGACCTAATGAGAAGAGGGGACTTACCAGTCTGGTTGGCCTACA AAGTGGCAGCTGAAGGCATCAACTACGCAGACAGAAAGTGGTGTTTTGATGGAATTAAGAAC AACCAAATACTGGAAGAAAATATGGAAGTGGAAATCTGGACAAAAGAAGGGGAAAGGAAAA AATTAAAACCCAGATGGTTGGATGCTAGGATCTATTCTGACCCACTAGCACTAAAAGAATTCA AGGAATTTGCAGCTGGAAGAAAATCTTTGACCCTGAACCTAATCACAGAAATGGGTAGGCTTC CAACTTTCATGACTCAGAAAGCAAGAAACGCACTGGACAACCTGGCTGTGCTGCATACGGCTG AGGCAGGTGGAAGGGCGTACAATCATGCTCTCAGTGAACTGCCGGAGACCCTGGAGACACTG CTCCTACTGACACTTCTGGCAACAGTCACAGGAGGAATCTTCTTATTCTTAATGAGCGGAAAAG GTATAGGGAAGATGACCCTGGGAATGTGTTGCATAATCACGGCTAGTATCCTCCTATGGTATG CACAGATACAACCACACTGGATAGCAGCTTCAATAATACTGGAGTTTTTCTCATAGTTTTGCT CATTCCAGAACCAGAAAAACAGAGAACACCCCAAGACAACCAATTGACCTACGTTGTCATAGC CATCCTCACAGTGGTGGCCGCAACCATGGCAAACGAGATGGGTTTCCTGGAAAAAACCAAGA AAGACCTCGGATTGGGAAGCATTACAACCCAGGAATCTGAGAGCAATATCCTGGACATAGATC TACGCCCTGCATCAGCATGGACGCTGTATGCCGTAGCTACAACATTTGTCACACCAATGTTGAG ACATAGCATTGAAAATTCCTCAGTGAATGTCTCCCTAACAGCCATTGCTAACCAAGCTACAGTG CTAATGGGTCTTGGGAAAGGATGGCCATTGTCAAAGATGGACATTGGAGTTCCCCTCCTTGCC ATTGGATGCTATTCACAAGTCAACCCTATAACTCTCACAGCAGCTCTCCTTTTATTGGTAGCAC ATTATGCCATTATAGGGCCAGGACTTCAAGCAAAAGCAACCAGAGAAGCCCAGAAAAGAGCA GCAGCAGGCATCATGAAAAACCCAACAGTCGATGGAATAACAGTGATTGACCTAGAACCAAT ACCCTATGATCCAAAATTTGAAAAGCAGTTAGGACAAGTAATGCTCCTAATCCTCTGCGTGACT CAAGTATTAATGATGAGGACTACATGGGCTTTATGTGAGGCTCTAACCCTAGCGACCGGGCCC ATCTCCACACTGTGGGAAGGAAATCCAGGGAGGTTTTGGAACACTACCATTGCAGTGTCAATG GCTAACATCTTTAGGGGGAGCTACTTGGCCGGAGCTGGACTTCTCTTTTCCATCATGAAAAACA CAACAAACACAAGAAGAGGAACTGGCAACATAGGAGAGACACTTGGAGAAAAATGGAAAAG TCGATTAAACGCACTGGGGAAAGTGAATTTCAAATCTACAAGAAAAGTGGAATCCAGGAAG

TGGATAGAACCCTAGCAAAAGAAGGTATCAAAAGAGGAGAAACGGACCACCATGCTGTGTCG
CGAGGTTCAGCAAAACTGAGATGGTTCGTCGAGAGAAATATGGTCACACCGGAAGGGAAGGT
GGTGGATCTCGGTTGCGGCAGAGGGGGCTGGTCATACTATTGCGGGGGACTAAAGAATGTAAG
AGAAGTCAAAGGCCTAACAAAAGGAGGACCAGGACATGAAGAACCCATCCCCATGTCAACAT
ATGGGTGGAATCTAGTGCGTCTGCAAAGTGGAGTTGACGTTTCTTCACCCCGCCAGAAAAGT
GTGATACATTGTTGTGTGACATAGGGGAGTCGTCACCAAATCCCACGATAGAAGCAGGACGAA
CACTCAGAGTCCTCAACTTAGTGGAAAATTGGTTGAACAATAACACCCAATTTTGCATAAAGG
TCCTCAACCCATATATGCCTTCAGTCATAGAAAAAATGGAAGCATTACAAAGGAAATATGGAG
GAGCCTTAGTGAGGAATCCACTCTCACGAAACTCCACGCATGAAATGTACTGGGTATCTAATG
CCACCGGGAACATAGTGTCATCAGTGAACATGATTTCAAGGATGTTGATTAACAGATTCACAA
TGAAACACAAGAAAGCCACTTACGAGCCAGATGTTGACCTAGGAAGTGGAACCCGCAACATT
GGAATTGAAAGTGAGATACCAAATCTAGACATAATAGGAAAGAGAATAGAGAAAATAAAACA
AGAGCATGAAACATCATGGCACTATGATCAAGACCACCCATACAAAACGTGGGCTTACCATGG
CAGCTATGAAACAAAACAAACTGGATCAGCATCATCTATGGTGAACGGAGTGGTCAGATTGCT
GACAAAACCTTGGGACGTCGTCCCTATGGTGACACAGATGGCAATGACAGACACGACTCCTTT
TGGACAACAGCGCGTTTTCAAAGAGAAAGTAGACACGAGAACCCAAGAACCGAAGGAAGGCA
CAAAGAAACTGATGAAAATTACGGCAGAGTGGCTTTGGAAAGAACTAGGAAAGAAAAAGACA
CCTAGGATGTGTACCAGAGAAGAATTCACAAGAAAAGTGAGAAGCAATGCAGCCTTGGGGGC
CATATTCACTGATGAGAACAAATGGAAATCGGCACGTGAGGCTGTTGAAGATGGTAGGTTCTG
GGAGCTGGTTGACAGGGAAAGAAATCTCCATCTTGAAGGAAAGTGTGAAACATGTGTGTACA
ACATGATGGGAAAAAGAGAGAAGAAACTAGGGGAGTTCGGCAAGGCAAAAGGTAGCAGAGC
CATATGGTACATGTGGCTTGGAGCACGCTTCTTAGAGTTTGAAGCCCTAGGATTCCTGAATGAA
GATCACTGGTTCTCCAGAGGGAACTCCCTGAGTGGAGTGGAAGGAGAAGGGCTGCACAGGCT
AGGCTACATTTTAAGAGACGTGGGCAAGAAGGAAGGGGGGGCAATGTACGCCGATGATACAG
CAGGATGGGACACAAGAATCACACTAGAAGACTTAAAAAATGAAGAAATGGTAACGAACCAC
ATGAAAGGAGAACACAAGAAACTAGCCGAGGCCATATTCAAACTAACGTACCAAAACAAGGT
GGTGCGTGTGCAAAGACCAACACCAAGAGGTACAGTAATGGATATCATATCGAGAAGAGACC
AAAGAGGCAGTGGGCAAGTCGGCACCTATGGCCTTAATACCTTCACCAATATGGAAGCCCAAT
TAATTAGACAGATGGAGGGAGAAGGAATCTTCAAAAGCATTCAGCACCTGACAGCCACAGAA
GAAATCGCTGTACAGAACTGGCTAGCAAGAGTGGGGCGTGAAAGGCTATCAAGAATGGCAAT
CAGTGGAGATGATTGTGTTGTAAAACCTATAGATGACAGATTTGCAAGTGCTTTAACAGCTCTA
AATGACATGGGAAAAGTTAGAAAAGATATACAACAATGGGAACCTTCAAGAGGATGGAACGA
TTGGACACAAGTGCCTTTCTGTTCACACCACTTTCATGAGTTAGTCATGAAAGATGGTCGCGTG
CTCGTAGTCCCATGCAGAAACCAAGATGAACTGATTGGTAGAGCCCGAATTTCCCAGGGAGCT
GGGTGGTCTTTGAAAGAGACGGCCTGTTTGGGAAAGTCTTACGCCCAAATGTGGACTCTGATG
TACTTCCACAGACGTGACCTCAGACTGGCGGCAAATGCCATCTGCTCGGCAGTCCCGTCACATT
GGGTTCCAACAAGTCGAACAACCTGGTCCATACACGCTAAGCATGAATGGATGACGACGGAA

GACATGCTGGCAGTCTGGAACAGGGTGTGGATCCAAGAAAACCCGTGGATGGAAGATAAAAC
TCCAGTGGAATCATGGGAAGAAGTCCCATACTTGGGAAAAAGAGAAGACCAATGGTGCGGCT
CATTGATTGGGCTAACAAGCAGGGCTACCTGGGCAAAGAACATCCAAACAGCAATAAATCAA
GTCAGATCCCTTATAGGCAATGAGGAATACACAGACTACATGCCATCCATGAAGAGATTTAGA
AGGGAAGAGGAAGAGGCAGGTGTCCTGTGGTAGAAGGCAAAACTAACATGAAACAAGGCTAA
AAGTCAGGTCGGATTAAGCCATAGTACGGAAAAAACTATGCTACCTGTGAGCCCCGTCCAAGG
ACGTTAAAAGAAGTCAGGCCATCACAAAATGCCACAGCTTGAGTAAACTGTGCAGCCTGTAGC
TCCACCTGAGGAGGTGTAAAAAACCTGGGAGGCCACAAACCATGGAAGCTGTACGCATGGCG
TAGTGGACTAGCGGTTAGAGGAGACCCCTCCCTTACAAATCGCAGCAAACAACGGGGGCCCA
AGGTGAGATGAAGCTGTAATCTCACTGGAAGGACTAGAGGTTAGAGGAGACCCCCCCAAAAC
AAAAAACAGCATATTGACGCTGGGAAAGACCAGAGATCCTGCTGTCTCCTCAGCATCATTCCA
GGCACAGAACGCCAGAAAATGGAATGGTGCTGTTGAATCAACAGGTTCT

SEQ ID NO:11 - DENV-3-VE-BID-V2268-2008-E-W/Min

AGTTGTTAGTCTACGTGGACCGACAAGAACAGTTTCGACTCGGAAGCTTGCTTAACGTAGTGCT
AACAGTTTTTTATTAGAGAGCAGATCTCTGATGAACAACCAACGGAAGAAGACGGGAAAACC
GTCTATCAATATGCTGAAACGCGTGAGAAACCGTGTGTCAACTGGATCACAGTTGGCGAAGAG
ATTCTCAAAAGGACTGCTGAACGGCCAGGGACCAATGAAATTGGTTATGGCGTTCATAGCTTT
CCTCAGATTTCTAGCCATTCCACCAACAGCAGGAGTCTTGGCTAGATGGGGAACCTTCAAGAA
GTCGGGGGCCATTAAGGTCCTGAAAGGCTTTAAGAAGGAGATCTCAAACATGCTGAGCATAAT
CAACAAACGGAAAAAGACATCGCTCTGTCTCATGATGATATTGCCAGCAGCACTTGCTTTCCA
CTTGACTTCACGAGATGGAGAGCCGCGCATGATTGTGGGGAAGAATGAAAGAGGAAAATCCC
TACTTTTTAAGACAGCCTCTGGAATTAACATGTGCACACTCATAGCCATGGACTTGGGAGAGAT
GTGTGATGACACGGTCACTTACAAATGCCCCCATATTACCGAAGTGGAACCTGAAGACATTGA
CTGCTGGTGCAACCTCACATCAACATGGGTGACTTATGGAACGTGCAATCAAGCCGGAGAGCA
TAGACGCGATAAGAGATCAGTGGCGTTAGCTCCCCATGTCGGCATGGGACTGGATACACGCAC
CCAAACTTGGATGTCGGCTGAAGGAGCTTGGAGGCAAGTCGAGAAGGTAGAGACATGGGCCC
TTAGGCACCCAGGGTTCACCATACTAGCCCTATTTCTTGCCCATTACATAGGCACTTCCTTGAC
CCAGAAGGTGGTTATTTTTATACTGCTAATGCTGGTCACCCCATCCATGACAATGAGATGTGTG
GGAGTAGGAAACAGAGATTTTGTGGAAGGACTATCAGGAGCTACGTGGGTTGACGTGGTGCTC
GAGCACGGGGGGTGTGTGACCACCATGGCTAAGAACAAGCCCACGTTGGATATAGAGCTTCA
GAAGACCGAGGCCACCCAACTGGCGACCCTAAGGAAGCTATGCATTGAGGGGAAAATCACCA
ACATAACAACTGACTCAAGATGTCCTACCCAAGGGGAAGCGGTTTTGCCTGAGGAGCAGGACC
AAAACTACGTGTGTAAGCATACATACGTAGACAGAGGCTGGGGGAACGGATGTGGTTTGTTTG
GTAAGGGAAGCTTGGTAACATGTGCGAAATTTCAATGCCTGGAACCAATAGAGGGAAAAGTG
GTGCAATATGAGAACCTCAAATACACCGTTATCATCACAGTGCACACAGGAGACCAACACCAG
GTGGGAAATGAAACGCAGGGAGTCACGGCTGAGATAACACCTCAGGCATCAACCACTGAAGC

CATCTTGCCTGAATATGGAACCCTTGGGCTAGAATGCTCACCACGGACAGGTTTGGACTTCAAT
GAAATGATCTTGCTAACAATGAAGAACAAAGCATGGATGGTACATAGACAATGGTTTTTGAC
CTACCTCTACCATGGACATCAGGAGCTACAACGGAAACACCAACCTGGAACAGGAAGGAGCT
TCTTGTGACATTTAAAAACGCACATGCGAAGAAACAAGAAGTAGTCGTTTTAGGGTCACAGGA
GGGAGCTATGCATACCGCACTAACCGGAGCGACTGAGATACAGAATAGCGGAGGGACATCAA
TCTTCGCCGGACACCTTAAGTGTAGATTGAAAATGGACAAACTTGAGCTTAAGGGGATGTCAT
ACGCTATGTGTACGAACACATTCGTGCTGAAAAAAGAGGTAAGCGAAACGCAACACGGAACG
ATACTGATTAAGGTTGAGTATAAGGGCGAAGACGTCCCATGTAAGATACCTTTTTCGACAGAG
GACGGACAGGGTAAGGCCCATAACGGAAGACTGATTACCGCTAACCCAGTGGTGACCAAAAA
AGAGGAACCAGTCAATATCGAAGCCGAACCTCCATTCGGAGAGTCAAACATAGTGATAGGGA
TAGGCGATAACGCACTTAAGATTAACTGGTACAAAAAAGGGTCATCAATCGGTAAGATGTTTG
AGGCAACCGCTAGGGGGGCTAGACGGATGGCCATACTCGGAGACACCGCATGGGACTTCGGA
TCCGTGGGGGGGTACTTAACTCACTCGGTAAGATGGTGCACCAAATTTTCGGATCCGCATAT
ACCGCTCTATTTAGCGGAGTGTCATGGGTGATGAAAATCGGAATCGGAGTTCTATTGACATGG
ATCGGATTGAACTCTAAGAATACATCTATGTCATTTTCATGTATCGCAATCGGAATTATTACGC
TATATCTCGGAGCCGTAGTGCAAGCCGACATGGGGTGTGTTATAAACTGGAAAGGCAAAGAAC
TCAAGTGTGGAAGTGGAATCTTCGTCACCAACGAGGTCCATACCTGGACAGAGCAATACAAAT
TCCAAGCAGACTCCCCAAAAAGATTGGCGACAGCCATTGCAGGCGCTTGGGAGAATGGAGTGT
GCGGAATTAGGTCAACAACCAGAATGGAGAATCTCCTGTGGAAGCAAATAGCCAATGAACTG
AACTACATATTGTGGGAAAACAATATCAAATTAACGGTTGTTGTGGGCGATATAATTGGGGTC
TTAGAGCAAGGGAAAAGAACACTAACACCACAACCCATGGAGCTAAAATACTCATGGAAAAC
GTGGGGAAAGGCAAAAATAGTGACAGCTGAAACACAAAATTCTTCTTTCATAATAGATGGACC
AAACACACCGGAGTGTCCAAGTGCCTCAAGAGCATGGAATGTGTGGGAGGTGGAAGATTACG
GGTTCGGAGTCTTCACAACCAACATATGGCTGAAACTCCGAGAGGTGTATACCCAACTATGTG
ACCATAGGTTAATGTCGGCAGCCGTCAAGGATGAAAGGGCCGTACATGCCGACATGGGCTATT
GGATAGAAAGTCAAAAGAATGGAAGTTGGAAGCTAGAAAAAGCATCCCTCATAGAGGTGAAA
ACCTGCACATGGCCAAAATCACATACCCTTTGGAGTAATGGTGTGTTAGAGAGTGACATGATC
ATTCCAAAAAGTCTAGCTGGTCCTATCTCGCAACACAACTACAGGCCCGGGTACCACACCCAG
ACGGCGGGACCTTGGCATTTAGGAAAATTAGAGCTGGACTTCAACTATTGTGAAGGAACAACA
GTTGTCATCACAGAAAACTGTGGGACAAGAGGCCCATCATTGAGAACAACAACAGTGTCAGG
GAAGTTAATACACGAATGGTGCTGCCGCTCGTGCACACTTCCTCCCCTGCGATACATGGGAGA
AGACGGTTGCTGGTATGGCATGGAAATCAGACCCATCAGTGAGAAAGAAGAAAACATGGTAA
AGTCTTTAGTCTCAGCGGGAAGTGGAGAGGTGGACAACTTCACAATGGGTGTCTTGTGTTTGG
CAATCCTCTTTGAAGAGGTGATGAGAGGAAAATTTGGGAAGAAACACATGATTGCGGGGGTTT
TCTTCACGTTTGTACTCCTTCTCTCAGGGCAAATAACATGGAGAGATATGGCGCACACACTAAT
AATGATTGGGTCCAATGCATCTGACAGGATGGGAATGGGCGTCACCTACCTAGCTTTAATTGC
AACATTTAAAATCCAGCCATTTTTGGCTTTGGGATTTTTCCTAAGAAAACTGACATCCAGAGAA

AATTTATTGTTAGGAGTTGGGCTGGCTATGGCAACAACGTTACAACTGCCAGAGGACATTGAA
CAAATGGCAAATGGAATCGCTCTGGGGCTCATGGCTCTCAAACTGATAACACAATTTGAAACA
TACCAACTATGGACAGCATTAATCTCCTTAACGTGTTCAAATACAATTTTTACGTTGACTGTTG
CCTGGAGAACAGCCACCCTGATTTTGGCTGGAGTTTCACTTTTACCAGTGTGCCAGTCTTCGAG
TATGAGGAAAACAGACTGGCTTCCAATGACAGTGGCAGCTATGGGAGTTCCACCTCTACCACT
TTTTATTTTTAGCTTAAAAGACACACTCAAAAGGAGAAGCTGGCCACTGAATGAAGGGGTGAT
GGCTGTTGGGCTTGTGAGCATTCTGGCCAGTTCTCTCCTTAGAAATGATGTACCCATGGCTGGA
CCATTAGTGGCCGGGGGCTTGCTGATAGCGTGCTACGTCATAACTGGCACGTCAGCAGACCTC
ACCGTAGAAAAAGCAGCAGATGTAACATGGGAGGAAGAGGCTGAGCAAACAGGAGTGTCCCA
CAACTTAATGATCACAGTTGATGATGATGGAACAATGAGAATAAAAGATGATGAGACTGAGA
ATATCTTAACAGTGCTTTTGAAAACAGCATTACTAATAGTGTCAGGAATCTTTCCATACTCCAT
ACCCGCAACATTGTTGGTCTGGCATACTTGGCAAAAGCAAACCCAAAGATCCGGCGTTCTATG
GGATGTACCCAGCCCTCCAGAGACACAGAAAGCAGAACTGGAAGAAGGGGTCTATAGGATCA
AACAGCAAGGAATTTTTGGGAAAACCCAAGTAGGGGTTGGAGTACAGAAAGAAGGAGTCTTT
CACACCATGTGGCACGTTACAAGAGGGGCAGTGTTGACATATAATGGGAAAAGACTGGAACC
AAATTGGGCTAGCGTGAAAAAAGATCTGATTTCATACGGAGGAGGATGGAGATTGAGCGCAC
AATGGCAAAAGGGGGAGGAGGTGCAGGTTATTGCCGTAGAGCCTGGGAAGAACCCAAAGAAC
TTTCAAACCATGCCAGGCACTTTTCAGACTACAACAGGGGAAATAGGAGCAATTGCACTGGAT
TTCAAGCCCGGAACTTCAGGATCTCCTATCATAAACAGAGAGGGAAAGGTAGTGGGACTGTAT
GGCAATGGAGTGGTTACAAAGAATGGTGGCTACGTCAGCGGAATAGCGCAAACGAATGCAGA
ACCAGATGGACCGACACCAGAATTGGAAGAAGAGATGTTCAAAAAGCGAAATCTAACCATAA
TGGATCTTCATCCTGGGTCAGGAAAGACACGGAAATACCTTCCAGCTATTGTTAGAGAGGCAA
TCAAGAGACGTTTGAGAACTCTAATTCTGGCACCAACAAGGGTGGTTGCAGCTGAGATGGAAG
AAGCATTGAAAGGGCTCCCAATAAGGTACCAAACAACAGCAACAAAATCTGAACACACAGGA
AGAGAGATTGTTGATCTGATGTGCCACGCAACGTTCACAATGCGTCTGCTGTCACCAGTTAGG
GTTCCAAACTATAACTTGATAATAATGGATGAAGCCCATTTCACAGACCCAGCCAGTATAGCT
GCTAGAGGGTACATATCAACTCGTGTTGGAATGGGAGAAGCAGCCGCAATATTCATGACAGCA
ACGCCCCCTGGAACAGCTGATGCCTTTCCCCAGAGCAACGCTCCAATTCAAGATGAAGAAAGG
GACATACCAGAACGCTCATGGAATTCAGGCAATGAATGGATAACCGACTTCGCTGGGAAAAC
GGTGTGGTTTGTCCCCAGCATTAAAGCCGGAAATGACATAGCAAATTGCCTGCGGAAAAACGG
GAAAAAGGTCATTCAACTTAGTAGGAAGACTTTTGACACAGAATATCAGAAAACCAAACTGA
ATGATTGGGACTTTGTGGTGACGACTGACATTTCAGAAATGGGGGCCAATTTCAAAGCAGATA
GAGTGATCGACCCAAGAAGATGTCTCAAACCAGTGATCCTGACAGATGGACCAGAGCGGGTG
ATCCTGGCTGGACCAATGCCAGTCACCGCAGCGAGTGCTGCGCAAAGGAGAGGGAGAGTTGG
CAGGAACCCACAAAAAGAAAATGACCAGTACATATTCACGGGCCAGCCTCTCAACAATGATG
AAGACCATGCTCACTGGACAGAAGCAAAAATGCTGCTGGACAACATTAACACACCAGAAGGG
ATCATACCAGCTCTCTTTGAGCCAGAAAGGGAGAAGTCAGCCGCCATAGACGGTGAGTATCGC

CTGAAAGGTGAGTCCAGGAAGACTTTCGTGGAACTCATGAGGAGGGGTGACCTTCCAGTCTGG TTAGCCCATAAAGTAGCATCAGAAGGGATCAAATATACAGATAGAAAATGGTGCTTTGATGGA CAACGTAATAATCAAATTTTAGAAGAGAACATGGATGTGGAAATCTGGACAAAGGAAGGAGA AAAGAAAAAATTGAGACCTAGGTGGCTTGATGCTCGCACCTATTCAGATCCCTTAGCACTCAA GGAATTCAAGGACTTTGCGGCTGGCAGGAAGTCAATAGCCCTTGATCTTGTGACAGAAATAGG AAGAGTGCCTTCACACCTAGCTCATAGAACGAGAAACGCTCTGGACAATCTGGTGATGCTGCA TACGTCAGAACATGGCGGTAAGGCCTACAGGCATGCGGTGGAGGAACTACCAGAGACAATGG AAACACTCCTACTCTTGGGACTCATGATCTTGTTGACAGGTGGAGCAATGCTTTTCTTAATATC AGGTAAAGGGATTGGAAAGACTTCAATAGGACTCATTTGTGTAATTGCTTCCAGCGGCATGTT GTGGATGGCCGAAATCCCACTCCAATGGATCGCGTCGGCCATAGTCCTGGAGTTTTTATGATG GTGTTGCTTATACCAGAACCAGAGAAGCAGAGAACCCCCCAAGACAACCAACTCGCATATGTC GTGATAGGCATACTTACACTGGCAGCAATAATAGCAGCCAATGAAATGGGATTGTTGGAAACT ACAAAGAGAGATTTAGGAATGTCTAAGGAGCCAGGTGTTGTTTCTCCAACCAGCTATTTAGAT GTGGACTTGCACCCAGCATCAGCCTGGACATTGTACGCTGTGGCCACTACAGTAATAACACCA ATGTTAAGACATACCATAGAGAATTCCACAGCAAATGTGTCCTTGGCAGCTATAGCCAACCAG GCAGTGGTCCTGATGGGTTTGGACAAAGGATGGCCAATATCAAAAATGGACTTAGGAGTACCC CTGCTGGCATTGGGTTGCTATTCACAAGTGAACCCACTGACTCTAACAGCGGCAGTACTCTTGC TGATCACACATTATGCCATTATAGGTCCAGGATTGCAGGCAAAAGCCACCCGTGAAGCTCAGA AAAGGACAGCTGCTGGAATAATGAAGAATCCAACAGTGGATGGGATAATGACAATAGACCTA GATCCTGTAATATATGATTCAAAATTTGAAAAGCAACTGGGACAGGTTATGCTCCTGGTTTTGT GTGCAGTTCAACTTTTGTTAATGAGAACATCATGGGCCTTGTGTGAAGCTTTAACCCTAGCTAC AGGACCAATAACAACACTCTGGGAAGGATCACCTGGGAAGTTTTGGAACACCACGATAGCTGT TTCCATGGCGAACATTTTTAGAGGGAGCTATTTAGCAGGAGCTGGGCTTGCTTTCTCTATTATG AAATCAGTTGGAACAGGGAAAAGAGGAACAGGCTCACAGGGTGAAACTTTGGGAGAAAAATG GAAAAAGAAGTTAAATCAATTATCCCGGAAAGAGTTTGACCTTTACAAGAAATCTGGAATCAC TGAGGTGGATAGAACAGAAGCCAAAGAAGGGTTGAAAAGAGGAGAAATAACACATCATGCCG TGTCCAGAGGTAGTGCAAAACTTCAATGGTTTGTGGAGAGAAACATGGTCATTCCCGAAGGAA GAGTTATAGACTTGGGCTGTGGAAGAGGAGGCTGGTCATATTACTGTGCAGGACTGAAAAAAG TCACAGAAGTGCGAGGATACACAAAAGGCGGTCCAGGACACGAAGAACCAGTACCTATGTCC ACATATGGATGGAACATAGTTAAGTTAATGAGTGGAAAGGATGTGTTTTATCTTCCACCTGAA AAGTGTGACACCCTGTTGTGCGACATTGGAGAATCTTCACCAAGCCCAACAGTGGAAGAAAGC AGAACTATAAGAGTTTTGAAGATGGTTGAACCATGGCTAAAGAACAACCAATTTTGTATTAAA GTATTGAACCCTTACATGCCAACTGTGATTGAGCACCTAGAAAGACTACAAAGGAAACATGGA GGAATGCTTGTGAGAAATCCACTTTCACGAAACTCCACGCACGAAATGTACTGGATATCCAAT GGCACAGGTAACATTGTCGCTTCAGTCAACATGGTATCTAGACTGCTACTGAACAGGTTCACG ATGACACACAGAAGACCCACCATTGAGAAAGATGTGGATTTAGGAGCAGGAACTCGACATGT TAATGCGGAACCAGAAACACCCAACATGGATGTCATTGGGGAAAGAATAAAAAGGATCAAGG

AGGAGCATAATTCAACATGGCACTACGATGACGAAAACCCCTACAAAACGTGGGCTTACCATG
GATCTTATGAAGTCAAAGCCACAGGCTCAGCCTCCTCCATGATAAATGGAGTCGTGAAACTCC
TCACTAAACCATGGGATGTGGTGCCCATGGTGACACAGATGGCAATGACAGATACAACTCCAT
TTGGCCAGCAGAGAGTCTTTAAAGAGAAAGTGGACACCAGGACACCCAGGTCCATGCCAGGA
ACAAGAAGGGTTATGGGGATCACAGCGGAGTGGCTCTGGAGAACCCTGGGAAGGAATAAAAA
ACCCAGGTTATGCACAAGGGAAGAGTTTACAAAAAAGGTCAGAACTAACGCAGCCATGGGAG
CTGTTTTCACAGAGGAGAACCAATGGGACAGCGCGAAAGCTGCTGTTGAGGATGAGGATTTTT
GGAAACTTGTGGACAGAGAACGTGAACTCCACAAACTGGGCAAGTGTGGAAGCTGTGTTTACA
ACATGATGGGTAAGAGAGAGAAGAAACTTGGAGAGTTTGGCAAAGCAAAAGGCAGTAGAGCT
ATATGGTACATGTGGTTGGGAGCCAGGTACCTTGAGTTCGAAGCCCTTGGATTCTTAAATGAA
GACCACTGGTTCTCGCGTGAGAACTCTTACAGTGGAGTGGAAGGAGAAGGACTGCACAAGCTA
GGCTATATATTAAGGGACATTTCCAAGATACCCGGAGGAGCTATGTATGCTGATGACACAGCT
GGTTGGGACACAAGAATAACAGAAGATGACCTGCACAATGAGGAAAAGATCACACAGCAAAT
GGACCCTGAACACAGGCAGTTAGCGAACGCTATATTTAAGCTCACATACCAAAACAAAGTGGT
CAAAGTTCAACGACCGACTCCAACAGGCACGGTAATGGATATCATATCTAGGAAAGACCAAA
GAGGCAGTGGACAGGTAGGAACTTATGGTCTGAATACATTCACCAACATGGAAGCCCAGTTAA
TCAGACAAATGGAAGGAGAAGGTGTGCTGTCAAAGGCAGACCTCGAGAACCCTCATCTGCCA
GAGAAGAAAATTACACAATGGTTGGAAACCAAAGGAGTGGAGAGGTTAAAAAGAATGGCCAT
AAGTGGGGATGACTGCGTGGTGAAACCAATCGATGACAGGTTCGCTAATGCCCTGCTCGCTCT
GAACGACATGGGAAAGGTTCGGAAAGACATACCTCAATGGCAGCCATCAAAGGGATGGCATG
ATTGGCAACAGGTTCCTTTCTGCTCCCACCACTTTCATGAATTGATCATGAAAGATGGAAGAAA
GTTGGTGGTTCCCTGCAGACCCCAGGACGAACTAATAGGAAGGGCAAGAATCTCTCAAGGAGC
GGGATGGAGCCTTAGAGAAACCGCATGTCTGGGGAAAGCCTACGCTCAAATGTGGAGTCTCAT
GTATTTTCACAGAAGAGACCTCAGACTAGCATCCAACGCCATATGTTCAGCAGTACCAGTCCA
CTGGGTCCCCACAAGTAGAACGACATGGTCTATTCACGCTCACCATCAGTGGATGACCACAGA
AGACATGCTTACTGTCTGGAACAGAGTGTGGATCGAGGACAATCCATGGATGGAAGACAAAA
CTCCAGTCACAACCTGGGAAAATGTTCCATATCTAGGGAAGAGAGAAGACCAATGGTGCGGAT
CACTTATTGGTCTCACTTCCAGAGCAACCTGGGCCCAGAACATACCCACAGCAATTCAACAGG
TTAGAAGCCTTATAGGCAATGAAGAGTTTCTGGACTACATGCCTTCAATGAAGAGATTTAGGA
AGGAGGAGGAGTCGGAGGGAGCCATTTGGTAAAACGTAGGAAGTGAAAAAGAGGTTAACTGT
CAGGCCATATTAAGCCACAGTACGGAAGAAGCTGTGCTGCCTGTGAGCCCCGTCCAAGGACGT
TAAAAGAAGAAGTCAGGCCCCAAAGCCACGGTTTGAGCAAACCGTGCTGCCTGTAGCTCCGTC
GTGGGGACGTAAAACCTGGGAGGCTGCAAACTGTGGAAGCTGTACGCACGGTGTAGCAGACT
AGCGGTTAGAGGAGACCCCTCCCATGACACAACGCAGCAGCGGGGCCCGAGCACTGAGGGAA
GCTGTACCTCCTTGCAAAGGACTAGAGGTTAGAGGAGACCCCCCGCAAATAAAAACAGCATAT
TGACGCTGGGAGAGACCAGAGATCCTGCTGTCTCCTCAGCATCATTCCAGGCACAGAACGCCA
GAAAATGGAATGGTGCTGTTGAATCAACAGGTTCT

SEQ ID NO:12 - DENV4_Syn_E-W/Min

AGTTGTTAGTCTGTGTGGACCGACAAGGACAGTTCCAAATCGGAAGCTTGCTTAACACAGTTCT
AACAGTTTGTTTAAATAGAGAGCAGATCTCTGGAAAAATGAACCAACGAAAAAAGGTGGTTA
GACCACCTTTCAATATGCTGAAACGCGAGAGAAACCGCGTATCAACCCCTCAAGGGTTGGTGA
AGAGATTCTCAACCGGACTTTTTTCTGGGAAAGGACCCTTACGGATGGTGCTAGCATTCATCAC
GTTTTTGCGAGTCCTTTCCATCCCACCAACAGCAGGGATTCTGAAGAGATGGGGACAGTTGAA
GAAAAATAAGGCCATCAAGATACTGATTGGATTCAGGAAGGAGATAGGCCGCATGCTGAACA
TCTTGAACGGGAGAAAAAGGTCAACGATAACATTGTTGTGCTTGATTCCCACCGTAATGGCGT
TTCACTTGTCAACAAGAGATGGCGAACCCCTCATGATAGTGGCAAAACATGAAAGGGGGAGA
CCTCTCTTGTTTAAGACAACAGAGGGGATCAACAAATGCACTCTCATTGCCATGGACTTGGGTG
AAATGTGTGAGGACACTGTCACGTATAAATGCCCCCTACTGGTCAATACCGAACCTGAAGACA
TTGATTGCTGGTGCAACCTCACGTCTACCTGGGTCATGTATGGGACATGCACCCAGAGCGGAG
AACGGAGACGAGAGAAGCGCTCAGTAGCTTTGACACCACATTCAGGAATGGGATTGGAAACA
AGAGCTGAGACATGGATGTCATCGGAAGGGGCTTGGAAGCATGCTCAGAGAGTAGAGAGCTG
GATACTCAGAAACCCAGGATTTGCGCTCTTGGCAGGATTTATGGCTTATATGATTGGGCAAAC
AGGAATCCAGCGAACTGTCTTCTTTGTCCTAATGATGCTGGTCGCCCCATCCTACGGAATGCGG
TGCGTAGGAGTAGGAAACAGAGACTTTGTGGAAGGAGTCTCAGGTGGAGCATGGGTCGACCT
GGTGCTAGAACATGGAGGATGCGTCACAACCATGGCCCAGGGAAAACCAACCTTGGATTTTGA
ACTGACTAAGACAACAGCTAAGGAAGTGGCTCTGTTAAGAACCTATTGCATTGAAGCCTCAAT
ATCAAACATAACTACGGCAACAAGATGTCCAACGCAAGGAGAGCCTTATCTGAAAGAGGAAC
AGGACCAACAGTACATCTGCCGGAGAGATGTGGTAGACAGAGGATGGGGCAATGGCTGTGGC
TTGTTTGGAAAAGGAGGAGTTGTGACATGTGCGAAGTTTTCATGTTCGGGGAAGATAACAGGC
AATCTGGTCCAAATTGAGAACCTTGAATACACAGTGGTTGTAACAGTCCACAATGGAGACACC
CATGCAGTAGGAAATGACACATCCAATCATGGAGTTACAGCCACGATAACTCCCAGGTCACCA
TCGGTAGAAGTCAAATTGCCGGACTATGGAGAACTAACACTCGATTGTGAACCCAGGTCTGGA
ATTGACTTTAATGAGATGATCCTAATGAAAATGAAAAAGAAAACATGGCTCGTGCATAAGCAA
TGGTTTTTGGATCTGCCTCTTCCATGGACAGCAGGAGCAGACACATCAGAGGTTCACTGGAATT
ACAAAGAGAGAATGGTGACGTTCAAGGTTCCTCATGCCAAGAGACAGGACGTTACAGTGTTAG
GGTCACAGGAGGGAGCTATGCATAGCGCACTAGCCGGAGCAACCGAAGTCGATAGCGGAGAC
GGAAATCATATGTTCGCCGGACATCTGAAATGCAAAGTGAGAATGGAGAAATTGCGGATTAA
GGGAATGTCATACACTATGTGTTCCGGTAAGTTTTCGATTGACAAAGAGATGGCCGAAACGCA
ACACGGAACAACAGTCGTTAAGGTTAAGTACGAAGGAGCCGGAGCTCCGTGTAAAGTGCCAA
TCGAAATTAGAGACGTTAACAAAGAGAAAGTGGTCGGAAGAGTGATATCTAGTACACCCCTA
GCCGAAAATACGAATTCCGTTACGAATATCGAACTCGAACCCCCCTTTGGAGACTCATACATA
GTGATAGGAGTGGGTAATTCCGCACTCACGTTGCACTGGTTCAGAAAGGGATCATCAATCGGG
AAGATGTTCGAATCAACATATAGGGGAGCGAAAAGAATGGCTATATTGGGCGAAACCGCATG

GGACTTCGGATCAGTCGGAGGACTGTTTACGAGTCTGGGTAAGGCCGTACATCAGGTATTCGG ATCAGTGTATACAACAATGTTTGGCGGAGTGTCATGGATGATACGGATACTGATAGGGTTTCT AGTGTTGTGGATCGGAACGAACTCACGTAATACGTCTATGGCTATGACATGTATTGCCGTAGG GGGGATTACACTGTTTCTGGGATTTACAGTGCAAGCAGACATGGGTTGTGTGGTGTCATGGAG TGGGAGAGAATTGAAGTGTGGAAGCGGAATTTTTGTGGTTGACAACGTGCACACTTGGACAGA ACAGTACAAATTCCAACCAGAGTCCCCAGCGAGACTAGCGTCTGCAATATTAAATGCCCACAA AGATGGGGTCTGTGGAATTAGATCAACCACGAGGCTGGAAAATGTTATGTGGAAGCAAATAA CCAATGAGCTAAACTATGTTCTCTGGGAAGGAGGACATGATCTCACTGTAGTGGCTGGGGATG TGAAAGGGGTGTTGACCAAGGGCAAGAGAGCACTCACACCCCCAGCGAGTGATCTGAAATAT TCATGGAAGACATGGGGGAAAGCAAAAATCTTCACCCCTGAAGCAAGAAACAGCACATTTTTA ATAGACGGACCAGACACCTCTGAATGCCCCAATGAACGAAGGGCATGGAATTCTTTTGAGGTG GAAGACTATGGATTTGGCATGTTCACGACCAACATATGGATGAAATTCCGAGAAGGAAGTTCA GAAGTGTGTGACCACAGGTTAATGTCAGCTGCAATTAAAGACCAGAAAGCTGTGCATGCTGAC ATGGGTTATTGGATAGAGAGCTCAAAAAACCAGACCTGGCAGATAGAGAGAGCATCTCTTATT GAAGTGAAAACATGTCTGTGGCCCAAGACCCATACACTGTGGAGCAATGGAGTGCTGGAAAG CCAGATGCTTATTCCAAAATCATATGCAGGCCCTTTTTCACAGCACAATTACCGCCAGGGCTAT GCTACGCAAACCGTGGGTCCATGGCACTTAGGCAAACTAGAGATAGACTTTGGAGAATGCCCC GGAACAACAGTCACAATTCAGGAGAATTGTGACCATAGAGGCCCATCTTTGAGGACCACCACT GCATCTGGAAAACTAGTCACGCAATGGTGTTGCCGCTCCTGCACGATGCCCCCCTTAAGGTTCT TAGGAGAAGATGGGTGCTGGTATGGGATGGAGATTAGGCCCTTGAGTGAAAAAGAAGAGAAC ATGGTCAAATCACAGGTGACGGCCGGACAGGGCACATCGGAAACTTTTCAATGGGTCTGTTG TGCCTGACCTTGTTTGTGGAAGAATGCTTGAGGAGAAGAGTCACCAGGAAACACATGATATTA GCTGTGGTAATCACTCTTTGTGCTATCATCCTGGGGGGCCTCACATGGATGGACTTGCTACGAG CCCTCATCATGTTGGGGGACACTATGTCTGGTAGAATAGGAGGACAGACCCACCTAGCCATCA TGGCAGTGTTCAAGATGTCACCGGGATACGTGCTGGGTGTGTTTTTAAGGAAACTCACTTCAAG AGAGACAGCACTAATGGTAATAGGAATGGCCATGACAACAACACTTTCAATTCCACATGACCT CATGGAACTCATTGATGGAATATCACTAGGACTAATTTTGCTAAAAATAGTAACACAGTTTGA CAACACCCAAGTGGGAACCTTAGCTCTTTCCTTGACTTTCATAAGATCAACAATGTCATTGGTC ATGGCTTGGAGGACCATTATGGCTGTGTTGTTTGTGGTCACACTCATTCCTTTGTGCAGGACAA GCTGTCTTCAAAAACAGTCTCATTGGGTAGAAATAACAGCACTCATCCTAGGAGCCCAAGCTC TGCCAGTGTACCTAATGACTCTTATGAAAGGAGCCTCAAGAAGATCTTGGCCTCTTAACGAAG GCATAATGGCTGTGGGTTTGGTTAGTCTCTTAGGAAGCGCTCTTTTAAAGAATGATGTCCCTTT AGCTGGCCCAATGGTGGCAGGAGGCTTACTTCTGGCGGCTTACGTAATGAGTGGCAGCTCAGC AGATCTGTCACTAGAGAAGGCCGCTAATGTGCAGTGGGATGAAATGGCAGACATAACAGGCT CAAGTCCAATCATAGAAGTGAAGCAAGATGAGGATGGCTCTTTCTCCATACGGGACGTCGAGG AAACCAATATGATAACCCTTTTGGTGAAACTGGCACTGATAACGGTGTCAGGTCTCTACCCCTT GGCAATTCCAATCACAATGACCTTATGGTACATGTGGCAAGTGAAAACACAAAGATCAGGAGC

CCTGTGGGACGTCCCTTCACCCGCCGCCACTCAAAAAGCCGCACTGTCTGAAGGAGTGTACAG
GATCATGCAAAGAGGGTTATTCGGGAAAACTCAGGTTGGAGTAGGGATACACATGGAAGGTG
TATTTCACACAATGTGGCATGTTACAAGAGGATCGGTGATCTGCCACGAGACTGGGAGATTGG
AGCCATCTTGGGCTGATGTCAGGAATGACATGATATCATACGGTGGGGGATGGAGGCTTGGAG
ATAAATGGGACAAAGAAGAAGACGTTCAGGTCCTCGCTATAGAACCAGGGAAAAATCCCAAA
CATGTCCAAACGAAACCTGGCCTTTTCAAGACCCTAACTGGAGAAATTGGAGCAGTAACATTA
GATTTCAAACCCGGAACGTCTGGTTCTCCCATTATCAACAGGAAAGGAAAAGTCATCGGACTC
TATGGAAATGGAGTGGTCACCAAATCAGGTGATTACGTCAGTGCCATAACACAAGCCGAAAG
AATTGGAGAGCCAGATTATGAAGTGGATGAGGACATTTTTCGAAAGAAAAGACTAACTATAAT
GGACTTACACCCCGGAGCCGGAAAGACAAAAAGAATTCTTCCATCAATAGTGAGAGAAGCCTT
AAAAAGGAGGCTGCGAACTTTGATTTTGGCTCCCACGAGAGTGGTGGCGGCCGAGATGGAAG
AGGCCCTACGTGGACTGCCAATCCGTTACCAAACCCCAGCTGTAAAATCAGAACACACAGGAA
GAGAGATTGTAGACCTCATGTGCCATGCAACCTTCACAACAAGACTTTTGTCATCAACCAGAG
TTCCAAACTACAACCTTATAGTAATGGATGAAGCACATTTCACCGATCCTTCCAGTGTCGCGGC
TAGAGGATACATTTCGACCAGGGTGGAAATGGGAGAGGCAGCAGCCATCTTCATGACCGCAA
CCCCTCCCGGAGCGACAGATCCCTTTCCCCAGAGCAACAGCCCAATAGAAGACATCGAGAGAG
AGATTCCGGAAAGGTCATGGAACACAGGGTTCGACTGGATAACAGACTACCAAGGGAAAACT
GTGTGGTTTGTTCCCAGCATAAAAGCTGGAAATGACATTGCAAATTGTTTGAGAAAGTCGGGA
AAGAAAGTTATCCAGTTGAGTAGGAAAACCTTTGATACAGAATATCCAAAAACGAAACTCACG
GACTGGGACTTTGTGGTCACTACAGACATATCTGAAATGGGGGCTAACTTTAGAGCTGGGAGA
GTGATAGACCCTAGAAGATGCCTCAAGCCAGTTATCCTAACAGATGGGCCAGAGAGAGTCATC
TTAGCAGGTCCTATTCCAGTGACTCCAGCAAGCGCTGCTCAGAGAAGAGGGCGAATAGGAAG
GAACCCAGCACAAGAAGACGACCAATACGTTTTCTCCGGAGACCCACTAAAAAATGATGAAG
ACCATGCCCACTGGACAGAAGCAAAGATGCTGCTTGACAATATCTACACCCCAGAAGGGATCA
TTCCAACATTGTTTGGTCCGGAAAGGGAAAAAACCCAAGCTATTGATGGAGAGTTTCGCCTCA
GAGGGGAACAAAGGAAGACTTTTGTGGAATTAATGAGGAGAGGAGACCTTCCGGTGTGGCTG
AGTTATAAGGTAGCTTCTGCTGGCATTTCTTACAAAGATCGGGAATGGTGCTTTACTGGGGAAA
GAAATAACCAAATTTTAGAAGAAAACATGGAGGTTGAAATTTGGACTAGAGAGGGAGAAAAG
AAAAAACTGAGGCCAAAATGGTTAGATGCACGTGTATACGCTGACCCCATGGCTTTGAAGGAT
TTCAAGGAGTTTGCCAGTGGAAGGAAGAGTATAACTCTCGACATCCTAACAGAGATCGCCAGT
TTGCCAACTTACCTTTCCTCTAGGGCCAAGCTCGCCCTTGACAACATAGTTATGCTCCACACAA
CAGAAAGAGGAGGGAGGGCCTATCAACATGCCCTGAACGAACTTCCGGAGTCACTGGAAACA
CTCATGCTTGTAGCCTTACTAGGAGCTATGACAGCAGGTATCTTCCTGTTTTTCATGCAAGGGA
AAGGAATAGGGAAATTGTCAATGGGTTTGATAACCATTGCGGTGGCTAGTGGCTTGCTCTGGG
TAGCAGAAATTCAACCCCAGTGGATAGCGGCCTCAATCATACTGGAGTTTTTCTCATGGTACT
GTTGATACCAGAACCAGAAAAACAAAGGACCCCACAAGACAATCAATTGATCTACGTCATATT
GACCATTCTCACCATTATTGGTCTAATAGCAGCCAACGAGATGGGGCTGATAGAAAAAACAAA

AACGGATTTTGGGTTTTACCAGGTAAAAACAGAAACCACCATCCTCGATGTGGACCTGAGACC
AGCTTCAGCATGGACGCTCTATGCGGTAGCCACCACAATTCTGACTCCCATGCTGAGACACAC
CATAGAAAATACGTCGGCCAACCTATCTTTAGCAGCCATCGCCAACCAGGCAGCCGTCCTAAT
GGGGCTTGGAAAAGGATGGCCACTCCACAGAATGGACCTCGGTGTGCCGCTGTTAGCAATGGG
ATGCTATTCTCAAGTGAACCCAACAACCTTGACAGCATCCTTAGTCATGCTTTTAGTCCATTAT
GCAATAATAGGCCCAGGATTGCAGGCAAAAGCCACAAGAGAGGCCCAGAAAAGGACAGCTGC
TGGAATCATGAAAAATCCCACAGTGGACGGGATAACAGTGATAGATCTAGAACCAATATCCTA
TGACCCAAAATTTGAAAAGCAATTAGGGCAGGTCATGTTACTCGTCTTGTGTGCTGGACAACT
ACTCTTGATGAGAACAACATGGGCTTTCTGTGAAGTTTTGACTTTGGCCACAGGACCAATCTTG
ACCTTGTGGGAGGGCAACCCGGGAAGGTTTTGGAACACGACCATAGCCGTATCTACCGCCAAC
ATTTTCAGGGGAAGTTATTTGGCAGGAGCTGGACTGGCTTTTTCACTCATAAAGAATGCACAA
ACCCCCAGGAGGGGAACTGGGACCACAGGAGAGACACTGGGAGAGAAGTGGAAGAGACAGC
TAAACTCATTAGACAGGAAAGAGTTTGAAGAGTATAAAAGAAGTGGAATACTAGAAGTGGAC
AGGACTGAAGCCAAGTCCGCCCTGAAAGATGGGTCTAAAATCAAGCATGCAGTATCTAGAGG
GTCCAGTAAGATCAGATGGATTGTTGAGAGAGGGATGGTAAAGCCAAAGGGGAAAGTTGTAG
ATCTTGGCTGTGGGAGAGGAGGATGGTCTTATTACATGGCGACACTCAAGAACGTGACTGAAG
TGAAAGGGTATACAAAAGGAGGTCCAGGACATGAAGAACCGATTCCCATGGCTACTTATGGCT
GGAATTTGGTCAAACTCCATTCAGGGGTTGACGTGTTCTACAAACCCACAGAGCAAGTGGACA
CCCTGCTCTGTGATATTGGGGAGTCATCTTCTAATCCAACAATAGAGGAAGGAAGAACATTAA
GAGTTTTGAAGATGGTGGAGCCATGGCTCTCTTCAAAACCTGAATTCTGCATCAAAGTCCTCAA
CCCCTACATGCCAACAGTCATAGAGGAGCTGGAGAAACTGCAGAGAAAACACGGTGGGAACC
TTGTCAGATGCCCGCTGTCCAGGAACTCCACCCATGAGATGTATTGGGTGTCAGGAGCGTCGG
GAAATATTGTGAGCTCTGTGAACACAACATCAAAGATGTTGTTGAACAGGTTCACAACAAGGC
ATAGGAAACCCACTTATGAGAAGGACGTAGATCTTGGGGCAGGAACGAGAAGTGTCTCTACTG
AAACAGAAAAACCAGACATGACAATCATTGGGAGAAGGCTTCAGCGATTGCAAGAGGAGCAC
AAAGAAACATGGCATTATGATCAGGAAAACCCATACAGAACCTGGGCGTATCATGGAAGCTA
TGAAGCTCCTTCGACAGGCTCTGCATCCTCCATGGTGAACGGGGTGGTGAAACTGCTAACAAA
ACCCTGGGATGTGATTCCAATGGTGACTCAGTTAGCCATGACAGATACAACCCCTTTTGGGCA
ACAAAGAGTGTTCAAAGAGAAGGTGGATACCAGAACGCCGCAACCAAAACCAGGCACACGAA
TGGTTATGACCACGACAGCCAATTGGCTATGGGCCCTCCTTGGAAAGAAGAAAAATCCCAGAC
TGTGTACAAGGGAAGAGTTCATCTCAAAAGTTAGATCAAACGCAGCCATAGGCGCAGTCTTTC
AGGAAGAACAAGGATGGACATCAGCCAGTGAAGCTGTGAATGACAGCCGGTTTTGGGAACTG
GTTGACAAAGAAAGGGCCCTTCACCAGGAAGGGAAATGTGAATCGTGTGTCTATAACATGATG
GGAAAACGTGAGAAAAAGTTAGGAGAGTTTGGCAGAGCCAAGGGAAGCCGAGCAATCTGGTA
CATGTGGCTGGGAGCGCGGTTTCTGGAATTTGAAGCCCTGGGTTTTTTGAATGAAGATCACTGG
TTTGGCAGAGAAAATTCATGGAGTGGAGTGGAAGGGGAAGGTCTGCACAGATTGGGATACAT
CCTGGAGGAGATAGACAAGAAGGATGGAGACCTAATGTATGCTGATGACACAGCAGGTTGGG

ACACAAGAATCACTGAGGATGACCTTCAAAATGAGGAACTGATCACGGAACAGATGGCTCCC
CACCACAAGATCCTAGCCAAAGCCATTTTCAAACTAACCTATCAAAACAAAGTGGTGAAAGTC
CTCAGACCCACACCGAGAGGAGCGGTGATGGATATCATATCCAGGAAAGACCAAAGAGGTAG
TGGACAAGTTGGAACATATGGTTTGAACACATTCACCAACATGGAAGTTCAACTCATCCGCCA
AATGGAAGCTGAAGGAGTCATCACACAAGATGACATGCAGAACCCAAAAGGGTTGAAAGAAA
GAGTTGAGAAATGGCTGAGAGAGTGTGGTGTCGACAGGTTAAAGAGGATGGCAATCAGTGGA
GACGATTGCGTGGTGAAGCCCCTAGATGAGAGGTTTGGCACTTCCCTCCTCTTCTTGAACGACA
TGGGAAAGGTGAGGAAAGACATTCCGCAGTGGGAACCATCTAAGGGATGGAAAAACTGGCAA
GAGGTTCCTTTTTGCTCCCATCACTTTCACAAGATCTTTATGAAGGATGGCCGCTCACTAGTTGT
TCCATGTAGAAACCAGGATGAACTGATAGGGAGAGCCAGAATCTCGCAGGGGGCTGGATGGA
GCTTAAGAGAAACAGCCTGCCTGGGCAAAGCTTACGCCCAGATGTGGTCGCTTATGTACTTCC
ACAGAAGGGATCTGCGTTTAGCCTCCATGGCCATATGCTCAGCAGTTCCAACGGAATGGTTTCC
AACAAGCAGAACAACATGGTCAATCCATGCTCATCACCAATGGATGACCACTGAAGACATGCT
CAAAGTGTGGAACAGAGTGTGGATAGAAGACAACCCCAATATGATTGACAAGACTCCAGTCC
ATTCGTGGGAAGATATACCTTACCTAGGGAAAAGAGAGGATTTGTGGTGTGGATCCCTGATTG
GACTTTCTTCTAGAGCCACCTGGGCGAAGAACATTCACACGGCCATAACTCAGGTCAGGAACT
TGATCGGAAAAGAGGAATACGTGGATTACATGCCAGTAATGAAAAGATACAGTGCTCCTTCAG
AGAGTGAAGGAGTTCTGTAATTACCAACAACAAACACCAAAGGCTATTGAAGTCAGGCCACTT
GTGCCACGGTTTGAGCAAACCGTGCTGCCTGTAGCTCCGCCAATAATGGGAGGCGTAATAATC
CCTAGGGAGGCCATGCGCCACGGAAGCTGTACGCGTGGCATATTGGACTAGCGGTTAGAGGA
GACCCCTCCCATCACTGACAAAACGCAGCAAAAGGGGGCCCGAAGCCAGGAGGAAGCTGTAC
TCCTGGTGGAAGGACTAGAGGTTAGAGGAGACCCCCCCAACACAAAAACAGCATATTGACGC
TGGGAAAGACCAGAGATCCTGCTGTCTCTACAACATCAATCCAGGCACAGAGCGCCGCAAGAT
GGATTGGTGTTGTTGATCCAACAGGTTCT

The present invention provides a composition for inducing an immune response in a subject comprising any of the attenuated viruses described herein and a pharmaceutically acceptable carrier. In various embodiments, the composition is a vaccine composition and the immune response is a protective immune response.

It should be understood that an attenuated virus of the invention, where used to elicit an immune response in a subject (or protective immune response) or to prevent a subject from or reduce the likelihood of becoming afflicted with a virus-associated disease, is administered to the subject in the form of a composition additionally comprising a pharmaceutically acceptable carrier. Pharmaceutically acceptable carriers are well known to those skilled in the art and include, but are not limited to, one or more of 0.01-0.1M and preferably 0.05M phosphate buffer, phosphate-buffered saline (PBS), or 0.9% saline. Such carriers also include aqueous or non-aqueous solutions, suspensions, and emulsions. Aqueous carriers include water, alcoholic/aqueous solutions, emulsions or suspensions, saline and buffered media. Examples of non-aqueous solvents are propylene glycol, polyethylene glycol, vegetable oils such as olive oil, and injectable organic esters such as ethyl oleate. Parenteral vehicles include sodium chloride solution, Ringer's dextrose, dextrose and sodium chloride, lactated Ringer's and fixed oils. Intravenous vehicles include fluid and nutrient replenishers, electrolyte replenishers such as those based on Ringer's dextrose, and the like. Solid compositions may comprise nontoxic solid carriers such as, for example, glucose, sucrose, mannitol, sorbitol, lactose, starch, magnesium stearate, cellulose or cellulose derivatives, sodium carbonate and magnesium carbonate. For administration in an aerosol, such as for pulmonary and/or intranasal delivery, an agent or composition is preferably formulated with a nontoxic surfactant, for example, esters or partial esters of C6 to C22 fatty acids or natural glycerides, and a propellant. Additional carriers such as lecithin may be included to facilitate intranasal delivery. Pharmaceutically acceptable carriers can further comprise minor amounts of auxiliary substances such as wetting or emulsifying agents, preservatives and other additives, such as, for example, antimicrobials, antioxidants and chelating agents, which enhance the shelf life and/or effectiveness of the active ingredients. The instant compositions can, as is well known in the art, be formulated so as to provide quick, sustained or delayed release of the active ingredient after administration to a subject.

In various embodiments of the instant composition or vaccine composition, the attenuated virus (i) does not substantially alter the synthesis and processing of viral proteins in an infected cell; (ii) produces similar amounts of virions per infected cell as wt virus; and/or (iii) exhibits substantially lower virion-specific infectivity than wt virus. In further embodiments, the attenuated virus induces a substantially similar immune response in a host animal as the corresponding wt virus.

This invention also provides a modified host cell line specially isolated or engineered to be permissive for an attenuated virus that is inviable in a wild type host cell. IN embodiments wherein the attenuated virus cannot grow in normal (wild type) host cells, it is absolutely dependent on the specific helper cell line for growth. This provides a very high level of safety for the generation of virus for vaccine production. Various embodiments of the instant modified cell line permit the growth of an attenuated virus, wherein the genome of said cell line has been altered to increase the number of genes encoding rare tRNAs.

### *Methods of eliciting an immune response*

Various embodiments provide for a method of eliciting an immune response in a subject comprising administering to the subject an effective dose of a composition comprising a modified Flavivirus of the present invention. Particular embodiments provide for a method of eliciting a protective immune response in a subject comprising administering to the subject a prophylactically or therapeutically effective dose of a vaccine composition comprising a modified Flavivirus of the present invention. In various embodiments, the immune response is cross-protective against a heterologous Flavivirus virus.

In various embodiments, the method further comprises administering to the subject at least one adjuvant. Non-limiting examples of adjuvants are discussed herein. Particular embodiments provide for a method of eliciting an immune response in a subject, comprising: administering to the subject a prophylactically or therapeutically effective dose of a vaccine composition comprising a modified dengue virus the present invention.

Various embodiments provide for a method of eliciting an immune response in a subject, comprising: administering to the subject an effective dose of a composition comprising a modified dengue virus the present invention.

Various embodiments provide for a method of eliciting a protective immune response in a subject, comprising: administering to the subject a prophylactically or therapeutically effective dose of a vaccine composition comprising a modified dengue virus the present invention.

In various embodiments, the method further comprises administering to the subject at least one adjuvant. Non-limiting examples of adjuvants are described herein.

In various embodiments, the immune response is cross-protective against a heterologous dengue virus.

Various embodiments of the present invention provide for a method of eliciting an immune response in a subject in need thereof, comprising: administering a prime dose of an attenuated Flavivirus produced by a method other than codon-pair deoptimization or codon deoptimization, or increasing of CpG or UpA di-nucleotides, or a modified Flavivirus in which expression of viral proteins is reduced compared to a parent virus, wherein the reduction in expression is the result of recoding the prM, or envelope (E) region; and administering one or more boost dose of the attenuated Flavivirus produced by methods other than codon-pair deoptimization or codon deoptimization, or increasing of CpG or UpA di-nucleotides, or the modified Flavivirus to the subject in need thereof, wherein at least the prime dose or the one or more boost dose is the modified virus. In various embodiments, a first of the one or more boost dose is administered about 2 weeks after the prime dose.

In various embodiments, the Flavivirus is a dengue virus.

In various embodiments, one or both of the E protein-encoding sequence protein-encoding sequence is recoded by reducing the codon pair bias or codon usage bias of the protein-encoding sequence. In various embodiments, reducing the codon-pair bias comprises identifying a codon pair in the parent protein-encoding sequence having a codon-pair score that can be reduced, and reducing the codon-pair bias by substituting the codon pair with a codon pair that has a lower codon-pair score.

In various embodiments, reducing the codon-pair bias comprises rearranging the codons of a parent protein-encoding sequence. In various embodiments, the E protein-encoding sequence is recoded by increasing the number of CpG or UpA di nucleotides compared to a parent virus. In various embodiments, each of the recoded prM/E protein-encoding sequence have a codon pair bias less than -0.05, or less than -0.06, or less than -0.07, or less than -0.08, or less than -0.09, or less than -0.1, or less than -0.11, or less than -0.12, or less than -0.13, or less than -0.14, or less than -0.15, or less than -0.16, or less than -0.17, or less than -0.18, or less than -0.19, or less than -0.2, or less than -0.25, or less than -0.3, or less than -0.35, or less than -0.4, or less than -0.45, or less than -0.5. In various embodiments, one or both of the E protein-encoding sequence is recoded by replacing one or more codons with synonymous codons that are less frequent in the viral host (e.g., human). In some embodiments, the number of codons substituted with synonymous codons is at least 5, or at least 10, or at least 30, or at least 30, or at least 40, or at least 50, or at least 75, or at least 100, or at least 200 or at least 300, or at least 400, or at least 500.

In addition, the present invention provides a method for eliciting a protective immune response in a subject comprising administering to the subject a prophylactically or therapeutically effective dose of any of the vaccine compositions described herein. This invention also provides a method for preventing a subject from becoming afflicted with a virus-associated disease comprising administering to the subject a prophylactically effective dose of any of the instant vaccine compositions. In embodiments of the above methods, the subject has been exposed to a pathogenic virus. "Exposed" to a pathogenic virus means contact with the virus such that infection could result.

The invention further provides a method for delaying the onset, or slowing the rate of progression, of a virus-associated disease in a virus-infected subject comprising administering to the subject a therapeutically effective dose of any of the instant vaccine compositions.

As used herein, "administering" means delivering using any of the various methods and delivery systems known to those skilled in the art. Administering can be performed, for example, intranasally, intraperitoneally, intracerebrally, intravenously, orally, transmucosally, subcutaneously, transdermally, intradermally, intramuscularly, topically, parenterally, via implant, intrathecally, intralymphatically, intralesionally, pericardially, or epidurally. An agent or composition may also be administered in an aerosol, such as for pulmonary and/or intranasal delivery. Administering may be performed, for example, once, a plurality of times, and/or over one or more extended periods.

Eliciting a protective immune response in a subject can be accomplished, for example, by administering a primary dose of a vaccine to a subject, followed after a suitable period of time by one or more subsequent administrations of the vaccine. A suitable period of time between administrations of the vaccine may readily be determined by one skilled in the art, and is usually on the order of several weeks to months. The present invention is not limited, however, to any particular method, route or frequency of administration.

In various embodiments, the present invention provides for a method of eliciting an immune response in a subject in need thereof, comprising: administering a prime dose of an attenuated Flavivirus produced by a method other than codon-pair deoptimization or codon deoptimization, or increasing of CpG or UpA di-nucleotides, or a modified Flavivirus in which expression of viral proteins is reduced compared to a parent virus, wherein the reduction in expression is the result of recoding the prM, or envelope (E) region, or both; and administering one or more boost dose of the attenuated Flavivirus by methods other than codon-pair deoptimization or codon deoptimization, or increasing of CpG or UpA di-nucleotides, or the modified Flavivirus to the subject in need thereof, wherein at least the prime dose or the one or more boost dose is the modified virus.

In various embodiments, the one or more boost dose is administered about 2 weeks after a prime dose. In various embodiments, 2, 3, 4, or 5 boost doses are administered. In various embodiments, the intervals between the boost doses can be 1, 2, 3, 4, 5, 6, 7, 8, 9, or 10 weeks. In additional embodiments, the intervals between the boost doses can be 1, 2, 3, 4, 5, 6, 7, 8, 9, 10, 11 or 12 months. As a non-limiting example, the prime dose can be administered, about two weeks thereafter a first boost dose can be administered, about one month after the first boost dose, a second boost dose can be administered, about 6 months after the second boost dose, a third boost dose can be administered. As another non-limiting example, the prime dose can be administered, about two weeks thereafter a first boost dose can be administered, about six months after the first boost dose, a second boost dose can be administered, about 12 months after the second boost dose, a third boost dose can be administered. In further embodiments, additional boost dosages can be periodically administered; for example, every 5 years, every 10 years, etc.

In various embodiments, the Flavivirus is a dengue virus. In various embodiments, the Flavivirus is selected from the group consisting of dengue fever virus, Zika virus, West Nile virus, yellow fever virus, Japanese encephalitis virus, Spondweni virus, Saint Louis encephalitis virus, and Powassan virus.

In various embodiments, one or both of the E protein-encoding sequence is recoded by lowering the codon pair bias or codon usage bias of the protein-encoding sequence. In various embodiments, the E protein-encoding sequence is recoded by increasing the number of CpG or UpA di nucleotides compared to a parent virus.

In various embodiments, reducing the codon-pair bias comprises identifying a codon pair in the parent protein-encoding sequence having a codon-pair score that can be reduced, and reducing the codon-pair bias by substituting the codon pair with a codon pair that has a lower codon-pair score.

In various embodiments, reducing the codon-pair bias comprises rearranging the codons of a parent protein-encoding sequence.

In various embodiments it includes the increase of the CpG dinucleotide in the modified virus

In various embodiments it includes the increase of the UpA dinucleotide in the modified virus

In various embodiments, the recoded prM/E protein-encoding sequence each have a codon pair bias less than -0.05, or less than -0.06, or less than -0.07, or less than -0.08, or less than -0.09, or less than -0.1, or less than -0.11, or less than -0.12, or less than -0.13, or less than -0.14, or less than -0.15, or less than -0.16, or less than -0.17, or less than -0.18, or less than -0.19, or less than -0.2, or less than -0.25, or less than -0.3, or less than -0.35, or less than -0.4, or less than -0.45, or less than -0.5.

In certain embodiments, the codon pair bias of the recoded prM-protein encoding sequence, E protein-encoding sequence, or both is reduced by at least 0.05, or at least 0.06, or at least 0.07, or at least 0.08, or at least 0.09, or at least 0.1, or at least 0.11, or at least 0.12, or at least 0.13, or at least 0.14, or at least 0.15, or at least 0.16, or at least 0.17, or at least 0.18, or at least 0.19, or at least 0.2, or at least 0.25, or at least 0.3, or at least 0.35, or at least 0.4, or at least 0.45, or at least 0.5, compared to the parent prM/E protein encoding sequence from which it is derived.

In various embodiments, the prM-protein encoding sequence, E protein-encoding sequence, or both are recoded by replacing one or more codons with synonymous codons that are less frequent in the viral host. In some embodiments, the number of codons substituted with synonymous codons is at least 5, or at least 10, or at least 30, or at least 30, or at least 40, or at least 50, or at least 75, or at least 100, or at least 200 or at least 300, or at least 400, or at least 500.

In various embodiments, the prime dose is administered subcutaneously, intramuscularly, intradermally, or intranasally.

In various embodiments, the one or more boost dose is administered intratumorally, intravenously, or intrathecally.

The timing between the prime and boost dosages can vary, for example, depending on the stage of infection or disease (e.g., non-infected, infected, number of days post infection), and the patient's health. In various embodiments, the one or more boost dose is administered about 2 weeks after the prime dose. That is, the prime dose is administered and about two weeks thereafter, a boost dose is administered.

In various embodiments, the dosage amount can vary between the prime and boost dosages. As a non-limiting example, the prime dose can contain fewer copies of the virus compared to the boost dose.

In other embodiments, the type of attenuated virus produced by a method other than codon-pair deoptimization or codon deoptimization, or increasing of CpG or UpA di-nucleotides or modified virus of the present invention can vary between the prime and boost dosages. In one non-limiting example, a modified virus of the present invention can be used in the prime dose and an attenuated virus (produced by a method other than codon-pair deoptimization or codon deoptimization, or increasing of CpG or UpA di-nucleotides) of the same or different family, genus, species, group or order can be used in the boost dose.

In other embodiments, the route of administration can vary between the prime and the boost dose. In a non-limiting example, the prime dose can be administered subcutaneously, and the boost dose can be administered via injection into the tumor; for tumors that are in accessible, or are difficult to access, the boost dose can be administered intravenously.

Certain embodiments of any of the instant immunization and therapeutic methods further comprise administering to the subject at least one adjuvant. An "adjuvant" shall mean any agent suitable for enhancing the immunogenicity of an antigen and boosting an immune response in a subject. Numerous adjuvants, including particulate adjuvants, suitable for use with both protein- and nucleic acid-based vaccines, and methods of combining adjuvants with antigens, are well known to those skilled in the art. Suitable adjuvants for nucleic acid based vaccines include, but are not limited to, Quil A, imiquimod, resiquimod, and interleukin-12 delivered in purified protein or nucleic acid form. Adjuvants suitable for use with protein immunization include, but are not limited to, alum, Freund's incomplete adjuvant (FIA), saponin, Quil A, and QS-21.

The invention also provides a kit for immunization of a subject with an attenuated virus of the invention. The kit comprises the attenuated virus, a pharmaceutically acceptable carrier, an applicator, and an instructional material for the use thereof. In further embodiments, the attenuated virus may be one or more dengue virus, one or more Japanese encephalitis virus, one or more West Nile virus, one or more yellow fever virus, one or more Zika virus, etc. More than one virus may be preferred where it is desirable to immunize a host against a number of different isolates of a particular virus. The invention includes other embodiments of kits that are known to those skilled in the art. The instructions can provide any information that is useful for directing the administration of the attenuated viruses.

Throughout this application, various publications, reference texts, textbooks, technical manuals, patents, and patent applications have been referred to. The teachings and disclosures of these publications, patents, patent applications and other documents in their entireties are hereby incorporated by reference into this application to more fully describe the state of the art to which the present invention pertains. However, the citation of a reference herein should not be construed as an acknowledgement that such reference is prior art to the present invention.

It is to be understood and expected that variations in the principles of invention herein disclosed can be made by one skilled in the art and it is intended that such modifications are to be included within the scope of the present invention. The following Examples further illustrate the invention but should not be construed to limit the scope of the invention in any way. Detailed descriptions of conventional methods, such as those employed in the construction of recombinant plasmids, transfection of host cells with viral constructs, polymerase chain reaction (PCR), and immunological techniques can be obtained from numerous publications, including Sambrook et al. (1989) and Coligan et al. (1994). All references mentioned herein are incorporated in their entirety by reference into this application. The contents of WO 2008/121992 and WO 2011/044561 are incorporated by reference.

EXAMPLES

*Example 1*

*Rapid design and construction of SAVE-deoptimized, live-attenuated dengue vaccine candidates.* To select our target sequences, we started with all prM-E sequences of all DENV virus isolates of the last decade and performed an amino acid consensus sequence analysis, available at the NCBI Virus Variation Database. As the consensus sequence usually never exists as an actual virus, it is unwise to use it as the basis of our designs. We prefer natural virus evolution to tell us what works and what does not. Thus, the consensus sequence for each serotype is then searched against the entire Genbank using the tblastn algorithm to identify an actual genome of a replicating virus which is most identical to the consensus sequence; we then used this sequence as our target sequence. In addition, the target sequence must not be unique in the database (possible sequencing artifacts), in which case we select the next highest identity sequence to the consensus, which is represented by two or more independent virus isolates. This search algorithm to determine the most relevant and replicating human isolate result in a sequence that is 1) a genuine human isolate and 2) as homologous to the rest of DENV sequence space as a single sequence can be. Using this approach, we identified the following viruses from which to select out target prM-E antigen sequences: DENV-1/VN/BID-V1774/2007, DENV-2/NI/BID-V533/2005, DENV-3/VE/BID-V2268/2008, DENV-4/US/BID-V2448/1999. We initiated our DENV vaccine program using the E-Min of DENV-2/NI/BID-V533/2005 and generated live-attenuated strains containing the other three E genes selected via this process (Figure 1). The CPB of wild-type prM/E genes fall within the normal range of human host cell's genes. Following codon pair-'deoptimization' via SAVE, the resulting deoptimized prM/E gene segments were now encoded predominately by under-represented human codon-pairs as evident by their extremely negative CPB, and are drastically different from any human gene. The SAVE-deoptimized synthetic prM/E from Figure 1 were synthesized *de novo* and using overlapping PCR subcloned individually into the WT DENV genomes - yielding six independent cDNA genomes each containing a synthetically 'de-optimized' fragment(s). We constructed infectious cDNA genomes for wt DENV1-4 and then recovered fully infectious, replicating virus for the deoptimized DENV vaccine candidates via transfection of RNA into Vero or BHK cells.

*Example 2*

*Leveraging SAVE platform flexibility to create second-generation Dengue virus vaccine candidates*

To fine-tune attenuation and immunogenicity, a second generation of Dengue virus vaccine candidates were constructed using the SAVE platform (Figure 2). The second generation of dengue vaccine candidates leverages the flexibility of the SAVE platform to reduce the deoptimized region from 2014 bp (E-min) to 997 bp (W-E-Min) or further to 664 bp (W-W-E-Min) while keeping the amino acid sequence 100% identical in a homologous backbone.

*Example 3*

*Growth of heterologous backbone dengue vaccine candidates in Vero cells under animal-component-free conditions*

To optimize production conditions for future cGMP manufacture and test for attenuation *in vitro,* DENV1-4 E-Min were used to infect Vero cells under animal-component free conditions at a MOI of 0.01 and supernatant titrated daily in a multiple-step growth curve over the course of 10 days post-infection. DENV2, DENV3, and DENV4 candidates reached titers of 1-2 x $10^5$ FFU/ml while DENV1 E-Min titer was reduced by ~1 $log_{10}$ compared to the others. (Figure 3).

*Example 4*

*Growth of homologous backbone dengue virus type 1 vaccine candidates in Vero cells and tests for temperature-sensitivity as a mechanism of attenuation*

Growth kinetics conducted at low (33°C), medium (37°C), and high (39°C) temperatures were used to optimize production conditions for the deoptimized candidates, test for attenuation *in vitro* and examine CPD variants for increase temperature sensitivity as a means of explaining the mechanism of attenuation. Multiple step growth curves were conducted in Vero cells for synthetic DENV1 WT virus (in a full-length DENV1 backbone) and attenuated live vaccine candidates DENV1 E-W/MIN and E-MIN derived from DENV1 WT. Synthetic DENV1 WT grows well in Vero cells at 33°C and 37°C. Typical of DENV, a transient reduction in virus yield was observed at 39 °C, however, titers recovered to 37°C levels by 7 dpi. DENV1 E-W/MIN, however, reached serviceable titers ~10-fold lower than DENV1 WT at both 33°C and 37°C. DENV1 E-MIN was highly attenuated in Vero cells and only reached detectable titers at 33°C starting on days 10-14 post-infection.

*Example 5*

*Growth of homologous backbone dengue virus type 2 vaccine candidates in Vero cells and tests for temperature-sensitivity as a mechanism of attenuation*

Growth kinetics conducted at low (33°C), medium (37°C), and high (39°C) temperatures were used to optimize production conditions for the deoptimized candidates, test for attenuation *in vitro* and examine CPD variants for increase temperature sensitivity as a means of explaining the mechanism of attenuation. Multiple step growth curves were conducted in Vero cells for synthetic DENV2 WT virus (in a full-length, homologous DENV2 backbone) and attenuated live vaccine candidates DENV2 E-W/MIN and E-MIN derived from DENV2 WT. DENV2 E-W/MIN and E-MIN were both attenuated *in vitro* with a 1-2 $log_{10}$ FFU/ml reduction for E-W/MIN and a more pronounced 2-3 $log_{10}$ FFU/ml reduction for DENV2 E-MIN (Figure 3). While growth kinetics were delayed, with regular medium replacement every 1-2 days virus titers reached >$10^6$ FFU/ml for DENV2 E-W/MIN. Importantly, attenuation was correlated with the extent of deoptimization in the E region. While temperature sensitivity (difference between titers at 37°C and 39°C) was similar for DENV2-WT and DENV2 E-W/MIN through day 4 post-infection, there were significant differences on days 5, 6, and 7. Temperature sensitivity was not observed for DENV2 E-MIN because there was no replication at any temperature through 6 DPI. However, starting at day 7-14 DENV2 E-MIN started producing detectable virus (but only at 33°C and 37°C).

*Example 6*

*Growth of homologous backbone dengue virus type 3 vaccine candidates in Vero cells and tests for temperature-sensitivity as a mechanism of attenuation*

Growth kinetics conducted at low (33°C), medium (37°C), and high (39°C) temperatures were used to optimize production conditions for the deoptimized candidates, test for attenuation *in vitro* and examine CPD variants for increase temperature sensitivity as a means of explaining the mechanism of attenuation. Multiple step growth curves were conducted in Vero cells for synthetic DENV3 WT virus (in a full-length DENV3 backbone) and attenuated live vaccine candidates DENV3 E-W/MIN and E-MIN derived from DENV3 WT. It was apparent that DENV3 E-W/MIN was more temperature sensitive at 39 °C than the WT with a significantly greater difference observable on days 3, 4, 5, and 7 post-infection. Because DENV3 E-MIN had minimal levels of replication in Vero cells at 39°C, the difference in virus yield from 37°C to 39°C were as high as $10^7$ FFU/ml. When grown at permissible temperatures, however, DENV3 E-MIN reached high titers which is promising for cGMP manufacture.

*Example 7*

*Growth of homologous backbone dengue virus type 4 vaccine candidates in Vero cells and tests for temperature-sensitivity as a mechanism of attenuation*

Growth kinetics conducted at low (33°C), medium (37°C), and high (39°C) temperatures were used to optimize production conditions for the deoptimized candidates, test for attenuation *in vitro* and examine CPD variants for increase temperature sensitivity as a means of explaining the mechanism of attenuation. Multiple step growth curves were conducted in Vero cells for synthetic DENV4 WT virus (in a full-length DENV4 backbone) and attenuated live vaccine candidates DENV4 E-W/MIN and E-MIN derived from DENV4 WT. Both DENV4 WT and E-W/MIN grew to high titers in Vero cells, with the peak DENV4 E-W/MIN titers approximately 10-fold lower but still high (~$10^7$ FFU/ml) at 33 °C and 37 °C. Both viruses were partially restricted at 39°C, but not to the extent seen with DENV1-3. On day 5 a small but significant (~5-fold) change in titer from 37 °C to 39 °C compared to WT was observed. DENV4 E-MIN was attenuated in Vero cells with peak titers 100-fold lower than WT. DENV4 E-MIN replication at 33°C and 37°C was similar, however, DENV4 E-MIN was not viable at 39°C with no detectable virus on days 1-14 post-infection.

*Example 8*

*Evaluation of the attenuation, immunogenicity and efficacy of DENV2 vaccine candidates in AG129 mice*

As described above, the AG129 adult mouse model is gaining acceptance for studies of flavivirus vaccines and therapeutics. We used AG129 mice to test: 1) each synthetically derived wild-type virus DENV at a dose of $10^6$ (positive control); 2) two doses ($10^4$ and $10^6$ PFU) of the vaccine candidates. Survival, weight, and clinical sign data were collected daily throughout the course of the experiment. SAVE deoptimized DENV strains were attenuated compared to synthetic wild-type viruses. DENV-2 E-min based on a DENV2 16681 backbone has been shown to be attenuated and immunogenic in neonatal ICR mice. In this immunogenicity/dose escalation study, the vaccine was evaluated for the first time in AG129 mice. Animals were immunized with DENV-2 E-min or DENV-2 16681 at two different concentrations. Resultant neutralizing antibody titers were determined, and the protective efficacy afforded evaluated against a mouse-adapted lethal strain of DENV2, D2S10[3]. None of the animals immunized with either virus at either dose showed clinical signs. Serum was collected from the immunized mice two days after the first immunization and viremia determined by RT-PCR. Viral RNA was detected in 55% (5/9) animals immunized with the higher inoculum of DENV-2 16681 and 11% (1/9) that received the lower inoculum, but was not detected in any of the mice immunized with DENV-2 E-min. The RT-PCR primers used do not target the viral E gene which has been codon deoptimized in this virus and, they successfully amplified DENV-2 E-min viral RNA included in the assay. Consequently, we do not believe that lack of detection of viremia in this study is a result of suboptimal RT-PCR amplification but indicates that the vaccine virus produced very low-level viremia *in vivo.* Upon completion of the immunization regimen serum was collected from all mice prior to virus challenge. All of the immunized animals had detectable DENV-2 neutralizing antibody titers prior to virus challenge with D2S10. A scatterplot of the data for each vaccine group demonstrating that there was an increase in neutralizing antibody titers with increasing vaccine dose for both DENV-2 E-min and DENV-2 16681, and that titers seen in E-min immunized mice were lower than those in mice immunized with the corresponding dose of DENV-2 16681. Animals in the media control group developed a rapid progressive infection with all animals dying. Mice immunized with the low dose of DENV-2 E-min vaccine were not protected, experiencing high lethality (67%) and a mean day of death (MDD) that was not significantly different to that seen in the media controls. In contrast, animals immunized with the high dose of DENV-2 E-min vaccine and with both doses of DENV-2 16681 experienced significant protection against DENV-2 D2S10 challenge. There was no lethality, or morbidity as indicated by a >10% loss in body weight, in any of these groups during the 30 day observation period. As anticipated high levels of viral RNA were detected in the sera of all media control animals on both of the days sampled. Viral RNA was also detected in all of the animals immunized with the lower dose of E-min on both days. Levels in these animals were comparable to those in controls on day 2 but were significantly lower on day 3. For animals immunized with the high dose of Emin, viral RNA was detected in the serum of 2/4 animals on day 2 with the RNA levels in the viremic animals being significantly lower than controls. On day 3, viral RNA was not detected in the sera of any of the high dose E-min animals. Overall, immunization with the high dose of Emin significantly reduced the number of animals in which viremia was detected (2/8 vs 8/8; p<0.01). For mice immunized with DENV-2 16681, no viral RNA was detected in the serum from any of the animals at either immunization dose on either of the two days sampled.

*Example 9*

*Titration of Codon-Pair Deoptimization leads to an improved DENV2 vaccine candidate in AG129 mice*

To improve the clinical relevance of our DENV2 candidate, we next constructed D2-E-min and D2-1/2-E-min with E sequence derived from DENV-2/NI/BID-V533/2005 in the heterologous DENV2 16681 backbone. We tested D2-1/2-E-min, with the E region consisting of half wt DENV-2/NI/BID-V533/2005 sequence and half CPD, to improve the immunogenicity of our virus as previous studies have shown that the extent of attenuation can be titrated in DENV2 by reduction of CPD sequence. AG129 and BALB/c mice (n=5) were vaccinated with $10^6$ or $10^4$ FFU of D2-E-min, D2-1/2-E-min, or D2-WTE (DENV2 16681 backbone with WT DENV-2/NI/BID-V533/2005 E region). Interestingly, the introduction of the modern DENV-2/NI/BID-V533/2005 E region resulted in increased virulence, with all AG129 mice infected with $10^6$ or $10^4$ FFU requiring euthanasia. No mortality or morbidity was observed in infected BALB/c mice or in any CPD DENV2 group.

Reduction of CPD sequence in the E region was associated with a significant, approximately 4-fold, increase (p=-.0002, Student's t-test) in the PRNT50 values of serum from D2-1/2-E-min compared to D2-E-Min vaccinated AG129 mice. Additionally, high neutralizing antibody titers were maintained with D2-1/2-E-min vaccination as no significant difference was observed between the $10^6$ (GM=3447) and $10^4$ (GM= 2867) FFU vaccinated groups. In immune-competent BALB/c mice, both D2-E-min and D2-1/2-E-min engendered levels of neutralizing antibody comparable to wild-type.

*Example 10*

*Evaluation of the Immunogenicity and Protective Efficacy of DENV3 vaccine candidates in AG129 mice*

AG129 mice were used to test the immunogenicity and protective efficacy of DENV-3 vaccine candidate DENV D2/D3-E-min, which comprises the DENV-2 strain 16681 backbone with a codon-deoptimized prME cassette from DENV-3/VE/BID-V2268/2008. This study was designed to evaluate the immunogenicity and efficacy of this candidate vaccine against challenge with virulent DENV-3 CO360/94. The first immunization was well tolerated by all of the animals with no major associated weight loss and none of the animals developed clinical signs. Similar results were seen after the second immunization although unexpectedly, one animal in Group 3 ($10^6$ FFU DENV D2/D3-WT) developed rapid progressive weight loss and became moribund requiring euthanasia on day 2 after the immunization. A second animal from the same group subsequently died unexpectedly two weeks after the immunization and prior to challenge with virulent DENV-3. After lethal challenge with 1.2 x $10^7$ PFU DENV-3 CO360/94, animals in the media control group developed a rapid progressive infection that was lethal in 8/9 animals with the mean day of death (MDD) among the animals that died of 4.0 $\pm$ 0.8 days. Immunization with the higher inoculum of DENV D2/D3-E-min provided significant protection with no lethality or morbidity (as measured by >10% weight loss) among the animals in the group. Immunization with the lower inoculum of DENV D2/D3-E-min was also protective with only a single animal developing a lethal infection. Animals that received the DENV D2/D3-WT virus prior to DENV-3 challenge were also protected with no lethality in either group, although, 2 animals in the higher inoculum group lost >10% of their original body weight.

*Example 11*

*Evaluation of the Immunogenicity and Protective Efficacy of DENV4 vaccine candidates in AG129 mice*

AG129 mice (n=12) were used to test the immunogenicity and protective efficacy of heterologous D2/D4-E-min and D2/D4-1/2-E-Min viruses compared to D2/D4-WTE (n=12) and media-immunized mock control mice (n=9) at a dose of $10^6$ FFU delivered subcutaneously. All mice were immunized on days 0 and 21 of the study and sera collected on day 2 to determine viremia and on day 35 for titration of neutralizing antibodies. On day 35, the mice were challenged with interperitoneal injection of $10^7$ PFU DENV4 703/4. Challenged mice were weighed and monitored for morbidity to assess the impact of viral challenge. In addition, 5 animals from each group were anesthetized and blood collected by retro-orbital bleed on day 2 post challenge and the remaining animals on day 3 to determine viremia levels by qRT-PCR. On day 30 post challenge the study was concluded, and serum collected from all surviving animals for determination of post-challenge PRNT50 values. The first immunization with all strains was well tolerated by all groups of animals with no sustained weight loss over the 5 day observation period, however, in the D2/D4-WTE group one mouse had to be euthanized prior to the second immunization and additional animals died after the second immunization. No animals in any of the other vaccine groups experienced progressive weight loss. At 2 days post-challenge (DPC), viremia was detected in 4/12 mice inoculated with D2/D4-WTE. For all of the mice immunized with the other two viruses, viremia levels were below the limit of detection of the assay (500 genome equivalents/ml). DENV-4 703/4 challenge produced rapid progressive infection with universal lethality in the media control group. In addition, three animals immunized with D2/D4-E-min experienced lethal infection with the other animals in this group remaining healthy for the duration of the study. Importantly, no lethality or evidence of morbidity as determined by weight loss was seen in any of the D2/D4-1/2-E-min immunized animals after DENV-4 703/4 challenge.

*Example 12*

*Immunogenicity of homologous DENY1-4 vaccine candidates in A129 mice*

We have tested DENV1-4 WT as well as vaccine candidates for DENV2-4 for immunogenicity in IFN$\alpha$ receptor knockout mice. All WT viruses were highly immunogenic in these mice, with neutralizing antibody titers >1024. DENV2-E-W/MIN was equally immunogenic to DENV2 WT virus at both a $10^6$ and $10^4$ FFU dose. Immunogenicity of DENV3 viruses waned with decreasing dose and increased deoptimization, however, DENV3 E-W/MIN was still highly immunogenic at a $10^4$ FFU dose (~1024 FRNT50). We noticed a pronounced improvement in the immunogenicity of DENV4 E-W/MIN and WT as compared to the heterologous DENV4 WT in the DENV2 16681 backbone, which was poorly immunogenic in A129 mice.

*Example 13*

*Tetravalent Vaccination in AG129 Mice and Efficacy vs DENV3 Challenge*

AG129 mice, homozygous for a double-knockout of IFN$\alpha$/$\beta$ and IFN$\gamma$ receptors are commonly used to test *Flavivirus* vaccine candidates due to increased susceptibility to infection. We used AG129 mice to test a tetravalent formulation of our DENV vaccine by vaccinating them on days 0 and 21 with $10^6$ IFU monovalent D2/D3-E-min vaccine (N=16; 10 male and 6 female), tetravalent vaccine containing $10^6$ IFU of each of the four monovalent DENV E-min viruses (N=16; 10 male and 6 female), or with media (N=13; 8 male and 5 female). Both of the immunizations were well tolerated by all of the animals with none of the animals developing clinical signs and no major weight loss ($\geq$10%). Serum was collected prior to challenge for measurement of neutralizing antibodies by FRNT50. All of the mice immunized with the monovalent vaccine developed detectable neutralizing antibody titers (range 20 - 160). In mice immunized with the tetravalent vaccine formulation, titers were generally lower (range <20 - 80). On day 35, all remaining mice were challenged with $10^7$ IFU

DENV3 CO360/94 delivered by the intraperitoneal route. As anticipated, animals in the media control group developed a rapid progressive infection that was lethal in 8/9 (89%) animals with the mean day of death (MDD) among the animals that died of 4.1 ± 0.4 days. Immunization with monovalent DENV D2/D3-E-min or the tetravalent vaccine provided significant protection with no lethality or morbidity (as measured by >10% weight loss) among the animals in either group. Viral RNA was detected in 8/9 control animals with the titer being comparable on days 2 and 3 post challenge.

*Example 14*

*In vivo* LD50 data for tetravalent and each strain of the homologous vaccine shows neuroattenuation. Week-old mice were injected with 10μl of inoculum containing synthetic wt DENV1-4 viruses as indicated doses. Each mouse was weighed daily for up to 2 weeks and mortality determined by humane early-endpoints (absence of weight gain, paresis, hind-limb paralysis) whenever possible.

| Virus | LD50 (FFU) | MTD (10 FFU) |
|---|---|---|
| DENV1 WT | 0.4 | 7 |
| DENV1 E-W/MIN | 2.2 | 10 |
| DENV2 WT | ≤3.4 | 9 |
| DENV2 E-W/MIN | 17.8 | >14 |
| DENV3 WT | 3.7 | 10 |
| DENV3 E-W/MIN | 1,931 | N.D. |
| DENV4 WT | 0.13 | 8 |
| DENV4 E-W/MIN | 2.5 | 11.5 |
| Tetravalent E-W/Min | 7.9 | 10 |

Various embodiments of the invention are described above in the Detailed Description. While these descriptions directly describe the above embodiments, it is understood that those skilled in the art may conceive modifications and/or variations to the specific embodiments shown and described herein. Any such modifications or variations that fall within the purview of this description are intended to be included therein as well. Unless specifically noted, it is the intention of the inventors that the words and phrases in the specification and claims be given the ordinary and accustomed meanings to those of ordinary skill in the applicable art(s).

The foregoing description of various embodiments of the invention known to the applicant at this time of filing the application has been presented and is intended for the purposes of illustration and description. The present description is not intended to be exhaustive nor limit the invention to the precise form disclosed and many modifications and variations are possible in the light of the above teachings. The embodiments described serve to explain the principles of the invention and its practical application and to enable others skilled in the art to utilize the invention in various embodiments and with various modifications as are suited to the particular use contemplated. Therefore, it is intended that the invention not be limited to the particular embodiments disclosed for carrying out the invention.

While particular embodiments of the present invention have been shown and described, it will be obvious to those skilled in the art that, based upon the teachings herein, changes and modifications may be made without departing from this invention and its broader aspects and, therefore, the appended claims are to encompass within their scope all such changes and modifications as are within the true spirit and scope of this invention. It will be understood by those within the art that, in general, terms used herein are generally intended as "open" terms (e.g., the term "including" should be interpreted as "including but not limited to," the term "having" should be interpreted as "having at least," the term "includes" should be interpreted as "includes but is not limited to," etc.).

As used herein the term "comprising" or "comprises" is used in reference to compositions, methods, and respective component(s) thereof, that are useful to an embodiment, yet open to the inclusion of unspecified elements, whether useful or not. It will be understood by those within the art that, in general, terms used herein are generally intended as "open" terms (e.g., the term "including" should be interpreted as "including but not limited to," the term "having" should be interpreted as "having at least," the term "includes" should be interpreted as "includes but is not limited to," etc.). Although the open-ended term "comprising," as a synonym of terms such as including, containing, or having, is used herein to describe and claim the invention, the present invention, or embodiments thereof, may alternatively be described using alternative terms such as "consisting of" or "consisting essentially of."

Numbered clauses:

The following numbered clauses form part of the description and not part of the claims, and do not define the scope of the claimed subject matter.

1. A modified dengue virus, comprising

a recoded prM protein, a recoded envelope (E) protein, or both,
wherein the recoded prM protein has a reduced codon pair bias compared to its parent prM protein encoding sequence, or has at least 5 codons substituted with synonymous codons less frequently used, or has an increased number of CpG or UpA di-nucleotides compared its parent prM protein encoding sequence, and
wherein the recoded E protein has a reduced codon pair bias compared to its parent E protein encoding sequence, or has at least 5 codons substituted with synonymous codons less frequently, or has an increased number of CpG or UpA di-nucleotides compared its parent E protein encoding sequence.

2. The modified dengue virus of clause 1, wherein expression of the prM protein or E protein or both are reduced compared to its parent dengue virus.
3. The modified dengue virus of clause 1, wherein the recoded prM protein has a reduced codon pair bias compared to its parent prM protein encoding sequence.
4. The modified dengue virus of clause 1, wherein the recoded prM protein has at least 5 codons substituted with synonymous codons less frequently used.
5. The modified dengue virus of clause 1, wherein the recoded prM protein has an increased number of CpG or UpA di-nucleotides compared its parent prM protein encoding sequence.
6. The modified dengue virus of clause 1, wherein the recoded E protein has a reduced codon pair bias compared to its parent E protein encoding sequence.
7. The modified dengue virus of clause 1, wherein the recoded E protein has at least 5 codons substituted with synonymous codons less frequently used.
8. The modified dengue virus of clause 1, wherein the recoded E protein has an increased number of CpG or UpA di-nucleotides compared its parent E protein encoding sequence.
9. The modified dengue virus of clause 1, wherein each of the recoded prM or E protein-encoding sequence has a codon pair bias of less than -0.05.
10. The modified dengue virus of clause 1, wherein the codon pair bias of each of the recoded prM or E protein-encoding sequence is reduced by at least 0.05.
11. The modified dengue virus of clause 1, wherein the modified dengue virus is selected from type 1, type 2, type 3, type 4 or a combination thereof.
12. The modified dengue virus of clause 1, where the modified dengue virus is a modified tetravalent dengue virus.
13. A dengue vaccine composition for inducing a protective immune response in a subject, comprising a modified dengue virus of any one of clauses 1-12, and a pharmaceutically acceptable excipient or carrier.
14. A method of eliciting an immune response in a subject, comprising: administering to the subject an effective dose of a composition comprising a modified dengue virus of any of clauses 1-12.
15. The method of clause 14, wherein the immune response is a protective immune response, and a prophylactically effective or therapeutically effective dose of a vaccine composition of clause 13 is administered.
16. The method of clause 14, further comprising administering to the subject at least one adjuvant.
17. The method of clause 14, wherein the immune response is cross-protective against a heterologous dengue virus.
18. A method of eliciting an immune response in a subject in need thereof, comprising:

administering a prime dose of (i) an attenuated dengue virus produced by a method other than codon-pair deoptimization or codon deoptimization, or increasing of CpG or UpA di-nucleotides, or (ii) a modified dengue virus comprising a recoded prM protein, a recoded envelope (E) protein, or both, wherein the recoded prM protein has a reduced codon pair bias compared to its parent prM protein encoding sequence, or has at least 5 codons substituted with synonymous codons less frequently used, or has an increased number of CpG or UpA di-nucleotides compared its parent prM protein encoding sequence, and wherein the recoded E protein has a reduced codon pair bias compared to its parent E protein encoding sequence, or has at least 5 codons substituted with synonymous codons less frequently, or has an increased number of CpG or UpA di-nucleotides compared its parent E protein encoding sequence; and
administering one or more boost dose of (i) the attenuated dengue virus produced by methods other than codon-pair deoptimization or codon deoptimization, or increasing of CpG or UpA di-nucleotides, or (ii) the modified dengue virus to the subject in need thereof, wherein at least the prime dose or the one or more boost dose is the modified dengue virus.

19. The method of clause 18, wherein a first of the one or more boost dose is administered about 2 weeks after the prime dose.
20. The method of clause 18, wherein expression of the prM protein or E protein or both are reduced compared to its parent dengue virus.
21. The method of clause 18, wherein the recoded prM protein has a reduced codon pair bias compared to its parent prM protein encoding sequence.
22. The method of clause 18, wherein the recoded prM protein has at least 5 codons substituted with synonymous codons less frequently used.
23. The method of clause 18, wherein the recoded prM protein has an increased number of CpG or UpA di-nucleotides compared its parent prM protein encoding sequence.
24. The method of clause 18, wherein the recoded E protein has a reduced codon pair bias compared to its parent E protein encoding sequence.
25. The method of clause 18, wherein the recoded E protein has at least 5 codons substituted with synonymous codons less frequently used.
26. The method of clause 18, wherein the recoded E protein has an increased number of CpG or UpA di-nucleotides compared its parent E protein encoding sequence.
27. The method of clause 18, wherein each of the recoded prM or E protein-encoding sequence has a codon pair bias of less than -0.05.
28. The method of clause 18, wherein the codon pair bias of each of the recoded prM or E protein-encoding sequence is reduced by at least 0.05.
29. The method of clause 18, wherein the modified dengue virus is selected from type 1, type 2, type 3, type 4 or a combination thereof.
30. The method of clause 18, where the modified dengue virus is a modified tetravalent dengue virus.
31. A method of making a modified dengue virus genome comprising:

obtaining a nucleotide sequence encoding the envelope protein of a dengue virus;
recoding the envelope encoding nucleotide sequence to reduce protein expression, and
substituting a nucleic acid having the recoded envelope-encoding nucleotide sequence into a parent dengue virus genome to make a modified dengue virus genome; whereby expression of the recoded envelope-encoding nucleotide sequence is reduced compared to the parent virus.

## Claims

**1.** A modified dengue virus, comprising

a recoded envelope (E) protein encoding sequence, or both the recoded envelope (E) protein encoding sequence and a recoded prM protein encoding sequence,
wherein the modified dengue virus is selected from serotype 1, serotype 2, serotype 3, or serotype 4, and
wherein the recoded E protein encoding sequence has the E protein encoding sequence in SEQ ID NO: 9, SEQ ID NO:10, SEQ ID NO:11, or SEQ ID NO:12, or wherein the recoded E protein encoding sequence has the E protein encoding sequence in SEQ ID NO: 9, SEQ ID NO:10, SEQ ID NO:11, or SEQ ID NO:12, with up to 15 nucleotide substitutions in the E protein encoding sequence.

**2.** The modified dengue virus of claim 1, wherein expression of the E protein or both the E protein and the prM protein are reduced compared to its parent dengue virus.

**3.** The modified dengue virus of claim 1, wherein the recoded E protein encoding sequence has the E protein encoding sequence in SEQ ID NO: 9, SEQ ID NO:10, SEQ ID NO:11, or SEQ ID NO:12, with up to 15 nucleotide substitutions in the E protein encoding sequence

**4.** The modified dengue virus of claim 1, wherein the recoded E protein encoding sequence has the E protein encoding sequence in SEQ ID NO: 9, SEQ ID NO:10, SEQ ID NO:11, or SEQ ID NO:12.

**5.** The modified dengue virus of claim 1, wherein the modified dengue virus comprises a recoded sequence of SEQ ID NO: 9, SEQ ID NO:10, SEQ ID NO:11, or SEQ ID NO:12.

6. The modified dengue virus of any one of claims 1-5, wherein the recoded E protein encoding sequence is recoded from a parent E protein encoding sequence from DENV-1/VN/BID-V1774/2007 (SEQ ID NO:1), DENV-2/NI/BID-V533/2005 (SEQ ID NO:3), DENV-3/VE/BID-V2268/2008 (SEQ ID NO:5), or DENV-4/US/BID-V2448/1999 (SEQ ID NO:7).

7. A tetravalent dengue virus composition, comprising: a modified dengue virus of any one of claims 1-6 of serotype 1, a modified dengue virus of any one of claims 1-6 of serotype 2, a modified dengue virus of any one of claims 1-6 of serotype 3, and a modified dengue virus of any one of claims 1-6 of serotype 4.

8. The tetravalent dengue virus composition of claim 7, wherein the recoded E protein encoding sequence is recoded from a parent E protein encoding sequence of serotype 1 from DENV-1/VN/BID-V1774/2007 (SEQ ID NO:1), a parent E protein encoding sequence of serotype 2 is from DENV-2/NI/BID-V533/2005 (SEQ ID NO:3), a parent E protein encoding sequence of serotype 3 from DENV-3/VE/BID-V2268/2008 (SEQ ID NO:5), and a parent E protein encoding sequence of serotype 4 from DENV-4/US/BID-V2448/1999 (SEQ ID NO:7).

9. The tetravalent dengue virus composition of claim 7 or claim 8, wherein

   the modified dengue virus of serotype 1 has a recoded E protein encoding sequence of the E protein encoding sequence in SEQ ID NO: 9 with up to 15 nucleic acid substitutions in the E protein encoding sequence,
   the modified dengue virus of serotype 2 has a recoded E protein encoding sequence of the E protein encoding sequence in SEQ ID NO: 10 with up to 15 nucleic acid substitutions in the E protein encoding sequence,
   the modified dengue virus of serotype 3 has a recoded E protein encoding sequence of the E protein encoding sequence in SEQ ID NO: 11 with up to 15 nucleic acid substitutions in the E protein encoding sequence, and
   the modified dengue virus of serotype 4 has a recoded E protein encoding sequence of the E protein encoding sequence in SEQ ID NO: 12 with up to 15 nucleic acid substitutions in the E protein encoding sequence.

10. The tetravalent dengue virus composition of claim 7 or claim 8, wherein

   the modified dengue virus of serotype 1 has a recoded E protein encoding sequence of the E protein encoding sequence in SEQ ID NO: 9,
   the modified dengue virus of serotype 2 has a recoded E protein encoding sequence of the E protein encoding sequence in SEQ ID NO: 10,
   the modified dengue virus of serotype 3 has a recoded E protein encoding sequence of the E protein encoding sequence in SEQ ID NO: 11, and
   the modified dengue virus of serotype 4 has a recoded E protein encoding sequence of the E protein encoding sequence in SEQ ID NO: 12.

11. The tetravalent dengue virus composition of claim 7 or claim 8, comprising modified dengue viruses having

   a sequence of SEQ ID NO: 9 with up to 15 nucleic acid substitutions in its E protein encoding sequence,
   a sequence of SEQ ID NO:10 with up to 15 nucleic acid substitutions in its E protein encoding sequence,
   a sequence of SEQ ID NO:11 with up to 15 nucleic acid substitutions in its E protein encoding sequence, and
   a sequence of SEQ ID NO:12 with up to 15 nucleic acid substitutions in its E protein encoding sequence.

12. The tetravalent dengue virus composition of claim 7 or claim 8, comprising modified dengue viruses having sequences of SEQ ID NO: 9, SEQ ID NO:10, SEQ ID NO:11, and SEQ ID NO:12.

13. A dengue immune composition for inducing an immune response in a subject, comprising one or more modified dengue viruses of any one of claims 1-6 or the tetravalent dengue virus composition of claims 7 or claim 8, and a pharmaceutically acceptable excipient or carrier.

14. The dengue immune composition of claim 13, further comprising at least one adjuvant.

15. The dengue immune composition of claim 13 or claim 14, wherein the immune response is cross-reactive against a heterologous dengue virus.

16. A dengue immune composition for inducing an immune response in a subject, comprising:

a prime dose of (i) an attenuated dengue virus produced by a method other than codon-pair deoptimization or codon deoptimization, or increasing of CpG or UpA di-nucleotides, or (ii) one or more modified dengue viruses of any one of claims 1-6 or the tetravalent dengue virus composition of claims 7 or claim 8; and
one or more boost dose of (i) the attenuated dengue virus produced by methods other than codon-pair deoptimization or codon deoptimization, or increasing of CpG or UpA di-nucleotides, or (ii) the one or more modified dengue viruses, wherein at least the prime dose or the one or more boost dose is the modified dengue virus.

**17.** A method of making a modified dengue virus genome comprising:

obtaining a parent E protein encoding sequence of a parent dengue virus;
recoding the E protein encoding sequence to reduce protein expression,

wherein the recoded E protein-encoding sequence or both the recoded E-protein encoding sequence and the recoded prM encoding sequence has a codon pair bias of less than - 0.05, and
wherein the recoded envelope (E) protein encoding sequence has the E protein encoding sequence in SEQ ID NO: 9, SEQ ID NO: 10, SEQ ID NO: 11, or SEQ ID NO:12, or wherein the recoded E protein encoding sequence has the E protein encoding sequence in SEQ ID NO: 9, SEQ ID NO: 10, SEQ ID NO:11, or SEQ ID NO:12, with up to 15 nucleotide substitutions in the E protein encoding sequence,

substituting the recoded envelope (E) protein encoding sequence into the parent dengue virus genome to make a modified dengue virus genome; whereby expression of the recoded envelope (E) protein encoding sequence is reduced compared to the parent virus, wherein the modified dengue virus is selected from type 1, type 2, type 3, or type 4.

FIG. 1A

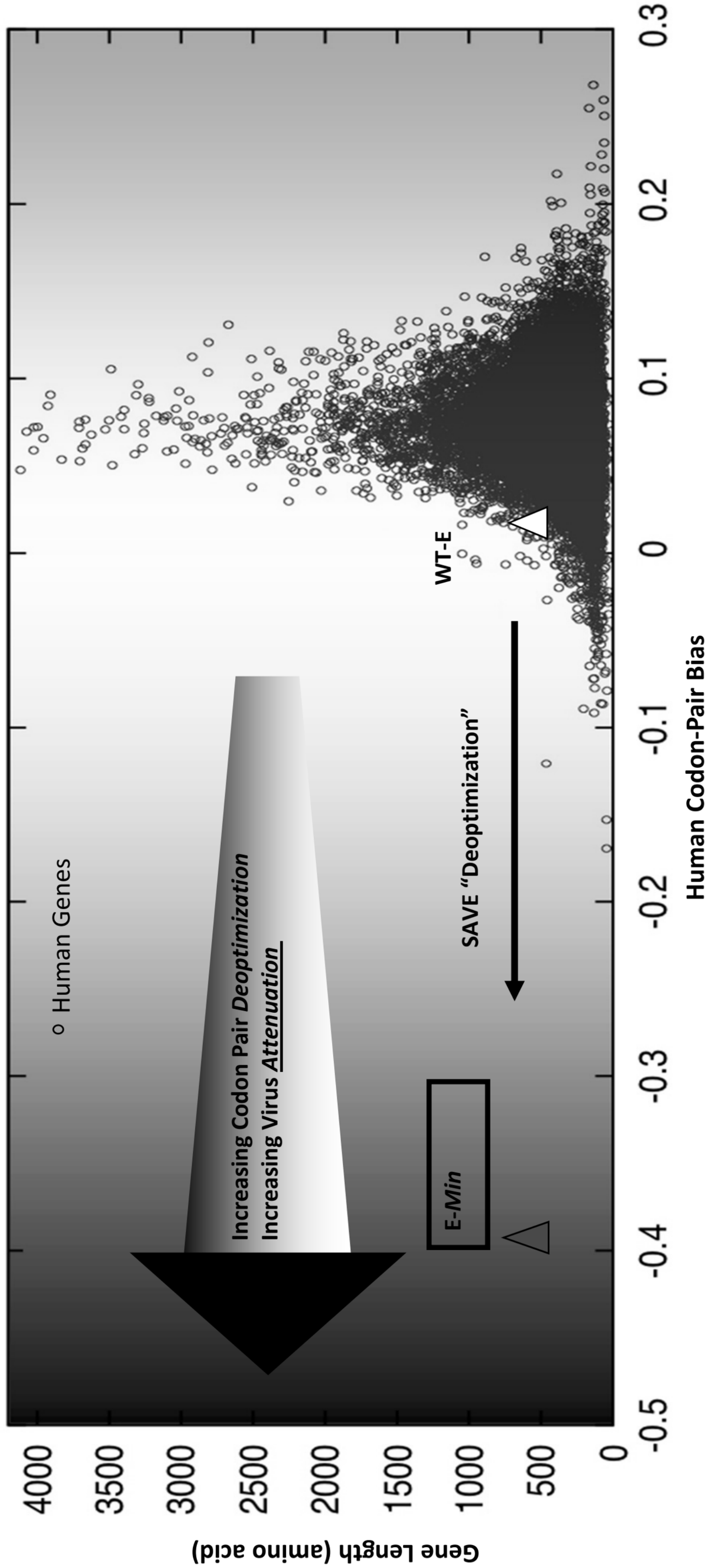

Human Genes
Increasing Codon Pair *Deoptimization*
Increasing Virus *Attenuation*
E-*Min*
SAVE "Deoptimization"
WT-E
Human Codon-Pair Bias
Gene Length (amino acid)
-0.5
-0.4
-0.3
-0.2
-0.1
0
0.1
0.2
0.3
0
500
1000
1500
2000
2500
3000
3500
4000

FIG. 1B

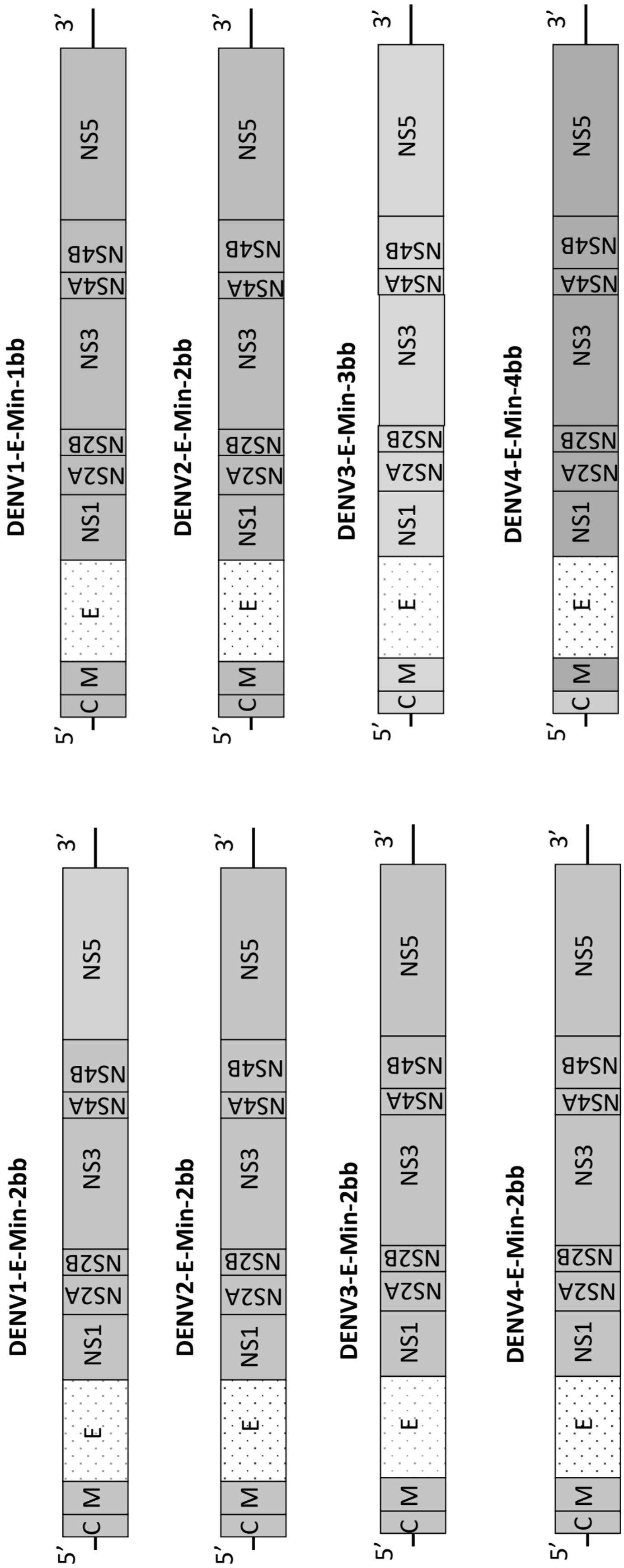
DENV1-E-Min-1bb
DENV2-E-Min-2bb
DENV3-E-Min-3bb
DENV4-E-Min-4bb
DENV1-E-Min-2bb
DENV2-E-Min-2bb
DENV3-E-Min-2bb
DENV4-E-Min-2bb
5'
C
M
E
NS1
NS2A
NS2B
NS3
NS4A
NS4B
NS5
3'

FIG. 2

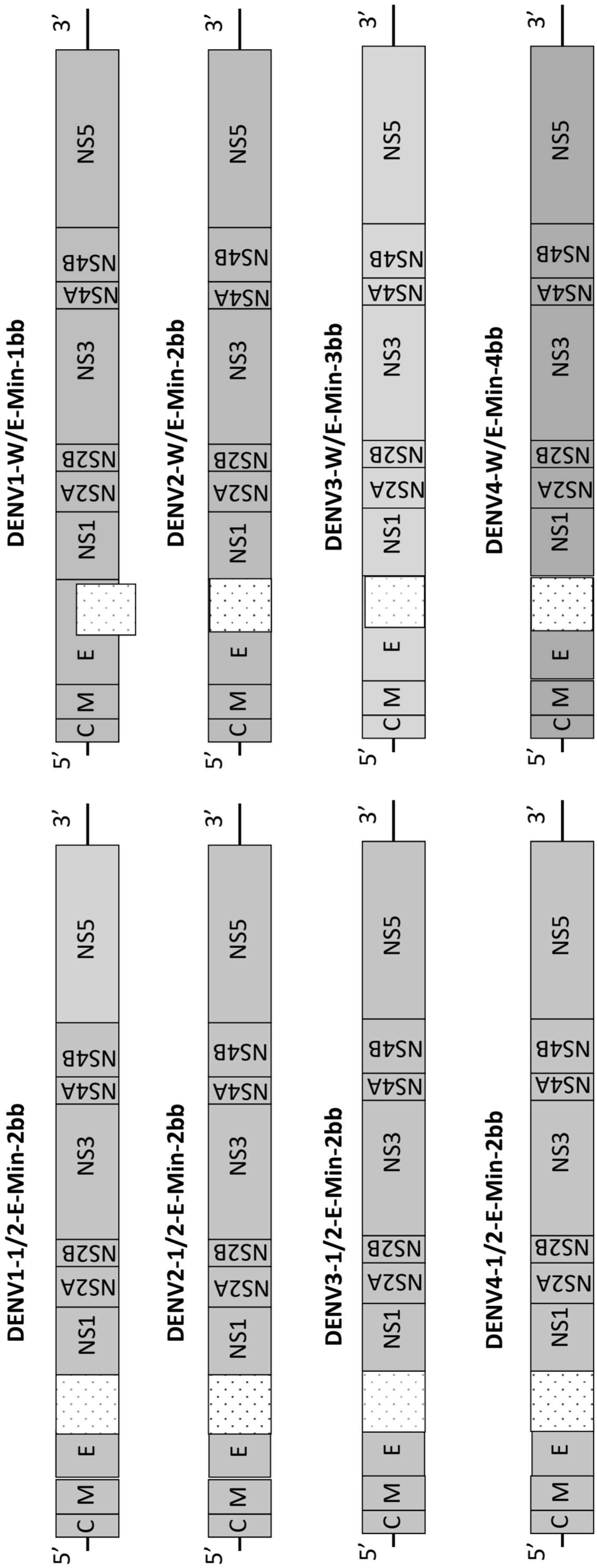
DENV1-W/E-Min-1bb
DENV2-W/E-Min-2bb
DENV3-W/E-Min-3bb
DENV4-W/E-Min-4bb
DENV1-1/2-E-Min-2bb
DENV2-1/2-E-Min-2bb
DENV3-1/2-E-Min-2bb
DENV4-1/2-E-Min-2bb
5'
C
M
E
NS1
NS2A
NS2B
NS3
NS4A
NS4B
NS5
3'

FIG. 3

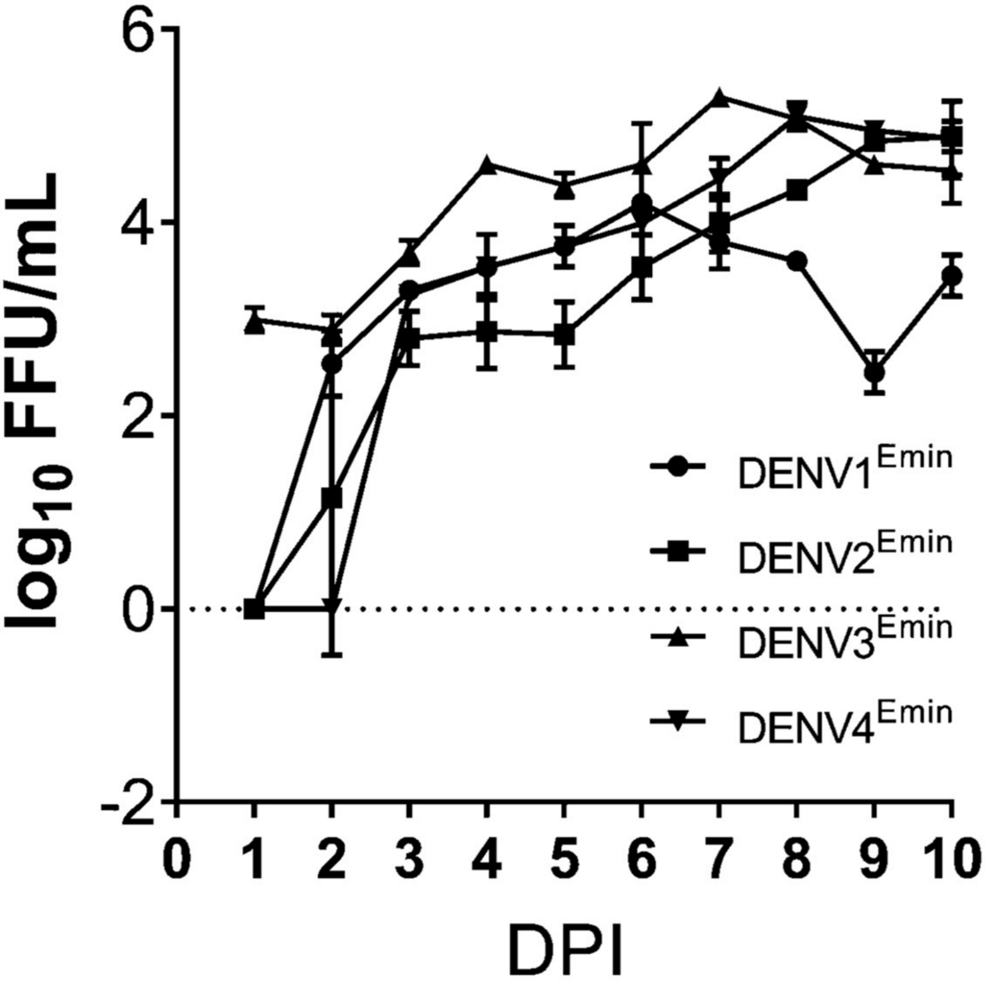
6
4
2
0
-2
log10 FFU/mL
0 1 2 3 4 5 6 7 8 9 10
DPI
DENV1Emin
DENV2Emin
DENV3Emin
DENV4Emin

FIG. 4A

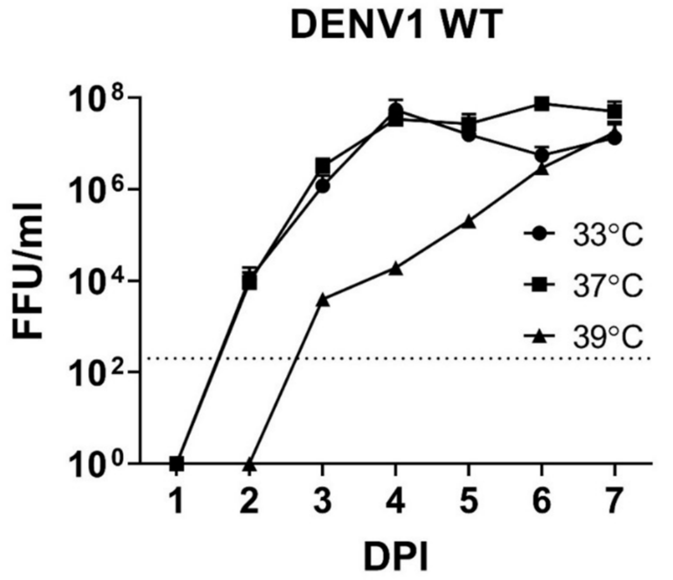


FIG. 4B

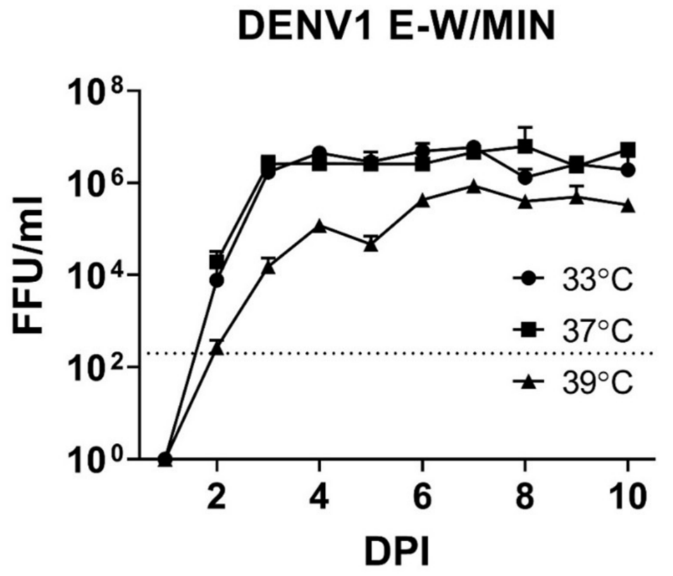


FIG. 4C

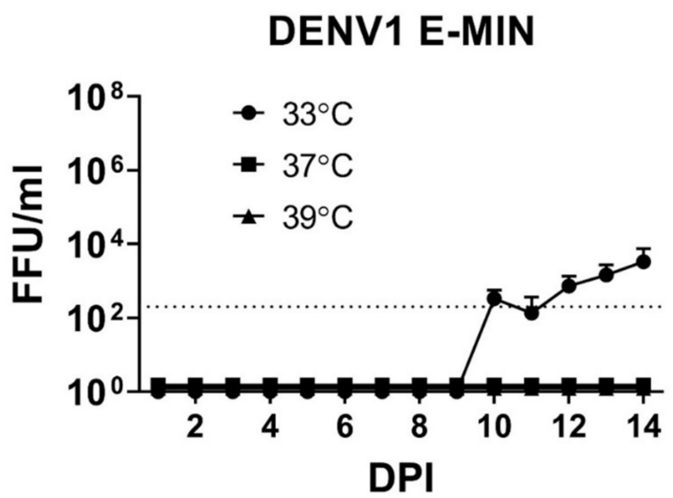

FIG. 5

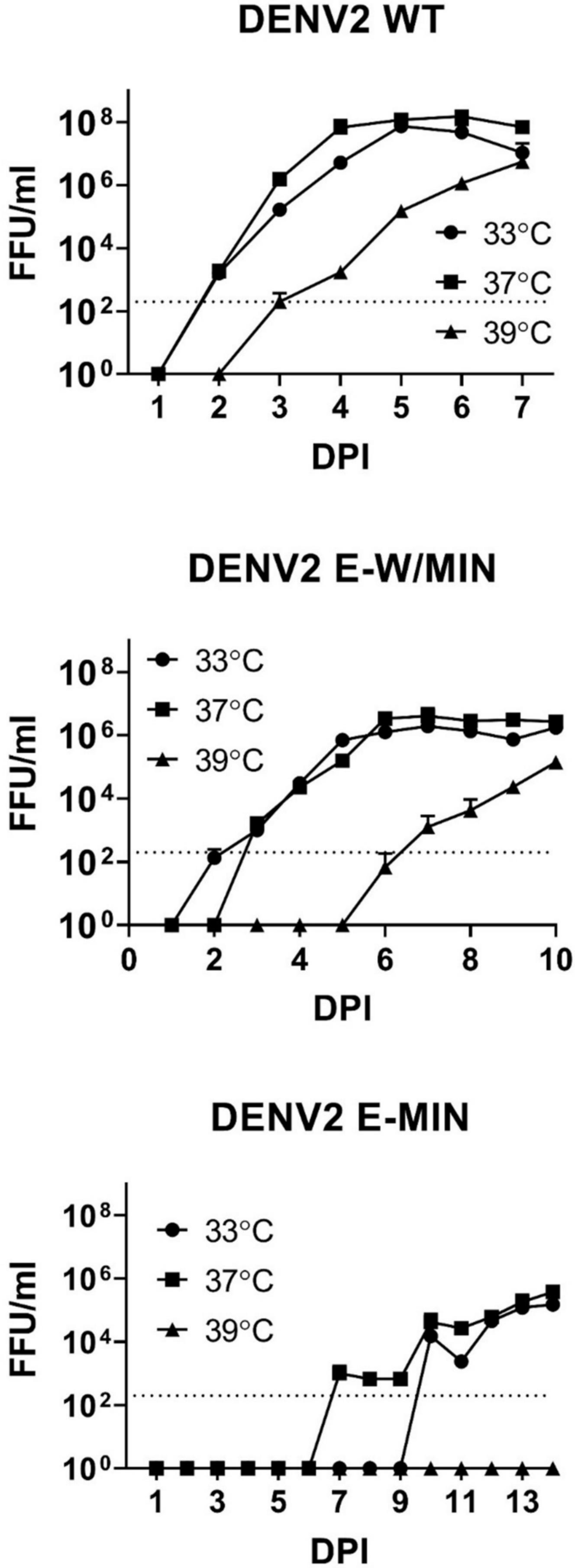
DENV2 WT
FFU/ml
10⁸
10⁶
10⁴
10²
10⁰
33°C
37°C
39°C
1 2 3 4 5 6 7
DPI
DENV2 E-W/MIN
FFU/ml
33°C
37°C
39°C
0 2 4 6 8 10
DPI
DENV2 E-MIN
FFU/ml
33°C
37°C
39°C
1 3 5 7 9 11 13
DPI

FIG. 6

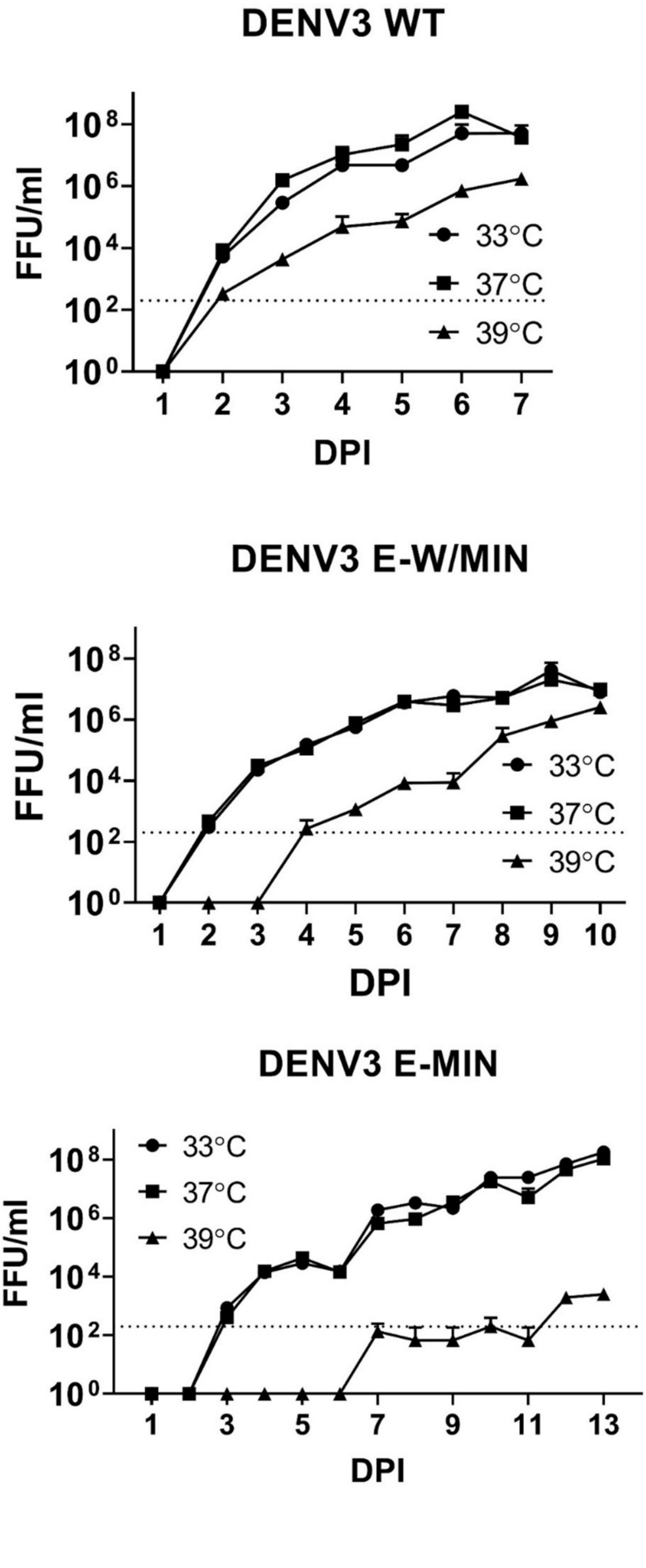
DENV3 WT
FFU/ml
10^8
10^6
10^4
10^2
10^0
1 2 3 4 5 6 7
DPI
33°C
37°C
39°C
DENV3 E-W/MIN
FFU/ml
10^8
10^6
10^4
10^2
10^0
1 2 3 4 5 6 7 8 9 10
DPI
33°C
37°C
39°C
DENV3 E-MIN
FFU/ml
10^8
10^6
10^4
10^2
10^0
1 3 5 7 9 11 13
DPI
33°C
37°C
39°C

FIG. 7

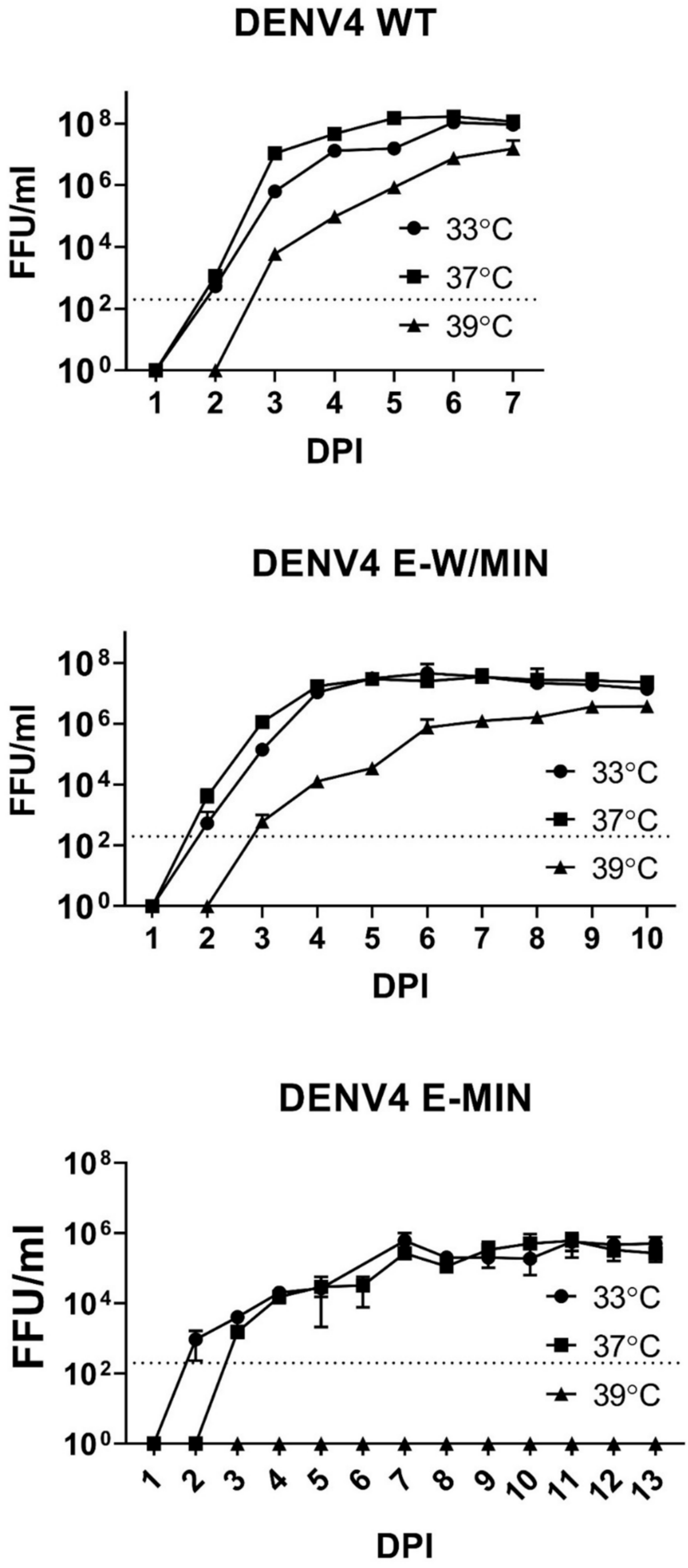
DENV4 WT
FFU/ml
$10^8$
$10^6$
$10^4$
$10^2$
$10^0$
1 2 3 4 5 6 7
DPI
33°C
37°C
39°C
DENV4 E-W/MIN
FFU/ml
$10^8$
$10^6$
$10^4$
$10^2$
$10^0$
1 2 3 4 5 6 7 8 9 10
DPI
33°C
37°C
39°C
DENV4 E-MIN
FFU/ml
$10^8$
$10^6$
$10^4$
$10^2$
$10^0$
1 2 3 4 5 6 7 8 9 10 11 12 13
DPI
33°C
37°C
39°C

FIG. 8A

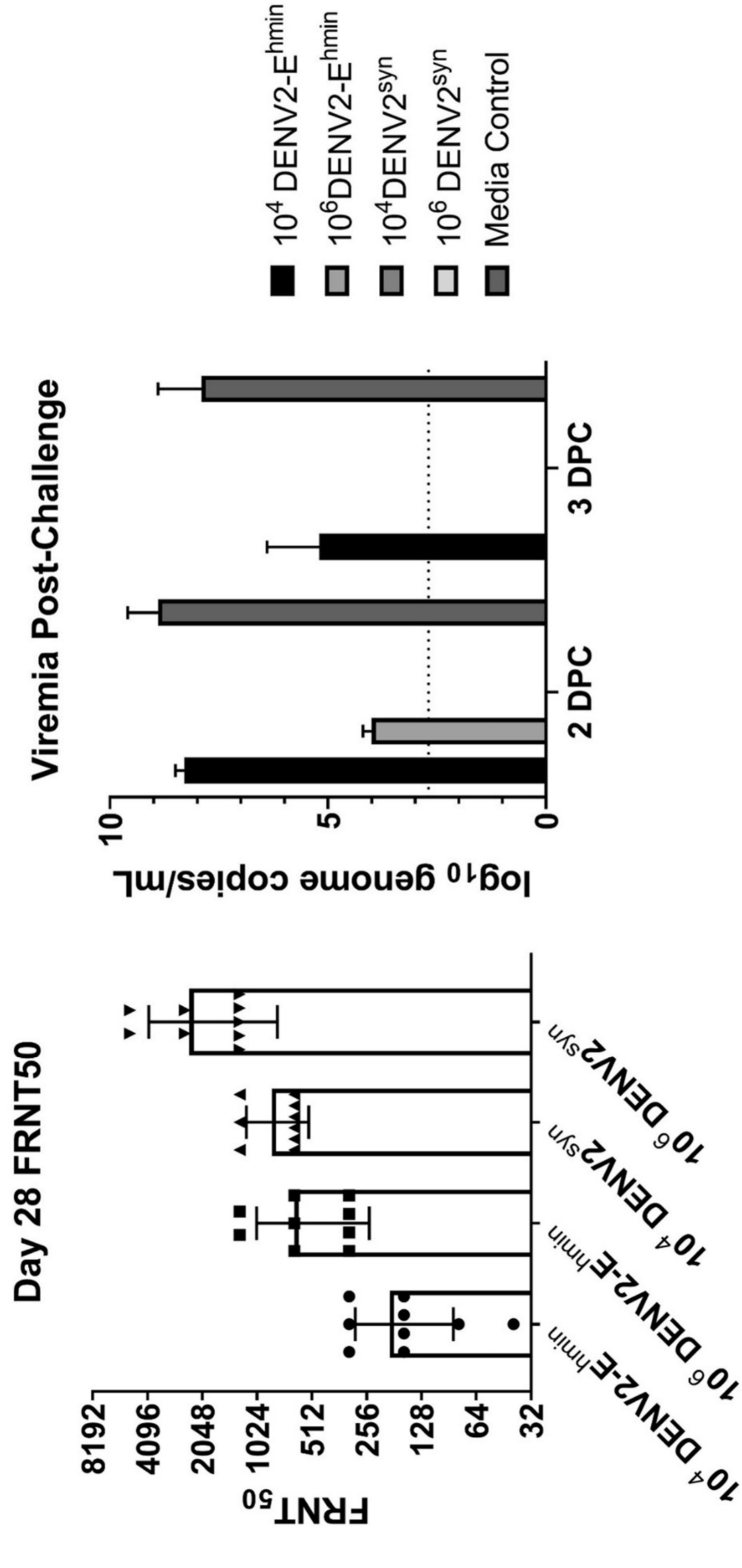
Day 28 FRNT50
FRNT50
8192
4096
2048
1024
512
256
128
64
32
10^4 DENV2-E^hmin
10^6 DENV2-E^hmin
10^4 DENV2^syn
10^6 DENV2^syn
Viremia Post-Challenge
log10 genome copies/mL
10
5
0
2 DPC
3 DPC
10^4 DENV2-E^hmin
10^6DENV2-E^hmin
10^4DENV2^syn
10^6 DENV2^syn
Media Control

FIG. 8B

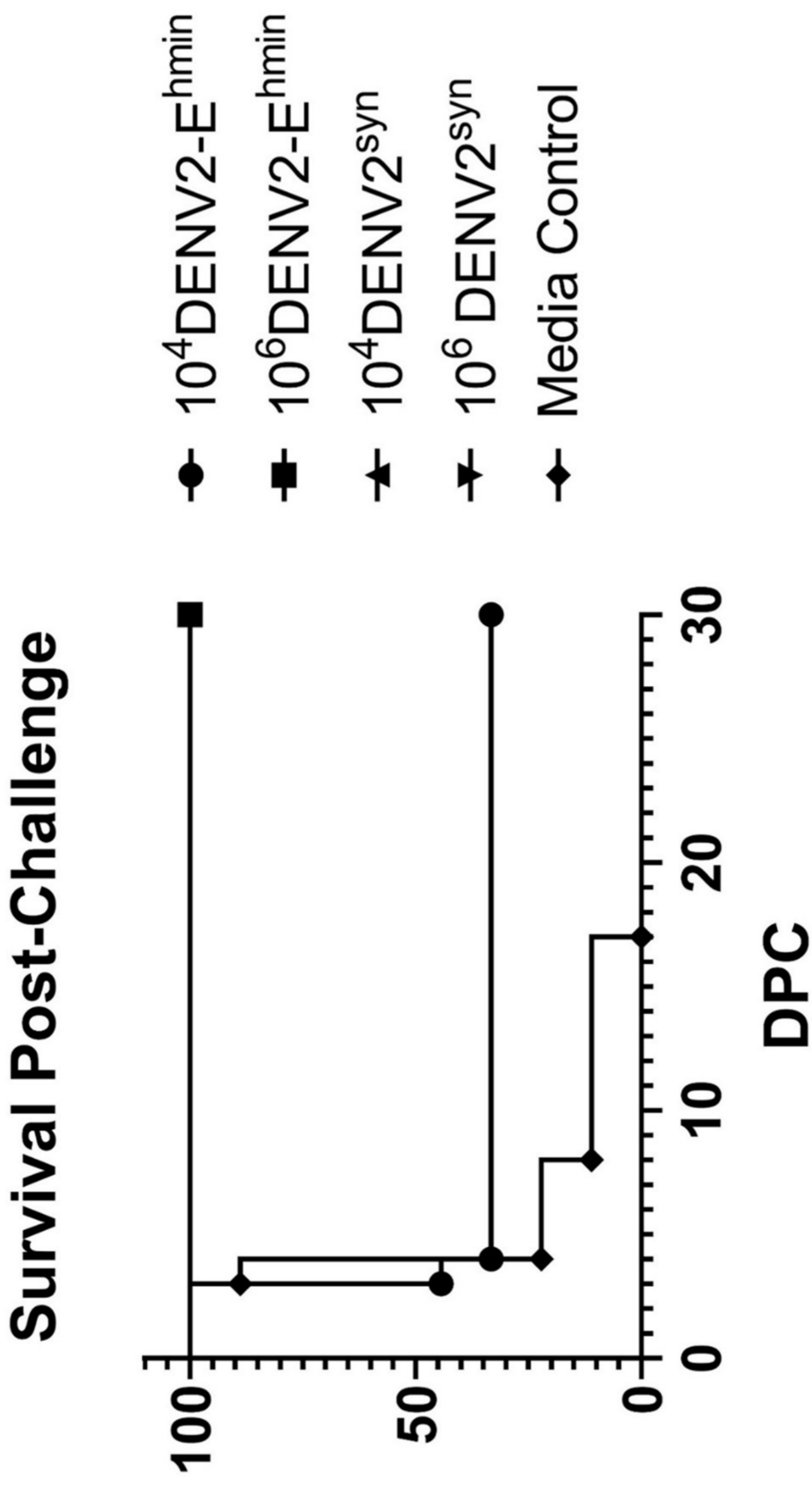
Survival Post-Challenge
Survival (%)
100
50
0
0
10
20
30
DPC
$10^4$DENV2-$E^{hmin}$
$10^6$DENV2-$E^{hmin}$
$10^4$DENV2$^{syn}$
$10^6$ DENV2$^{syn}$
Media Control

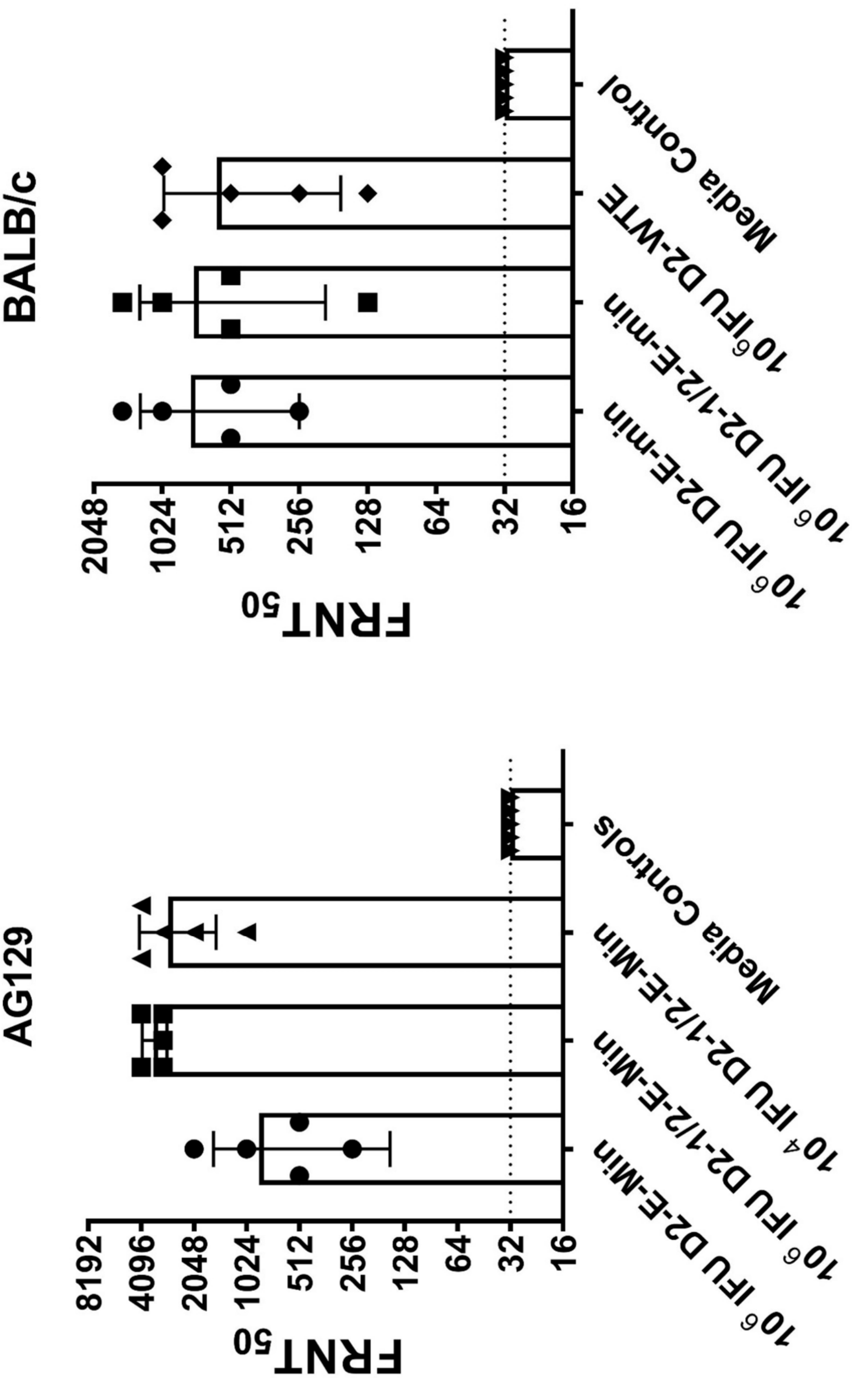
BALB/c
FRNT50
2048
1024
512
256
128
64
32
16
10^6 IFU D2-E-min
10^6 IFU D2-1/2-E-min
10^6 IFU D2-WTE
Media Control
AG129
FRNT50
8192
4096
2048
1024
512
256
128
64
32
16
10^6 IFU D2-E-Min
10^6 IFU D2-1/2-E-Min
10^4 IFU D2-1/2-E-Min
Media Controls

FIG. 9

FIG. 10A

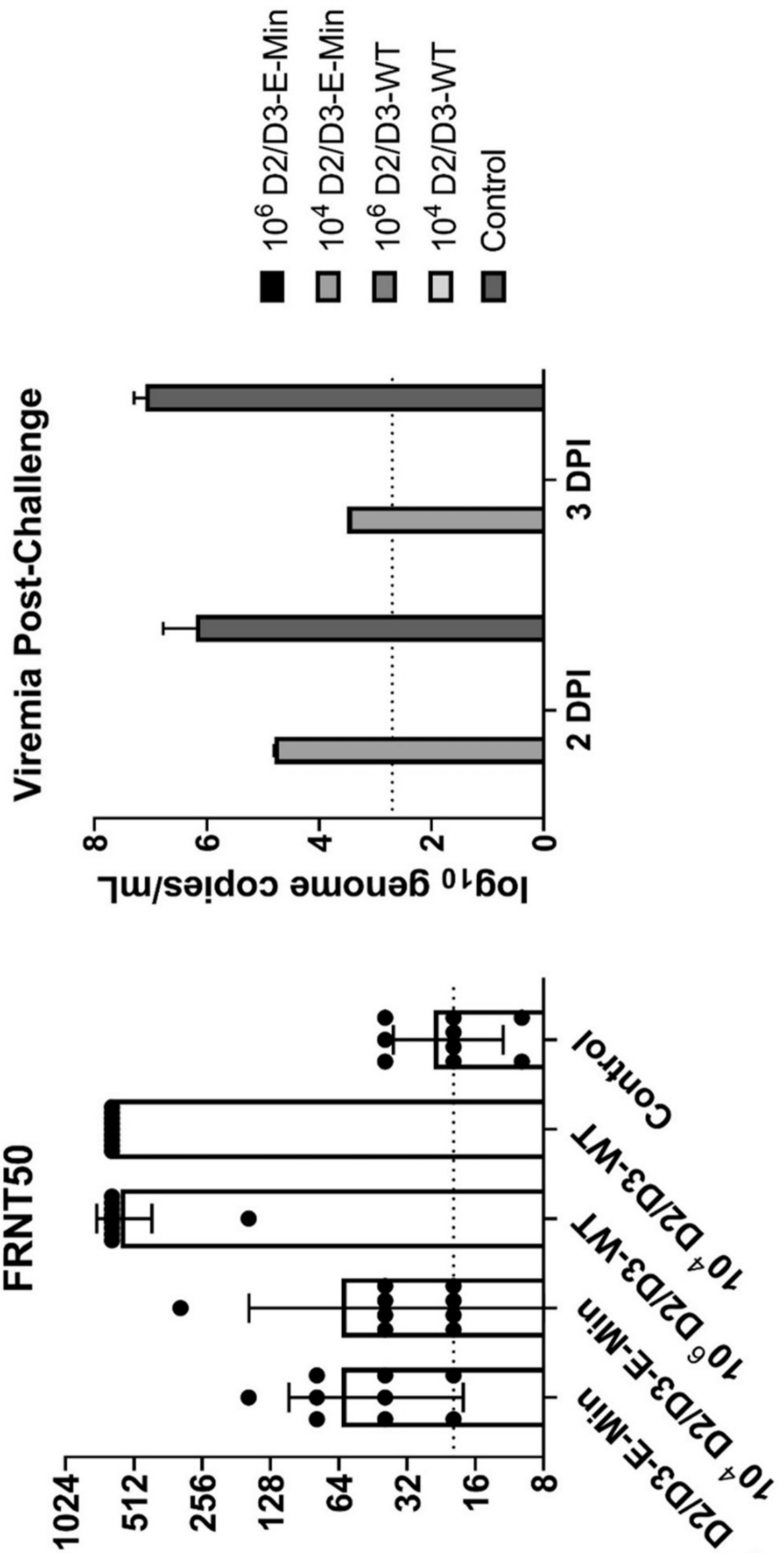
Viremia Post-Challenge
log10 genome copies/mL
8
6
4
2
0
2 DPI
3 DPI
10^6 D2/D3-E-Min
10^4 D2/D3-E-Min
10^6 D2/D3-WT
10^4 D2/D3-WT
Control
FRNT50
FRNT50
1024
512
256
128
64
32
16
8
10^6 D2/D3-E-Min
10^4 D2/D3-E-Min
10^6 D2/D3-WT
10^4 D2/D3-WT
Control

FIG. 10B

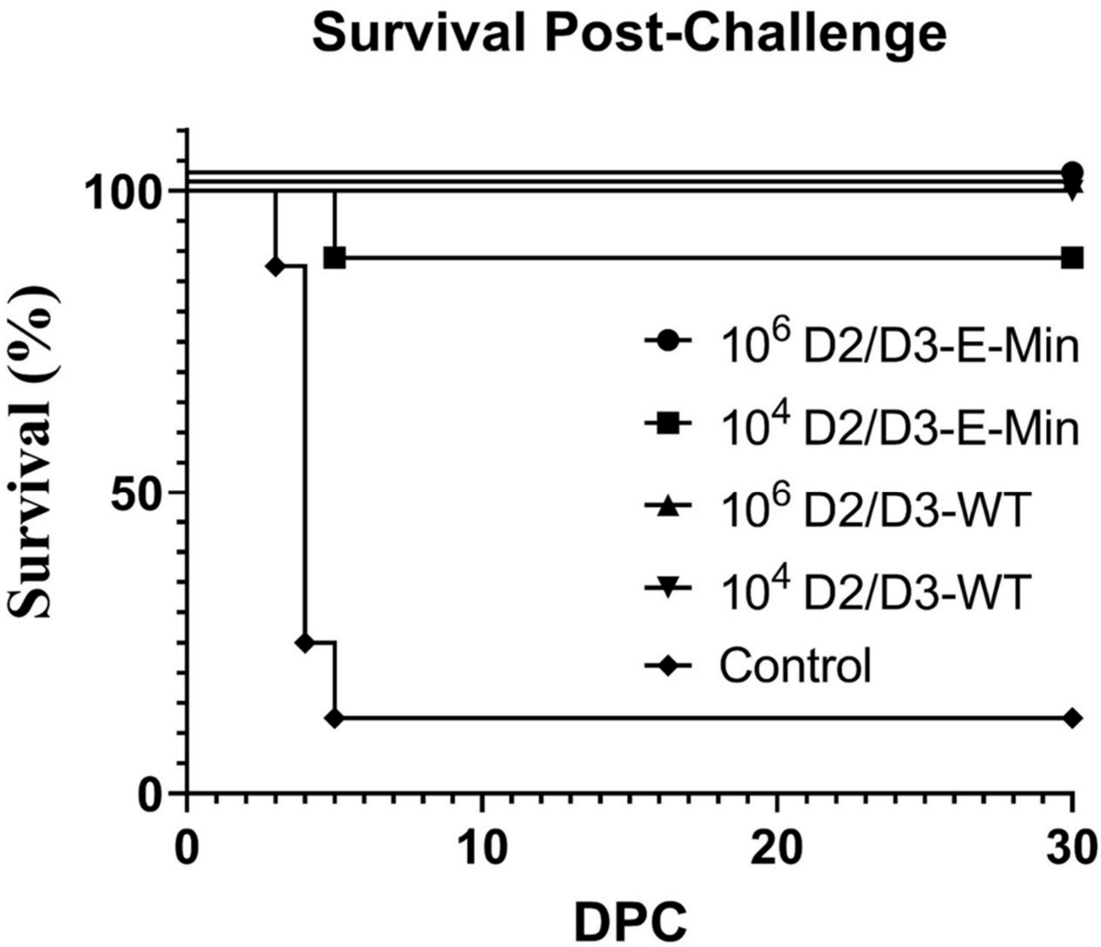
Survival Post-Challenge
Survival (%)
100
50
0
0
10
20
30
DPC
10^6 D2/D3-E-Min
10^4 D2/D3-E-Min
10^6 D2/D3-WT
10^4 D2/D3-WT
Control

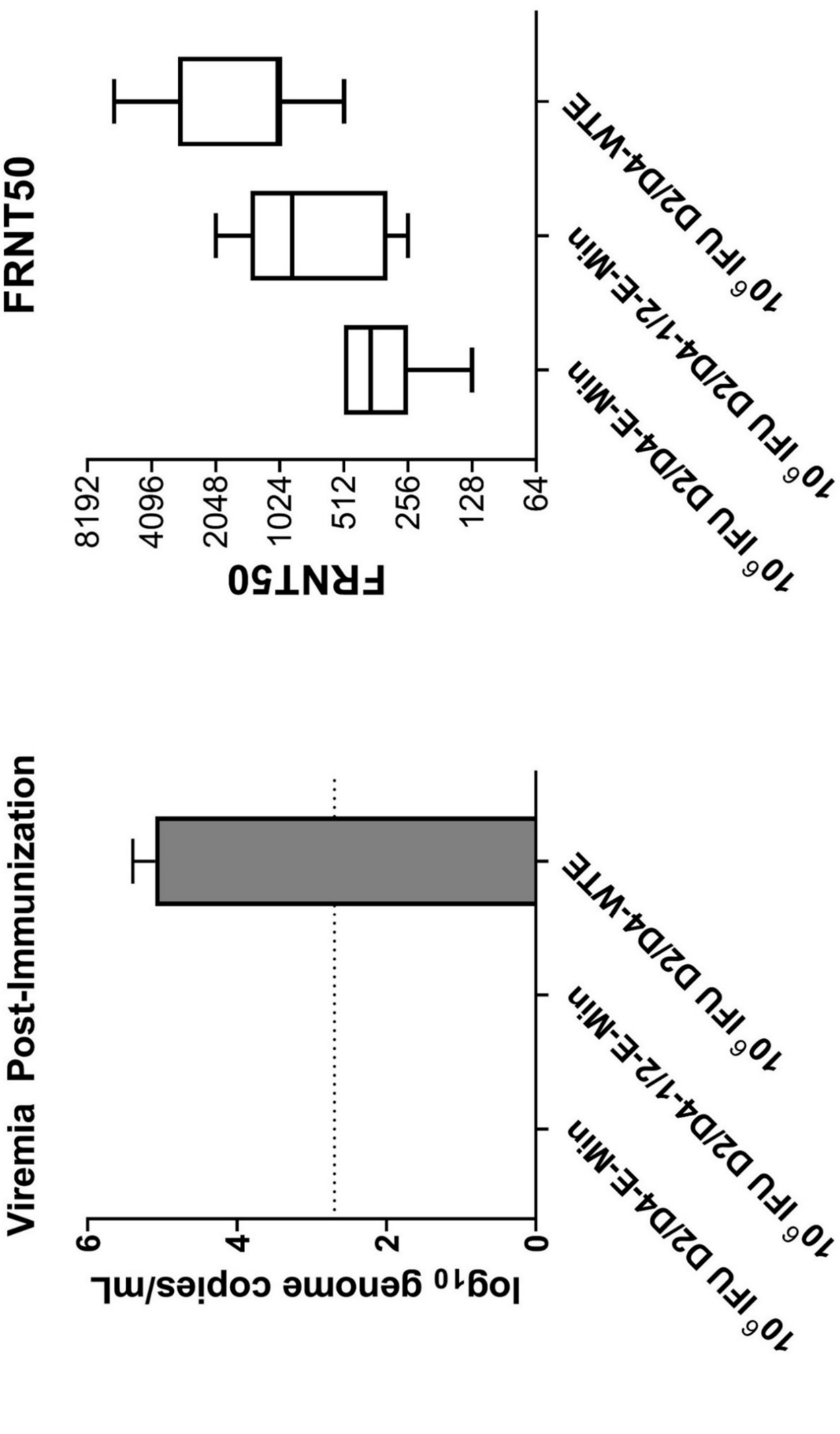
FRNT50
FRNT50
8192
4096
2048
1024
512
256
128
64
10^6 IFU D2/D4-E-Min
10^6 IFU D2/D4-1/2-E-Min
10^6 IFU D2/D4-WTE
Viremia Post-Immunization
log10 genome copies/mL
6
4
2
0
10^6 IFU D2/D4-E-Min
10^6 IFU D2/D4-1/2-E-Min
10^6 IFU D2/D4-WTE

FIG. 11A

FIG. 11B

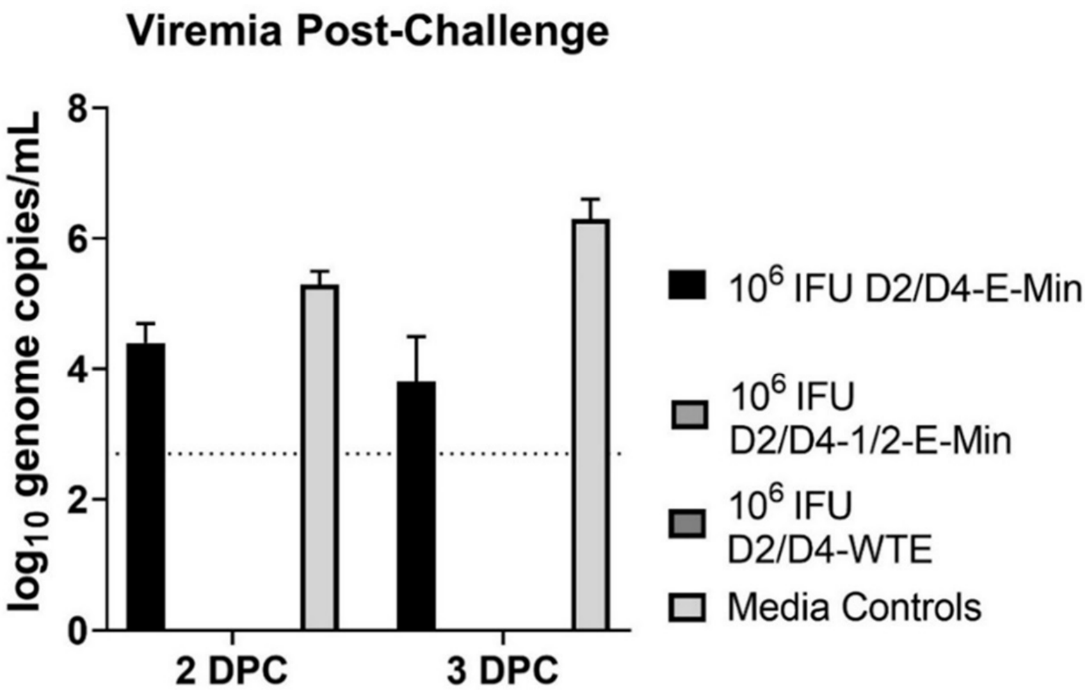
Viremia Post-Challenge
log10 genome copies/mL
8
6
4
2
0
2 DPC
3 DPC
10^6 IFU D2/D4-E-Min
10^6 IFU D2/D4-1/2-E-Min
10^6 IFU D2/D4-WTE
Media Controls

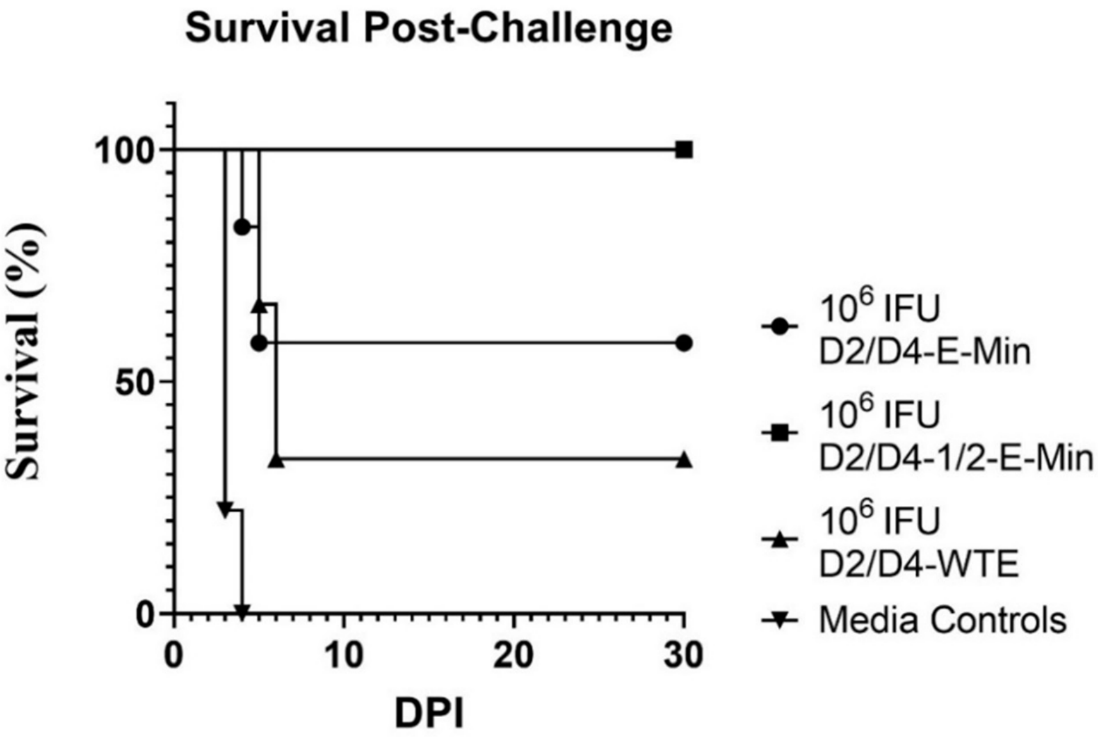
Survival Post-Challenge
Survival (%)
100
50
0
0
10
20
30
DPI
10^6 IFU D2/D4-E-Min
10^6 IFU D2/D4-1/2-E-Min
10^6 IFU D2/D4-WTE
Media Controls

FIG. 12

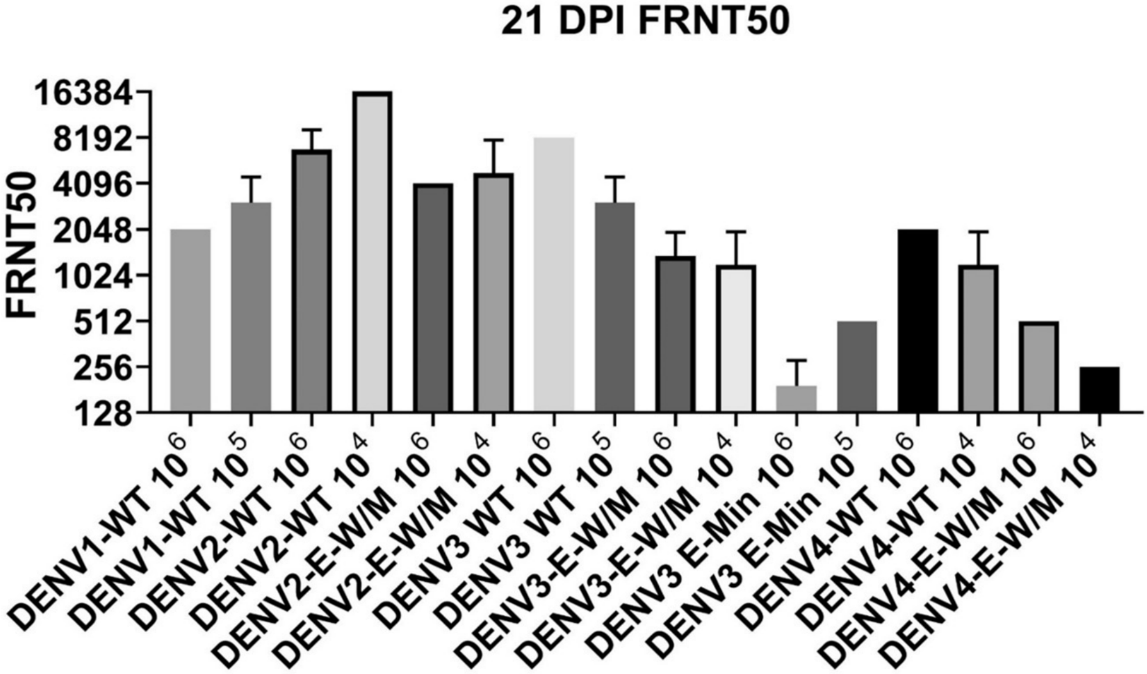
21 DPI FRNT50
16384
8192
4096
2048
1024
512
256
128
FRNT50
DENV1-WT 10^6
DENV1-WT 10^5
DENV2-WT 10^6
DENV2-WT 10^4
DENV2-E-W/M 10^6
DENV2-E-W/M 10^4
DENV3 WT 10^6
DENV3 WT 10^5
DENV3-E-W/M 10^6
DENV3-E-W/M 10^4
DENV3 E-Min 10^6
DENV3 E-Min 10^5
DENV4-WT 10^6
DENV4-WT 10^4
DENV4-E-W/M 10^6
DENV4-E-W/M 10^4

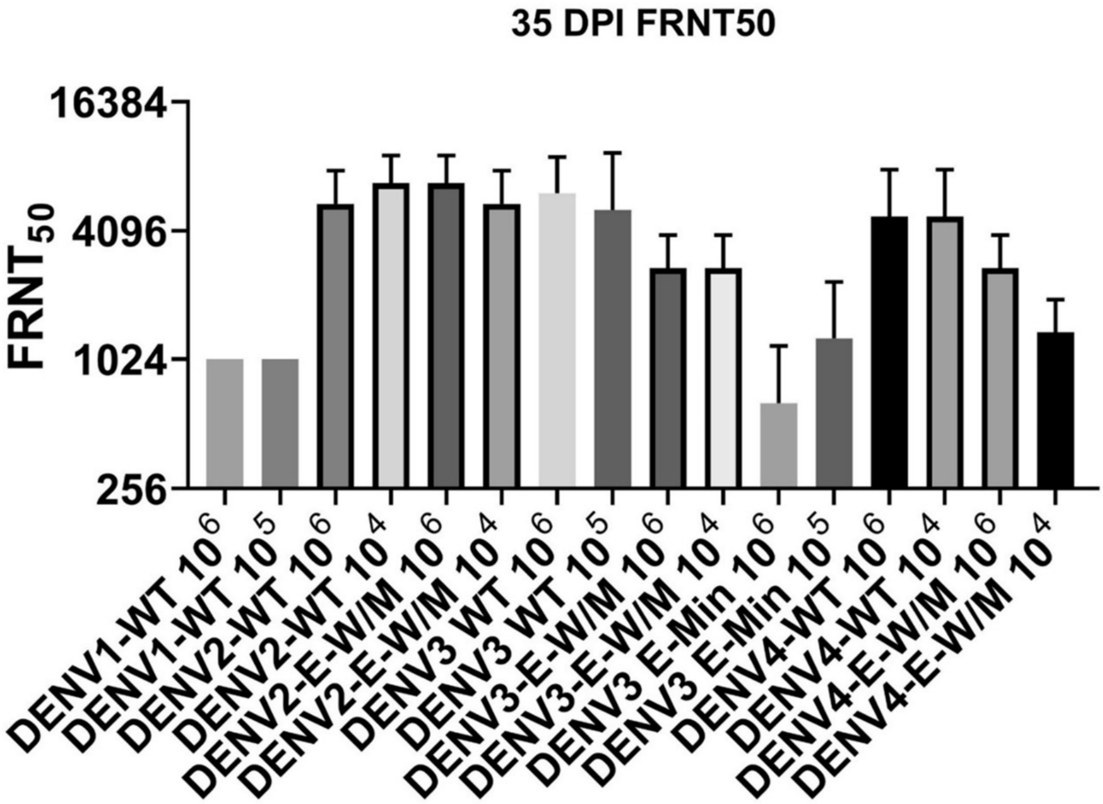
35 DPI FRNT50
16384
4096
1024
256
FRNT50
DENV1-WT 10^6
DENV1-WT 10^5
DENV2-WT 10^6
DENV2-WT 10^4
DENV2-E-W/M 10^6
DENV2-E-W/M 10^4
DENV3 WT 10^6
DENV3 WT 10^5
DENV3-E-W/M 10^6
DENV3-E-W/M 10^4
DENV3 E-Min 10^6
DENV3 E-Min 10^5
DENV4-WT 10^6
DENV4-WT 10^4
DENV4-E-W/M 10^6
DENV4-E-W/M 10^4

FIG. 13

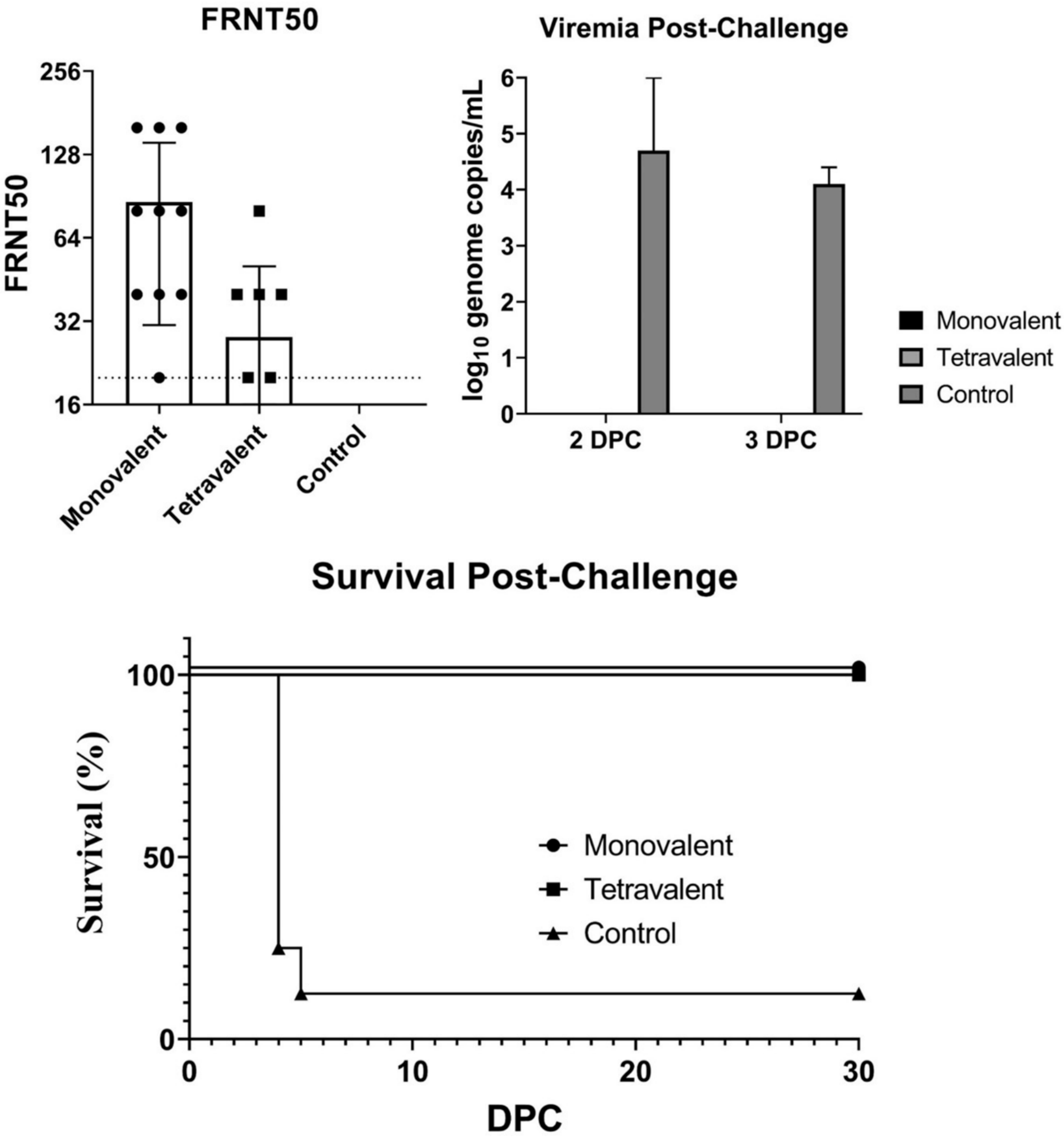
FRNT50
256
128
64
32
16
FRNT50
Monovalent
Tetravalent
Control
Viremia Post-Challenge
6
5
4
3
2
1
0
$log_{10}$ genome copies/mL
2 DPC
3 DPC
Monovalent
Tetravalent
Control
Survival Post-Challenge
100
50
0
Survival (%)
Monovalent
Tetravalent
Control
0
10
20
30
DPC

FIG. 14

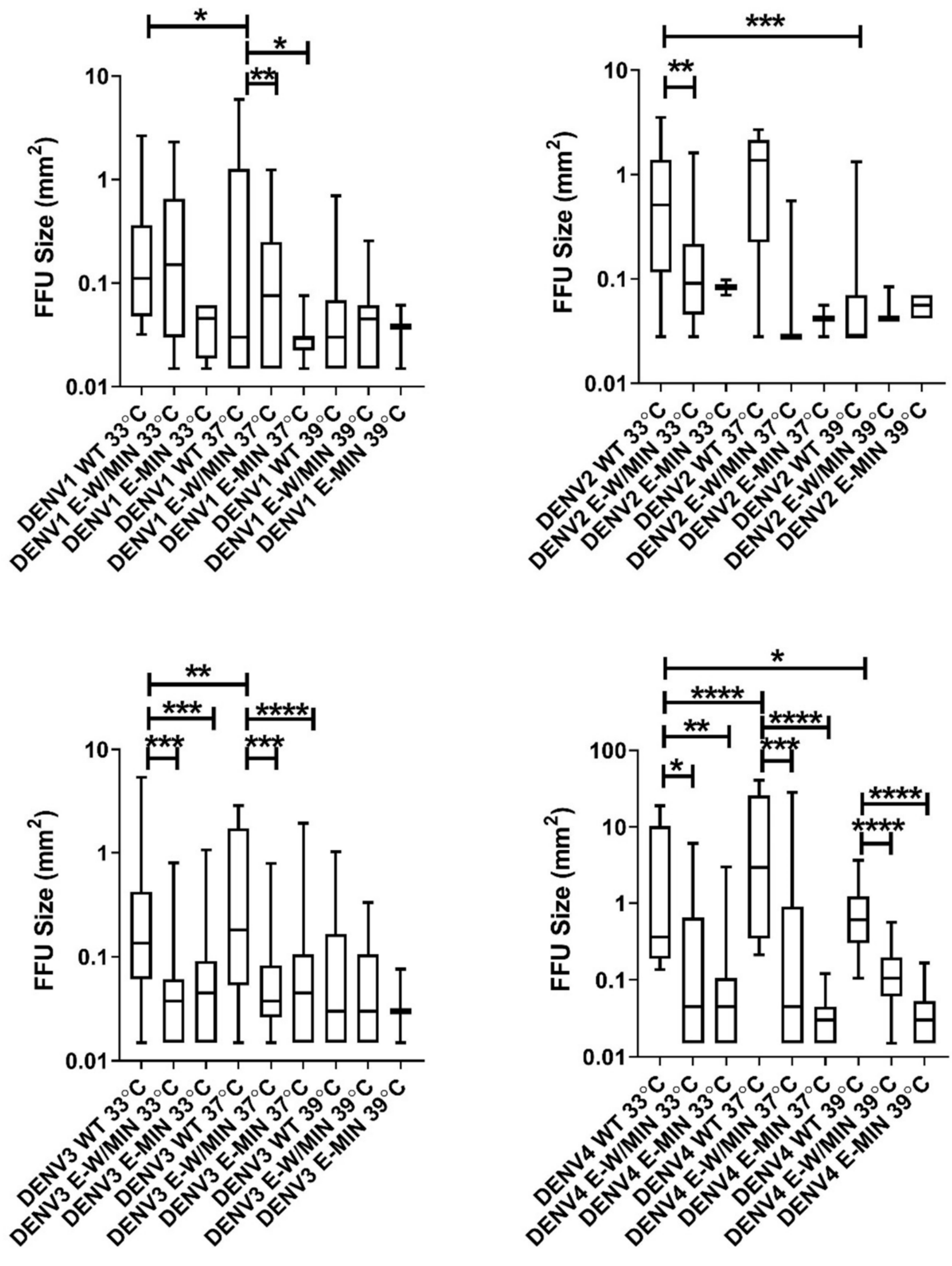
FFU Size (mm2)
DENV1 WT 33°C
DENV1 E-W/MIN 33°C
DENV1 E-MIN 33°C
DENV1 WT 37°C
DENV1 E-W/MIN 37°C
DENV1 E-MIN 37°C
DENV1 WT 39°C
DENV1 E-W/MIN 39°C
DENV1 E-MIN 39°C
DENV2 WT 33°C
DENV2 E-W/MIN 33°C
DENV2 E-MIN 33°C
DENV2 WT 37°C
DENV2 E-W/MIN 37°C
DENV2 E-MIN 37°C
DENV2 WT 39°C
DENV2 E-W/MIN 39°C
DENV2 E-MIN 39°C
DENV3 WT 33°C
DENV3 E-W/MIN 33°C
DENV3 E-MIN 33°C
DENV3 WT 37°C
DENV3 E-W/MIN 37°C
DENV3 E-MIN 37°C
DENV3 WT 39°C
DENV3 E-W/MIN 39°C
DENV3 E-MIN 39°C
DENV4 WT 33°C
DENV4 E-W/MIN 33°C
DENV4 E-MIN 33°C
DENV4 WT 37°C
DENV4 E-W/MIN 37°C
DENV4 E-MIN 37°C
DENV4 WT 39°C
DENV4 E-W/MIN 39°C
DENV4 E-MIN 39°C

FIG. 15

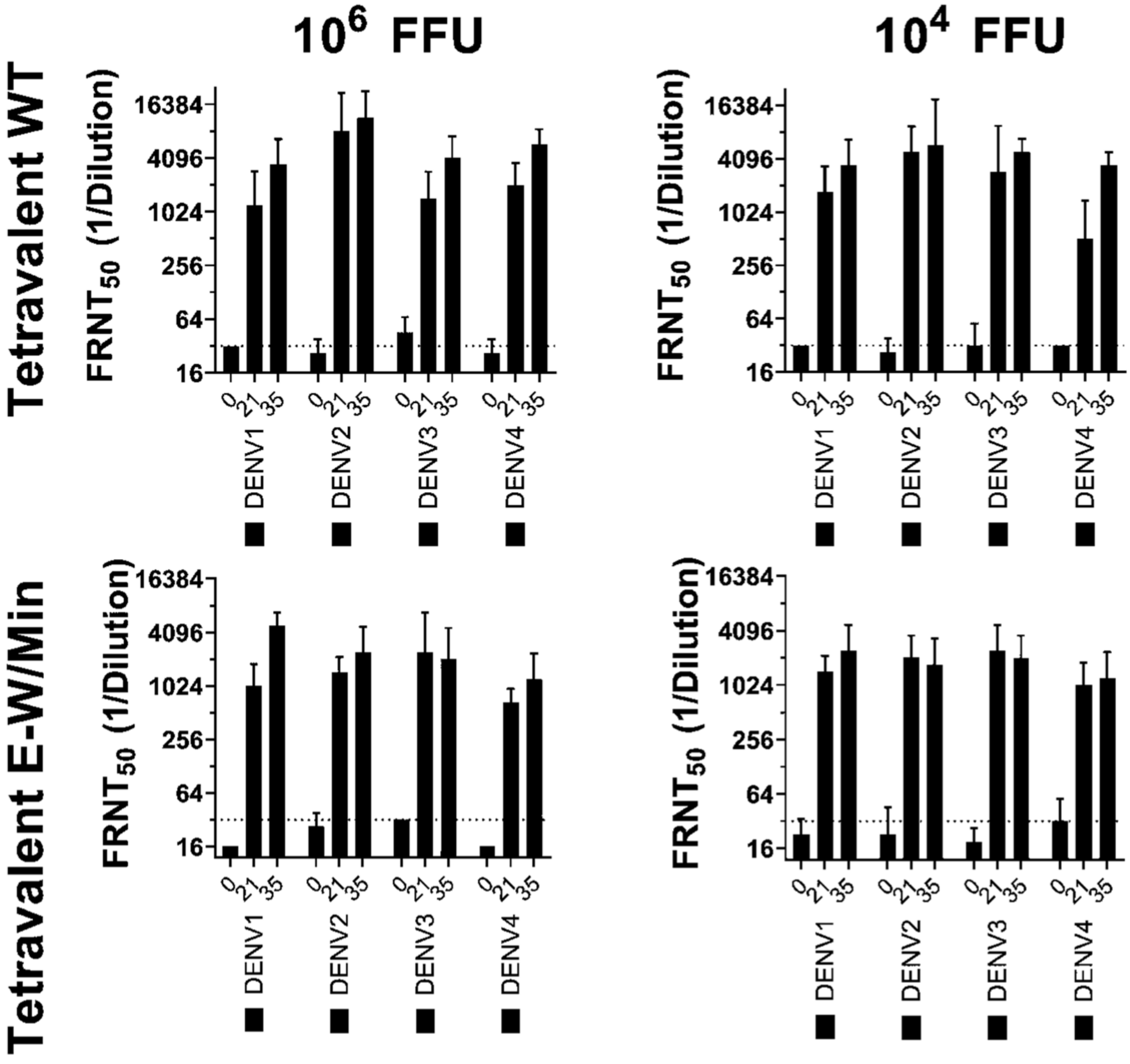
10^6 FFU
10^4 FFU
Tetravalent WT
Tetravalent E-W/Min
FRNT50 (1/Dilution)
16384
4096
1024
256
64
16
0
21
35
DENV1
DENV2
DENV3
DENV4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 62866477
- WO 2008121992 A
- WO 2011044561 A